# EUROPEAN PATENT APPLICATION

(11) **EP 2 107 127 A1**
(43) Date of publication of application: **07.10.2009**
(21) Application number: 08290307.1
(22) Date of filing: 31.03.2008
(51) Int. Cl.: C12Q 1/68

(54) **In vitro diagnostic method for the diagnosis of somatic and ovarian cancers**

(71) Applicant: Université Joseph Fourier, 38400 St Martin d'Hères (FR); INSERM (Institut National de la Santé et de la Recherche Médicale), 75654 Paris Cedex 13 (FR)
(72) Inventor: Pison-Rousseaux, Sophie, 38410 Saint Martin d'Uriage (FR); Khochbin, Saadi, 38240 Meylan (FR)
(74) Representative: Grosset-Fournier, Chantal Catherine

(57) **Abstract**

The invention relates to the use of one element chosen among a nucleic acid molecule, a fragment of said nucleic acid molecule and a variant of the said nucleic acid molecule for the *in vitro* or *ex vivo* diagnosis of any type of somatic or ovarian cancers, wherein at least one of any of the above described elements is abnormally expressed in cancer cells of at least one type of the somatic or ovarian cancers, and wherein each type of somatic or ovarian cancer cells abnormally expresses at least one of the above described elements.

## Description

The present invention relates to the *in vitro* diagnostic method for the diagnosis of somatic and ovarian cancers.

Spermatogenesis is a unique process of cell differentiation, involving the concerted action of a large number of factors, among which many show a testis-restricted expression pattern. Although several lists of testis-specific genes have been established for several species, including mouse (Chalmel et al. 2007; Schultz et al. 2003), until recently none was yet available for the human genes. Recently, two groups have proposed a list of human genes expressed in testis (Bock-Axelsen et al. 2007; Chen et al. 2005) . However the methods used did not allow sorting the genes according to their strict expression in testis.

Testis-specific (TS) genes are actively repressed in somatic cells. However, during cell transformation, it has been observed that some testis-specific genes are de-repressed, leading to the illegitimate expression of the encoded factors. These factors have been named "Cancer Testis" (CT) antigens, due to their ability to induce an immune response directed against them. Initially, CT factors were found deregulated in some somatic cancers. By contrast, CT factors are generally absent in the undifferentiated testicular tumors.

Actually, CT factors, coded by CT genes, correspond to genes with an expression restricted to germ cells of the testis (testis-specific genes; TS), and placenta (placenta-specific genes; PS). More particularly, their expression is confined to immature cells such as spermatogonia, spermatocytes, spermatids, and placental cells such as trophoblasts.

Some CTs can be expressed in nongametogenic tissues such as the pancreas, liver, and spleen at levels far below that observed in germ cells.

CT genes belong to families of genes that share common characteristics:
- they are expressed in a wide variety of malignant tumors,
- their expression programs are strictly regulated by epigenetic mechanisms such as DNA methylation, and
- they are immunogenic.

More than 40 families of CT genes have been identified so far on immunogenic properties, expression profiles, and by bioinformatic methods (for reviews see (Costa et al. 2007; Kalejs and Erenpreisa 2005; Meklat et al. 2007; Scanlan et al. 2002; Scanlan et al. 2004; Simpson et al. 2005)), but little is known about their specific functions, and their functional connection with stem cell biology and cancer is widely unexplored.

The discovery of CT genes led to the theory that aberrant expression of germline genes in cancer reflects the activation of the silenced gametogenic programme in somatic cells, and that this programmatic acquisition is one of the driving forces of tumorigenesis.
Although the discovery and study of CTs have raised a lot of excitement and interest, the sporadic nature of the expression of the already known CTs in cancer cells hampers their use as reliable markers for cancer diagnosis, follow-up and treatment.

Furthermore, studies have proposed strategies to identify large scale of CT genes in order to provide cancer diagnosis makers.

WO/2006/029176 (Scanlan et al.) relates to the use of the nucleic acid molecules, polypeptides and fragments thereof in methods and compositions for the diagnosis and treatment of diseases, such as cancer. Some putative CT testis-specific genes have been tested for their expression in somatic cancer tissues by a RT-PCR. However, this study identified too few CT genes, for use as a reliable marker of somatic cancers.

Bock-Axelsen et al.(PNAS, 2007, vol 204 pp13122-13127) have recently proposed a new method to identify genes overexpressed in human solid tumors, using a micro-array strategy. This document discloses that cancers overexpress only a few genes that are selectively expressed in the same tissue in which tumor is originated. In particular, Bock-Axelsen *et al.* describe some testis-specific genes mis-regulated in a panel of somatic tumors. Using a transcriptomic-based approach, Bock-Aselsen et al. Found testis-overexpressed genes, which are deregulated in somatic cancer, but, according to EST data (which they did not look at), most of the genes they have identified as "testis specific" or "CT" do not show a testis-restricted pattern of expression in normal cells.

In spite of these works, determining new TS genes as putative CT genes, no method gives satisfactory results about either the testis-specific restriction of expression of some genes, or their putative deregulation of expression of CT-genes.

So the invention provides a reliable global identification of TS and PS liable to be miss-regulated in somatic tumor, i.e. CT genes, said CT genes being used as universal biomarkers of malignant somatic cell transformation.
The invention also provides simple, rapid and easy-to-use methods using nucleic acid molecules of CT genes, or the corresponding proteins, for the *in vitro* and *ex vivo* diagnosis of somatic and ovarian cancer.
The invention provides kits for the detection of ovarian and somatic cancers, using specific CT genes.
Moreover, the invention provides pharmaceutical compositions comprising nucleic acid molecules or proteins for the therapy of cancer.

The invention relates to the use of one element chosen among:
- at least a nucleic acid molecule of the group comprising or constituted by :
   - a nucleotide sequence of the group consisting in SEQ ID NO 641 to SEQ ID NO 754, or,
   - a nucleotide sequence of the group consisting in SEQ ID NO 2q-1, q varying from 1 to 320, coding for a protein comprising or constituted by an amino acid sequence belonging to the group consisting in SEQ ID NO 2q, q varying from 1 to 320, or
   - the complementary sequence of the nucleic acid molecule thereof,
- at least a fragment of said nucleic acid molecule, said fragment comprising at least 15 contiguous nucleotides of said nucleic acid molecule,
- at least a variant of the said nucleic acid molecule, wherein the variant presents a sequence homology of at least 70%, particularly 80%, and more particularly 90% compared to said nucleic acid molecule,
for the *in vitro* or *ex vivo* diagnosis of any type of somatic or ovarian cancers, wherein at least one of any of the above described elements is abnormally expressed in cancer cells of at least one type of the somatic or ovarian cancers, and wherein each type of somatic or ovarian cancer cells abnormally expresses at least one of the above described elements.

The prior art does not allow to determine a clear cut association between cancer and CT genes, i.e. any CT gene is miss-regulated in at least one cancer tissue and any cancer expresses at an abnormal level at least one CT gene.

The invention is based on the unexpected observation that any CT gene is miss-regulated in at least one somatic and ovarian tumor, and reciprocally any somatic and ovarian cancer expresses at least a miss-regulated CT gene.

According to the invention, terms "nucleic acid molecules", "nucleic acids", "oligonucleotides" and "polynucleotides" are uniformly used to define a chain of bases that characterizing a DNA molecules or an RNA molecule.
The term "base" is used to define the components of the DNA or RNA, i.e. deoxyribonucleotides and ribonucleotides respectively. All the deoxyribonucleotides and ribonucleotides known in the art are concerned by the invention.
DNA molecules in the invention correspond to a gene, its transcripts when said gene is expressed, variants of said gene when they exist, or any other molecules constituted or comprising at least two bases. DNA molecules also concern the complementary DNA (cDNA), which result from the natural or artificial reverse transcription, i.e. DNA synthesis from RNA. RNA molecules of the invention corresponds to a mRNA, rRNA, miRNA, or any other molecule constituted or comprising at least two bases that characterize RNA.

Preferably, the invention concerns mRNA molecules, that include, but is not limited to, full length mRNA corresponding to the complete transcription of a gene during the transcription process. All the variants, isoforms and fragments of said RNA are also considered in the invention.

According to the invention, a "variant" is defined as a polynucleotide molecule that differs from the reference polynucleotide molecule (the gene), but retains essential properties. The gene and its variants share similar polynucleotide sequences with, for example, 70 % of nucleic acids identity, preferably 80% of nucleic acids identity, more preferably or particularly 90% of nucleic acids identity, more preferably or particularly 92% of nucleic acids identity, more preferably or particularly 95% of nucleic acids identity, more preferably or particularly 98% of nucleic acids identity and more preferably or particularly 99% of nucleic acids identity. The variants of the invention can be also considered as isoforms. These variants can be the result of an alternative splicing, which result of an addition or deletion of one or more exons naturally contained in the nucleic acid sequence of the gene. Moreover, variants in the invention also concerns, but is not limited to, products of pseudo-genes, that have diverged in their sequence from the gene.

All the variants are characterized in that they have retained the essential properties of the nucleic acid molecule from which they derive.

According to the invention, fragments of nucleic acid molecule are defined by the fact that they contain at least 15 nucleotides, advantageously they contain at least 20 nucleotides, preferably 30 nucleotides, more preferably 40 nucleotides, more preferably 60 nucleotides, more preferably 100 nucleotides. Fragments of a nucleic acid molecule can also correspond to the nucleic acid molecule corresponding to a gene wherein at least one nucleotide is suppressed. These fragments can retain some important genetic information of said nucleic acid molecule or simply can serve as oligonucleotides allowing DNA amplification, or oligonucleotides probes allowing nucleic acid molecule hybridization.
The "fragments" according to the invention corresponds then to a part of said nucleic acid molecule, and can also correspond to the complementary sequence of said part of said nucleic acid molecule. The complementarity is a concept well known in the art based on the possible interaction between purine and pyrimidine bases.

According to the invention, "abnormally expressed in cancer cells" means that the above-mentioned elements are expressed at a level which is not the normal level of expression of said elements.
In the invention, the elements mentioned above are expressed specifically in testis or placenta. Their expression can be measured by commonly used methods known in the art. For example, expression level of nucleic acid molecules can be measured by methods such as Reverse-Transcription Quantitative PCR (RT-QPCR) or Northern Blotting according to a routine protocol These methods allow measuring the levels of mRNA corresponding to a particular gene (or sequence). In the first approach, the RNA from the sample (total or polyA, the latter corresponding to mRNA) is submitted to reverse transcription, in order to obtain the DNA corresponding to the complementary sequences. In Q-PCR, this DNA is then amplified by PCR, in conditions allowing a quantification of the initial amount of DNA. By using specific primers the amount of DNA corresponding to a particular sequence can be quantified. Northern blotting involves the electrophoretic separation of the RNA molecules, followed by the detection of specific sequences by hybridizing complementary sequences, used as probes (these probes are labeled).
In the testicular or placental cells of a healthy individual, said elements are expressed at a level which corresponds to a "normal level". According to the invention, said elements are not expressed in the corresponding somatic cells of said healthy individual, their expression level is null.
When somatic cells become malignant, according to the invention said malignant somatic cells express the previously described elements, which are normally not expressed in the corresponding normal somatic cells. Therefore, said elements have an expression level in malignant somatic cells higher than zero. So, in malignant somatic cells, when an element is absent in a healthy condition, its expression in a malignant condition is considered as abnormal.

According to the invention, terms "abnormally regulated", "miss-regulated" and "deregulated" are uniformly used hereafter to define a regulation in an abnormal condition, i.e. a cancer. Also, a normal condition, which refers to a normal regulation, corresponds to a condition in which cells are healthy.

According to the invention, nucleic acid molecules are characterized by their nucleic acid sequence among the nucleic acids sequences consisting in SEQ ID NO 2q-1, q varying from 1 to 320, and SEQ ID NO 641 to SEQ ID NO 754.
These nucleic acid molecules mentioned above are expressed either in testis, or in placenta, in a healthy condition.
The above-mentioned nucleic acid sequences SEQ ID NO 2q-1, q varying from 1 to 320, correspond to the following nucleic acid sequences: SEQ ID NO 1, SEQ ID NO 3, SEQ ID NO 5, SEQ ID NO 7, SEQ ID NO 9, SEQ ID NO 11, SEQ ID NO 13, SEQ ID NO 15, SEQ ID NO 17, SEQ ID NO 19, SEQ ID NO 21, SEQ ID NO 23, SEQ ID NO 25, SEQ ID NO 27, SEQ ID NO 29, SEQ ID NO 31, SEQ ID NO 33, SEQ ID NO 35, SEQ ID NO 37, SEQ ID NO 39, SEQ ID NO 41, SEQ ID NO 43, SEQ ID NO 45, SEQ ID NO 47, SEQ ID NO 49, SEQ ID NO 51, SEQ ID NO 53, SEQ ID NO 55, SEQ ID NO 57, SEQ ID NO 59, SEQ ID NO 61, SEQ ID NO 63, SEQ ID NO 65, SEQ ID NO 67, SEQ ID NO 69, SEQ ID NO 71, SEQ ID NO 73, SEQ ID NO 75, SEQ ID NO 77, SEQ ID NO 79, SEQ ID NO 81, SEQ ID NO 83, SEQ ID NO 85, SEQ ID NO 87, SEQ ID NO 89, SEQ ID NO 91, SEQ ID NO 93, SEQ ID NO 95, SEQ ID NO 97, SEQ ID NO 99, SEQ ID NO 101, SEQ ID NO 103, SEQ ID NO 105, SEQ ID NO 107, SEQ ID NO 109, SEQ ID NO 111, SEQ ID NO 113, SEQ ID NO 115, SEQ ID NO 117, SEQ ID NO 119, SEQ ID NO 121, SEQ ID NO 123, SEQ ID NO 125, SEQ ID NO 127, SEQ ID NO 129, SEQ ID NO 131, SEQ ID NO 133, SEQ ID NO 135, SEQ ID NO 137, SEQ ID NO 139, SEQ ID NO 141, SEQ ID NO 143, SEQ ID NO 145, SEQ ID NO 147, SEQ ID NO 149, SEQ ID NO 151, SEQ ID NO 153, SEQ ID NO 155, SEQ ID NO 157, SEQ ID NO 159, SEQ ID NO 161, SEQ ID NO 163, SEQ ID NO 165, SEQ ID NO 167, SEQ ID NO 169, SEQ ID NO 171, SEQ ID NO 173, SEQ ID NO 175, SEQ ID NO 177, SEQ ID NO 179, SEQ ID NO 181, SEQ ID NO 183, SEQ ID NO 185, SEQ ID NO 187, SEQ ID NO 189, SEQ ID NO 191, SEQ ID NO 193, SEQ ID NO 195, SEQ ID NO 197, SEQ ID NO 199, SEQ ID NO 201, SEQ ID NO 203, SEQ ID NO 205, SEQ ID NO 207, SEQ ID NO 209, SEQ ID NO 211, SEQ ID NO 213, SEQ ID NO 215, SEQ ID NO 217, SEQ ID NO 219, SEQ ID NO 221, SEQ ID NO 223, SEQ ID NO 225, SEQ ID NO 227, SEQ ID NO 229, SEQ ID NO 231, SEQ ID NO 233, SEQ ID NO 235, SEQ ID NO 237, SEQ ID NO 239, SEQ ID NO 241, SEQ ID NO 243, SEQ ID NO 245, SEQ ID NO 247, SEQ ID NO 249, SEQ ID NO 251, SEQ ID NO 253, SEQ ID NO 255, SEQ ID NO 257, SEQ ID NO 259, SEQ ID NO 261, SEQ ID NO 263, SEQ ID NO 265, SEQ ID NO 267, SEQ ID NO 269, SEQ ID NO 271, SEQ ID NO 273, SEQ ID NO 275, SEQ ID NO 277, SEQ ID NO 279, SEQ ID NO 281, SEQ ID NO 283, SEQ ID NO 285, SEQ ID NO 287, SEQ ID NO 289, SEQ ID NO 291, SEQ ID NO 293, SEQ ID NO 295, SEQ ID NO 297, SEQ ID NO 299, SEQ ID NO 301, SEQ ID NO 303, SEQ ID NO 305, SEQ ID NO 307, SEQ ID NO 309, SEQ ID NO 311, SEQ ID NO 313, SEQ ID NO 315, SEQ ID NO 317, SEQ ID NO 319, SEQ ID NO 321, SEQ ID NO 323, SEQ ID NO 325, SEQ ID NO 327, SEQ ID NO 329, SEQ ID NO 331, SEQ ID NO 333, SEQ ID NO 335, SEQ ID NO 337, SEQ ID NO 339, SEQ ID NO 341, SEQ ID NO 343, SEQ ID NO 345, SEQ ID NO 347, SEQ ID NO 349, SEQ ID NO 351, SEQ ID NO 353, SEQ ID NO 355, SEQ ID NO 357, SEQ ID NO 359, SEQ ID NO 361, SEQ ID NO 363, SEQ ID NO 365, SEQ ID NO 367, SEQ ID NO 369, SEQ ID NO 371, SEQ ID NO 373, SEQ ID NO 375, SEQ ID NO 377, SEQ ID NO 379, SEQ ID NO 381, SEQ ID NO 383, SEQ ID NO 385, SEQ ID NO 387, SEQ ID NO 389, SEQ ID NO 391, SEQ ID NO 393, SEQ ID NO 395, SEQ ID NO 397, SEQ ID NO 399, SEQ ID NO 401, SEQ ID NO 403, SEQ ID NO 405, SEQ ID NO 407, SEQ ID NO 409, SEQ ID NO 411, SEQ ID NO 413, SEQ ID NO 415, SEQ ID NO 417, SEQ ID NO 419, SEQ ID NO 421, SEQ ID NO 423, SEQ ID NO 425, SEQ ID NO 427, SEQ ID NO 429, SEQ ID NO 431, SEQ ID NO 433, SEQ ID NO 435, SEQ ID NO 437, SEQ ID NO 439, SEQ ID NO 441, SEQ ID NO 443, SEQ ID NO 445, SEQ ID NO 447, SEQ ID NO 449, SEQ ID NO 451, SEQ ID NO 453, SEQ ID NO 455, SEQ ID NO 457, SEQ ID NO 459, SEQ ID NO 461, SEQ ID NO 463, SEQ ID NO 465, SEQ ID NO 467, SEQ ID NO 469, SEQ ID NO 471, SEQ ID NO 473, SEQ ID NO 475, SEQ ID NO 477, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 497, SEQ ID NO 499, SEQ ID NO 501, SEQ ID NO 503, SEQ ID NO 505, SEQ ID NO 507, SEQ ID NO 509, SEQ ID NO 511, SEQ ID NO 513, SEQ ID NO 515, SEQ ID NO 517, SEQ ID NO 519, SEQ ID NO 521, SEQ ID NO 523, SEQ ID NO 525, SEQ ID NO 527, SEQ ID NO 529, SEQ ID NO 531, SEQ ID NO 533, SEQ ID NO 535, SEQ ID NO 537, SEQ ID NO 539, SEQ ID NO 541, SEQ ID NO 543, SEQ ID NO 545, SEQ ID NO 547, SEQ ID NO 549, SEQ ID NO 551, SEQ ID NO 553, SEQ ID NO 555, SEQ ID NO 557, SEQ ID NO 559, SEQ ID NO 561, SEQ ID NO 563, SEQ ID NO 565, SEQ ID NO 567, SEQ ID NO 569, SEQ ID NO 571, SEQ ID NO 573, SEQ ID NO 575, SEQ ID NO 577, SEQ ID NO 579, SEQ ID NO 581, SEQ ID NO 583, SEQ ID NO 585, SEQ ID NO 587, SEQ ID NO 589, SEQ ID NO 591, SEQ ID NO 593, SEQ ID NO 595, SEQ ID NO 597, SEQ ID NO 599, SEQ ID NO 601, SEQ ID NO 603, SEQ ID NO 605, SEQ ID NO 607, SEQ ID NO 609, SEQ ID NO 611, SEQ ID NO 613, SEQ ID NO 615, SEQ ID NO 617, SEQ ID NO 619, SEQ ID NO 621, SEQ ID NO 623, SEQ ID NO 625, SEQ ID NO 627, SEQ ID NO 629, SEQ ID NO 631, SEQ ID NO 633, SEQ ID NO 635, SEQ ID NO 637, and SEQ ID NO 639.
The above-mentioned nucleic acid sequences SEQ ID NO 641 to SEQ ID NO 754 correspond to the following sequences: SEQ ID NO 641, SEQ ID NO 642, SEQ ID NO 643, SEQ ID NO 644, SEQ ID NO 645, SEQ ID NO 646, SEQ ID NO 647, SEQ ID NO 648, SEQ ID NO 649, SEQ ID NO 650, SEQ ID NO 651, SEQ ID NO 652, SEQ ID NO 653, SEQ ID NO 654, SEQ ID NO 655, SEQ ID NO 656, SEQ ID NO 657, SEQ ID NO 658, SEQ ID NO 659, SEQ ID NO 660, SEQ ID NO 661, SEQ ID NO 662, SEQ ID NO 663, SEQ ID NO 664, SEQ ID NO 665, SEQ ID NO 666, SEQ ID NO 667, SEQ ID NO 668, SEQ ID NO 669, SEQ ID NO 670, SEQ ID NO 671, SEQ ID NO 672, SEQ ID NO 673, SEQ ID NO 674, SEQ ID NO 675, SEQ ID NO 676, SEQ ID NO 677, SEQ ID NO 678, SEQ ID NO 679, SEQ ID NO 680, SEQ ID NO 681, SEQ ID NO 682, SEQ ID NO 683, SEQ ID NO 684, SEQ ID NO 685, SEQ ID NO 686, SEQ ID NO 687, SEQ ID NO 688, SEQ ID NO 689, SEQ ID NO 690, SEQ ID NO 691, SEQ ID NO 692, SEQ ID NO 693, SEQ ID NO 694, SEQ ID NO 695, SEQ ID NO 696, SEQ ID NO 697, SEQ ID NO 698, SEQ ID NO 699, SEQ ID NO 700, SEQ ID NO 701, SEQ ID NO 702, SEQ ID NO 703, SEQ ID NO 704, SEQ ID NO 705, SEQ ID NO 706, SEQ ID NO 707, SEQ ID NO 708, SEQ ID NO 709, SEQ ID NO 710, SEQ ID NO 711, SEQ ID NO 712, SEQ ID NO 713, SEQ ID NO 714, SEQ ID NO 715, SEQ ID NO 716, SEQ ID NO 717, SEQ ID NO 718, SEQ ID NO 719, SEQ ID NO 720, SEQ ID NO 721, SEQ ID NO 722, SEQ ID NO 723, SEQ ID NO 724, SEQ ID NO 725, SEQ ID NO 726, SEQ ID NO 727, SEQ ID NO 728, SEQ ID NO 729, SEQ ID NO 730, SEQ ID NO 731, SEQ ID NO 732, SEQ ID NO 733, SEQ ID NO 734, SEQ ID NO 735, SEQ ID NO 736, SEQ ID NO 737, SEQ ID NO 738, SEQ ID NO 739, SEQ ID NO 740, SEQ ID NO 741, SEQ ID NO 742, SEQ ID NO 743, SEQ ID NO 744, SEQ ID NO 745, SEQ ID NO 746, SEQ ID NO 747, SEQ ID NO 748, SEQ ID NO 749, SEQ ID NO 750, SEQ ID NO 751, SEQ ID NO 752, SEQ ID NO 753 and SEQ ID NO 754.

According to the invention, nucleic acids molecules characterized by the nucleic acid sequence chosen among the group consisting in SEQ ID NO 2q-1, q varying from 1 to 320, are able to code for proteins. Said proteins are characterized by their amino acids sequences chosen among the group consisting in SEQ ID NO 2q, q varying from 1 to 320.
The above-mentioned amino acid sequences SEQ ID NO 2q, q varying from 1 to 320, correspond to the following amino acid sequences: SEQ ID NO 2, SEQ ID NO 4, SEQ ID NO 6, SEQ ID NO 8, SEQ ID NO 10, SEQ ID NO 12, SEQ ID NO 14, SEQ ID NO 16, SEQ ID NO 18, SEQ ID NO 20, SEQ ID NO 22, SEQ ID NO 24, SEQ ID NO 26, SEQ ID NO 28, SEQ ID NO 30, SEQ ID NO 32, SEQ ID NO 34, SEQ ID NO 36, SEQ ID NO 38, SEQ ID NO 40, SEQ ID NO 42, SEQ ID NO 44, SEQ ID NO 46, SEQ ID NO 48, SEQ ID NO 50, SEQ ID NO 52, SEQ ID NO 54, SEQ ID NO 56, SEQ ID NO 58, SEQ ID NO 60, SEQ ID NO 62, SEQ ID NO 64, SEQ ID NO 66, SEQ ID NO 68, SEQ ID NO 70, SEQ ID NO 72, SEQ ID NO 74, SEQ ID NO 76, SEQ ID NO 78, SEQ ID NO 80, SEQ ID NO 82, SEQ ID NO 84, SEQ ID NO 86, SEQ ID NO 88, SEQ ID NO 90, SEQ ID NO 92, SEQ ID NO 94, SEQ ID NO 96, SEQ ID NO 98, SEQ ID NO 100, SEQ ID NO 102, SEQ ID NO 104, SEQ ID NO 106, SEQ ID NO 108, SEQ ID NO 110, SEQ ID NO 112, SEQ ID NO 114, SEQ ID NO 116, SEQ ID NO 118, SEQ ID NO 120, SEQ ID NO 122, SEQ ID NO 124, SEQ ID NO 126, SEQ ID NO 128, SEQ ID NO 130, SEQ ID NO 132, SEQ ID NO 134, SEQ ID NO 136, SEQ ID NO 138, SEQ ID NO 140, SEQ ID NO 142, SEQ ID NO 144, SEQ ID NO 146, SEQ ID NO 148, SEQ ID NO 150, SEQ ID NO 152, SEQ ID NO 154, SEQ ID NO 156, SEQ ID NO 158, SEQ ID NO 160, SEQ ID NO 162, SEQ ID NO 164, SEQ ID NO 166, SEQ ID NO 168, SEQ ID NO 170, SEQ ID NO 172, SEQ ID NO 174, SEQ ID NO 176, SEQ ID NO 178, SEQ ID NO 180, SEQ ID NO 182, SEQ ID NO 184, SEQ ID NO 186, SEQ ID NO 188, SEQ ID NO 190, SEQ ID NO 192, SEQ ID NO 194, SEQ ID NO 196, SEQ ID NO 198, SEQ ID NO 200, SEQ ID NO 202, SEQ ID NO 204, SEQ ID NO 206, SEQ ID NO 208, SEQ ID NO 210, SEQ ID NO 212, SEQ ID NO 214, SEQ ID NO 216, SEQ ID NO 218, SEQ ID NO 220, SEQ ID NO 222, SEQ ID NO 224, SEQ ID NO 226, SEQ ID NO 228, SEQ ID NO 230, SEQ ID NO 232, SEQ ID NO 234, SEQ ID NO 236, SEQ ID NO 238, SEQ ID NO 240, SEQ ID NO 242, SEQ ID NO 244, SEQ ID NO 246, SEQ ID NO 248, SEQ ID NO 250, SEQ ID NO 252, SEQ ID NO 254, SEQ ID NO 256, SEQ ID NO 258, SEQ ID NO 260, SEQ ID NO 262, SEQ ID NO 264, SEQ ID NO 266, SEQ ID NO 268, SEQ ID NO 270, SEQ ID NO 272, SEQ ID NO 274, SEQ ID NO 276, SEQ ID NO 278, SEQ ID NO 280, SEQ ID NO 282, SEQ ID NO 284, SEQ ID NO 286, SEQ ID NO 288, SEQ ID NO 290, SEQ ID NO 292, SEQ ID NO 294, SEQ ID NO 296, SEQ ID NO 298, SEQ ID NO 300, SEQ ID NO 302, SEQ ID NO 304, SEQ ID NO 306, SEQ ID NO 308, SEQ ID NO 310, SEQ ID NO 312, SEQ ID NO 314, SEQ ID NO 316, SEQ ID NO 318, SEQ ID NO 320, SEQ ID NO 322, SEQ ID NO 324, SEQ ID NO 326, SEQ ID NO 328, SEQ ID NO 330, SEQ ID NO 332, SEQ ID NO 334, SEQ ID NO 336, SEQ ID NO 338, SEQ ID NO 340, SEQ ID NO 342, SEQ ID NO 344, SEQ ID NO 346, SEQ ID NO 348, SEQ ID NO 350, SEQ ID NO 352, SEQ ID NO 354, SEQ ID NO 356, SEQ ID NO 358, SEQ ID NO 360, SEQ ID NO 362, SEQ ID NO 364, SEQ ID NO 366, SEQ ID NO 368, SEQ ID NO 370, SEQ ID NO 372, SEQ ID NO 374, SEQ ID NO 376, SEQ ID NO 378, SEQ ID NO 380, SEQ ID NO 382, SEQ ID NO 384, SEQ ID NO 386, SEQ ID NO 388, SEQ ID NO 390, SEQ ID NO 392, SEQ ID NO 394, SEQ ID NO 396, SEQ ID NO 398, SEQ ID NO 400, SEQ ID NO 402, SEQ ID NO 404, SEQ ID NO 406, SEQ ID NO 408, SEQ ID NO 410, SEQ ID NO 412, SEQ ID NO 414, SEQ ID NO 416, SEQ ID NO 418, SEQ ID NO 420, SEQ ID NO 422, SEQ ID NO 424, SEQ ID NO 426, SEQ ID NO 428, SEQ ID NO 430, SEQ ID NO 432, SEQ ID NO 434, SEQ ID NO 436, SEQ ID NO 438, SEQ ID NO 440, SEQ ID NO 442, SEQ ID NO 444, SEQ ID NO 446, SEQ ID NO 448, SEQ ID NO 450, SEQ ID NO 452, SEQ ID NO 454, SEQ ID NO 456, SEQ ID NO 458, SEQ ID NO 460, SEQ ID NO 462, SEQ ID NO 464, SEQ ID NO 466, SEQ ID NO 468, SEQ ID NO 470, SEQ ID NO 472, SEQ ID NO 474, SEQ ID NO 476, SEQ ID NO 478, SEQ ID NO 480, SEQ ID NO 482, SEQ ID NO 484, SEQ ID NO 486, SEQ ID NO 488, SEQ ID NO 490, SEQ ID NO 492, SEQ ID NO 494, SEQ ID NO 496, SEQ ID NO 498, SEQ ID NO 500, SEQ ID NO 502, SEQ ID NO 504, SEQ ID NO 506, SEQ ID NO 508, SEQ ID NO 510, SEQ ID NO 512, SEQ ID NO 514, SEQ ID NO 516, SEQ ID NO 518, SEQ ID NO 520, SEQ ID NO 522, SEQ ID NO 524, SEQ ID NO 526, SEQ ID NO 528, SEQ ID NO 530, SEQ ID NO 532, SEQ ID NO 534, SEQ ID NO 536, SEQ ID NO 538, SEQ ID NO 540, SEQ ID NO 542, SEQ ID NO 544, SEQ ID NO 546, SEQ ID NO 548, SEQ ID NO 550, SEQ ID NO 552, SEQ ID NO 554, SEQ ID NO 556, SEQ ID NO 558, SEQ ID NO 560, SEQ ID NO 562, SEQ ID NO 564, SEQ ID NO 566, SEQ ID NO 568, SEQ ID NO 570, SEQ ID NO 572, SEQ ID NO 574, SEQ ID NO 576, SEQ ID NO 578, SEQ ID NO 580, SEQ ID NO 582, SEQ ID NO 584, SEQ ID NO 586, SEQ ID NO 588, SEQ ID NO 590, SEQ ID NO 592, SEQ ID NO 594, SEQ ID NO 596, SEQ ID NO 598, SEQ ID NO 600, SEQ ID NO 602, SEQ ID NO 604, SEQ ID NO 606, SEQ ID NO 608, SEQ ID NO 610, SEQ ID NO 612, SEQ ID NO 614, SEQ ID NO 616, SEQ ID NO 618, SEQ ID NO 620, SEQ ID NO 622, SEQ ID NO 624, SEQ ID NO 626, SEQ ID NO 628, SEQ ID NO 630, SEQ ID NO 632, SEQ ID NO 634, SEQ ID NO 636, SEQ ID NO 638, and SEQ ID NO 640.

According to the invention, "any type of somatic and ovarian cancers" means "any type of somatic cancers" and "any type of ovarian cancer". By the way, the invention does not relate to the male gonad cancer, i.e. testicular cancer.

The term "diagnosis" means in the invention the process of identifying a medical condition or disease by its signs, symptoms, and from the results of various diagnostic procedures. It means also the recognition of a disease or condition by its outward signs and symptoms. Diagnosis corresponds also to the analysis of the underlying physiological/biochemical cause(s) of a disease or condition.
According to the invention, in vitro or ex vivo diagnosis also concerns the characterization of the type or the stage or the therapeutic follow-up of somatic and ovarian cancer.

In one preferred embodiment, the invention relates to the use of at least one nucleic acid molecule described above, wherein the nucleic acid molecule belongs to the group consisting in SEQ ID NO 17, SEQ ID NO 37, SEQ ID NO 39, SEQ ID NO 41, SEQ ID NO 43, SEQ ID NO 45, SEQ ID NO 47, SEQ ID NO 49, SEQ ID NO 51, SEQ ID NO 55, SEQ ID NO 59, SEQ ID NO 61, SEQ ID NO 63, SEQ ID NO 65, SEQ ID NO 67, SEQ ID NO 69, SEQ ID NO 71, SEQ ID NO 73, SEQ ID NO 79, SEQ ID NO 81, SEQ ID NO 83, SEQ ID NO 85, SEQ ID NO 89, SEQ ID NO 91, SEQ ID NO 93, SEQ ID NO 95, SEQ ID NO 97, SEQ ID NO 99, SEQ ID NO 101, SEQ ID NO 103, SEQ ID NO 113, SEQ ID NO 115, SEQ ID NO 117, SEQ ID NO 121, SEQ ID NO 155, SEQ ID NO 157, SEQ ID NO 161, SEQ ID NO 165, SEQ ID NO 167, SEQ ID NO 169, SEQ ID NO 171, SEQ ID NO 189, SEQ ID NO 191, SEQ ID NO 195, SEQ ID NO 197, SEQ ID NO 199, SEQ ID NO 203, SEQ ID NO 205, SEQ ID NO 207, SEQ ID NO 213, SEQ ID NO 215, SEQ ID NO 217, SEQ ID NO 219, SEQ ID NO 223, SEQ ID NO 225, SEQ ID NO 229, SEQ ID NO 231, SEQ ID NO 233, SEQ ID NO 235, SEQ ID NO 237, SEQ ID NO 239, SEQ ID NO 241, SEQ ID NO 243, SEQ ID NO 247, SEQ ID NO 263, SEQ ID NO 281, SEQ ID NO 293, SEQ ID NO 295, SEQ ID NO 297, SEQ ID NO 303, SEQ ID NO 317, SEQ ID NO 319, SEQ ID NO 321, SEQ ID NO 323, SEQ ID NO 325, SEQ ID NO 327, SEQ ID NO 329, SEQ ID NO 331, SEQ ID NO 333, SEQ ID NO 335, SEQ ID NO 337, SEQ ID NO 339, SEQ ID NO 341, SEQ ID NO 343, SEQ ID NO 345, SEQ ID NO 347, SEQ ID NO 351, SEQ ID NO 353, SEQ ID NO 355, SEQ ID NO 357, SEQ ID NO 359, SEQ ID NO 361, SEQ ID NO 363, SEQ ID NO 365, SEQ ID NO 367, SEQ ID NO 369, SEQ ID NO 371, SEQ ID NO 373, SEQ ID NO 375, SEQ ID NO 377, SEQ ID NO 379, SEQ ID NO 381, SEQ ID NO 387, SEQ ID NO 389, SEQ ID NO 395, SEQ ID NO 397, SEQ ID NO 399, SEQ ID NO 401, SEQ ID NO 403, SEQ ID NO 405, SEQ ID NO 407, SEQ ID NO 409, SEQ ID NO 413, SEQ ID NO 423, SEQ ID NO 425, SEQ ID NO 435, SEQ ID NO 437, SEQ ID NO 441, SEQ ID NO 445, SEQ ID NO 461, SEQ ID NO 463, SEQ ID NO 497, SEQ ID NO 517, SEQ ID NO 527, SEQ ID NO 551, SEQ ID NO 567, SEQ ID NO 579, SEQ ID NO 595, SEQ ID NO 599, SEQ ID NO 601, SEQ ID NO 609, SEQ ID NO 623, SEQ ID NO 625, SEQ ID NO 627, SEQ ID NO 641, SEQ ID NO 642, SEQ ID NO 643, SEQ ID NO 644, SEQ ID NO 645, SEQ ID NO 646, SEQ ID NO 647, SEQ ID NO 648, SEQ ID NO 649, SEQ ID NO 650, SEQ ID NO 651, SEQ ID NO 652, SEQ ID NO 653, SEQ ID NO 654, SEQ ID NO 655, SEQ ID NO 656, SEQ ID NO 657, SEQ ID NO 658, SEQ ID NO 659, SEQ ID NO 660, SEQ ID NO 661, SEQ ID NO 662, SEQ ID NO 663, SEQ ID NO 664, SEQ ID NO 665, SEQ ID NO 666, SEQ ID NO 667, SEQ ID NO 668, SEQ ID NO 669, SEQ ID NO 670, SEQ ID NO 671, SEQ ID NO 672, SEQ ID NO 673, SEQ ID NO 674, SEQ ID NO 675, SEQ ID NO 676, SEQ ID NO 677, SEQ ID NO 678, SEQ ID NO 679, SEQ ID NO 680, SEQ ID NO 681, SEQ ID NO 682, SEQ ID NO 683, SEQ ID NO 684, SEQ ID NO 685, SEQ ID NO 686, SEQ ID NO 687, SEQ ID NO 688, SEQ ID NO 689, SEQ ID NO 690, SEQ ID NO 691, SEQ ID NO 692, SEQ ID NO 693, SEQ ID NO 694, SEQ ID NO 695, SEQ ID NO 696, SEQ ID NO 697, SEQ ID NO 698, SEQ ID NO 699, SEQ ID NO 700, SEQ ID NO 702, SEQ ID NO 703, SEQ ID NO 704, SEQ ID NO 705, SEQ ID NO 706, SEQ ID NO 707, SEQ ID NO 708, SEQ ID NO 709, SEQ ID NO 710, SEQ ID NO 711, SEQ ID NO 712, SEQ ID NO 713, SEQ ID NO 714, SEQ ID NO 715, SEQ ID NO 717, SEQ ID NO 718, SEQ ID NO 719, SEQ ID NO 720, SEQ ID NO 722, SEQ ID NO 724, SEQ ID NO 725, SEQ ID NO 726, SEQ ID NO 727, SEQ ID NO 728, SEQ ID NO 729, SEQ ID NO 730, SEQ ID NO 731, SEQ ID NO 732, SEQ ID NO 733, SEQ ID NO 734, SEQ ID NO 735, SEQ ID NO 736, SEQ ID NO 737, SEQ ID NO 738, SEQ ID NO 739, SEQ ID NO 740, SEQ ID NO 741, SEQ ID NO 742, SEQ ID NO 743, SEQ ID NO 744, SEQ ID NO 745, SEQ ID NO 746, SEQ ID NO 747, SEQ ID NO 748, SEQ ID NO 749, SEQ ID NO 750, SEQ ID NO 751, SEQ ID NO 752, SEQ ID NO 753, SEQ ID NO 754.

According to the invention, the above mentioned nucleic acid molecules correspond to new genes. These genes are new because nothing has been so far identified with respect to their function.

Moreover, these genes have been found, according to the invention, to be expressed either in testis or placenta, but not in other tissues or cells constituting an animal or a human. Therefore, these genes are considered to be specifically expressed in testis or placenta.

In one other specific embodiment, the invention relates to the use of nucleic acid molecules as described above, wherein nucleic acid molecule belongs to the group consisting in SEQ ID NO 17, SEQ ID NO 37, SEQ ID NO 39, SEQ ID NO 41, SEQ ID NO 43, SEQ ID NO 45, SEQ ID NO 47, SEQ ID NO 49, SEQ ID NO 51, SEQ ID NO 55, SEQ ID NO 59, SEQ ID NO 61, SEQ ID NO 63, SEQ ID NO 65, SEQ ID NO 67, SEQ ID NO 69, SEQ ID NO 71, SEQ ID NO 73, SEQ ID NO 81, SEQ ID NO 83, SEQ ID NO 85, SEQ ID NO 89, SEQ ID NO 91, SEQ ID NO 93, SEQ ID NO 95, SEQ ID NO 97, SEQ ID NO 99, SEQ ID NO 101, SEQ ID NO 103, SEQ ID NO 113, SEQ ID NO 115, SEQ ID NO 117, SEQ ID NO 121, SEQ ID NO 155, SEQ ID NO 157, SEQ ID NO 161, SEQ ID NO 165, SEQ ID NO 167, SEQ ID NO 169, SEQ ID NO 171, SEQ ID NO 189, SEQ ID NO 191, SEQ ID NO 195, SEQ ID NO 197, SEQ ID NO 199, SEQ ID NO 203, SEQ ID NO 207, SEQ ID NO 213, SEQ ID NO 215, SEQ ID NO 217, SEQ ID NO 219, SEQ ID NO 223, SEQ ID NO 225, SEQ ID NO 229, SEQ ID NO 231, SEQ ID NO 233, SEQ ID NO 235, SEQ ID NO 237, SEQ ID NO 239, SEQ ID NO 241, SEQ ID NO 263, SEQ ID NO 281, SEQ ID NO 293, SEQ ID NO 295, SEQ ID NO 297, SEQ ID NO 303, SEQ ID NO 317, SEQ ID NO 319, SEQ ID NO 321, SEQ ID NO 323, SEQ ID NO 325, SEQ ID NO 327, SEQ ID NO 329, SEQ ID NO 331, SEQ ID NO 333, SEQ ID NO 335, SEQ ID NO 337, SEQ ID NO 339, SEQ ID NO 343, SEQ ID NO 345, SEQ ID NO 347, SEQ ID NO 351, SEQ ID NO 353, SEQ ID NO 355, SEQ ID NO 357, SEQ ID NO 359, SEQ ID NO 361, SEQ ID NO 363, SEQ ID NO 365, SEQ ID NO 367, SEQ ID NO 369, SEQ ID NO 371, SEQ ID NO 373, SEQ ID NO 375, SEQ ID NO 377, SEQ ID NO 379, SEQ ID NO 381, SEQ ID NO 387, SEQ ID NO 389, SEQ ID NO 395, SEQ ID NO 397, SEQ ID NO 399, SEQ ID NO 401, SEQ ID NO 403, SEQ ID NO 405, SEQ ID NO 407, SEQ ID NO 409, SEQ ID NO 413, SEQ ID NO 423, SEQ ID NO 425, SEQ ID NO 435, SEQ ID NO 437, SEQ ID NO 441, SEQ ID NO 445, SEQ ID NO 461, SEQ ID NO 497, SEQ ID NO 517, SEQ ID NO 527, SEQ ID NO 551, SEQ ID NO 567, SEQ ID NO 579, SEQ ID NO 595, SEQ ID NO 599, SEQ ID NO 601, SEQ ID NO 609, SEQ ID NO 623, SEQ ID NO 625, SEQ ID NO 627, SEQ ID NO 641, SEQ ID NO 642, SEQ ID NO 643, SEQ ID NO 644, SEQ ID NO 645, SEQ ID NO 646, SEQ ID NO 647, SEQ ID NO 648, SEQ ID NO 649, SEQ ID NO 650, SEQ ID NO 651, SEQ ID NO 652, SEQ ID NO 653, SEQ ID NO 654, SEQ ID NO 656, SEQ ID NO 657, SEQ ID NO 658, SEQ ID NO 659, SEQ ID NO 660, SEQ ID NO 661, SEQ ID NO 662, SEQ ID NO 663, SEQ ID NO 664, SEQ ID NO 665, SEQ ID NO 666, SEQ ID NO 667, SEQ ID NO 668, SEQ ID NO 669, SEQ ID NO 670, SEQ ID NO 672, SEQ ID NO 673, SEQ ID NO 674, SEQ ID NO 675, SEQ ID NO 676, SEQ ID NO 677, SEQ ID NO 678, SEQ ID NO 679, SEQ ID NO 680, SEQ ID NO 681, SEQ ID NO 682, SEQ ID NO 683, SEQ ID NO 687, SEQ ID NO 688, SEQ ID NO 689, SEQ ID NO 690, SEQ ID NO 691, SEQ ID NO 692, SEQ ID NO 693, SEQ ID NO 694, SEQ ID NO 695, SEQ ID NO 696, SEQ ID NO 697, SEQ ID NO 698, SEQ ID NO 700, SEQ ID NO 702, SEQ ID NO 703, SEQ ID NO 704, SEQ ID NO 705, SEQ ID NO 706, SEQ ID NO 707, SEQ ID NO 708, SEQ ID NO 709, SEQ ID NO 710, SEQ ID NO 711, SEQ ID NO 712, SEQ ID NO 713, SEQ ID NO 714, SEQ ID NO 715, SEQ ID NO 718, SEQ ID NO 720, SEQ ID NO 722, SEQ ID NO 724, SEQ ID NO 725, SEQ ID NO 727, SEQ ID NO 728, SEQ ID NO 729, SEQ ID NO 730, SEQ ID NO 731, SEQ ID NO 732, SEQ ID NO 733, SEQ ID NO 735, SEQ ID NO 736, SEQ ID NO 737, SEQ ID NO 738, SEQ ID NO 739, SEQ ID NO 740, SEQ ID NO 741, SEQ ID NO 742, SEQ ID NO 743, SEQ ID NO 744, SEQ ID NO 745, SEQ ID NO 746, SEQ ID NO 747, SEQ ID NO 748, SEQ ID NO 749, SEQ ID NO 750, SEQ ID NO 752, SEQ ID NO 753 and SEQ ID NO 754.

According to the invention, the above-mentioned nucleic acid molecules are specifically expressed in testis.

In another specific embodiment, the invention relates to the use of nucleic acid molecules for the *in vitro* or *ex vivo* diagnosis of any type of somatic or ovarian cancers, wherein the nucleic acid molecule belongs to the group consisting in SEQ ID NO 79, SEQ ID NO 205, SEQ ID NO 243, SEQ ID NO 247, SEQ ID NO 341, SEQ ID NO 463, SEQ ID NO 655, SEQ ID NO 671, SEQ ID NO 684, SEQ ID NO 685, SEQ ID NO 686, SEQ ID NO 699, SEQ ID NO 717, SEQ ID NO 719, SEQ ID NO 726, SEQ ID NO 734 and SEQ ID NO 751.
According to the invention, the above-mentioned nucleic acid molecules are specifically expressed in placenta.

In another particular embodiment, the invention relates to the use of nucleic acid molecule for *the in vitro* or *ex vivo* diagnosis of any type of somatic or ovarian cancers, wherein the nucleic acid molecule belongs to the group consisting in SEQ ID NO 17, SEQ ID NO 37, SEQ ID NO 41, SEQ ID NO 43, SEQ ID NO 59, SEQ ID NO 67, SEQ ID NO 69, SEQ ID NO 73, SEQ ID NO 79, SEQ ID NO 85, SEQ ID NO 89, SEQ ID NO 91, SEQ ID NO 95, SEQ ID NO 97, SEQ ID NO 99, SEQ ID NO 101, SEQ ID NO 115, SEQ ID NO 155, SEQ ID NO 157, SEQ ID NO 161, SEQ ID NO 165, SEQ ID NO 167, SEQ ID NO 169, SEQ ID NO 189, SEQ ID NO 199, SEQ ID NO 203, SEQ ID NO 217, SEQ ID NO 223, SEQ ID NO 231, SEQ ID NO 233, SEQ ID NO 237, SEQ ID NO 239, SEQ ID NO 243, SEQ ID NO 293, SEQ ID NO 297, SEQ ID NO 303, SEQ ID NO 319, SEQ ID NO 321, SEQ ID NO 323, SEQ ID NO 325, SEQ ID NO 327, SEQ ID NO 337, SEQ ID NO 347, SEQ ID NO 351, SEQ ID NO 365, SEQ ID NO 369, SEQ ID NO 371, SEQ ID NO 375, SEQ ID NO 381, SEQ ID NO 387, SEQ ID NO 389, SEQ ID NO 401, SEQ ID NO 403, SEQ ID NO 407, SEQ ID NO 425, SEQ ID NO 435, SEQ ID NO 437, SEQ ID NO 445, SEQ ID NO 461, SEQ ID NO 463, SEQ ID NO 497, SEQ ID NO 517, SEQ ID NO 551, SEQ ID NO 623, SEQ ID NO 627, SEQ ID NO 642, SEQ ID NO 644, SEQ ID NO 645, SEQ ID NO 647, SEQ ID NO 649, SEQ ID NO 652, SEQ ID NO 653, SEQ ID NO 655, SEQ ID NO 656, SEQ ID NO 657, SEQ ID NO 661, SEQ ID NO 664, SEQ ID NO 665, SEQ ID NO 671, SEQ ID NO 672, SEQ ID NO 673, SEQ ID NO 674, SEQ ID NO 675, SEQ ID NO 677, SEQ ID NO 681, SEQ ID NO 684, SEQ ID NO 688, SEQ ID NO 691, SEQ ID NO 692, SEQ ID NO 695, SEQ ID NO 697, SEQ ID NO 700, SEQ ID NO 704, SEQ ID NO 705, SEQ ID NO 706, SEQ ID NO 710, SEQ ID NO 712, SEQ ID NO 714, SEQ ID NO 717, SEQ ID NO 718, SEQ ID NO 719, SEQ ID NO 720, SEQ ID NO 722, SEQ ID NO 724, SEQ ID NO 725, SEQ ID NO 728, SEQ ID NO 729, SEQ ID NO 734, SEQ ID NO 736, SEQ ID NO 737 and SEQ ID NO 738.
According to the invention, the above-mentioned sequences correspond to new placenta and testis specific genes are miss-regulated in somatic and/or ovarian cancer.

In one advantageous embodiment, the invention relates to the use of nucleic acid molecules for *the in vitro* and ex vivo diagnosis of somatic and ovarian cancer, wherein the nucleic acid molecule belongs to the group consisting in SEQ ID NO 17, SEQ ID NO 37, SEQ ID NO 41, SEQ ID NO 43, SEQ ID NO 59, SEQ ID NO 67, SEQ ID NO 69, SEQ ID NO 73, SEQ ID NO 85, SEQ ID NO 89, SEQ ID NO 91, SEQ ID NO 95, SEQ ID NO 97, SEQ ID NO 99, SEQ ID NO 101, SEQ ID NO 115, SEQ ID NO 155, SEQ ID NO 157, SEQ ID NO 161, SEQ ID NO 165, SEQ ID NO 167, SEQ ID NO 169, SEQ ID NO 189, SEQ ID NO 199, SEQ ID NO 203, SEQ ID NO 217, SEQ ID NO 223, SEQ ID NO 231, SEQ ID NO 233, SEQ ID NO 237, SEQ ID NO 239, SEQ ID NO 293, SEQ ID NO 297, SEQ ID NO 303, SEQ ID NO 319, SEQ ID NO 321, SEQ ID NO 323, SEQ ID NO 325, SEQ ID NO 327, SEQ ID NO 337, SEQ ID NO 347, SEQ ID NO 351, SEQ ID NO 365, SEQ ID NO 369, SEQ ID NO 371, SEQ ID NO 375, SEQ ID NO 381, SEQ ID NO 387, SEQ ID NO 389, SEQ ID NO 401, SEQ ID NO 403, SEQ ID NO 407, SEQ ID NO 425, SEQ ID NO 435, SEQ ID NO 437, SEQ ID NO 445, SEQ ID NO 461, SEQ ID NO 497, SEQ ID NO 517, SEQ ID NO 551, SEQ ID NO 623, SEQ ID NO 627, SEQ ID NO 642, SEQ ID NO 644, SEQ ID NO 645, SEQ ID NO 647, SEQ ID NO 649, SEQ ID NO 652, SEQ ID NO 653, SEQ ID NO 656, SEQ ID NO 657, SEQ ID NO 661, SEQ ID NO 664, SEQ ID NO 665, SEQ ID NO 672, SEQ ID NO 673, SEQ ID NO 674, SEQ ID NO 675, SEQ ID NO 677, SEQ ID NO 681, SEQ ID NO 688, SEQ ID NO 691, SEQ ID NO 692, SEQ ID NO 695, SEQ ID NO 697, SEQ ID NO 700, SEQ ID NO 704, SEQ ID NO 705, SEQ ID NO 706, SEQ ID NO 710, SEQ ID NO 712, SEQ ID NO 714, SEQ ID NO 718, SEQ ID NO 720, SEQ ID NO 722, SEQ ID NO 724, SEQ ID NO 725, SEQ ID NO 728, SEQ ID NO 729, SEQ ID NO 736, SEQ ID NO 737 and SEQ ID NO 738.
According to the invention, the above-mentioned nucleic acid molecules are expressed in testis, and are miss-regulated in somatic or ovarian tumor.

In another particular embodiment the invention relates to the use of nucleic acid molecules as described above, wherein the nucleic acid molecule belongs to the group consisting in SEQ ID NO 79, SEQ ID NO 243, SEQ ID NO 463, SEQ ID NO 655, SEQ ID NO 671, SEQ ID NO 684, SEQ ID NO 717, SEQ ID NO 719 and SEQ ID NO 734.
Theses genes are normally expressed in placental tissues, and are miss-regulated in somatic and ovarian tumors.

The invention also relates to the use of nucleic acid molecules for the in vitro and ex vivo diagnosis of somatic and ovarian cancer, wherein the nucleic acid molecule belongs to the group consisting in SEQ ID NO 1, SEQ ID NO 3, SEQ ID NO 5, SEQ ID NO 7, SEQ ID NO 9, SEQ ID NO 11, SEQ ID NO 13, SEQ ID NO 15, SEQ ID NO 19, SEQ ID NO 21, SEQ ID NO 23, SEQ ID NO 25, SEQ ID NO 27, SEQ ID NO 29, SEQ ID NO 31, SEQ ID NO 33, SEQ ID NO 35, SEQ ID NO 53, SEQ ID NO 57, SEQ ID NO 75, SEQ ID NO 77, SEQ ID NO 87, SEQ ID NO 105, SEQ ID NO 107, SEQ ID NO 109, SEQ ID NO 111, SEQ ID NO 119, SEQ ID NO 123, SEQ ID NO 125, SEQ ID NO 127, SEQ ID NO 129, SEQ ID NO 131, SEQ ID NO 133, SEQ ID NO 135, SEQ ID NO 137, SEQ ID NO 139, SEQ ID NO 141, SEQ ID NO 143, SEQ ID NO 145, SEQ ID NO 147, SEQ ID NO 149, SEQ ID NO 151, SEQ ID NO 153, SEQ ID NO 159, SEQ ID NO 163, SEQ ID NO 173, SEQ ID NO 175, SEQ ID NO 177, SEQ ID NO 179, SEQ ID NO 181, SEQ ID NO 183, SEQ ID NO 185, SEQ ID NO 187, SEQ ID NO 193, SEQ ID NO 201, SEQ ID NO 209, SEQ ID NO 211, SEQ ID NO 221, SEQ ID NO 227, SEQ ID NO 245, SEQ ID NO 249, SEQ ID NO 251, SEQ ID NO 253, SEQ ID NO 255, SEQ ID NO 257, SEQ ID NO 259, SEQ ID NO 261, SEQ ID NO 265, SEQ ID NO 267, SEQ ID NO 269, SEQ ID NO 271, SEQ ID NO 273, SEQ ID NO 275, SEQ ID NO 277, SEQ ID NO 279, SEQ ID NO 283, SEQ ID NO 285, SEQ ID NO 287, SEQ ID NO 289, SEQ ID NO 291, SEQ ID NO 299, SEQ ID NO 301, SEQ ID NO 305, SEQ ID NO 307, SEQ ID NO 309, SEQ ID NO 311, SEQ ID NO 313, SEQ ID NO 315, SEQ ID NO 349, SEQ ID NO 383, SEQ ID NO 385, SEQ ID NO 391, SEQ ID NO 393, SEQ ID NO 411, SEQ ID NO 415, SEQ ID NO 417, SEQ ID NO 419, SEQ ID NO 421, SEQ ID NO 427, SEQ ID NO 429, SEQ ID NO 431, SEQ ID NO 433, SEQ ID NO 439, SEQ ID NO 443, SEQ ID NO 447, SEQ ID NO 449, SEQ ID NO 451, SEQ ID NO 453, SEQ ID NO 455, SEQ ID NO 457, SEQ ID NO 459, SEQ ID NO 465, SEQ ID NO 467, SEQ ID NO 469, SEQ ID NO 471, SEQ ID NO 473, SEQ ID NO 475, SEQ ID NO 477, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 499, SEQ ID NO 501, SEQ ID NO 503, SEQ ID NO 505, SEQ ID NO 507, SEQ ID NO 509, SEQ ID NO 511, SEQ ID NO 513, SEQ ID NO 515, SEQ ID NO 519, SEQ ID NO 521, SEQ ID NO 523, SEQ ID NO 525, SEQ ID NO 529, SEQ ID NO 531, SEQ ID NO 533, SEQ ID NO 535, SEQ ID NO 537, SEQ ID NO 539, SEQ ID NO 541, SEQ ID NO 543, SEQ ID NO 545, SEQ ID NO 547, SEQ ID NO 549, SEQ ID NO 553, SEQ ID NO 555, SEQ ID NO 557, SEQ ID NO 559, SEQ ID NO 561, SEQ ID NO 563, SEQ ID NO 565, SEQ ID NO 569, SEQ ID NO 571, SEQ ID NO 573, SEQ ID NO 575, SEQ ID NO 577, SEQ ID NO 581, SEQ ID NO 583, SEQ ID NO 585, SEQ ID NO 587, SEQ ID NO 589, SEQ ID NO 591, SEQ ID NO 593, SEQ ID NO 597, SEQ ID NO 603, SEQ ID NO 605, SEQ ID NO 607, SEQ ID NO 611, SEQ ID NO 613, SEQ ID NO 615, SEQ ID NO 617, SEQ ID NO 619, SEQ ID NO 621, SEQ ID NO 629, SEQ ID NO 631, SEQ ID NO 633, SEQ ID NO 635, SEQ ID NO 637, SEQ ID NO 639, SEQ ID NO 701, SEQ ID NO 716, SEQ ID NO 721 and SEQ ID NO 723.

According to the invention, the above-mentioned nucleic acid molecules are expressed in testis or placental tissues.

In particular, the invention relates to the use of nucleic acid molecule for the in vitro and ex vivo diagnosis of somatic and ovarian tumor, wherein the nucleic acid molecule belongs to the group consisting in SEQ ID NO 1, SEQ ID NO 3, SEQ ID NO 5, SEQ ID NO 7, SEQ ID NO 11, SEQ ID NO 13, SEQ ID NO 15, SEQ ID NO 19, SEQ ID NO 21, SEQ ID NO 25, SEQ ID NO 27, SEQ ID NO 29, SEQ ID NO 31, SEQ ID NO 33, SEQ ID NO 35, SEQ ID NO 53, SEQ ID NO 57, SEQ ID NO 75, SEQ ID NO 77, SEQ ID NO 87, SEQ ID NO 105, SEQ ID NO 107, SEQ ID NO 109, SEQ ID NO 119, SEQ ID NO 123, SEQ ID NO 125, SEQ ID NO 127, SEQ ID NO 129, SEQ ID NO 135, SEQ ID NO 137, SEQ ID NO 139, SEQ ID NO 143, SEQ ID NO 145, SEQ ID NO 147, SEQ ID NO 149, SEQ ID NO 151, SEQ ID NO 153, SEQ ID NO 159, SEQ ID NO 163, SEQ ID NO 173, SEQ ID NO 175, SEQ ID NO 177, SEQ ID NO 179, SEQ ID NO 183, SEQ ID NO 185, SEQ ID NO 187, SEQ ID NO 193, SEQ ID NO 201, SEQ ID NO 209, SEQ ID NO 221, SEQ ID NO 227, SEQ ID NO 245, SEQ ID NO 249, SEQ ID NO 251, SEQ ID NO 253, SEQ ID NO 255, SEQ ID NO 257, SEQ ID NO 259, SEQ ID NO 261, SEQ ID NO 265, SEQ ID NO 267, SEQ ID NO 269, SEQ ID NO 271, SEQ ID NO 273, SEQ ID NO 277, SEQ ID NO 279, SEQ ID NO 285, SEQ ID NO 287, SEQ ID NO 291, SEQ ID NO 299, SEQ ID NO 301, SEQ ID NO 305, SEQ ID NO 309, SEQ ID NO 311, SEQ ID NO 313, SEQ ID NO 383, SEQ ID NO 385, SEQ ID NO 391, SEQ ID NO 393, SEQ ID NO 411, SEQ ID NO 415, SEQ ID NO 417, SEQ ID NO 419, SEQ ID NO 421, SEQ ID NO 427, SEQ ID NO 429, SEQ ID NO 431, SEQ ID NO 433, SEQ ID NO 439, SEQ ID NO 443, SEQ ID NO 447, SEQ ID NO 449, SEQ ID NO 453, SEQ ID NO 455, SEQ ID NO 457, SEQ ID NO 459, SEQ ID NO 465, SEQ ID NO 467, SEQ ID NO 469, SEQ ID NO 473, SEQ ID NO 475, SEQ ID NO 477, SEQ ID NO 499, SEQ ID NO 501, SEQ ID NO 503, SEQ ID NO 505, SEQ ID NO 507, SEQ ID NO 509, SEQ ID NO 511, SEQ ID NO 513, SEQ ID NO 515, SEQ ID NO 519, SEQ ID NO 525, SEQ ID NO 529, SEQ ID NO 531, SEQ ID NO 533, SEQ ID NO 535, SEQ ID NO 537, SEQ ID NO 539, SEQ ID NO 541, SEQ ID NO 543, SEQ ID NO 545, SEQ ID NO 547, SEQ ID NO 549, SEQ ID NO 553, SEQ ID NO 555, SEQ ID NO 557, SEQ ID NO 559, SEQ ID NO 561, SEQ ID NO 563, SEQ ID NO 565, SEQ ID NO 569, SEQ ID NO 571, SEQ ID NO 573, SEQ ID NO 575, SEQ ID NO 577, SEQ ID NO 581, SEQ ID NO 585, SEQ ID NO 587, SEQ ID NO 589, SEQ ID NO 591, SEQ ID NO 593, SEQ ID NO 603, SEQ ID NO 605, SEQ ID NO 607, SEQ ID NO 611, SEQ ID NO 613, SEQ ID NO 615, SEQ ID NO 617, SEQ ID NO 619, SEQ ID NO 621, SEQ ID NO 629, SEQ ID NO 631, SEQ ID NO 633, SEQ ID NO 635, SEQ ID NO 637, SEQ ID NO 639, SEQ ID NO 701, SEQ ID NO 716, SEQ ID NO 721 and SEQ ID NO 723.
These nucleic acid molecules are expressed in testis.

In another particular embodiment, the invention relates to the use of nucleic acid molecule for the in vitro and ex vivo diagnosis of somatic and ovarian tumor, wherein the nucleic acid molecule belongs to the group consisting SEQ ID NO 9; SEQ ID NO 23; SEQ ID NO 111; SEQ ID NO 131; SEQ ID NO 133; SEQ ID NO 141; SEQ ID NO 181; SEQ ID NO 211; SEQ ID NO 275; SEQ ID NO 283; SEQ ID NO 289; SEQ ID NO 307; SEQ ID NO 315; SEQ ID NO 349; SEQ ID NO 451; SEQ ID NO 471; SEQ ID NO 479; SEQ ID NO 481; SEQ ID NO 483; SEQ ID NO 485; SEQ ID NO 487; SEQ ID NO 489; SEQ ID NO 491; SEQ ID NO 493; SEQ ID NO 495; SEQ ID NO 521; SEQ ID NO 523; SEQ ID NO 583 and; SEQ ID NO 597. These nucleic acid molecules are expressed in placenta.

In another specific embodiment, the invention relates to the use of nucleic acid molecule for the in vitro and ex vivo diagnosis of somatic and ovarian tumor, wherein the nucleic acid molecule belongs to the group consisting in SEQ ID NO 1, SEQ ID NO 3, SEQ ID NO 5, SEQ ID NO 7, SEQ ID NO 9, SEQ ID NO 11, SEQ ID NO 15, SEQ ID NO 19, SEQ ID NO 21, SEQ ID NO 23, SEQ ID NO 27, SEQ ID NO 31, SEQ ID NO 35, SEQ ID NO 77, SEQ ID NO 87, SEQ ID NO 111, SEQ ID NO 119, SEQ ID NO 123, SEQ ID NO 125, SEQ ID NO 127, SEQ ID NO 129, SEQ ID NO 131, SEQ ID NO 133, SEQ ID NO 135, SEQ ID NO 139, SEQ ID NO 141, SEQ ID NO 143, SEQ ID NO 145, SEQ ID NO 151, SEQ ID NO 153, SEQ ID NO 159, SEQ ID NO 163, SEQ ID NO 181, SEQ ID NO 183, SEQ ID NO 185, SEQ ID NO 201, SEQ ID NO 227, SEQ ID NO 249, SEQ ID NO 251, SEQ ID NO 253, SEQ ID NO 257, SEQ ID NO 259, SEQ ID NO 265, SEQ ID NO 267, SEQ ID NO 269, SEQ ID NO 271, SEQ ID NO 273, SEQ ID NO 275, SEQ ID NO 277, SEQ ID NO 279, SEQ ID NO 283, SEQ ID NO 285, SEQ ID NO 287, SEQ ID NO 289, SEQ ID NO 299, SEQ ID NO 301, SEQ ID NO 305, SEQ ID NO 307, SEQ ID NO 309, SEQ ID NO 311, SEQ ID NO 313, SEQ ID NO 315, SEQ ID NO 349, SEQ ID NO 385, SEQ ID NO 393, SEQ ID NO 411, SEQ ID NO 417, SEQ ID NO 419, SEQ ID NO 421, SEQ ID NO 427, SEQ ID NO 429, SEQ ID NO 431, SEQ ID NO 439, SEQ ID NO 443, SEQ ID NO 447, SEQ ID NO 449, SEQ ID NO 451, SEQ ID NO 453, SEQ ID NO 455, SEQ ID NO 457, SEQ ID NO 459, SEQ ID NO 469, SEQ ID NO 471, SEQ ID NO 473, SEQ ID NO 475, SEQ ID NO 477, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 501, SEQ ID NO 503, SEQ ID NO 507, SEQ ID NO 511, SEQ ID NO 513, SEQ ID NO 515, SEQ ID NO 519, SEQ ID NO 521, SEQ ID NO 523, SEQ ID NO 525, SEQ ID NO 531, SEQ ID NO 533, SEQ ID NO 535, SEQ ID NO 537, SEQ ID NO 539, SEQ ID NO 541, SEQ ID NO 547, SEQ ID NO 549, SEQ ID NO 553, SEQ ID NO 555, SEQ ID NO 557, SEQ ID NO 561, SEQ ID NO 563, SEQ ID NO 565, SEQ ID NO 569, SEQ ID NO 571, SEQ ID NO 573, SEQ ID NO 575, SEQ ID NO 577, SEQ ID NO 581, SEQ ID NO 583, SEQ ID NO 585, SEQ ID NO 591, SEQ ID NO 593, SEQ ID NO 597, SEQ ID NO 603, SEQ ID NO 611, SEQ ID NO 613, SEQ ID NO 617, SEQ ID NO 629, SEQ ID NO 631, SEQ ID NO 633, SEQ ID NO 635 and SEQ ID NO 637.

These nucleic acid molecules are expressed in testis or placenta, and are also abnormally expressed in somatic or ovarian cancers.

In another particular embodiment, the invention relates to the use of nucleic acid molecule for the in vitro and ex vivo diagnosis of somatic and ovarian tumor, wherein the nucleic acid molecule belongs to the group consisting in SEQ ID NO 1, SEQ ID NO 3, SEQ ID NO 5, SEQ ID NO 7, SEQ ID NO 11, SEQ ID NO 15, SEQ ID NO 19, SEQ ID NO 21, SEQ ID NO 27, SEQ ID NO 31, SEQ ID NO 35, SEQ ID NO 77, SEQ ID NO 87, SEQ ID NO 119, SEQ ID NO 123, SEQ ID NO 125, SEQ ID NO 127, SEQ ID NO 129, SEQ ID NO 135, SEQ ID NO 139, SEQ ID NO 143, SEQ ID NO 145, SEQ ID NO 151, SEQ ID NO 153, SEQ ID NO 159, SEQ ID NO 163, SEQ ID NO 183, SEQ ID NO 185, SEQ ID NO 201, SEQ ID NO 227, SEQ ID NO 249, SEQ ID NO 251, SEQ ID NO 253, SEQ ID NO 257, SEQ ID NO 259, SEQ ID NO 265, SEQ ID NO 267, SEQ ID NO 269, SEQ ID NO 271, SEQ ID NO 273, SEQ ID NO 277, SEQ ID NO 279, SEQ ID NO 285, SEQ ID NO 287, SEQ ID NO 299, SEQ ID NO 301, SEQ ID NO 305, SEQ ID NO 309, SEQ ID NO 311, SEQ ID NO 313, SEQ ID NO 385, SEQ ID NO 393, SEQ ID NO 411, SEQ ID NO 417, SEQ ID NO 419, SEQ ID NO 421, SEQ ID NO 427, SEQ ID NO 429, SEQ ID NO 431, SEQ ID NO 439, SEQ ID NO 443, SEQ ID NO 447, SEQ ID NO 449, SEQ ID NO 453, SEQ ID NO 455, SEQ ID NO 457, SEQ ID NO 459, SEQ ID NO 469, SEQ ID NO 473, SEQ ID NO 475, SEQ ID NO 477, SEQ ID NO 501, SEQ ID NO 503, SEQ ID NO 507, SEQ ID NO 511, SEQ ID NO 513, SEQ ID NO 515, SEQ ID NO 519, SEQ ID NO 525, SEQ ID NO 531, SEQ ID NO 533, SEQ ID NO 535, SEQ ID NO 537, SEQ ID NO 539, SEQ ID NO 541, SEQ ID NO 547, SEQ ID NO 549, SEQ ID NO 553, SEQ ID NO 555, SEQ ID NO 557, SEQ ID NO 561, SEQ ID NO 563, SEQ ID NO 565, SEQ ID NO 569, SEQ ID NO 571, SEQ ID NO 573, SEQ ID NO 575, SEQ ID NO 577, SEQ ID NO 581, SEQ ID NO 585, SEQ ID NO 591, SEQ ID NO 593, SEQ ID NO 603, SEQ ID NO 611, SEQ ID NO 613, SEQ ID NO 617, SEQ ID NO 629, SEQ ID NO 631, SEQ ID NO 633, SEQ ID NO 635 and SEQ ID NO 637.
These nucleic acid molecules are expressed in testis and are also abnormally expressed in somatic or ovarian cancers.

In another particular embodiment, the invention relates to the use of nucleic acid molecule for the in vitro and ex vivo diagnosis of somatic and ovarian tumor, wherein the nucleic acid molecule belongs to the group consisting in SEQ ID NO 9, SEQ ID NO 23, SEQ ID NO 111, SEQ ID NO 131, SEQ ID NO 133, SEQ ID NO 141, SEQ ID NO 181, SEQ ID NO 275, SEQ ID NO 283, SEQ ID NO 289, SEQ ID NO 307, SEQ ID NO 315, SEQ ID NO 349, SEQ ID NO 451, SEQ ID NO 471, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 521, SEQ ID NO 523, SEQ ID NO 583 and SEQ ID NO 597.
These nucleic acid molecules are expressed in placenta, and are also abnormally expressed in somatic or ovarian cancers.

In another particular embodiment, the invention relates to the use of nucleic acid molecule for the in vitro and ex vivo diagnosis of somatic and ovarian tumor, wherein the nucleic acid molecule belongs to the group consisting in SEQ ID NO 9, SEQ ID NO 11, SEQ ID NO 23, SEQ ID NO 25, SEQ ID NO 29, SEQ ID NO 31, SEQ ID NO 33, SEQ ID NO 35, SEQ ID NO 53, SEQ ID NO 57, SEQ ID NO 75, SEQ ID NO 77, SEQ ID NO 87, SEQ ID NO 105, SEQ ID NO 107, SEQ ID NO 109, SEQ ID NO 111, SEQ ID NO 123, SEQ ID NO 125, SEQ ID NO 127, SEQ ID NO 129, SEQ ID NO 131, SEQ ID NO 133, SEQ ID NO 139, SEQ ID NO 143, SEQ ID NO 147, SEQ ID NO 151, SEQ ID NO 153, SEQ ID NO 159, SEQ ID NO 163, SEQ ID NO 173, SEQ ID NO 175, SEQ ID NO 177, SEQ ID NO 179, SEQ ID NO 181, SEQ ID NO 185, SEQ ID NO 187, SEQ ID NO 193, SEQ ID NO 201, SEQ ID NO 209, SEQ ID NO 211, SEQ ID NO 221, SEQ ID NO 227, SEQ ID NO 249, SEQ ID NO 253, SEQ ID NO 257, SEQ ID NO 259, SEQ ID NO 261, SEQ ID NO 265, SEQ ID NO 269, SEQ ID NO 275, SEQ ID NO 277, SEQ ID NO 279, SEQ ID NO 283, SEQ ID NO 285, SEQ ID NO 289, SEQ ID NO 301, SEQ ID NO 305, SEQ ID NO 307, SEQ ID NO 311, SEQ ID NO 315, SEQ ID NO 349, SEQ ID NO 383, SEQ ID NO 391, SEQ ID NO 393, SEQ ID NO 411, SEQ ID NO 415, SEQ ID NO 419, SEQ ID NO 421, SEQ ID NO 427, SEQ ID NO 429, SEQ ID NO 431, SEQ ID NO 433, SEQ ID NO 439, SEQ ID NO 443, SEQ ID NO 447, SEQ ID NO 451, SEQ ID NO 465, SEQ ID NO 467, SEQ ID NO 471, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 499, SEQ ID NO 501, SEQ ID NO 503, SEQ ID NO 505, SEQ ID NO 509, SEQ ID NO 511, SEQ ID NO 519, SEQ ID NO 521, SEQ ID NO 523, SEQ ID NO 525, SEQ ID NO 529, SEQ ID NO 531, SEQ ID NO 539, SEQ ID NO 543, SEQ ID NO 545, SEQ ID NO 547, SEQ ID NO 553, SEQ ID NO 555, SEQ ID NO 557, SEQ ID NO 559, SEQ ID NO 563, SEQ ID NO 565, SEQ ID NO 573, SEQ ID NO 575, SEQ ID NO 581, SEQ ID NO 583, SEQ ID NO 587, SEQ ID NO 589, SEQ ID NO 593, SEQ ID NO 597, SEQ ID NO 605, SEQ ID NO 607, SEQ ID NO 611, SEQ ID NO 615, SEQ ID NO 617, SEQ ID NO 621, SEQ ID NO 629, SEQ ID NO 631, SEQ ID NO 639, SEQ ID NO 701, SEQ ID NO 716 and SEQ ID NO 721.
These nucleic acid molecules are expressed in testis or placenta.

In another particular embodiment, the invention relates to the use of nucleic acid molecule for the in vitro and ex vivo diagnosis of somatic and ovarian tumor, wherein the nucleic acid molecule belongs to the group consisting in SEQ ID NO 9; SEQ ID NO 11; SEQ ID NO 23; SEQ ID NO 27; SEQ ID NO 31; SEQ ID NO 35; SEQ ID NO 77; SEQ ID NO 87; SEQ ID NO 111; SEQ ID NO 123; SEQ ID NO 125; SEQ ID NO 127; SEQ ID NO 129; SEQ ID NO 131; SEQ ID NO 133; SEQ ID NO 135; SEQ ID NO 139; SEQ ID NO 143; SEQ ID NO 151; SEQ ID NO 153; SEQ ID NO 159; SEQ ID NO 163; SEQ ID NO 181; SEQ ID NO 185; SEQ ID NO 201; SEQ ID NO 227; SEQ ID NO 249; SEQ ID NO 253; SEQ ID NO 257; SEQ ID NO 259; SEQ ID NO 265; SEQ ID NO 267; SEQ ID NO 269; SEQ ID NO 275; SEQ ID NO 277; SEQ ID NO 279; SEQ ID NO 283; SEQ ID NO 285; SEQ ID NO 289; SEQ ID NO 301; SEQ ID NO 305; SEQ ID NO 307; SEQ ID NO 309; SEQ ID NO 311; SEQ ID NO 315; SEQ ID NO 349; SEQ ID NO 393; SEQ ID NO 411; SEQ ID NO 419; SEQ ID NO 421; SEQ ID NO 427; SEQ ID NO 429; SEQ ID NO 431; SEQ ID NO 439; SEQ ID NO 443; SEQ ID NO 447; SEQ ID NO 449; SEQ ID NO 451; SEQ ID NO 469; SEQ ID NO 471; SEQ ID NO 475; SEQ ID NO 479; SEQ ID NO 481; SEQ ID NO 483; SEQ ID NO 485; SEQ ID NO 487; SEQ ID NO 489; SEQ ID NO 491; SEQ ID NO 493; SEQ ID NO 495; SEQ ID NO 501; SEQ ID NO 503; SEQ ID NO 507; SEQ ID NO 511; SEQ ID NO 515; SEQ ID NO 519; SEQ ID NO 521; SEQ ID NO 523; SEQ ID NO 525; SEQ ID NO 531; SEQ ID NO 533; SEQ ID NO 535; SEQ ID NO 537; SEQ ID NO 539; SEQ ID NO 541; SEQ ID NO 547; SEQ ID NO 549; SEQ ID NO 553; SEQ ID NO 555; SEQ ID NO 557; SEQ ID NO 561; SEQ ID NO 563; SEQ ID NO 565; SEQ ID NO 569; SEQ ID NO 573; SEQ ID NO 575; SEQ ID NO 577; SEQ ID NO 581; SEQ ID NO 583; SEQ ID NO 593; SEQ ID NO 597; SEQ ID NO 611; SEQ ID NO 617; SEQ ID NO 629; SEQ ID NO 631 and SEQ ID NO 637.

These genes nucleic acid molecules are expressed in testis or placenta, and are also abnormally expressed in somatic or ovarian cancers.

In another particular embodiment, the invention relates to the use of nucleic acid molecule for the in vitro and ex vivo diagnosis of somatic and ovarian tumor, wherein the nucleic acid molecule belongs to the group consisting in SEQ ID NO 11; SEQ ID NO 27; SEQ ID NO 31; SEQ ID NO 35; SEQ ID NO 77; SEQ ID NO 87; SEQ ID NO 123; SEQ ID NO 125; SEQ ID NO 127; SEQ ID NO 129; SEQ ID NO 135; SEQ ID NO 139; SEQ ID NO 143; SEQ ID NO 151; SEQ ID NO 153; SEQ ID NO 159; SEQ ID NO 163; SEQ ID NO 185; SEQ ID NO 201; SEQ ID NO 227; SEQ ID NO 249; SEQ ID NO 253; SEQ ID NO 257; SEQ ID NO 259; SEQ ID NO 265; SEQ ID NO 267; SEQ ID NO 269; SEQ ID NO 277; SEQ ID NO 279; SEQ ID NO 285; SEQ ID NO 301; SEQ ID NO 305; SEQ ID NO 309; SEQ ID NO 311; SEQ ID NO 393; SEQ ID NO 411; SEQ ID NO 419; SEQ ID NO 421; SEQ ID NO 427; SEQ ID NO 429; SEQ ID NO 431; SEQ ID NO 439; SEQ ID NO 443; SEQ ID NO 447; SEQ ID NO 449; SEQ ID NO 469; SEQ ID NO 475; SEQ ID NO 501; SEQ ID NO 503; SEQ ID NO 507; SEQ ID NO 511; SEQ ID NO 515; SEQ ID NO 519; SEQ ID NO 525; SEQ ID NO 531; SEQ ID NO 533; SEQ ID NO 535; SEQ ID NO 537; SEQ ID NO 539; SEQ ID NO 541; SEQ ID NO 547; SEQ ID NO 549; SEQ ID NO 553; SEQ ID NO 555; SEQ ID NO 557; SEQ ID NO 561; SEQ ID NO 563; SEQ ID NO 565; SEQ ID NO 569; SEQ ID NO 573; SEQ ID NO 575; SEQ ID NO 577; SEQ ID NO 581; SEQ ID NO 593; SEQ ID NO 611; SEQ ID NO 617; SEQ ID NO 629; SEQ ID NO 631 and SEQ ID NO 637.

These nucleic acid molecules are expressed in testis, and are also abnormally expressed in somatic or ovarian cancers.

In another particular embodiment, the invention relates to the use of nucleic acid molecule for the in vitro and ex vivo diagnosis of somatic and ovarian tumor, wherein the nucleic acid molecule belongs to the group consisting in SEQ ID NO 9, SEQ ID NO 23, SEQ ID NO 111, SEQ ID NO 131, SEQ ID NO 133, SEQ ID NO 181, SEQ ID NO 275, SEQ ID NO 283, SEQ ID NO 289, SEQ ID NO 307, SEQ ID NO 315, SEQ ID NO 349, SEQ ID NO 451, SEQ ID NO 471, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 521, SEQ ID NO 523, SEQ ID NO 583 and SEQ ID NO 597,
These genes nucleic acid molecules are expressed in placenta, and are also abnormally expressed in somatic or ovarian cancers.

In another particular embodiment, the invention relates to the use of nucleic acid molecule for the in vitro and ex vivo diagnosis of somatic and ovarian tumor, belonging to the group consisting in SEQ ID NO 1; SEQ ID NO 3; SEQ ID NO 5; SEQ ID NO 7; SEQ ID NO 9; SEQ ID NO 11; SEQ ID NO 15; SEQ ID NO 17; SEQ ID NO 19; SEQ ID NO 21; SEQ ID NO 23; SEQ ID NO 27; SEQ ID NO 31; SEQ ID NO 35; SEQ ID NO 37; SEQ ID NO 41; SEQ ID NO 43; SEQ ID NO 59; SEQ ID NO 67; SEQ ID NO 69; SEQ ID NO 73; SEQ ID NO 77; SEQ ID NO 79; SEQ ID NO 85; SEQ ID NO 87; SEQ ID NO 89; SEQ ID NO 91; SEQ ID NO 95; SEQ ID NO 97; SEQ ID NO 99; SEQ ID NO 101; SEQ ID NO 111; SEQ ID NO 115; SEQ ID NO 119; SEQ ID NO 123; SEQ ID NO 125; SEQ ID NO 127; SEQ ID NO 129; SEQ ID NO 131; SEQ ID NO 133; SEQ ID NO 135; SEQ ID NO 139; SEQ ID NO 141; SEQ ID NO 143; SEQ ID NO 145; SEQ ID NO 151; SEQ ID NO 153; SEQ ID NO 155; SEQ ID NO 157; SEQ ID NO 159; SEQ ID NO 161; SEQ ID NO 163; SEQ ID NO 165; SEQ ID NO 167; SEQ ID NO 169; SEQ ID NO 181; SEQ ID NO 183; SEQ ID NO 185; SEQ ID NO 189; SEQ ID NO 199; SEQ ID NO 201; SEQ ID NO 203; SEQ ID NO 217; SEQ ID NO 223; SEQ ID NO 227; SEQ ID NO 231; SEQ ID NO 233; SEQ ID NO 237; SEQ ID NO 239; SEQ ID NO 243; SEQ ID NO 249; SEQ ID NO 251; SEQ ID NO 253; SEQ ID NO 257; SEQ ID NO 259; SEQ ID NO 265; SEQ ID NO 267; SEQ ID NO 269; SEQ ID NO 271; SEQ ID NO 273; SEQ ID NO 275; SEQ ID NO 277; SEQ ID NO 279; SEQ ID NO 283; SEQ ID NO 285; SEQ ID NO 287; SEQ ID NO 289; SEQ ID NO 293; SEQ ID NO 297; SEQ ID NO 299; SEQ ID NO 301; SEQ ID NO 303; SEQ ID NO 305; SEQ ID NO 307; SEQ ID NO 309; SEQ ID NO 311; SEQ ID NO 313; SEQ ID NO 315; SEQ ID NO 319; SEQ ID NO 321; SEQ ID NO 323; SEQ ID NO 325; SEQ ID NO 327; SEQ ID NO 337; SEQ ID NO 347; SEQ ID NO 349; SEQ ID NO 351; SEQ ID NO 365; SEQ ID NO 369; SEQ ID NO 371; SEQ ID NO 375; SEQ ID NO 381; SEQ ID NO 385; SEQ ID NO 387; SEQ ID NO 389; SEQ ID NO 393; SEQ ID NO 401; SEQ ID NO 403; SEQ ID NO 407; SEQ ID NO 411; SEQ ID NO 417; SEQ ID NO 419; SEQ ID NO 421; SEQ ID NO 425; SEQ ID NO 427; SEQ ID NO 429; SEQ ID NO 431; SEQ ID NO 435; SEQ ID NO 437; SEQ ID NO 439; SEQ ID NO 443; SEQ ID NO 445; SEQ ID NO 447; SEQ ID NO 449; SEQ ID NO 451; SEQ ID NO 453; SEQ ID NO 455; SEQ ID NO 457; SEQ ID NO 459; SEQ ID NO 461; SEQ ID NO 463; SEQ ID NO 469; SEQ ID NO 471; SEQ ID NO 473; SEQ ID NO 475; SEQ ID NO 477; SEQ ID NO 479; SEQ ID NO 481; SEQ ID NO 483; SEQ ID NO 485; SEQ ID NO 487; SEQ ID NO 489; SEQ ID NO 491; SEQ ID NO 493; SEQ ID NO 495; SEQ ID NO 497; SEQ ID NO 501; SEQ ID NO 503; SEQ ID NO 507; SEQ ID NO 511; SEQ ID NO 513; SEQ ID NO 515; SEQ ID NO 517; SEQ ID NO 519; SEQ ID NO 521; SEQ ID NO 523; SEQ ID NO 525; SEQ ID NO 531; SEQ ID NO 533; SEQ ID NO 535; SEQ ID NO 537; SEQ ID NO 539; SEQ ID NO 541; SEQ ID NO 547; SEQ ID NO 549; SEQ ID NO 551; SEQ ID NO 553; SEQ ID NO 555; SEQ ID NO 557; SEQ ID NO 561; SEQ ID NO 563; SEQ ID NO 565; SEQ ID NO 569; SEQ ID NO 571; SEQ ID NO 573; SEQ ID NO 575; SEQ ID NO 577; SEQ ID NO 581; SEQ ID NO 583; SEQ ID NO 585; SEQ ID NO 591; SEQ ID NO 593; SEQ ID NO 597; SEQ ID NO 603; SEQ ID NO 611; SEQ ID NO 613; SEQ ID NO 617; SEQ ID NO 623; SEQ ID NO 627; SEQ ID NO 629; SEQ ID NO 631; SEQ ID NO 633; SEQ ID NO 635; SEQ ID NO 637; SEQ ID NO 642; SEQ ID NO 644; SEQ ID NO 645; SEQ ID NO 647; SEQ ID NO 649; SEQ ID NO 652; SEQ ID NO 653; SEQ ID NO 655; SEQ ID NO 656; SEQ ID NO 657; SEQ ID NO 661; SEQ ID NO 664; SEQ ID NO 665; SEQ ID NO 671; SEQ ID NO 672; SEQ ID NO 673; SEQ ID NO 674; SEQ ID NO 675; SEQ ID NO 677; SEQ ID NO 681; SEQ ID NO 684; SEQ ID NO 688; SEQ ID NO 691; SEQ ID NO 692; SEQ ID NO 695; SEQ ID NO 697; SEQ ID NO 700; SEQ ID NO 704; SEQ ID NO 705; SEQ ID NO 706; SEQ ID NO 710; SEQ ID NO 712; SEQ ID NO 714; SEQ ID NO 717; SEQ ID NO 718; SEQ ID NO 719; SEQ ID NO 720; SEQ ID NO 722; SEQ ID NO 724; SEQ ID NO 725; SEQ ID NO 728; SEQ ID NO 729; SEQ ID NO 734; SEQ ID NO 736; SEQ ID NO 737 and SEQ ID NO 738.

Some of these nucleic acid molecules are new or known genes, and all are expressed in testis or placenta, and are also abnormally expressed in somatic or ovarian cancers.

In another particular embodiment, the invention relates to the use of nucleic acid molecule for the in vitro and ex vivo diagnosis of somatic and ovarian tumor, wherein the nucleic acid molecule belongs to the group consisting in SEQ ID NO 9; SEQ ID NO 11; SEQ ID NO 17; SEQ ID NO 23; SEQ ID NO 27; SEQ ID NO 31; SEQ ID NO 35; SEQ ID NO 37; SEQ ID NO 41; SEQ ID NO 43; SEQ ID NO 59; SEQ ID NO 69; SEQ ID NO 73; SEQ ID NO 77; SEQ ID NO 79; SEQ ID NO 85; SEQ ID NO 87; SEQ ID NO 89; SEQ ID NO 91; SEQ ID NO 95; SEQ ID NO 97; SEQ ID NO 99; SEQ ID NO 101; SEQ ID NO 111; SEQ ID NO 115; SEQ ID NO 123; SEQ ID NO 125; SEQ ID NO 127; SEQ ID NO 129; SEQ ID NO 131; SEQ ID NO 133; SEQ ID NO 135; SEQ ID NO 139; SEQ ID NO 143; SEQ ID NO 151; SEQ ID NO 153; SEQ ID NO 155; SEQ ID NO 157; SEQ ID NO 159; SEQ ID NO 161; SEQ ID NO 163; SEQ ID NO 165; SEQ ID NO 167; SEQ ID NO 181; SEQ ID NO 185; SEQ ID NO 189; SEQ ID NO 199; SEQ ID NO 201; SEQ ID NO 203; SEQ ID NO 217; SEQ ID NO 223; SEQ ID NO 227; SEQ ID NO 231; SEQ ID NO 233; SEQ ID NO 237; SEQ ID NO 239; SEQ ID NO 243; SEQ ID NO 249; SEQ ID NO 253; SEQ ID NO 257; SEQ ID NO 259; SEQ ID NO 265; SEQ ID NO 267; SEQ ID NO 269; SEQ ID NO 275; SEQ ID NO 277; SEQ ID NO 279; SEQ ID NO 283; SEQ ID NO 285; SEQ ID NO 289; SEQ ID NO 293; SEQ ID NO 297; SEQ ID NO 301; SEQ ID NO 303; SEQ ID NO 305; SEQ ID NO 307; SEQ ID NO 309; SEQ ID NO 311; SEQ ID NO 315; SEQ ID NO 319; SEQ ID NO 321; SEQ ID NO 323; SEQ ID NO 325; SEQ ID NO 327; SEQ ID NO 337; SEQ ID NO 347; SEQ ID NO 349; SEQ ID NO 351; SEQ ID NO 365; SEQ ID NO 369; SEQ ID NO 371; SEQ ID NO 375; SEQ ID NO 381; SEQ ID NO 387; SEQ ID NO 389; SEQ ID NO 393; SEQ ID NO 401; SEQ ID NO 403; SEQ ID NO 407; SEQ ID NO 411; SEQ ID NO 419; SEQ ID NO 421; SEQ ID NO 425; SEQ ID NO 427; SEQ ID NO 429; SEQ ID NO 431; SEQ ID NO 435; SEQ ID NO 437; SEQ ID NO 439; SEQ ID NO 443; SEQ ID NO 445; SEQ ID NO 447; SEQ ID NO 449; SEQ ID NO 451; SEQ ID NO 461; SEQ ID NO 463; SEQ ID NO 469; SEQ ID NO 471; SEQ ID NO 475; SEQ ID NO 479; SEQ ID NO 481; SEQ ID NO 483; SEQ ID NO 485; SEQ ID NO 487; SEQ ID NO 489; SEQ ID NO 491; SEQ ID NO 493; SEQ ID NO 495; SEQ ID NO 497; SEQ ID NO 501; SEQ ID NO 503; SEQ ID NO 507; SEQ ID NO 511; SEQ ID NO 515; SEQ ID NO 517; SEQ ID NO 519; SEQ ID NO 521; SEQ ID NO 523; SEQ ID NO 525; SEQ ID NO 531; SEQ ID NO 533; SEQ ID NO 535; SEQ ID NO 537; SEQ ID NO 539; SEQ ID NO 541; SEQ ID NO 547; SEQ ID NO 549; SEQ ID NO 551; SEQ ID NO 553; SEQ ID NO 555; SEQ ID NO 557; SEQ ID NO 561; SEQ ID NO 563; SEQ ID NO 565; SEQ ID NO 569; SEQ ID NO 573; SEQ ID NO 575; SEQ ID NO 577; SEQ ID NO 581; SEQ ID NO 583; SEQ ID NO 593; SEQ ID NO 597; SEQ ID NO 611; SEQ ID NO 617; SEQ ID NO 623; SEQ ID NO 627; SEQ ID NO 629; SEQ ID NO 631; SEQ ID NO 637; SEQ ID NO 642; SEQ ID NO 644; SEQ ID NO 645; SEQ ID NO 647; SEQ ID NO 649; SEQ ID NO 652; SEQ ID NO 653; SEQ ID NO 655; SEQ ID NO 656; SEQ ID NO 657; SEQ ID NO 661; SEQ ID NO 664; SEQ ID NO 665; SEQ ID NO 671; SEQ ID NO 672; SEQ ID NO 673; SEQ ID NO 674; SEQ ID NO 675; SEQ ID NO 677; SEQ ID NO 681; SEQ ID NO 684; SEQ ID NO 688; SEQ ID NO 691; SEQ ID NO 692; SEQ ID NO 695; SEQ ID NO 697; SEQ ID NO 700; SEQ ID NO 704; SEQ ID NO 705; SEQ ID NO 706; SEQ ID NO 710; SEQ ID NO 712; SEQ ID NO 714; SEQ ID NO 717; SEQ ID NO 718; SEQ ID NO 719; SEQ ID NO 722; SEQ ID NO 724; SEQ ID NO 725; SEQ ID NO 728; SEQ ID NO 729; SEQ ID NO 734; SEQ ID NO 736; SEQ ID NO 737 and SEQ ID NO 738.

Some of these nucleic acid molecules are new or known genes, and all are expressed in testis or placenta, and are also abnormally expressed in somatic or ovarian cancers.

The invention also relates to the use of one element chosen among:
- at least a protein coded by a nucleic acid molecule described above, said protein comprising or constituted by an amino acid sequence belonging to the group consisting in SEQ ID NO 2q, q varying from 1 to 320, or
- at least a variant of said protein, said variant presenting a sequence identity of at least 70%, preferably 80%, more preferably 90% with respect to said protein, or
- at least a fragment of said protein, said fragment being able to be recognized by an antibody specifically directed against said protein,
for the *in vitro* or *ex vivo* diagnosis of any type of somatic or ovarian cancers,
wherein at least one of any of the above described elements is abnormally expressed in cancer cells of at least one type of the somatic or ovarian cancers, and
wherein each type of somatic or ovarian cancer cells abnormally expresses at least one of the above described elements.

The above mentioned protein SEQ ID NO 2q, q varying from 1 to 320, correspond to a protein coding by the nucleic acid molecules SEQ ID NO 2q-1, q varying from 1 to 320.
As examples, according to the invention, said proteins can be defined such that SEQ ID NO 2 coded by nucleic acid molecule SEQ ID NO 1, SEQ ID NO 4 coded by nucleic acid molecule SEQ ID NO 3, SEQ ID NO 6 coded by nucleic acid molecule SEQ ID NO 5, SEQ ID NO 8 coded by nucleic acid molecule SEQ ID NO 7, etc...

The following table 1 summarizes the correspondence between nucleic acid molecules SEQ ID NO 2q-1 and the protein SEQ ID NO 2q, coded by said nucleic acids.
The table 1 also describes the cells wherein nucleic acid molecules and protein are normally expressed. PS: Placental-specific genes, TS Testis-specific genes.

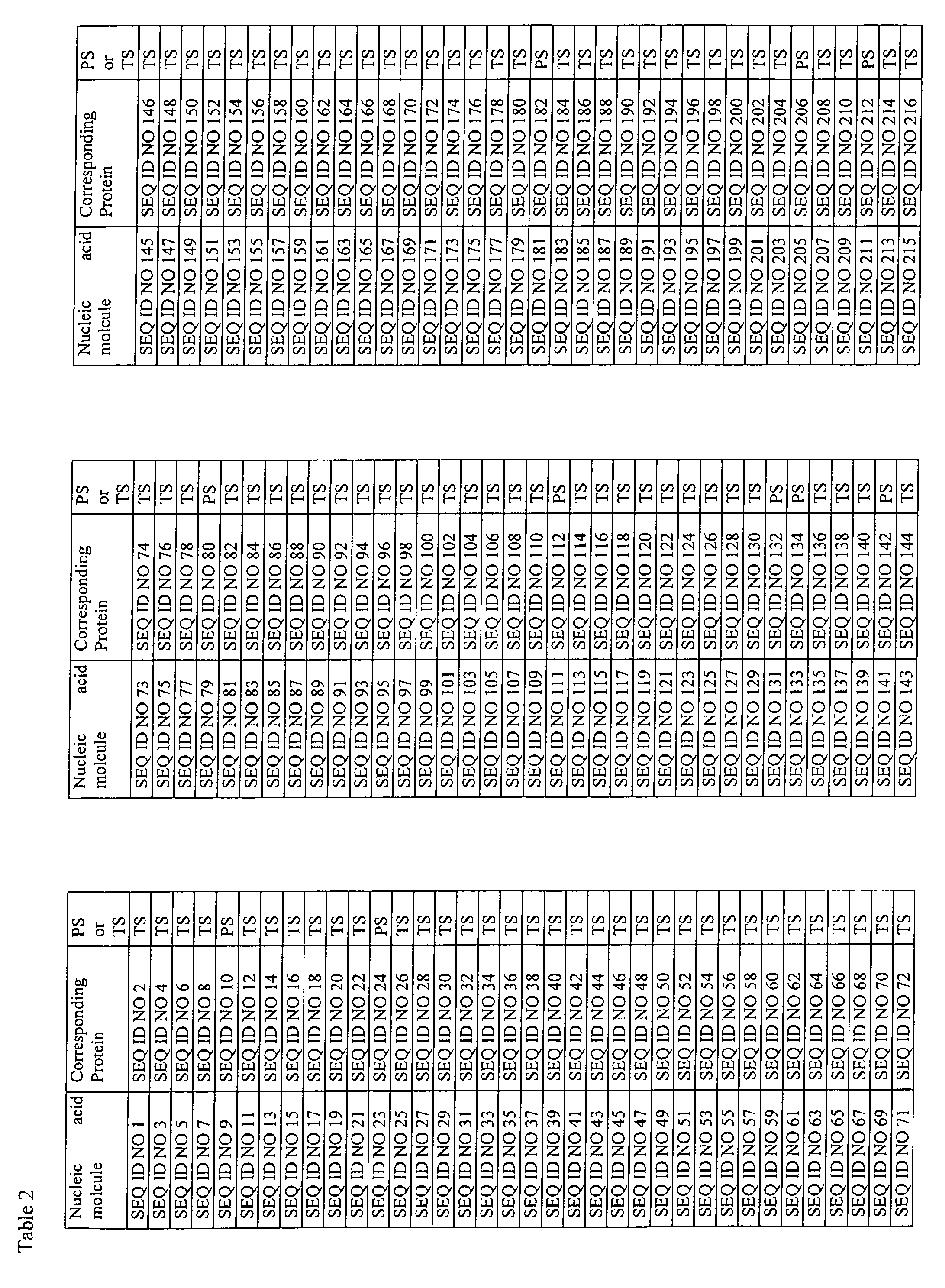

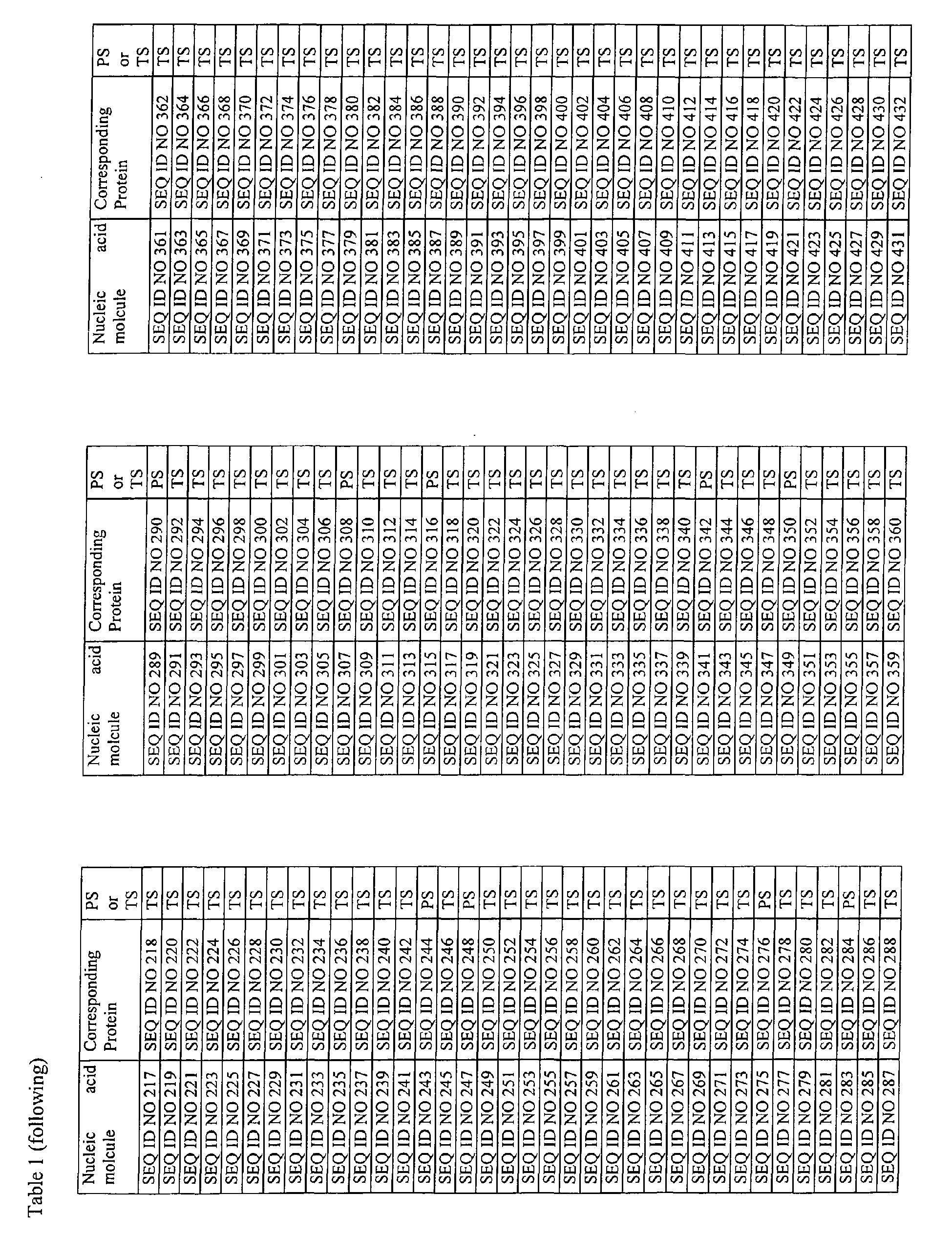

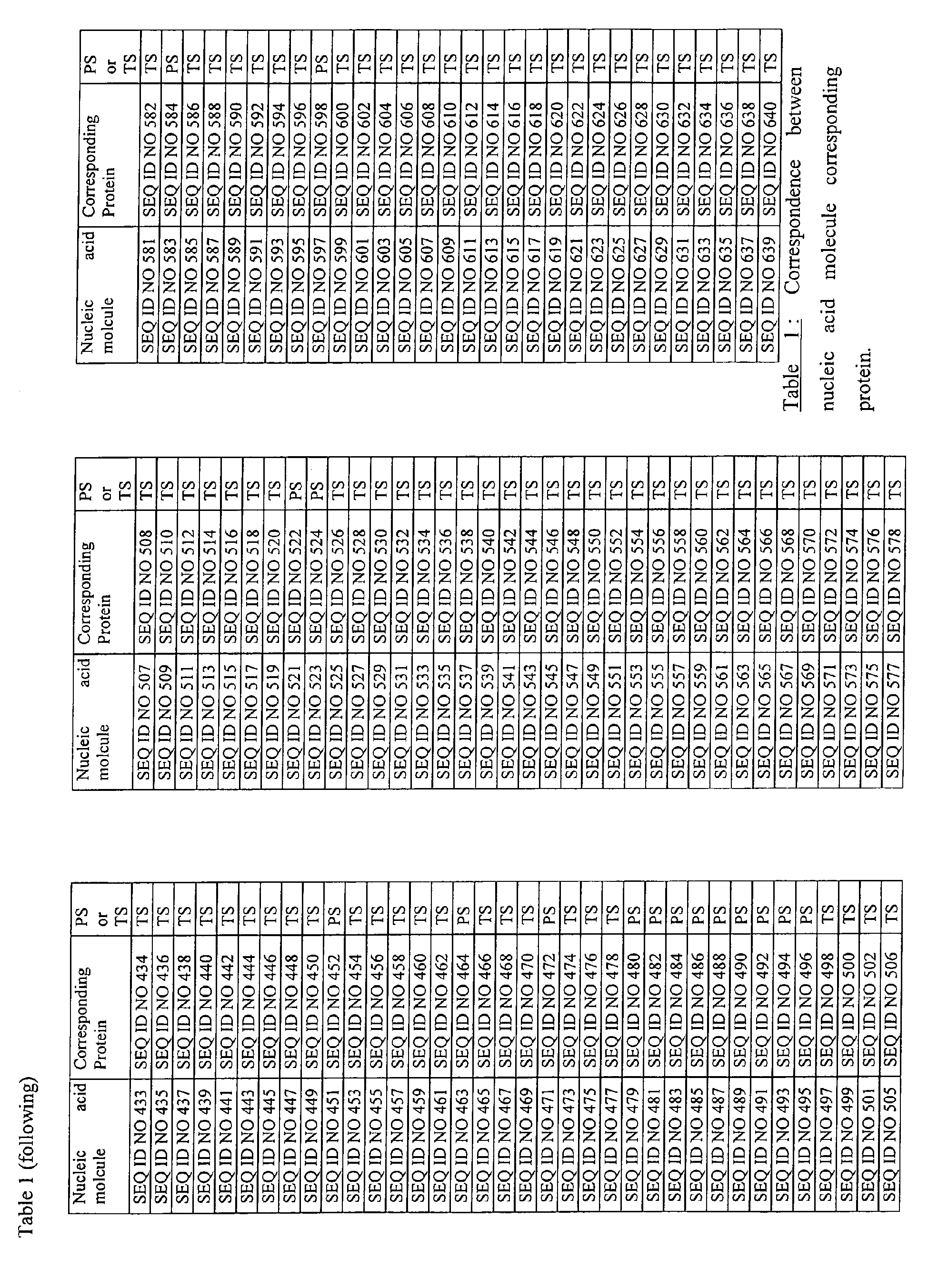

Protein and fragments thereof are defined such that they comprise amino acid sequences liable to be recognized by specific antibodies.

In one preferred embodiment, the invention also related to the use of one antibody characterized in that it specifically recognizes a protein described above.
According to the invention, the antibodies can be monoclonal antibodies or polyclonal antibodies.

In a preferred embodiment, the invention relates to the use of a nucleic acid molecule described above, or a protein described above for the for the *in vitro* or *ex vivo* diagnosis of any type of somatic or ovarian cancers, wherein the somatic or ovarian cancer cells is a solid tumor, or an hematological neoplasm.

According to the invention, "neoplasm" describes an abnormal proliferation of genetically altered cells. Neoplasms can be benign or malignant.
According to the invention, "tumors" means any abnormal swelling, lump or mass.
As commonly used in the art, according to the invention, terms "tumor" and "neoplasm" are synonymous with cancer.

Cancers are classified by the type of cell that resembles the tumor and, therefore, the tissue presumed to be the origin of the tumor. Examples of general categories include:
- Carcinoma: Malignant tumors derived from epithelial cells. This group represents the most common cancers, including the common forms of breast, prostate, lung and colon cancer.
- Sarcoma: Malignant tumors derived from connective tissue, or mesenchymal cells,
- Germ cell tumor: Tumors derived from totipotent cells. In adults most often found in the testicle and ovary. However, the invention does no relate to the testicle cancer,
- Blastic tumor: A tumor (usually malignant) which resembles an immature or embryonic tissue. Many of these tumors are most common in children,
- Lymphoma and leukemia: Malignancies derived from hematopoietic (blood-forming) cells.

According to the invention, "solid tumors" concern tumors derived from organs, and in particular concern:
- lung cancer, including small cell lung cancer and non-small lung cancer,
- pancreas cancer,
- bladder cancer,
- breast cancer,
- brain cancer, including glioblastomas medulloblastomas and neuroblastomas,
- cervical cancer,
- gastric cancer,
- colon cancer, including colorectal carcinoma,
- endometrial cancer,
- esophageal cancer,
- biliary tract cancer,
- head and neck cancer,
- oral cancer, including squamous cell carcinoma,
- liver cancer, including hepatocarcinoma,
- ovarian cancer, including those arising from epithelial cells, stromal cells, germ cells and mesenchymal cells,
- pancreatic cancer,
- prostate cancer,
- rectal cancer,
- sarcomas, including leiomyosarcoma, rhabdomyosarcoma, liposarcoma, fibrosarcoma, synovial sarcoma, neurosarcoma, chondrosarcoma, Ewing sarcoma, malignant fibrous histocytoma, glioma, hepatoma and osteosarcoma,
- skin cancer, including melanomas, Kaposi's sarcoma, basocellular cancer and squamous cell cancer,
- thyroid cancer, including thyroid adenocarcinoma and medullar carcinoma,
- kidney cancer, including adenocarcinoma and Wilms tumour,
- intraepithelial neoplasms, including Bowen's disease and Paget's disease, and
- placental cancer or choriocarcinoma.

According to the invention, "hematological neoplasms" concern all the neoplasms derived from blood cells or progeny of blood cells, and in particular concern:
- acute lymphocytic leukemias,
- acute myelogenous leukemias,
- multiple myelomas,
- AIDS-associated leukemias, and
- adult T-cell leukemia lymphomas

The invention concerns also lymphomas such as Hodgkin's disease, lymphocytic lymphoma and mantle cell lymphoma.

The invention also relates to one at least of the nucleic acid molecule belonging to the group consisting in SEQ ID NO 17, SEQ ID NO 37, SEQ ID NO 39, SEQ ID NO 41, SEQ ID NO 43, SEQ ID NO 45, SEQ ID NO 47, SEQ ID NO 49, SEQ ID NO 51, SEQ ID NO 55, SEQ ID NO 59, SEQ ID NO 61, SEQ ID NO 63, SEQ ID NO 65, SEQ ID NO 67, SEQ ID NO 69, SEQ ID NO 71, SEQ ID NO 73, SEQ ID NO 79, SEQ ID NO 81, SEQ ID NO 83, SEQ ID NO 85, SEQ ID NO 89, SEQ ID NO 91, SEQ ID NO 93, SEQ ID NO 95, SEQ ID NO 97, SEQ ID NO 99, SEQ ID NO 101, SEQ ID NO 103, SEQ ID NO 113, SEQ ID NO 115, SEQ ID NO 117, SEQ ID NO 121, SEQ ID NO 155, SEQ ID NO 157, SEQ ID NO 161, SEQ ID NO 165, SEQ ID NO 167, SEQ ID NO 169, SEQ ID NO 171, SEQ ID NO 189, SEQ ID NO 191, SEQ ID NO 195, SEQ ID NO 197, SEQ ID NO 199, SEQ ID NO 203, SEQ ID NO 205, SEQ ID NO 207, SEQ ID NO 213, SEQ ID NO 215, SEQ ID NO 217, SEQ ID NO 219, SEQ ID NO 223, SEQ ID NO 225, SEQ ID NO 229, SEQ ID NO 231, SEQ ID NO 233, SEQ ID NO 235, SEQ ID NO 237, SEQ ID NO 239, SEQ ID NO 241, SEQ ID NO 243, SEQ ID NO 247, SEQ ID NO 263, SEQ ID NO 281, SEQ ID NO 293, SEQ ID NO 295, SEQ ID NO 297, SEQ ID NO 303, SEQ ID NO 317, SEQ ID NO 319, SEQ ID NO 321, SEQ ID NO 323, SEQ ID NO 325, SEQ ID NO 327, SEQ ID NO 329, SEQ ID NO 331, SEQ ID NO 333, SEQ ID NO 335, SEQ ID NO 337, SEQ ID NO 339, SEQ ID NO 341, SEQ ID NO 343, SEQ ID NO 345, SEQ ID NO 347, SEQ ID NO 351, SEQ ID NO 353, SEQ ID NO 355, SEQ ID NO 357, SEQ ID NO 359, SEQ ID NO 361, SEQ ID NO 363, SEQ ID NO 365, SEQ ID NO 367, SEQ ID NO 369, SEQ ID NO 371, SEQ ID NO 373, SEQ ID NO 375, SEQ ID NO 377, SEQ ID NO 379, SEQ ID NO 381, SEQ ID NO 387, SEQ ID NO 389, SEQ ID NO 395, SEQ ID NO 397, SEQ ID NO 399, SEQ ID NO 401, SEQ ID NO 403, SEQ ID NO 405, SEQ ID NO 407, SEQ ID NO 409, SEQ ID NO 413, SEQ ID NO 423, SEQ ID NO 425, SEQ ID NO 435, SEQ ID NO 437, SEQ ID NO 441, SEQ ID NO 445, SEQ ID NO 461, SEQ ID NO 463, SEQ ID NO 497, SEQ ID NO 517, SEQ ID NO 527, SEQ ID NO 551, SEQ ID NO 567, SEQ ID NO 579, SEQ ID NO 595, SEQ ID NO 599, SEQ ID NO 601, SEQ ID NO 609, SEQ ID NO 623, SEQ ID NO 625, SEQ ID NO 627, SEQ ID NO 641, SEQ ID NO 642, SEQ ID NO 643, SEQ ID NO 644, SEQ ID NO 645, SEQ ID NO 646, SEQ ID NO 647, SEQ ID NO 648, SEQ ID NO 649, SEQ ID NO 650, SEQ ID NO 651, SEQ ID NO 652, SEQ ID NO 653, SEQ ID NO 654, SEQ ID NO 655, SEQ ID NO 656, SEQ ID NO 657, SEQ ID NO 658, SEQ ID NO 659, SEQ ID NO 660, SEQ ID NO 661, SEQ ID NO 662, SEQ ID NO 663, SEQ ID NO 664, SEQ ID NO 665, SEQ ID NO 666, SEQ ID NO 667, SEQ ID NO 668, SEQ ID NO 669, SEQ ID NO 670, SEQ ID NO 671, SEQ ID NO 672, SEQ ID NO 673, SEQ ID NO 674, SEQ ID NO 675, SEQ ID NO 676, SEQ ID NO 677, SEQ ID NO 678, SEQ ID NO 679, SEQ ID NO 680, SEQ ID NO 681, SEQ ID NO 682, SEQ ID NO 683, SEQ ID NO 684, SEQ ID NO 685, SEQ ID NO 686, SEQ ID NO 687, SEQ ID NO 688, SEQ ID NO 689, SEQ ID NO 690, SEQ ID NO 691, SEQ ID NO 692, SEQ ID NO 693, SEQ ID NO 694, SEQ ID NO 695, SEQ ID NO 696, SEQ ID NO 697, SEQ ID NO 698, SEQ ID NO 699, SEQ ID NO 700, SEQ ID NO 702, SEQ ID NO 703, SEQ ID NO 704, SEQ ID NO 705, SEQ ID NO 706, SEQ ID NO 707, SEQ ID NO 708, SEQ ID NO 709, SEQ ID NO 710, SEQ ID NO 711, SEQ ID NO 712, SEQ ID NO 713, SEQ ID NO 714, SEQ ID NO 715, SEQ ID NO 717, SEQ ID NO 718, SEQ ID NO 719, SEQ ID NO 720, SEQ ID NO 722, SEQ ID NO 724, SEQ ID NO 725, SEQ ID NO 726, SEQ ID NO 727, SEQ ID NO 728, SEQ ID NO 729, SEQ ID NO 730, SEQ ID NO 731, SEQ ID NO 732, SEQ ID NO 733, SEQ ID NO 734, SEQ ID NO 735, SEQ ID NO 736, SEQ ID NO 737, SEQ ID NO 738, SEQ ID NO 739, SEQ ID NO 740, SEQ ID NO 741, SEQ ID NO 742, SEQ ID NO 743, SEQ ID NO 744, SEQ ID NO 745, SEQ ID NO 746, SEQ ID NO 747, SEQ ID NO 748, SEQ ID NO 749, SEQ ID NO 750, SEQ ID NO 751, SEQ ID NO 752, SEQ ID NO 753 and SEQ ID NO 754, or fragment thereof, or variants of said nucleic acid molecules,
or a set comprising at least two of said nucleic acid molecules, or fragment thereof, or variants. These nucleic acid molecules are novel and expressed in placenta or in testis, according to the invention.

In a particular embodiment, the invention also relates to a one at least of the nucleic acid molecule belonging to the group consisting in SEQ ID NO 17, SEQ ID NO 37, SEQ ID NO 39, SEQ ID NO 41, SEQ ID NO 43, SEQ ID NO 45, SEQ ID NO 47, SEQ ID NO 49, SEQ ID NO 51, SEQ ID NO 55, SEQ ID NO 59, SEQ ID NO 61, SEQ ID NO 63, SEQ ID NO 65, SEQ ID NO 67, SEQ ID NO 69, SEQ ID NO 71, SEQ ID NO 73, SEQ ID NO 81, SEQ ID NO 83, SEQ ID NO 85, SEQ ID NO 89, SEQ ID NO 91, SEQ ID NO 93, SEQ ID NO 95, SEQ ID NO 97, SEQ ID NO 99, SEQ ID NO 101, SEQ ID NO 103, SEQ ID NO 113, SEQ ID NO 115, SEQ ID NO 117, SEQ ID NO 121, SEQ ID NO 155, SEQ ID NO 157, SEQ ID NO 161, SEQ ID NO 165, SEQ ID NO 167, SEQ ID NO 169, SEQ ID NO 171, SEQ ID NO 189, SEQ ID NO 191, SEQ ID NO 195, SEQ ID NO 197, SEQ ID NO 199, SEQ ID NO 203, SEQ ID NO 207, SEQ ID NO 213, SEQ ID NO 215, SEQ ID NO 217, SEQ ID NO 219, SEQ ID NO 223, SEQ ID NO 225, SEQ ID NO 229, SEQ ID NO 231, SEQ ID NO 233, SEQ ID NO 235, SEQ ID NO 237, SEQ ID NO 239, SEQ ID NO 241, SEQ ID NO 263, SEQ ID NO 281, SEQ ID NO 293, SEQ ID NO 295, SEQ ID NO 297, SEQ ID NO 303, SEQ ID NO 317, SEQ ID NO 319, SEQ ID NO 321, SEQ ID NO 323, SEQ ID NO 325, SEQ ID NO 327, SEQ ID NO 329, SEQ ID NO 331, SEQ ID NO 333, SEQ ID NO 335, SEQ ID NO 337, SEQ ID NO 339, SEQ ID NO 343, SEQ ID NO 345, SEQ ID NO 347, SEQ ID NO 351, SEQ ID NO 353, SEQ ID NO 355, SEQ ID NO 357, SEQ ID NO 359, SEQ ID NO 361, SEQ ID NO 363, SEQ ID NO 365, SEQ ID NO 367, SEQ ID NO 369, SEQ ID NO 371, SEQ ID NO 373, SEQ ID NO 375, SEQ ID NO 377, SEQ ID NO 379, SEQ ID NO 381, SEQ ID NO 387, SEQ ID NO 389, SEQ ID NO 395, SEQ ID NO 397, SEQ ID NO 399, SEQ ID NO 401, SEQ ID NO 403, SEQ ID NO 405, SEQ ID NO 407, SEQ ID NO 409, SEQ ID NO 413, SEQ ID NO 423, SEQ ID NO 425, SEQ ID NO 435, SEQ ID NO 437, SEQ ID NO 441, SEQ ID NO 445, SEQ ID NO 461, SEQ ID NO 497, SEQ ID NO 517, SEQ ID NO 527, SEQ ID NO 551, SEQ ID NO 567, SEQ ID NO 579, SEQ ID NO 595, SEQ ID NO 599, SEQ ID NO 601, SEQ ID NO 609, SEQ ID NO 623, SEQ ID NO 625, SEQ ID NO 627, SEQ ID NO 641, SEQ ID NO 642, SEQ ID NO 643, SEQ ID NO 644, SEQ ID NO 645, SEQ ID NO 646, SEQ ID NO 647, SEQ ID NO 648, SEQ ID NO 649, SEQ ID NO 650, SEQ ID NO 651, SEQ ID NO 652, SEQ ID NO 653, SEQ ID NO 654, SEQ ID NO 656, SEQ ID NO 657, SEQ ID NO 658, SEQ ID NO 659, SEQ ID NO 660, SEQ ID NO 661, SEQ ID NO 662, SEQ ID NO 663, SEQ ID NO 664, SEQ ID NO 665, SEQ ID NO 666, SEQ ID NO 667, SEQ ID NO 668, SEQ ID NO 669, SEQ ID NO 670, SEQ ID NO 672, SEQ ID NO 673, SEQ ID NO 674, SEQ ID NO 675, SEQ ID NO 676, SEQ ID NO 677, SEQ ID NO 678, SEQ ID NO 679, SEQ ID NO 680, SEQ ID NO 681, SEQ ID NO 682, SEQ ID NO 683, SEQ ID NO 687, SEQ ID NO 688, SEQ ID NO 689, SEQ ID NO 690, SEQ ID NO 691, SEQ ID NO 692, SEQ ID NO 693, SEQ ID NO 694, SEQ ID NO 695, SEQ ID NO 696, SEQ ID NO 697, SEQ ID NO 698, SEQ ID NO 700, SEQ ID NO 702, SEQ ID NO 703, SEQ ID NO 704, SEQ ID NO 705, SEQ ID NO 706, SEQ ID NO 707, SEQ ID NO 708, SEQ ID NO 709, SEQ ID NO 710, SEQ ID NO 711, SEQ ID NO 712, SEQ ID NO 713, SEQ ID NO 714, SEQ ID NO 715, SEQ ID NO 718, SEQ ID NO 720, SEQ ID NO 722, SEQ ID NO 724, SEQ ID NO 725, SEQ ID NO 727, SEQ ID NO 728, SEQ ID NO 729, SEQ ID NO 730, SEQ ID NO 731, SEQ ID NO 732, SEQ ID NO 733, SEQ ID NO 735, SEQ ID NO 736, SEQ ID NO 737, SEQ ID NO 738, SEQ ID NO 739, SEQ ID NO 740, SEQ ID NO 741, SEQ ID NO 742, SEQ ID NO 743, SEQ ID NO 744, SEQ ID NO 745, SEQ ID NO 746, SEQ ID NO 747, SEQ ID NO 748, SEQ ID NO 749, SEQ ID NO 750, SEQ ID NO 752, SEQ ID NO 753 and SEQ ID NO 754 or fragment thereof, or variants of said nucleic acid molecules,
or a set comprising at least two of said nucleic acid molecules, or fragment thereof, or variants.

These nucleic acid molecules are novel and are expressed in testis.

In another preferred embodiment, the invention relates to a one at least of the nucleic acid molecule belonging to the group consisting in SEQ ID NO 79, SEQ ID NO 205, SEQ ID NO 243, SEQ ID NO 247, SEQ ID NO 341, SEQ ID NO 463, SEQ ID NO 655, SEQ ID NO 671, SEQ ID NO 684, SEQ ID NO 685, SEQ ID NO 686, SEQ ID NO 699, SEQ ID NO 717, SEQ ID NO 719, SEQ ID NO 726, SEQ ID NO 734 and SEQ ID NO 751 or fragment thereof, or variants of said nucleic acid molecules,
or a set comprising at least two of said nucleic acid molecules, or fragment thereof, or variants. These nucleic acid molecules are novel and are expressed in placenta.

In one other particular embodiment, the invention relates to a one at least of the nucleic acid molecules belonging to the group consisting in SEQ ID NO 17, SEQ ID NO 37, SEQ ID NO 41, SEQ ID NO 43, SEQ ID NO 59, SEQ ID NO 67, SEQ ID NO 69, SEQ ID NO 73, SEQ ID NO 79, SEQ ID NO 85, SEQ ID NO 89, SEQ ID NO 91, SEQ ID NO 95, SEQ ID NO 97, SEQ ID NO 99, SEQ ID NO 101, SEQ ID NO 115, SEQ ID NO 155, SEQ ID NO 157, SEQ ID NO 161, SEQ ID NO 165, SEQ ID NO 167, SEQ ID NO 169, SEQ ID NO 189, SEQ ID NO 199, SEQ ID NO 203, SEQ ID NO 217, SEQ ID NO 223, SEQ ID NO 231, SEQ ID NO 233, SEQ ID NO 237, SEQ ID NO 239, SEQ ID NO 243, SEQ ID NO 293, SEQ ID NO 297, SEQ ID NO 303, SEQ ID NO 319, SEQ ID NO 321, SEQ ID NO 323, SEQ ID NO 325, SEQ ID NO 327, SEQ ID NO 337, SEQ ID NO 347, SEQ ID NO 351, SEQ ID NO 365, SEQ ID NO 369, SEQ ID NO 371, SEQ ID NO 375, SEQ ID NO 381, SEQ ID NO 387, SEQ ID NO 389, SEQ ID NO 401, SEQ ID NO 403, SEQ ID NO 407, SEQ ID NO 425, SEQ ID NO 435, SEQ ID NO 437, SEQ ID NO 445, SEQ ID NO 461, SEQ ID NO 463, SEQ ID NO 497, SEQ ID NO 517, SEQ ID NO 551, SEQ ID NO 623, SEQ ID NO 627, SEQ ID NO 642, SEQ ID NO 644, SEQ ID NO 645, SEQ ID NO 647, SEQ ID NO 649, SEQ ID NO 652, SEQ ID NO 653, SEQ ID NO 655, SEQ ID NO 656, SEQ ID NO 657, SEQ ID NO 661, SEQ ID NO 664, SEQ ID NO 665, SEQ ID NO 671, SEQ ID NO 672, SEQ ID NO 673, SEQ ID NO 674, SEQ ID NO 675, SEQ ID NO 677, SEQ ID NO 681, SEQ ID NO 684, SEQ ID NO 688, SEQ ID NO 691, SEQ ID NO 692, SEQ ID NO 695, SEQ ID NO 697, SEQ ID NO 700, SEQ ID NO 704, SEQ ID NO 705, SEQ ID NO 706, SEQ ID NO 710, SEQ ID NO 712, SEQ ID NO 714, SEQ ID NO 717, SEQ ID NO 718, SEQ ID NO 719, SEQ ID NO 720, SEQ ID NO 722, SEQ ID NO 724, SEQ ID NO 725, SEQ ID NO 728, SEQ ID NO 729, SEQ ID NO 734, SEQ ID NO 736, SEQ ID NO 737 and SEQ ID NO 738 or fragment thereof, or variants of said nucleic acid molecules,
or a set comprising at least two of said nucleic acid molecules, or fragment thereof, or variants.

These nucleic acid molecules are novel, are expressed in testis or placenta, and are also abnormally expressed in somatic or ovarian cancers.

In another preferred embodiment, the invention also relates to one at least of the nucleic acid molecules belonging to the group consisting in SEQ ID NO 17, SEQ ID NO 37, SEQ ID NO 41, SEQ ID NO 43, SEQ ID NO 59, SEQ ID NO 67, SEQ ID NO 69, SEQ ID NO 73, SEQ ID NO 85, SEQ ID NO 89, SEQ ID NO 91, SEQ ID NO 95, SEQ ID NO 97, SEQ ID NO 99, SEQ ID NO 101, SEQ ID NO 115, SEQ ID NO 155, SEQ ID NO 157, SEQ ID NO 161, SEQ ID NO 165, SEQ ID NO 167, SEQ ID NO 169, SEQ ID NO 189, SEQ ID NO 199, SEQ ID NO 203, SEQ ID NO 217, SEQ ID NO 223, SEQ ID NO 231, SEQ ID NO 233, SEQ ID NO 237, SEQ ID NO 239, SEQ ID NO 293, SEQ ID NO 297, SEQ ID NO 303, SEQ ID NO 319, SEQ ID NO 321, SEQ ID NO 323, SEQ ID NO 325, SEQ ID NO 327, SEQ ID NO 337, SEQ ID NO 347, SEQ ID NO 351, SEQ ID NO 365, SEQ ID NO 369, SEQ ID NO 371, SEQ ID NO 375, SEQ ID NO 381, SEQ ID NO 387, SEQ ID NO 389, SEQ ID NO 401, SEQ ID NO 403, SEQ ID NO 407, SEQ ID NO 425, SEQ ID NO 435, SEQ ID NO 437, SEQ ID NO 445, SEQ ID NO 461, SEQ ID NO 497, SEQ ID NO 517, SEQ ID NO 551, SEQ ID NO 623, SEQ ID NO 627, SEQ ID NO 642, SEQ ID NO 644, SEQ ID NO 645, SEQ ID NO 647, SEQ ID NO 649, SEQ ID NO 652, SEQ ID NO 653, SEQ ID NO 656, SEQ ID NO 657, SEQ ID NO 661, SEQ ID NO 664, SEQ ID NO 665, SEQ ID NO 672, SEQ ID NO 673, SEQ ID NO 674, SEQ ID NO 675, SEQ ID NO 677, SEQ ID NO 681, SEQ ID NO 688, SEQ ID NO 691, SEQ ID NO 692, SEQ ID NO 695, SEQ ID NO 697, SEQ ID NO 700, SEQ ID NO 704, SEQ ID NO 705, SEQ ID NO 706, SEQ ID NO 710, SEQ ID NO 712, SEQ ID NO 714, SEQ ID NO 718, SEQ ID NO 720, SEQ ID NO 722, SEQ ID NO 724, SEQ ID NO 725, SEQ ID NO 728, SEQ ID NO 729, SEQ ID NO 736, SEQ ID NO 737 and SEQ ID NO 738 or fragment thereof, or variants of said nucleic acid molecules,
or a set comprising at least two of said nucleic acid molecules, or fragment thereof, or variants. These nucleic acid molecules are novel, are expressed in testis, and are also abnormally expressed in somatic or ovarian cancers.

In another embodiment, the invention relates to one at least of the a nucleic acid molecule belonging to the group consisting in SEQ ID NO 79, SEQ ID NO 243, SEQ ID NO 463, SEQ ID NO 655, SEQ ID NO 671, SEQ ID NO 684, SEQ ID NO 717, SEQ ID NO 719 and SEQ ID NO 734 or fragment thereof, or variants of said nucleic acid molecules,
or a set comprising at least two of said nucleic acid molecules, or fragment thereof, or variants. These nucleic acid molecules are novel, are expressed in placenta, and are abnormally expressed in somatic or ovarian cancers.

In another embodiment, the invention relates to a complementary sequence of any nucleic acid molecule described above.

In another particular embodiment, the invention also relates to protein or fragment thereof encoded by a nucleic acid molecule described above.
The protein, according to the invention, comprises or is constituted by the following sequences: SEQ ID NO 17 coded by SEQ ID NO 18, SEQ ID NO 37 coded by SEQ ID NO 38, SEQ ID NO 39 coded by SEQ ID NO 40, SEQ ID NO 41 coded by SEQ ID NO 42, SEQ ID NO 43 coded by SEQ ID NO 44, SEQ ID NO 45 coded by SEQ ID NO 46, SEQ ID NO 47 coded by SEQ ID NO 48, SEQ ID NO 49 coded by SEQ ID NO 50, SEQ ID NO 51 coded by SEQ ID NO 52, SEQ ID NO 55 coded by SEQ ID NO 56, SEQ ID NO 59 coded by SEQ ID NO 60, SEQ ID NO 61 coded by SEQ ID NO 62, SEQ ID NO 63 coded by SEQ ID NO 64, SEQ ID NO 65 coded by SEQ ID NO 66, SEQ ID NO 67 coded by SEQ ID NO 68, SEQ ID NO 69 coded by SEQ ID NO 70, SEQ ID NO 71 coded by SEQ ID NO 72, SEQ ID NO 73 coded by SEQ ID NO 74, SEQ ID NO 79 coded by SEQ ID NO 80, SEQ ID NO 81 coded by SEQ ID NO 82, SEQ ID NO 83 coded by SEQ ID NO 84, SEQ ID NO 85 coded by SEQ ID NO 86, SEQ ID NO 89 coded by SEQ ID NO 90, SEQ ID NO 91 coded by SEQ ID NO 92, SEQ ID NO 93 coded by SEQ ID NO 94, SEQ ID NO 95 coded by SEQ ID NO 96, SEQ ID NO 97 coded by SEQ ID NO 98, SEQ ID NO 99 coded by SEQ ID NO 100, SEQ ID NO 101 coded by SEQ ID NO 102, SEQ ID NO 103 coded by SEQ ID NO 104, SEQ ID NO 113 coded by SEQ ID NO 114, SEQ ID NO 115 coded by SEQ ID NO 116, SEQ ID NO 117 coded by SEQ ID NO 118, SEQ ID NO 121 coded by SEQ ID NO 122, SEQ ID NO 155 coded by SEQ ID NO 156, SEQ ID NO 157 coded by SEQ ID NO 158, SEQ ID NO 161 coded by SEQ ID NO 162, SEQ ID NO 165 coded by SEQ ID NO 166, SEQ ID NO 167 coded by SEQ ID NO 168, SEQ ID NO 169 coded by SEQ ID NO 170, SEQ ID NO 171 coded by SEQ ID NO 172, SEQ ID NO 189 coded by SEQ ID NO 190, SEQ ID NO 191 coded by SEQ ID NO 192, SEQ ID NO 195 coded by SEQ ID NO 196, SEQ ID NO 197 coded by SEQ ID NO 198, SEQ ID NO 199 coded by SEQ ID NO 200, SEQ ID NO 203 coded by SEQ ID NO 204, SEQ ID NO 205 coded by SEQ ID NO 206, SEQ ID NO 207 coded by SEQ ID NO 208, SEQ ID NO 213 coded by SEQ ID NO 214, SEQ ID NO 215 coded by SEQ ID NO 216, SEQ ID NO 217 coded by SEQ ID NO 218, SEQ ID NO 219 coded by SEQ ID NO 220, SEQ ID NO 223 coded by SEQ ID NO 224, SEQ ID NO 225 coded by SEQ ID NO 226, SEQ ID NO 229 coded by SEQ ID NO 230, SEQ ID NO 231 coded by SEQ ID NO 232, SEQ ID NO 233 coded by SEQ ID NO 234, SEQ ID NO 235 coded by SEQ ID NO 236, SEQ ID NO 237 coded by SEQ ID NO 238, SEQ ID NO 239 coded by SEQ ID NO 240, SEQ ID NO 241 coded by SEQ ID NO 242, SEQ ID NO 243 coded by SEQ ID NO 244, SEQ ID NO 247 coded by SEQ ID NO 248, SEQ ID NO 263 coded by SEQ ID NO 264, SEQ ID NO 281 coded by SEQ ID NO 282, SEQ ID NO 293 coded by SEQ ID NO 294, SEQ ID NO 295 coded by SEQ ID NO 296, SEQ ID NO 297 coded by SEQ ID NO 298, SEQ ID NO 303 coded by SEQ ID NO 304, SEQ ID NO 317 coded by SEQ ID NO 318, SEQ ID NO 319 coded by SEQ ID NO 320, SEQ ID NO 321 coded by SEQ ID NO 322, SEQ ID NO 323 coded by SEQ ID NO 324, SEQ ID NO 325 coded by SEQ ID NO 326, SEQ ID NO 327 coded by SEQ ID NO 328, SEQ ID NO 329 coded by SEQ ID NO 330, SEQ ID NO 331 coded by SEQ ID NO 332, SEQ ID NO 333 coded by SEQ ID NO 334, SEQ ID NO 335 coded by SEQ ID NO 336, SEQ ID NO 337 coded by SEQ ID NO 338, SEQ ID NO 339 coded by SEQ ID NO 340, SEQ ID NO 341 coded by SEQ ID NO 342, SEQ ID NO 343 coded by SEQ ID NO 344, SEQ ID NO 345 coded by SEQ ID NO 346, SEQ ID NO 347 coded by SEQ ID NO 348, SEQ ID NO 351 coded by SEQ ID NO 352, SEQ ID NO 353 coded by SEQ ID NO 354, SEQ ID NO 355 coded by SEQ ID NO 356, SEQ ID NO 357 coded by SEQ ID NO 358, SEQ ID NO 359 coded by SEQ ID NO 360, SEQ ID NO 361 coded by SEQ ID NO 362, SEQ ID NO 363 coded by SEQ ID NO 364, SEQ ID NO 365 coded by SEQ ID NO 366, SEQ ID NO 367 coded by SEQ ID NO 368, SEQ ID NO 369 coded by SEQ ID NO 370, SEQ ID NO 371 coded by SEQ ID NO 372, SEQ ID NO 373 coded by SEQ ID NO 374, SEQ ID NO 375 coded by SEQ ID NO 376, SEQ ID NO 377 coded by SEQ ID NO 378, SEQ ID NO 379 coded by SEQ ID NO 380, SEQ ID NO 381 coded by SEQ ID NO 382, SEQ ID NO 387 coded by SEQ ID NO 388, SEQ ID NO 389 coded by SEQ ID NO 390, SEQ ID NO 395 coded by SEQ ID NO 396, SEQ ID NO 397 coded by SEQ ID NO 398, SEQ ID NO 399 coded by SEQ ID NO 400, SEQ ID NO 401 coded by SEQ ID NO 402, SEQ ID NO 403 coded by SEQ ID NO 404, SEQ ID NO 405 coded by SEQ ID NO 406, SEQ ID NO 407 coded by SEQ ID NO 408, SEQ ID NO 409 coded by SEQ ID NO 410, SEQ ID NO 413 coded by SEQ ID NO 414, SEQ ID NO 423 coded by SEQ ID NO 424, SEQ ID NO 425 coded by SEQ ID NO 426, SEQ ID NO 435 coded by SEQ ID NO 436, SEQ ID NO 437 coded by SEQ ID NO 438, SEQ ID NO 441 coded by SEQ ID NO 442, SEQ ID NO 445 coded by SEQ ID NO 446, SEQ ID NO 461 coded by SEQ ID NO 462, SEQ ID NO 463 coded by SEQ ID NO 464, SEQ ID NO 497 coded by SEQ ID NO 498, SEQ ID NO 517 coded by SEQ ID NO S 18, SEQ ID NO 527 coded by SEQ ID NO 528, SEQ ID NO 551 coded by SEQ ID NO 552, SEQ ID NO 567 coded by SEQ ID NO 568, SEQ ID NO 579 coded by SEQ ID NO 580, SEQ ID NO 595 coded by SEQ ID NO 596, SEQ ID NO 599 coded by SEQ ID NO 600, SEQ ID NO 601 coded by SEQ ID NO 602, SEQ ID NO 609 coded by SEQ ID NO 610, SEQ ID NO 623 coded by SEQ ID NO 624, SEQ ID NO 625 coded by SEQ ID NO 626 and SEQ ID NO 627 coded by SEQ ID NO 628.

In another embodiment, the invention relates to an antibody, characterized in that is specifically directed against one of the proteins previously described.

Said antibody described above can be a monoclonal antibody or a polyclonal antibody.

In another particular embodiment, the invention relates to an anti-idiotypic antibody directed against one of the antibodies describes above.

Also, the invention relates, in a preferred embodiment, to the Fab fragment of any anti-idiotypic antibodies above-mentioned.

The invention describes a method for the *in vitro* and/or *ex vivo* cancer diagnosis in a subject, by determining the presence or the variation of amount of at least one nucleic acid molecule comprising or constituted by a nucleotide acid sequence consisting in SEQ ID NO 2q-1, q varying from 1 to 320 and SEQ ID NO 641 to SEQ ID NO 754, or a fragment thereof, among nucleic acids from a biological sample from the subject, said presence or variation of amount of said nucleic acid molecule being assessed with respect to the absence or the given amount of said nucleic acid molecule from a sample isolated from an healthy subject, comprising:
- contacting nucleic acids from the biological sample with an agent, said agent being at least one nucleic acid molecule, or a complementary nucleic acid molecule of at least one nucleic acid molecule, comprising or constituted by the group consisting in SEQ ID NO 2q-1, q varying from 1 to 320 and SEQ ID NO 641 to SEQ ID NO 754 or a fragment thereof, and the said agent being able to selectively hybridize with at least one nucleic acid molecule comprising or constituted by the group consisting in SEQ ID NO 2q-1, q varying from 1 to 320 and SEQ ID NO 641 to SEQ ID NO 754 liable to be present among nucleic acids from the biological sample, to form a nucleic acid complex,
- determining the presence or the variation of amount of said nucleic acid complex indicating the fact that the subject is afflicted by cancer.

According to the invention, the "determination of the presence" of at least one nucleic acid molecule indicates that if a nucleic acid molecule can be detected in a biological sample, said nucleic acid molecule is considered as present in the biological sample. On the contrary, if said nucleic acid molecule can not be detected by the method of the invention, the nucleic acid molecule is considered as absent from the biological sample.
According to the invention, the "determination of variation of amount" of at least one nucleic acid molecule means that the quantity of said nucleic acid molecule is measured. The amount of nucleic acid molecule is measured using classical protocol of quantification, wherein the amount of nucleic acid molecule is compared with at least two control samples. These control samples are represented by at least a negative sample and a positive control sample. The value associated to the measure of the quantity of nucleic acid molecule is null in the control negative sample, and value associated to the measure of the quantity of nucleic acid molecule is positive in the control positive sample.
The negative sample corresponds to a biological sample of a healthy individual, or patient, wherein said nucleic acid molecule is either absent or present at a known level, said known level being defined as the standard level.
So, if the nucleic acid molecule is absent of the biologic sample, the value of the quantification is null. On the other hand, if the nucleic acid molecule is present, the value of the quantification is superior to zero.
The presence or amount of nucleic acid molecule may be determined by any routine protocols commonly used in the art. In particular, the nucleic acid molecule is detected by commonly used techniques based on the nucleic acid hybridization, such as Southern blot and Northern blot.
The extraction of the nucleic acid molecules of the samples is managed by a routine protocol used in the art. Advantageously, nucleic acid molecules extracted from the biological sample are RNA.

According to the invention, said agent comprising and/or being constituted by at least one polynucleotide molecule preferably corresponding to a fragment of said nucleic acid molecule, said polynucleotide molecule being such that it is able to specifically hybridize with said nucleic acid molecule, according to the base complementarity. Preferably in the invention, said polynucleotide molecule, also called hereafter nucleic acid, is a DNA molecule.

Then, the method of the invention consists in contacting nucleic acid molecules extracted from the biological sample of a subject, with an agent. Contact between nucleic acid molecule, when present, and agent allows to form a nucleic acid complex.

Preferably, before contacting the agent with the nucleic acid molecules, said nucleic acid molecules being labeled with any known labeling molecules (radioisotopes, enzymes, fluorescent molecules...). The hybridization is made according a standard procedure, by modulating if necessary saline concentration and temperature. The protocol used for hybridization is well known by the skilled man in the art.
Alternatively, said nucleic acid complex can be detected using known labeling molecules (e.g. fluorescent molecules) that specifically detect the formation of a double strand nucleic acid molecule, as the result of the hybridization.
The presence or amount of the formed nucleic acid complex is detected, by the detection of hybridized nucleic acid molecules with a specific detection method fitting to the used labeling molecule.
The presence or amount of nucleic acid molecule, compared with at least the absence or the amount of said nucleic acid molecule, allows defining if the individual from whom nucleic acid molecules derive from is afflicted by cancer.

In an advantageous embodiment, the invention relates to a method described above, wherein the above-defined agent is preferably immobilized on a micro-array, said micro-array comprising at least one nucleic acid comprising or consisting by a nucleic acid sequence of the group consisting SEQ ID NO 755 to SEQ ID NO 1088.
The above-mentioned SEQ ID NO 755 to SEQ ID NO 1088 correspond to polynucleotide molecule, or nucleic acid, of the group consisting in : SEQ ID NO 755, SEQ ID NO 756, SEQ ID NO 757, SEQ ID NO 758, SEQ ID NO 759, SEQ ID NO 760, SEQ ID NO 761, SEQ ID NO 762, SEQ ID NO 763, SEQ ID NO 764, SEQ ID NO 765, SEQ ID NO 766, SEQ ID NO 767_{,} SEQ ID NO 768_{,} SEQ ID NO 769, SEQ ID NO 770, SEQ ID NO 771, SEQ ID NO 772, SEQ ID NO 773, SEQ ID NO 774, SEQ ID NO 775, SEQ ID NO 776, SEQ ID NO 777, SEQ ID NO 778, SEQ ID NO 779, SEQ ID NO 780, SEQ ID NO 781, SEQ ID NO 782, SEQ ID NO 783, SEQ ID NO 784, SEQ ID NO 785, SEQ ID NO 786, SEQ ID NO 787, SEQ ID NO 788, SEQ ID NO 789, SEQ ID NO 790, SEQ ID NO 791, SEQ ID NO 792, SEQ ID NO 793, SEQ ID NO 794, SEQ ID NO 795, SEQ ID NO 796, SEQ ID NO 797, SEQ ID NO 798, SEQ ID NO 799, SEQ ID NO 800, SEQ ID NO 801, SEQ ID NO 802, SEQ ID NO 803, SEQ ID NO 804, SEQ ID NO 805, SEQ ID NO 806, SEQ ID NO 807, SEQ ID NO 808, SEQ ID NO 809, SEQ ID NO 810, SEQ ID NO 811, SEQ ID NO 812, SEQ ID NO 813, SEQ ID NO 814, SEQ ID NO 815, SEQ ID NO 816, SEQ ID NO 817, SEQ ID NO 818, SEQ ID NO 819, SEQ ID NO 820, SEQ ID NO 821, SEQ ID NO 822, SEQ ID NO 823, SEQ ID NO 824, SEQ ID NO 825, SEQ ID NO 826, SEQ ID NO 827, SEQ ID NO 828, SEQ ID NO 829, SEQ ID NO 830, SEQ ID NO 831, SEQ ID NO 832, SEQ ID NO 833, SEQ ID NO 834, SEQ ID NO 835, SEQ ID NO 836, SEQ ID NO 837, SEQ ID NO 838, SEQ ID NO 839, SEQ ID NO 840, SEQ ID NO 841, SEQ ID NO 842, SEQ ID NO 843, SEQ ID NO 844, SEQ ID NO 845, SEQ ID NO 846, SEQ ID NO 847, SEQ ID NO 848, SEQ ID NO 849, SEQ ID NO 850, SEQ ID NO 851, SEQ ID NO 852, SEQ ID NO 853, SEQ ID NO 854, SEQ ID NO 855, SEQ ID NO 856, SEQ ID NO 857, SEQ ID NO 858, SEQ ID NO 859, SEQ ID NO 860, SEQ ID NO 861, SEQ ID NO 862, SEQ ID NO 863, SEQ ID NO 864, SEQ ID NO 865, SEQ ID NO 866, SEQ ID NO 867, SEQ ID NO 868, SEQ ID NO 869, SEQ ID NO 870, SEQ ID NO 871, SEQ ID NO 872, SEQ ID NO 873, SEQ ID NO 874, SEQ ID NO 875, SEQ ID NO 876, SEQ ID NO 877, SEQ ID NO 878, SEQ ID NO 879, SEQ ID NO 880, SEQ ID NO 881, SEQ ID NO 882, SEQ ID NO 883, SEQ ID NO 884, SEQ ID NO 885, SEQ ID NO 886, SEQ ID NO 887, SEQ ID NO 888, SEQ ID NO 889, SEQ ID NO 890, SEQ ID NO 891, SEQ ID NO 892, SEQ ID NO 893, SEQ ID NO 894, SEQ ID NO 895, SEQ ID NO 896, SEQ ID NO 897, SEQ ID NO 898, SEQ ID NO 899, SEQ ID NO 900, SEQ ID NO 901, SEQ ID NO 902, SEQ ID NO 903, SEQ ID NO 904, SEQ ID NO 905, SEQ ID NO 906, SEQ ID NO 907, SEQ ID NO 908, SEQ ID NO 909, SEQ ID NO 910, SEQ ID NO 911, SEQ ID NO 912, SEQ ID NO 913, SEQ ID NO 914, SEQ ID NO 915, SEQ ID NO 916, SEQ ID NO 917, SEQ ID NO 918, SEQ ID NO 919, SEQ ID NO 920, SEQ ID NO 921, SEQ ID NO 922, SEQ ID NO 923, SEQ ID NO 924, SEQ ID NO 925, SEQ ID NO 926, SEQ ID NO 927, SEQ ID NO 928, SEQ ID NO 929, SEQ ID NO 930, SEQ ID NO 931, SEQ ID NO 932, SEQ ID NO 933, SEQ ID NO 934, SEQ ID NO 935, SEQ ID NO 936, SEQ ID NO 937, SEQ ID NO 938, SEQ ID NO 939, SEQ ID NO 940, SEQ ID NO 941, SEQ ID NO 942, SEQ ID NO 943, SEQ ID NO 944, SEQ ID NO 945, SEQ ID NO 946, SEQ ID NO 947, SEQ ID NO 948, SEQ ID NO 949, SEQ ID NO 950, SEQ ID NO 951, SEQ ID NO 952, SEQ ID NO 953, SEQ ID NO 954, SEQ ID NO 955, SEQ ID NO 956, SEQ ID NO 957, SEQ ID NO 958, SEQ ID NO 959, SEQ ID NO 960, SEQ ID NO 961, SEQ ID NO 962, SEQ ID NO 963, SEQ ID NO 964, SEQ ID NO 965, SEQ ID NO 966, SEQ ID NO 967, SEQ ID NO 968, SEQ ID NO 969, SEQ ID NO 970, SEQ ID NO 971, SEQ ID NO 972, SEQ ID NO 973, SEQ ID NO 974, SEQ ID NO 975, SEQ ID NO 976, SEQ ID NO 977, SEQ ID NO 978, SEQ ID NO 979, SEQ ID NO 980, SEQ ID NO 981, SEQ ID NO 982, SEQ ID NO 983, SEQ ID NO 984, SEQ ID NO 985, SEQ ID NO 986, SEQ ID NO 987, SEQ ID NO 988, SEQ ID NO 989, SEQ ID NO 990, SEQ ID NO 991, SEQ ID NO 992, SEQ ID NO 993, SEQ ID NO 994, SEQ ID NO 995, SEQ ID NO 996, SEQ ID NO 997, SEQ ID NO 998, SEQ ID NO 999, SEQ ID NO 1000, SEQ ID NO 1001, SEQ ID NO 1002, SEQ ID NO 1003, SEQ ID NO 1004, SEQ ID NO 1005, SEQ ID NO 1006, SEQ ID NO 1007, SEQ ID NO 1008, SEQ ID NO 1009, SEQ ID NO 1010, SEQ ID NO 1011, SEQ ID NO 1012, SEQ ID NO 1013, SEQ ID NO 1014, SEQ ID NO 1015, SEQ ID NO 1016, SEQ ID NO 1017, SEQ ID NO 1018, SEQ ID NO 1019, SEQ ID NO 1020, SEQ ID NO 1021, SEQ ID NO 1022, SEQ ID NO 1023, SEQ ID NO 1024, SEQ ID NO 1025, SEQ ID NO 1026, SEQ ID NO 1027, SEQ ID NO 1028, SEQ ID NO 1029, SEQ ID NO 1030, SEQ ID NO 1031, SEQ ID NO 1032, SEQ ID NO 1033, SEQ ID NO 1034, SEQ ID NO 1035, SEQ ID NO 1036, SEQ ID NO 1037, SEQ ID NO 1038, SEQ ID NO 1039, SEQ ID NO 1040, SEQ ID NO 1041, SEQ ID NO 1042, SEQ ID NO 1043, SEQ ID NO 1044, SEQ ID NO 1045, SEQ ID NO 1046, SEQ ID NO 1047, SEQ ID NO 1048, SEQ ID NO 1049, SEQ ID NO 1050, SEQ ID NO 1051, SEQ ID NO 1052, SEQ ID NO 1053, SEQ ID NO 1054, SEQ ID NO 1055, SEQ ID NO 1056, SEQ ID NO 1057, SEQ ID NO 1058, SEQ ID NO 1059, SEQ ID NO 1060, SEQ ID NO 1061, SEQ ID NO 1062, SEQ ID NO 1063, SEQ ID NO 1064, SEQ ID NO 1065, SEQ ID NO 1066, SEQ ID NO 1067, SEQ ID NO 1068, SEQ ID NO 1069, SEQ ID NO 1070, SEQ ID NO 1071, SEQ ID NO 1072, SEQ ID NO 1073, SEQ ID NO 1074, SEQ ID NO 1075, SEQ ID NO 1076, SEQ ID NO 1077, SEQ ID NO 1078, SEQ ID NO 1079, SEQ ID NO 1080, SEQ ID NO 1081, SEQ ID NO 1082, SEQ ID NO 1083, SEQ ID NO 1084, SEQ ID NO 1085, SEQ ID NO 1086, SEQ ID NO 1087 and SEQ ID NO 1088.

According to the invention, the above-mentioned polynucleotide molecule, or nucleic acid are also called polynucleotidic probes.

According to the invention, polynucleotidic probes comprising or constituted by SEQ ID NO 755 to SEQ ID NO 1088 are able to specifically recognize and hybridize with one nucleic acid sequence of the group consisting in SEQ ID NO 2q-1, q varying from 1 to 320, and SEQ ID NO 641 to SEQ ID NO 754. The hybridization is performed according to standard procedure described above.
More particularly, nucleic acid comprising or constituted by SEQ ID NO 755 to SEQ ID NO 1088 are able to specifically recognize the complementary sequence of one nucleic acid sequence of the following group : SEQ ID NO 1, SEQ ID NO 3, SEQ ID NO 5, SEQ ID NO 7, SEQ ID NO 9, SEQ ID NO 11, SEQ ID NO 13, SEQ ID NO 17, SEQ ID NO 19, SEQ ID NO 21, SEQ ID NO 23, SEQ ID NO 25, SEQ ID NO 27, SEQ ID NO 29, SEQ ID NO 31, SEQ ID NO 33, SEQ ID NO 35, SEQ ID NO 41, SEQ ID NO 43, SEQ ID NO 45, SEQ ID NO 47, SEQ ID NO 49, SEQ ID NO 51, SEQ ID NO 53, SEQ ID NO 55, SEQ ID NO 59, SEQ ID NO 61, SEQ ID NO 63, SEQ ID NO 65, SEQ ID NO 67, SEQ ID NO 71, SEQ ID NO 73, SEQ ID NO 75, SEQ ID NO 77, SEQ ID NO 79, SEQ ID NO 81, SEQ ID NO 83, SEQ ID NO 85, SEQ ID NO 87, SEQ ID NO 89, SEQ ID NO 91, SEQ ID NO 93, SEQ ID NO 97, SEQ ID NO 105, SEQ ID NO 107, SEQ ID NO 109, SEQ ID NO 111, SEQ ID NO 113, SEQ ID NO 115, SEQ ID NO 119, SEQ ID NO 123, SEQ ID NO 125, SEQ ID NO 127, SEQ ID NO 129, SEQ ID NO 131, SEQ ID NO 133, SEQ ID NO 135, SEQ ID NO 137, SEQ ID NO 139, SEQ ID NO 141, SEQ ID NO 145, SEQ ID NO 147, SEQ ID NO 149, SEQ ID NO 151, SEQ ID NO 153, SEQ ID NO 155, SEQ ID NO 157, SEQ ID NO 163, SEQ ID NO 167, SEQ ID NO 169, SEQ ID NO 173, SEQ ID NO 175, SEQ ID NO 177, SEQ ID NO 179, SEQ ID NO 181, SEQ ID NO 183, SEQ ID NO 185, SEQ ID NO 187, SEQ ID NO 189, SEQ ID NO 191, SEQ ID NO 193, SEQ ID NO 195, SEQ ID NO 197, SEQ ID NO 199, SEQ ID NO 201, SEQ ID NO 203, SEQ ID NO 205, SEQ ID NO 209, SEQ ID NO 213, SEQ ID NO 215, SEQ ID NO 217, SEQ ID NO 219, SEQ ID NO 221, SEQ ID NO 223, SEQ ID NO 225, SEQ ID NO 227, SEQ ID NO 229, SEQ ID NO 231, SEQ ID NO 233, SEQ ID NO 237, SEQ ID NO 239, SEQ ID NO 241, SEQ ID NO 245, SEQ ID NO 249, SEQ ID NO 251, SEQ ID NO 253, SEQ ID NO 255, SEQ ID NO 257, SEQ ID NO 265, SEQ ID NO 267, SEQ ID NO 269, SEQ ID NO 271, SEQ ID NO 273, SEQ ID NO 275, SEQ ID NO 277, SEQ ID NO 279, SEQ ID NO 283, SEQ ID NO 285, SEQ ID NO 287, SEQ ID NO 289, SEQ ID NO 291, SEQ ID NO 293, SEQ ID NO 295, SEQ ID NO 297, SEQ ID NO 303, SEQ ID NO 305, SEQ ID NO 307, SEQ ID NO 309, SEQ ID NO 311, SEQ ID NO 313, SEQ ID NO 315, SEQ ID NO 319, SEQ ID NO 321, SEQ ID NO 323, SEQ ID NO 325, SEQ ID NO 331, SEQ ID NO 335, SEQ ID NO 341, SEQ ID NO 345, SEQ ID NO 347, SEQ ID NO 353, SEQ ID NO 355, SEQ ID NO 371, SEQ ID NO 373, SEQ ID NO 375, SEQ ID NO 381, SEQ ID NO 383, SEQ ID NO 385, SEQ ID NO 389, SEQ ID NO 391, SEQ ID NO 393, SEQ ID NO 395, SEQ ID NO 397, SEQ ID NO 399, SEQ ID NO 401, SEQ ID NO 403, SEQ ID NO 405, SEQ ID NO 407, SEQ ID NO 409, SEQ ID NO 413, SEQ ID NO 417, SEQ ID NO 419, SEQ ID NO 421, SEQ ID NO 423, SEQ ID NO 427, SEQ ID NO 429, SEQ ID NO 431, SEQ ID NO 433, SEQ ID NO 435, SEQ ID NO 437, SEQ ID NO 439, SEQ ID NO 441, SEQ ID NO 443, SEQ ID NO 447, SEQ ID NO 449, SEQ ID NO 451, SEQ ID NO 453, SEQ ID NO 455, SEQ ID NO 457, SEQ ID NO 459, SEQ ID NO 461, SEQ ID NO 465, SEQ ID NO 467, SEQ ID NO 469, SEQ ID NO 471, SEQ ID NO 473, SEQ ID NO 475, SEQ ID NO 477, SEQ ID NO 481, SEQ ID NO 483, SEQ ID NO 485, SEQ ID NO 487, SEQ ID NO 491, SEQ ID NO 493, SEQ ID NO 495, SEQ ID NO 501, SEQ ID NO 503, SEQ ID NO 505, SEQ ID NO 507, SEQ ID NO 509, SEQ ID NO 511, SEQ ID NO 513, SEQ ID NO 515, SEQ ID NO 517, SEQ ID NO 519, SEQ ID NO 521, SEQ ID NO 523, SEQ ID NO 525, SEQ ID NO 527, SEQ ID NO 529, SEQ ID NO 531, SEQ ID NO 533, SEQ ID NO 535, SEQ ID NO 537, SEQ ID NO 539, SEQ ID NO 541, SEQ ID NO 543, SEQ ID NO 545, SEQ ID NO 547, SEQ ID NO 549, SEQ ID NO 551, SEQ ID NO 555, SEQ ID NO 559, SEQ ID NO 561, SEQ ID NO 563, SEQ ID NO 565, SEQ ID NO 567, SEQ ID NO 569, SEQ ID NO 571, SEQ ID NO 573, SEQ ID NO 575, SEQ ID NO 577, SEQ ID NO 579, SEQ ID NO 581, SEQ ID NO 583, SEQ ID NO 585, SEQ ID NO 587, SEQ ID NO 589, SEQ ID NO 591, SEQ ID NO 593, SEQ ID NO 595, SEQ ID NO 597, SEQ ID NO 599, SEQ ID NO 601, SEQ ID NO 603, SEQ ID NO 605, SEQ ID NO 607, SEQ ID NO 609, SEQ ID NO 611, SEQ ID NO 613, SEQ ID NO 617, SEQ ID NO 619, SEQ ID NO 623, SEQ ID NO 625, SEQ ID NO 629, SEQ ID NO 631, SEQ ID NO 633, SEQ ID NO 635, SEQ ID NO 637, SEQ ID NO 642, SEQ ID NO 644, SEQ ID NO 653, SEQ ID NO 664, SEQ ID NO 669, SEQ ID NO 673, SEQ ID NO 681, SEQ ID NO 683, SEQ ID NO 687, SEQ ID NO 689, SEQ ID NO 690, SEQ ID NO 691, SEQ ID NO 692, SEQ ID NO 693, SEQ ID NO 694, SEQ ID NO 695, SEQ ID NO 698, SEQ ID NO 701, SEQ ID NO 702, SEQ ID NO 703, SEQ ID NO 707, SEQ ID NO 710, SEQ ID NO 712, SEQ ID NO 714, SEQ ID NO 716, SEQ ID NO 717, SEQ ID NO 720, SEQ ID NO 721, SEQ ID NO 722, SEQ ID NO 723, SEQ ID NO 737 and SEQ ID NO 742.

According to the invention, said micro-array can also contain other nucleic acid than previously described nucleic acid.
The above-mentioned polynucleotidic probes correspond to a part of the above mentioned nucleic acid molecules. Said probes are able to form a nucleic acid complex with said nucleic acid molecules according to the complementary hybridization of nucleic acid well known in the art.

The following table 2 indicates the correspondence between nucleic acid molecules and the corresponding polynucleotide molecules.
Nucleic acid NO indicates the SEQ ID corresponding to nucleic acid sequence; PS or TS indicates if said nucleic acid molecule correspond to a PS or a TS gene; CT or not CT indicates is said nucleic acid molecule is a CT or a non CT gene; Nb of probe indicates the number of polynucleotides recognizing said nucleic acid molecule and NO of probe indicates the SEQ ID of polynucleotide molecule.

**Table 2**

| **Nucleic acid NO** | **PS or TS** | **CT or no CT** | **Nb of probes** | **NO of probes** | **NO of probes** | **NO of probes** | **NO of probes** |
|---|---|---|---|---|---|---|---|
| SEQ ID NO 1 | TS | CT | 1 | SEQ ID NO 755 | | | |
| SEQ ID NO 3 | TS | CT | 1 | SEQ ID NO 756 | | | |
| SEQ ID NO 5 | TS | CT | 1 | SEQ ID NO 757 | | | |
| SEQ ID NO 7 | TS | CT | 1 | SEQ ID NO 758 | | | |
| SEQ ID NO 9 | PS | CT | 2 | SEQ ID NO 759 | SEQ ID NO 760 | | |
| SEQ ID NO 11 | TS | CT | 1 | SEQ ID NO 761 | | | |
| SEQ ID NO 13 | TS | no CT | 1 | SEQ ID NO 762 | | | |
| SEQ ID NO 17 | TS | CT | 1 | SEQ ID NO 763 | | | |
| SEQ ID NO 19 | TS | CT | 1 | SEQ ID NO 764 | | | |
| SEQ ID NO 21 | TS | CT | 1 | SEQ ID NO 765 | | | |
| SEQ ID NO 23 | PS | CT | 2 | SEQ ID NO 766 | SEQ ID NO 767 | | |
| SEQ ID NO 25 | TS | no CT | 1 | SEQ ID NO 768 | | | |
| SEQ ID NO 27 | TS | CT | 1 | SEQ ID NO 769 | | | |
| SEQ ID NO 29 | TS | no CT | 1 | SEQ ID NO 770 | | | |
| SEQ ID NO 31 | TS | CT | 1 | SEQ ID NO 771 | | | |
| SEQ ID NO 33 | TS | no CT | 1 | SEQ ID NO 772 | | | |
| SEQ ID NO 35 | TS | CT | 1 | SEQ ID NO 773 | | | |
| SEQ ID NO 41 | TS | CT | 1 | SEQ ID NO 774 | | | |
| SEQ ID NO 43 | TS | CT | 1 | SEQ ID NO 775 | | | |
| SEQ ID NO 45 | TS | no CT | 1 | SEQ ID NO 776 | | | |
| SEQ ID NO 47 | TS | no CT | 1 | SEQ ID NO 777 | | | |
| SEQ ID NO 49 | TS | no CT | 1 | SEQ ID NO 778 | | | |
| SEQ ID NO 51 | TS | no CT | 1 | SEQ ID NO 779 | | | |
| SEQ ID NO 53 | TS | no CT | 1 | SEQ ID NO 780 | | | |
| SEQ ID NO 55 | TS | no CT | 1 | SEQ ID NO 781 | | | |
| SEQ ID NO 59 | TS | CT | 2 | SEQ ID NO 782 | SEQ ID NO 783 | | |
| SEQ ID NO 61 | TS | no CT | 1 | SEQ ID NO 784 | | | |
| SEQ ID NO 63 | TS | no CT | 1 | SEQ ID NO 785 | | | |
| SEQ ID NO 65 | TS | no CT | 1 | SEQ ID NO 786 | | | |
| SEQ ID NO 67 | TS | CT | 1 | SEQ ID NO 787 | | | |
| SEQ ID NO 71 | TS | no CT | 1 | SEQ ID NO 788 | | | |
| SEQ ID NO 73 | TS | CT | 1 | SEQ ID NO 789 | | | |
| SEQ ID NO 75 | TS | no CT | 1 | SEQ ID NO 790 | | | |
| SEQ ID NO 77 | TS | CT | 1 | SEQ ID NO 791 | | | |
| SEQ ID NO 79 | PS | CT | 1 | SEQ ID NO 792 | | | |
| SEQ ID NO 81 | TS | no CT | 1 | SEQ ID NO 793 | | | |
| SEQ ID NO 83 | TS | no CT | 1 | SEQ ID NO 794 | | | |
| SEQ ID NO 85 | TS | CT | 1 | SEQ ID NO 795 | | | |
| SEQ ID NO 87 | TS | CT | 2 | SEQ ID NO 796 | SEQ ID NO 797 | | |
| SEQ ID NO 89 | TS | CT | 1 | SEQ ID NO 798 | | | |
| SEQ ID NO 91 | TS | CT | 1 | SEQ ID NO 799 | | | |
| SEQ ID NO 93 | TS | no CT | 1 | SEQ ID NO 800 | | | |
| SEQ ID NO 97 | TS | CT | 1 | SEQ ID NO 801 | | | |
| SEQ ID NO 105 | TS | no CT | 2 | SEQ ID NO 802 | SEQ ID NO 803 | | |
| SEQ ID NO 107 | TS | no CT | 1 | SEQ ID NO 804 | | | |
| SEQ ID NO 109 | TS | no CT | 1 | SEQ ID NO 805 | | | |
| SEQ ID NO 111 | PS | CT | 1 | SEQ ID NO 806 | | | |
| SEQ ID NO 113 | TS | no CT | 1 | SEQ ID NO 807 | | | |
| SEQ ID NO 115 | TS | CT | 1 | SEQ ID NO 808 | | | |
| SEQ ID NO 119 | TS | CT | 1 | SEQ ID NO 809 | | | |
| SEQ ID NO 123 | TS | CT | 1 | SEQ ID NO 810 | | | |
| SEQ ID NO 125 | TS | CT | 1 | SEQ ID NO 811 | | | |
| SEQ ID NO 127 | TS | CT | 1 | SEQ ID NO 812 | | | |
| SEQ ID NO 129 | TS | CT | 1 | SEQ ID NO 813 | | | |
| SEQ ID NO 131 | PS | CT | 4 | SEQ ID NO 814 | SEQ ID NO 815 | SEQ ID NO 816 | SEQ ID NO 817 |
| SEQ ID NO 133 | PS | CT | 2 | SEQ ID NO 818 | SEQ ID NO 819 | | |
| SEQ ID NO 135 | TS | CT | 1 | SEQ ID NO 820 | | | |
| SEQ ID NO 137 | TS | no CT | 1 | SEQ ID NO 821 | | | |
| SEQ ID NO 139 | TS | CT | 1 | SEQ ID NO 822 | | | |
| SEQ ID NO 141 | PS | CT | 1 | SEQ ID NO 823 | | | |
| SEQ ID NO 145 | TS | CT | 1 | SEQ ID NO 824 | | | |
| SEQ ID NO 147 | TS | no CT | 1 | SEQ ID NO 825 | | | |
| SEQ ID NO 149 | TS | no CT | 1 | SEQ ID NO 826 | | | |
| SEQ ID NO 151 | TS | CT | 2 | SEQ ID NO 827 | SEQ ID NO 828 | | |
| SEQ ID NO 153 | TS | CT | 1 | SEQ ID NO 829 | | | |
| SEQ ID NO 155 | TS | CT_est | 1 | SEQ ID NO 830 | | | |
| SEQ ID NO 157 | TS | CT | 1 | SEQ ID NO 831 | | | |
| SEQ ID NO 163 | TS | CT | 1 | SEQ ID NO 832 | | | |
| SEQ ID NO 167 | TS | CT | 2 | SEQ ID NO 833 | SEQ ID NO 834 | | |
| SEQ ID NO 169 | TS | CT | 1 | SEQ ID NO 835 | | | |
| SEQ ID NO 173 | TS | no CT | 2 | SEQ ID NO 836 | SEQ ID NO 837 | | |
| SEQ ID NO 175 | TS | no CT | 1 | SEQ ID NO 838 | | | |
| SEQ ID NO 177 | TS | no CT | 1 | SEQ ID NO 839 | | | |
| SEQ ID NO 179 | TS | no CT | 1 | SEQ ID NO 840 | | | |
| SEQ ID NO 181 | PS | CT | 1 | SEQ ID NO 841 | | | |
| SEQ ID NO 183 | TS | CT | 1 | SEQ ID NO 842 | | | |
| SEQ ID NO 185 | TS | CT | 1 | SEQ ID NO 843 | | | |
| SEQ ID NO 187 | TS | no CT | 2 | SEQ ID NO 844 | SEQ ID NO 845 | | |
| SEQ ID NO 189 | TS | CT | 1 | SEQ ID NO 846 | | | |
| SEQ ID NO 191 | TS | no CT | 1 | SEQ ID NO 847 | | | |
| SEQ ID NO 193 | TS | no CT | 1 | SEQ ID NO 848 | | | |
| SEQ ID NO 195 | TS | no CT | 2 | SEQ ID NO 849 | SEQ ID NO 850 | | |
| SEQ ID NO 197 | TS | no CT | 1 | SEQ ID NO 851 | | | |
| SEQ ID NO 199 | TS | CT | 1 | SEQ ID NO 852 | | | |
| SEQ ID NO 201 | TS | CT | 1 | SEQ ID NO 853 | | | |
| SEQ ID NO 203 | TS | CT | 1 | SEQ ID NO 854 | | | |
| SEQ ID NO 205 | PS | no CT | 1 | SEQ ID NO 855 | | | |
| SEQ ID NO 209 | TS | no CT | 1 | SEQ ID NO 856 | | | |
| SEQ ID NO 213 | TS | no CT | 4 | SEQ ID NO 857 | SEQ ID NO 858 | SEQ ID NO 859 | SEQ ID NO 860 |
| SEQ ID NO 215 | TS | no CT | 1 | SEQ ID NO 861 | | | |
| SEQ ID NO 217 | TS | CT | 1 | SEQ ID NO 862 | | | |
| SEQ ID NO 219 | TS | no CT | 1 | SEQ ID NO 863 | | | |
| SEQ ID NO 221 | TS | no CT | 1 | SEQ ID NO 864 | | | |
| SEQ ID NO 223 | TS | CT | 1 | SEQ ID NO 865 | | | |
| SEQ ID NO 225 | TS | no CT | 1 | SEQ ID NO 866 | | | |
| SEQ ID NO 227 | TS | CT | 2 | SEQ ID NO 867 | SEQ ID NO 868 | | |
| SEQ ID NO 229 | TS | no CT | 1 | SEQ ID NO 869 | | | |
| SEQ ID NO 231 | TS | CT | 1 | SEQ ID NO 870 | | | |
| SEQ ID NO 233 | TS | CT | 3 | SEQ ID NO 871 | SEQ ID NO 872 | SEQ ID NO 873 | |
| SEQ ID NO 237 | TS | CT | 1 | SEQ ID NO 874 | | | |
| SEQ ID NO 239 | TS | CT | 1 | SEQ ID NO 875 | | | |
| SEQ ID NO 241 | TS | no CT | 1 | SEQ ID NO 876 | | | |
| SEQ ID NO 245 | TS | no CT | 2 | SEQ ID NO 877 | SEQ ID NO 878 | | |
| SEQ ID NO 249 | TS | CT | 1 | SEQ ID NO 879 | | | |
| SEQ ID NO 251 | TS | CT | 1 | SEQ ID NO 880 | | | |
| SEQ ID NO 253 | TS | CT | 2 | SEQ ID NO 881 | SEQ ID NO 882 | | |
| SEQ ID NO 255 | TS | no CT | 1 | SEQ ID NO 883 | | | |
| SEQ ID NO 257 | TS | CT | 1 | SEQ ID NO 884 | | | |
| SEQ ID NO 265 | TS | CT | 1 | SEQ ID NO 885 | | | |
| SEQ ID NO 267 | TS | CT | 1 | SEQ ID NO 886 | | | |
| SEQ ID NO 269 | TS | CT | 1 | SEQ ID NO 887 | | | |
| SEQ ID NO 271 | TS | CT | 1 | SEQ ID NO 888 | | | |
| SEQ ID NO 273 | TS | CT | 1 | SEQ ID NO 889 | | | |
| SEQ ID NO 275 | PS | CT | 1 | SEQ ID NO 890 | | | |
| SEQ ID NO 277 | TS | CT | 2 | SEQ ID NO 891 | SEQ ID NO 892 | | |
| SEQ ID NO 279 | TS | CT | 1 | SEQ ID NO 893 | | | |
| SEQ ID NO 283 | PS | CT | 1 | SEQ ID NO 894 | | | |
| SEQ ID NO 285 | TS | CT | 1 | SEQ ID NO 895 | | | |
| SEQ ID NO 287 | TS | CT | 1 | SEQ ID NO 896 | | | |
| SEQ ID NO 289 | PS | CT | 1 | SEQ ID NO 897 | | | |
| SEQ ID NO 291 | TS | no CT | 1 | SEQ ID NO 898 | | | |
| SEQ ID NO 293 | TS | CT | 1 | SEQ ID NO 899 | | | |
| SEQ ID NO 295 | TS | no CT | 1 | SEQ ID NO 900 | | | |
| SEQ ID NO 297 | TS | CT | 1 | SEQ ID NO 901 | | | |
| SEQ ID NO 303 | TS | CT | 2 | SEQ ID NO 902 | SEQ ID NO 903 | | |
| SEQ ID NO 305 | TS | CT | 1 | SEQ ID NO 904 | | | |
| SEQ ID NO 307 | PS | CT | 1 | SEQ ID NO 905 | | | |
| SEQ ID NO 309 | TS | CT | 1 | SEQ ID NO 906 | | | |
| SEQ ID NO 311 | TS | CT | 1 | SEQ ID NO 907 | | | |
| SEQ ID NO 313 | TS | CT | 1 | SEQ ID NO 908 | | | |
| SEQ ID NO 315 | PS | CT | 1 | SEQ ID NO 909 | | | |
| SEQ ID NO 319 | TS | CT | 2 | SEQ ID NO 910 | SEQ ID NO 911 | | |
| SEQ ID NO 321 | TS | CT | 1 | SEQ ID NO 912 | | | |
| SEQ ID NO 323 | TS | CT | 1 | SEQ ID NO 913 | | | |
| SEQ ID NO 325 | TS | CT | 1 | SEQ ID NO 914 | | | |
| SEQ ID NO 331 | TS | no CT | 1 | SEQ ID NO 915 | | | |
| SEQ ID NO 335 | TS | no CT | 1 | SEQ ID NO 916 | | | |
| SEQ ID NO 341 | PS | no CT | 2 | SEQ ID NO 917 | SEQ ID NO 918 | | |
| SEQ ID NO 345 | TS | no CT | 1 | SEQ ID NO 919 | | | |
| SEQ ID NO 347 | TS | CT | 1 | SEQ ID NO 920 | | | |
| SEQ ID NO 353 | TS | no CT | 1 | SEQ ID NO 921 | | | |
| SEQ ID NO 355 | TS | no CT | 2 | SEQ ID NO 922 | SEQ ID NO 923 | | |
| SEQ ID NO 371 | TS | CT | 1 | SEQ ID NO 924 | | | |
| SEQ ID NO 373 | TS | no CT | 1 | SEQ ID NO 925 | | | |
| SEQ ID NO 375 | TS | CT | 1 | SEQ ID NO 926 | | | |
| SEQ ID NO 381 | TS | CT_est | 1 | SEQ ID NO 927 | | | |
| SEQ ID NO 383 | TS | no CT | 1 | SEQ ID NO 928 | | | |
| SEQ ID NO 385 | TS | CT | 1 | SEQ ID NO 929 | | | |
| SEQ ID NO 389 | TS | CT | 1 | SEQ ID NO 930 | | | |
| SEQ ID NO 391 | TS | no CT | 1 | SEQ ID NO 931 | | | |
| SEQ ID NO 393 | TS | CT | 1 | SEQ ID NO 932 | | | |
| SEQ ID NO 395 | TS | no CT | 1 | SEQ ID NO 933 | | | |
| SEQ ID NO 397 | TS | no CT | 1 | SEQ ID NO 934 | | | |
| SEQ ID NO 399 | TS | no CT | 1 | SEQ ID NO 935 | | | |
| SEQ ID NO 401 | TS | CT | 1 | SEQ ID NO 936 | | | |
| SEQ ID NO 403 | TS | CT | 1 | SEQ ID NO 937 | | | |
| SEQ ID NO 405 | TS | no CT | 1 | SEQ ID NO 938 | | | |
| SEQ ID NO 407 | TS | CT | 2 | SEQ ID NO 939 | SEQ ID NO 940 | | |
| SEQ ID NO 409 | TS | no CT | 1 | SEQ ID NO 941 | | | |
| SEQ ID NO 413 | TS | no CT | 1 | SEQ ID NO 942 | | | |
| SEQ ID NO 417 | TS | CT | 1 | SEQ ID NO 943 | | | |
| SEQ ID NO 419 | TS | CT | 1 | SEQ ID NO 944 | | | |
| SEQ ID NO 421 | TS | CT | 1 | SEQ ID NO 945 | | | |
| SEQ ID NO 423 | TS | no CT | 1 | SEQ ID NO 946 | | | |
| SEQ ID NO 427 | TS | CT | 2 | SEQ ID NO 947 | SEQ ID NO 948 | | |
| SEQ ID NO 429 | TS | CT | 1 | SEQ ID NO 949 | | | |
| SEQ ID NO 431 | TS | CT | 1 | SEQ ID NO 950 | | | . |
| SEQ ID NO 433 | TS | no CT | 1 | SEQ ID NO 951 | | | |
| SEQ ID NO 435 | TS | CT | 1 | SEQ ID NO 952 | | | |
| SEQ ID NO 437 | TS | CT | 1 | SEQ ID NO 953 | | | |
| SEQ ID NO 439 | TS | CT | 2 | SEQ ID NO 954 | SEQ ID NO 955 | | |
| SEQ ID NO 441 | TS | no CT | 2 | SEQ ID NO 956 | SEQ ID NO 957 | | |
| SEQ ID NO 443 | TS | CT | 2 | SEQ ID NO 958 | SEQ ID NO 959 | | |
| SEQ ID NO 447 | TS | CT | 1 | SEQ ID NO 960 | | | |
| SEQ ID NO 449 | TS | CT | 1 | SEQ ID NO 961 | | | |
| SEQ ID NO 451 1 | PS | CT | 2 | SEQ ID NO 962 | SEQ ID NO 963 | | |
| SEQ ID NO 453 | TS | CT | 1 | SEQ ID NO 964 | | | |
| SEQ ID NO 455 | TS | CT | 1 | SEQ ID NO 965 | | | |
| SEQ ID NO 457 | TS | CT | 1 | SEQ ID NO 966 | | | |
| SEQ ID NO 459 | TS | CT | 1 | SEQ ID NO 967 | | | |
| SEQ ID NO 461 | TS | CT | 1 | SEQ ID NO 968 | | | . |
| SEQ ID NO 465 | TS | no CT | 1 | SEQ ID NO 969 | | | |
| SEQ ID NO 467 | TS | no CT | 1 | SEQ ID NO 970 | | | |
| SEQ ID NO 469 | TS | CT | 2 | SEQ ID NO 971 | SEQ ID NO 972 | | |
| SEQ ID NO 471 | PS | CT | 1 | SEQ ID NO 973 | | | |
| SEQ ID NO 473 | TS | CT | 1 | SEQ ID NO 974 | | | |
| SEQ ID NO 475 | TS | CT | 1 | SEQ ID NO 975 | | | |
| SEQ ID NO 477 | TS | CT | 1 | SEQ ID NO 976 | | | |
| SEQ ID NO 481 | PS | CT | 2 | SEQ ID NO 977 | SEQ ID NO 978 | | |
| SEQ ID NO 483 | PS | CT | 1 | SEQ ID NO 979 | | | |
| SEQ ID NO 485 | PS | CT | 1 | SEQ ID NO 980 | | | |
| SEQ ID NO 487 | PS | CT | 1 | SEQ ID NO 981 | | | |
| SEQ ID NO 491 | PS | CT | 1 | SEQ ID NO 982 | | | |
| SEQ ID NO 493 | PS | CT | 1 | SEQ ID NO 983 | | | |
| SEQ ID NO 495 | PS | CT | 2 | SEQ ID NO 984 | SEQ ID NO 985 | | |
| SEQ ID NO 501 | TS | CT | 1 | SEQ ID NO 986 | | | |
| SEQ ID NO 503 | TS | CT | 1 | SEQ ID NO 987 | | | |
| SEQ ID NO 505 | TS | no CT | 1 | SEQ ID NO 988 | | | |
| SEQ ID NO 507 | TS | CT | 3 | SEQ ID NO 989 | SEQ ID NO 990 | SEQ ID NO 991 | |
| SEQ ID NO 509 | TS | no CT | 1 | SEQ ID NO 992 | | | |
| SEQ ID NO 511 | TS | CT | 1 | SEQ ID NO 993 | | | |
| SEQ ID NO 513 | TS | CT_est | 1 | SEQ ID NO 994 | | | |
| SEQ ID NO 515 | TS | CT | 1 | SEQ ID NO 995 | | | |
| SEQ ID NO 517 | TS | CT | 1 | SEQ ID NO 996 | | | |
| SEQ ID NO 519 | TS | CT | 1 | SEQ ID NO 997 | | | |
| SEQ ID NO 521 | PS | CT | 2 | SEQ ID NO 998 | SEQ ID NO 999 | | |
| SEQ ID NO 523 | PS | CT | 1 | SEQ ID NO 1000 | | | |
| SEQ ID NO 525 | TS | CT | 2 | SEQ ID NO 1001 | SEQ ID NO 1002 | | |
| SEQ ID NO 527 | TS | no CT | 1 | SEQ ID NO 1003 | | | |
| SEQ ID NO 529 | TS | no CT | 1 | SEQ ID NO 1004 | | | |
| SEQ ID NO 531 | TS | CT | 1 | SEQ ID NO 1005 | | | |
| SEQ ID NO 533 | TS | CT | 1 | SEQ ID NO 1006 | | | |
| SEQ ID NO 535 | TS | CT | 1 | SEQ ID NO 1007 | | | |
| SEQ ID NO 537 | TS | CT | 1 | SEQ ID NO 1008 | | | |
| SEQ ID NO 539 | TS | CT | 1 | SEQ ID NO 1009 | | | |
| SEQ ID NO 541 | TS | CT | 1 | SEQ ID NO 1010 | | | |
| SEQ ID NO 543 | TS | no CT | 1 | SEQ ID NO 1011 | | | |
| SEQ ID NO 545 | TS | no CT | 1 | SEQ ID NO 1012 | | | |
| SEQ ID NO 547 | TS | CT | 1 | SEQ ID NO 1013 | | | |
| SEQ ID NO 549 | TS | CT | 1 | SEQ ID NO 1014 | | | |
| SEQ ID NO 551 | TS | CT | 1 | SEQ ID NO 1015 | | | 1 |
| SEQ ID NO 555 | TS | CT | 1 | SEQ ID NO 1016 | | | |
| SEQ ID NO 559 | TS | no CT | 1 | SEQ ID NO 1017 | | | |
| SEQ ID NO 561 | TS | CT | 1 | SEQ ID NO 1018 | | | |
| SEQ ID NO 563 | TS | CT | 1 | SEQ ID NO 1019 | | | |
| SEQ ID NO 565 | TS | CT | 1 | SEQ ID NO 1020 | | | |
| SEQ ID NO 567 | TS | no CT | 1 | SEQ ID NO 1021 | | | |
| SEQ ID NO 569 | TS | CT | 1 | SEQ ID NO 1022 | | | |
| SEQ ID NO 571 | TS | CT | 1 | SEQ ID NO 1023 | | | |
| SEQ ID NO 573 | TS | CT | 1 | SEQ ID NO 1024 | | | |
| SEQ ID NO 575 | TS | CT_est | 1 | SEQ ID NO 1025 | | | |
| SEQ ID NO 577 | TS | CT | 1 | SEQ ID NO 1026 | | | |
| SEQ ID NO 579 | TS | no CT | 1 | SEQ ID NO 1027 | | | |
| SEQ ID NO 581 | TS | CT | 1 | SEQ ID NO 1028 | | | |
| SEQ ID NO 583 | PS | CT | 1 | SEQ ID NO 1029 | | | |
| SEQ ID NO 585 | TS | CT | 1 | SEQ ID NO 1030 | | | |
| SEQ ID NO 587 | TS | no CT | 1 | SEQ ID NO 1031 | | | |
| SEQ ID NO 589 | TS | no CT | 1 | SEQ ID NO 1032 | | | |
| SEQ ID NO 591 | TS | CT | 1 | SEQ ID NO 1033 | | | |
| SEQ ID NO 593 | TS | CT | 2 | SEQ ID NO 1034 | SEQ ID NO 1035 | | |
| SEQ ID NO 595 | TS | no CT | 1 | SEQ ID NO 1036 | | | |
| SEQ ID NO 597 | PS | CT | 1 | SEQ ID NO 1037 | | | |
| SEQ ID NO 599 | TS | no CT | 1 | SEQ ID NO 1038 | | | |
| SEQ ID NO 601 | TS | no CT | 1 | SEQ ID NO 1039 | | | |
| SEQ ID NO 603 | TS | CT | 1 | SEQ ID NO 1040 | | | |
| SEQ ID NO 605 | TS | no CT | 1 | SEQ ID NO 1041 | | | |
| SEQ ID NO 607 | TS | no CT | 1 | SEQ ID NO 1042 | | | |
| SEQ ID NO 609 | TS | no CT | 1 | SEQ ID NO 1043 | | | |
| SEQ ID NO 611 | TS | CT | 1 | SEQ ID NO 1044 | | | |
| SEQ ID NO 613 | TS | CT | 1 | SEQ ID NO 1045 | | | |
| SEQ ID NO 617 | TS | CT | 1 | SEQ ID NO 1046 | | | |
| SEQ ID NO 619 | TS | no CT | 1 | SEQ ID NO 1047 | | | |
| SEQ ID NO 623 | TS | CT | 1 | SEQ ID NO 1048 | | | |
| SEQ ID NO 625 | TS | no CT | 1 | SEQ ID NO 1049 | | | |
| SEQ ID NO 629 | TS | CT | 1 | SEQ ID NO 1050 | | | |
| SEQ ID NO 631 | TS | CT | 2 | SEQ ID NO 1051 | SEQ ID NO 1052 | | |
| SEQ ID NO 633 | TS | CT | 1 | SEQ ID NO 1053 | | | |
| SEQ ID NO 635 | TS | CT | 1 | SEQ ID NO 1054 | | | |
| SEQ ID NO 637 | TS | CT | 1 | SEQ ID NO 1055 | | | |
| SEQ ID NO 642 | TS | CT | 1 | SEQ ID NO 1056 | | | |
| SEQ ID NO 644 | TS | CT | 1 | SEQ ID NO 1057 | | | |
| SEQ ID NO 653 | TS | CT | 2 | SEQ ID NO 1058 | SEQ ID NO 1059 | | |
| SEQ ID NO 664 | TS | CT | 1 | SEQ ID NO 1060 | | | |
| SEQ ID NO 669 | TS | no CT | 1 | SEQ ID NO 1061 | | | |
| SEQ ID NO 673 | TS | CT | 1 | SEQ ID NO 1062 | | | |
| SEQ ID NO 681 | TS | CT_est | 1 | SEQ ID NO 1063 | | | |
| SEQ ID NO 683 | TS | no CT | 1 | SEQ ID NO 1064 | | | |
| SEQ ID NO 687 | TS | no CT | 1 | SEQ ID NO 1065 | | | |
| SEQ ID NO 689 | TS | no CT | 1 | SEQ ID NO 1066 | | | |
| SEQ ID NO 690 | TS | no CT | 1 | SEQ ID NO 1067 | | | |
| SEQ ID NO 691 | TS | CT | 1 | SEQ ID NO 1068 | | | |
| SEQ ID NO 692 | TS | CT | 1 | SEQ ID NO 1069 | | | |
| SEQ ID NO 693 | TS | no CT | 1 | SEQ ID NO 1070 | | | |
| SEQ ID NO 694 | TS | no CT | 1 | SEQ ID NO 1071 | | | |
| SEQ ID NO 695 | TS | CT | 1 | SEQ ID NO 1072 | | | |
| SEQ ID NO 698 | TS | no CT | 1 | SEQ ID NO 1073 | | | |
| SEQ ID NO 701 | TS | no CT | 1 | SEQ ID NO 1074 | | | |
| SEQ ID NO 702 | TS | no CT | 1 | SEQ ID NO 1075 | | | |
| SEQ ID NO 703 | TS | no CT | 1 | SEQ ID NO 1076 | | | |
| SEQ ID NO 707 | TS | no CT | 1 | SEQ ID NO 1077 | | | |
| SEQ ID NO 710 | TS | CT | 1 | SEQ ID NO 1078 | | | |
| SEQ ID NO 712 | TS | CT | 1 | SEQ ID NO 1079 | | | |
| SEQ ID NO 714 | TS | CT | 1 | SEQ ID NO 1080 | | | |
| SEQ ID NO 716 | TS | no CT | 1 | SEQ ID NO 1081 | | | |
| SEQ ID NO 717 | PS | CT | 1 | SEQ ID NO 1082 | | | |
| SEQ ID NO 720 | TS | CT | 1 | SEQ ID NO 1083 | | | |
| SEQ ID NO 721 | TS | no CT | 1 | SEQ ID NO 1084 | | | |
| SEQ ID NO 722 | TS | CT | 1 | SEQ ID NO 1085 | | | |
| SEQ ID NO 723 | TS | no CT | 1 | SEQ ID NO 1086 | | | |
| SEQ ID NO 737 | TS | CT | 1 | SEQ ID NO 1087 | | | |
| SEQ ID NO 742 | TS | no CT | 1 | SEQ ID NO 1088 | | | |

In another advantageous embodiment, the invention relates to a method described above, wherein said agent is able to allow PCR amplification of at least one nucleic acid molecule comprising or constituted by the group consisting SEQ ID NO 2q-1, q varying from 1 to 320 and SEQ ID NO 641 to SEQ ID NO 754 liable to be present among nucleic acids from the biological sample, said PCR amplification being preferably reverse transcription-quantitative PCR, or PCR array.

The invention also relates to a method for the *in vitro* and/or *ex vivo* cancer diagnosis in a subject, by determining the presence or the variation of amount of at least one protein comprising or constituted by an amino acid sequence consisting in SEQ ID NO 2q, q varying from 1 to 320, or a fragment thereof, among polypeptides from a biological sample from the subject, said presence or variation of amount of said protein being assessed with respect to the absence or the given amount of said protein from a sample isolated from an healthy subject, comprising:
- contacting polypeptides from the biological sample with an agent, said agent being able to recognize at least one protein comprising or constituted by the group consisting in SEQ ID NO 2q, q varying from 1 to 320, or a fragment thereof, liable to be present among polypeptides from the biological sample, to form an immune complex,
- determining the presence or the variation of amount of said immune complex indicating the fact that the subject is afflicted by cancer.

In another preferred embodiment, the invention relates to a method for the *in vitro* and/or *ex vivo* cancer diagnosis, wherein immune complex results from the specific recognition of a protein comprising or constituted by an amino acid sequence consisting in SEQ ID NO 2q, q varying from 1 to 320, or a fragment thereof, by said agent, said immune complex being liable to be determined for instance by immunohistochemistry, immunocytochemistry, immunofluorescence, western blotting and immunoprecipitation.

The invention also relates to a method for the *in vitro* and/or *ex vivo* cancer diagnosis in a subject, by determining the presence or the variation of amount of at least one antibody that specifically recognizes a protein comprising or constituted by an amino acid sequence consisting in SEQ ID NO 2q, q varying from 1 to 320, or a fragment thereof, among antibodies that specifically recognize polypeptides from a biological sample from the subject, said presence or variation of amount of said antibody that specifically recognizes protein being assessed with respect to the absence or the given amount of said antibody that specifically recognizes protein from a sample isolated from an healthy subject, comprising:
- contacting antibodies that specifically recognize polypeptides from the biological sample with an agent, said agent being able to recognize at least one antibody that specifically recognize protein comprising or constituted by the group consisting in SEQ ID NO 2q, q varying from 1 to 320, or a fragment thereof, liable to be present among antibodies that specifically recognize polypeptides from the biological sample, to form an immune complex,
- determining the presence or the variation of amount of said immune complex indicating the fact that the subject is afflicted by cancer.

According to the invention, the determination of the presence of at least one antibody indicates that if an antibody can be detected in a biological sample, the antibody is considered as present in the biological sample. On the contrary, if the said antibody can not be detected by the method of the invention, the antibody is considered as absent from the biological sample.
By antibody, it is defined in the invention all the immunological molecules produced by B-cell: immunoglobulins (Ig). Then, according to the invention, all the soluble and insoluble immunoglobulins, such as IgG, IgM, IgA, and IgD, can be detected.
With regard to the determination of the quantification of amount of at least an antibody, it is heard in the invention, that the quantity of said antibody is measured.
The amount of antibody is measured using a classical protocol of quantification, wherein the amount of antibody is compared with at least two control samples. These control samples are represented by at least a negative sample and a positive control sample. The value associated to the measure of the quantity of antibody is null in the control negative sample, and value associated to the measure of the quantity of antibody is positive in the control positive sample. So, if the antibody is absent of the biologic sample, the value of the quantification is null. On the other hand, if the antibody is present, the value of the quantification is superior to zero. The presence or amount of antibodies may be determined by any routine protocols commonly used in the art.

According to the method of the invention, polypeptides are recognized specifically by at least one antibody liable to be present in a biological sample of a subject. When the antibody is present, the recognition is said specific, which means that the antibody only interact with said polypeptide, or the variants or isoforms of the polypeptides, but does not interact with another polypeptide.

Also, the invention relates to a method for the *in vitro* and/or *ex vivo* cancer diagnosis in a subject, by determining the presence of an immune response in a biological sample from the subject comprising:
- contacting a biological sample from the subject with an agent, said agent being able to recognize at least one fragment of a protein comprising or constituted by the group consisting in SEQ ID NO 2q, q varying from 1 to 320, liable to be presented by the MHC molecules of T-cells,
- determining the presence of said immune response indicating the fact that the subject is afflicted by cancer.

In one advantageous embodiment, the invention relates to any methods described above, wherein the sample is a body fluid, a body effusion, a cell, a tissue or a tumor.

The invention also relates to a kit for the *in vitro* and/or *ex vivo* cancer diagnosis comprising:
- a nucleic acid micro-array comprising or constituted by at least one nucleic acid molecule comprising or consisting by a nucleic acid sequence of the group consisting SEQ ID NO 755 to SEQ ID NO 1088,
- possibly material for preparation of nucleic acids of the biological sample from a patient suspected to be afflicted by cancer, in particular the preparation of cDNAs,
- possibly labelled molecules for labelling said nucleic nucleic acids,
- possibly a negative control corresponding to nucleic acids from a biological sample from an healthy subject.

The invention also relates to a kit for the *in vitro* and/or *ex vivo* cancer diagnosis comprising:
- ELISA support comprising or constituted by at least one protein comprising or constituted by an amino acid sequence of the group consisting in SEQ ID NO 2q, q varying from 1 to 320, or fragment thereof,
- possibly labelled antibodies directed against antibody that recognizes specifically said protein, said protein being liable to be present among polypeptides from a sample from a patient suspected to be afflicted by cancer,
possibly a negative control corresponding to antibodies polypeptides from a sample from an healthy subject.

The invention also relates to a kit for the *in vitro* and/or *ex vivo* cancer diagnosis comprising:
- ELISA support comprising or constituted by at least one antibody that recognizes specifically a protein comprising or consisting by an amino acid sequence of the group consisting in SEQ ID NO 2q, q varying from 1 to 320, or fragment thereof,
- possibly labelled antibody directed against a protein specifically recognized by said antibody, said antibody being liable to be present among antibodies from a sample from a patient suspected to be afflicted by cancer,
- possibly a negative control corresponding to polypeptides from a sample from an healthy subject.

The invention also relates to a kit for the *in vitro* and/or *ex vivo* cancer diagnosis comprising:
- ELISA support comprising or constituted by at least one antibody that recognizes specifically a protein comprising or consisting by an amino acid sequence of the group consisting in SEQ ID NO 2q, q varying from 1 to 320, or fragment thereof,
- possibly labelled antibody directed against a protein specifically recognized by said antibody, said antibody being liable to be present among antibodies from a sample from a patient suspected to be afflicted by cancer,
- possibly a negative control corresponding to polypeptides from a sample from an healthy subject.

The invention also relates to a pharmaceutical composition comprising at least, as active substance, one of the elements chosen among the group consisting in:
- a nucleic acid molecule described above,
- a protein described above, and
- an antibody described above,
in association with a pharmaceutically acceptable vehicle.

The invention also relates to a vaccine composition comprising as active ingredient an antibody, fragments or derivatives thereof described above, in association with a pharmaceutically acceptable vehicle.

The invention relates to a pharmaceutical composition for the treatment of cancers comprising as active ingredient is at least one RNAi molecule, said RNAi molecule being able to hybridize with a nucleic acid molecule described above, in association with a pharmaceutically acceptable vehicle.
RNA interference (RNAi) is a mechanism that inhibits gene expression by causing the degradation of specific RNA molecules or hindering the transcription of specific genes. RNAi plays a role in regulating development and genome maintenance. Small interfering RNA strands (siRNA) are keys to the RNAi process, and have complementary nucleotide sequences to the targeted RNA strand. Specific RNAi pathway proteins are guided by the siRNA to the targeted messenger RNA (mRNA), where they "cleave" the target, breaking it down into smaller portions that can no longer be translated into protein.

In an advantageous embodiment, the invention relates to a pharmaceutical composition described previously, wherein said RNAi specifically hybridize to at least a nucleic acid molecule of the group comprising or constituted by a nucleotide sequence of the group consisting in SEQ ID NO 1 to SEQ ID NO 476, or at least a nucleotide acid molecule coding for protein comprising or constituted by an amino acid sequence belonging to the group consisting in SEQ ID NO 2q, q varying from 1 to 320, said RNAi containing a 17-25 nucleotide sense sequence (siRNA).

In another advantageous embodiment, the invention relates to a pharmaceutical composition previously described, wherein said RNAi specifically binds to at least a nucleic acid molecule of the group comprising or constituted by a nucleotide sequence of the group consisting in SEQ ID NO 1 to SEQ ID NO 476, or at least a nucleotide acid molecule coding for protein comprising or constituted by an amino acid sequence belonging to the group consisting SEQ ID NO 2q, q varying from 1 to 320, said RNAi containing an oligonucleotide composed 17-25 nucleotides sense sequence, a 7-11 nucleotides hairpin loop sequence and an antisense sequence binding complementarily to the sense sequence (shRNA), said shRNA being contained in an expression vector allowing shRNA expression in mammal cells.

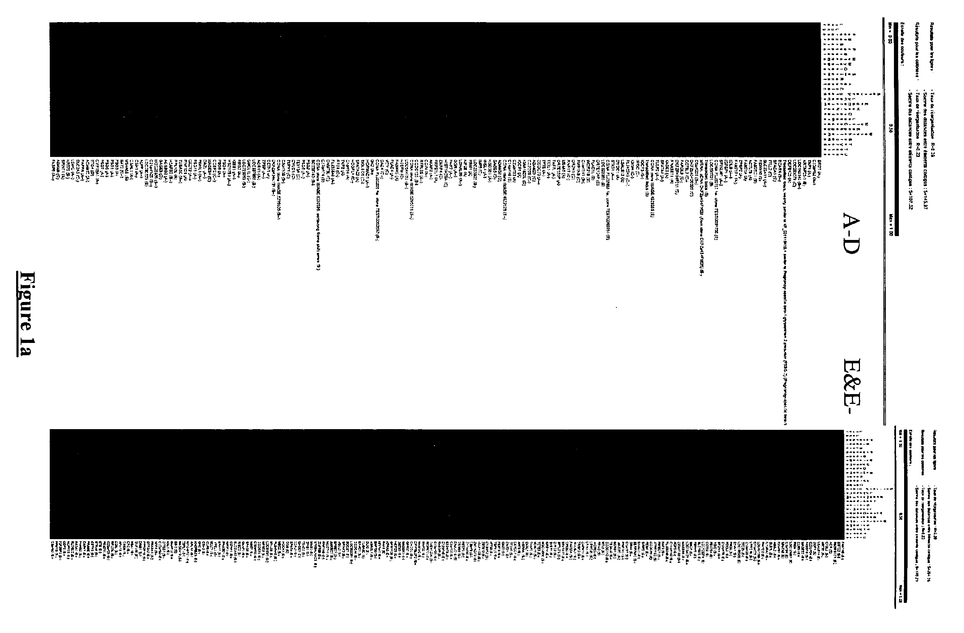

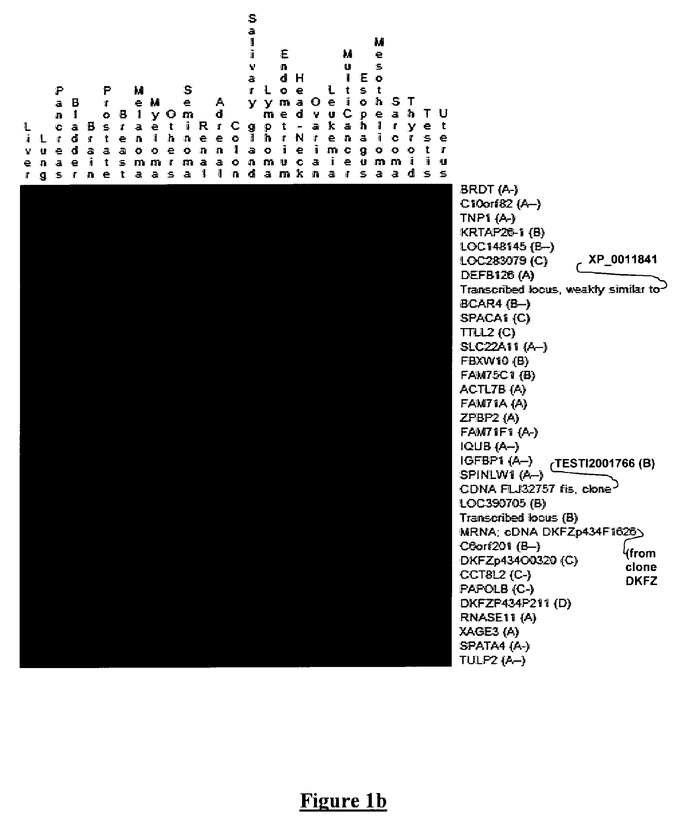

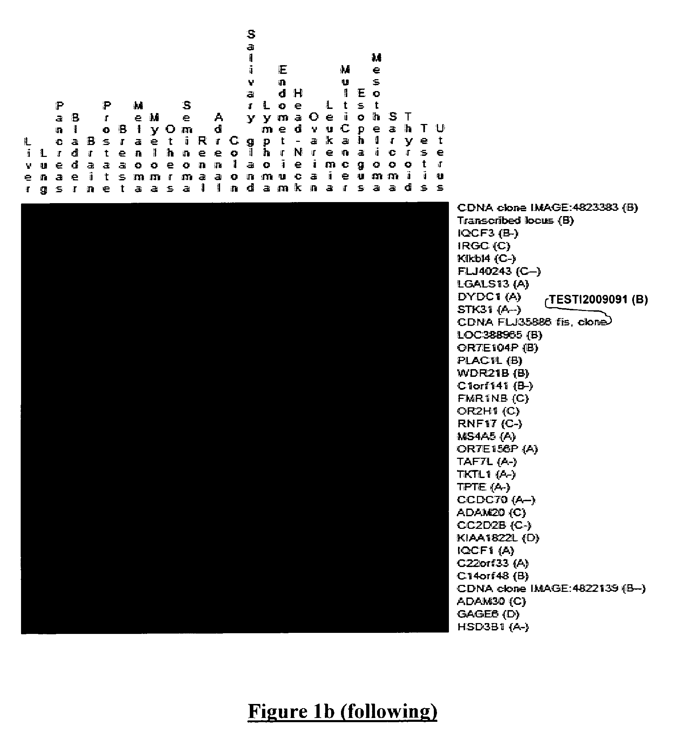

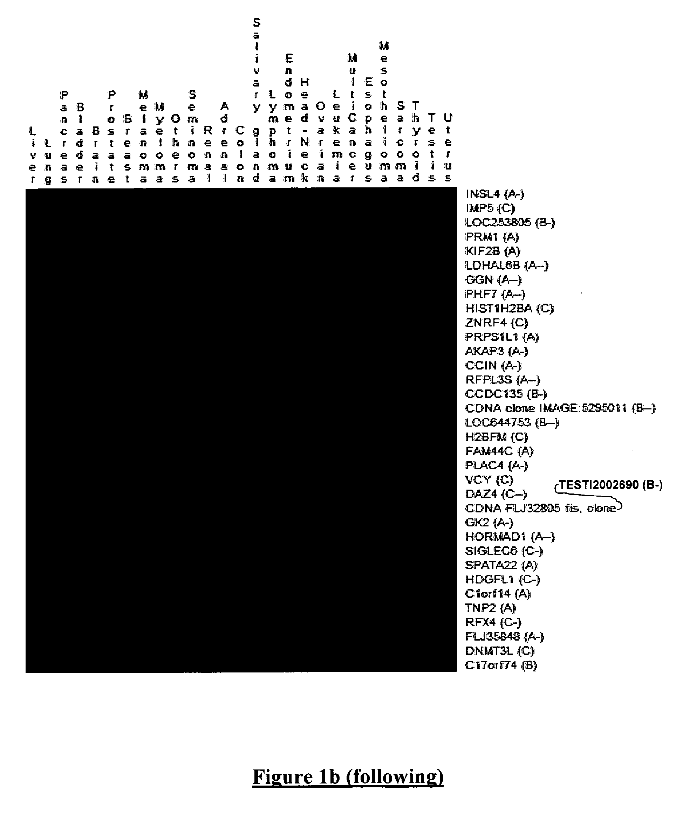

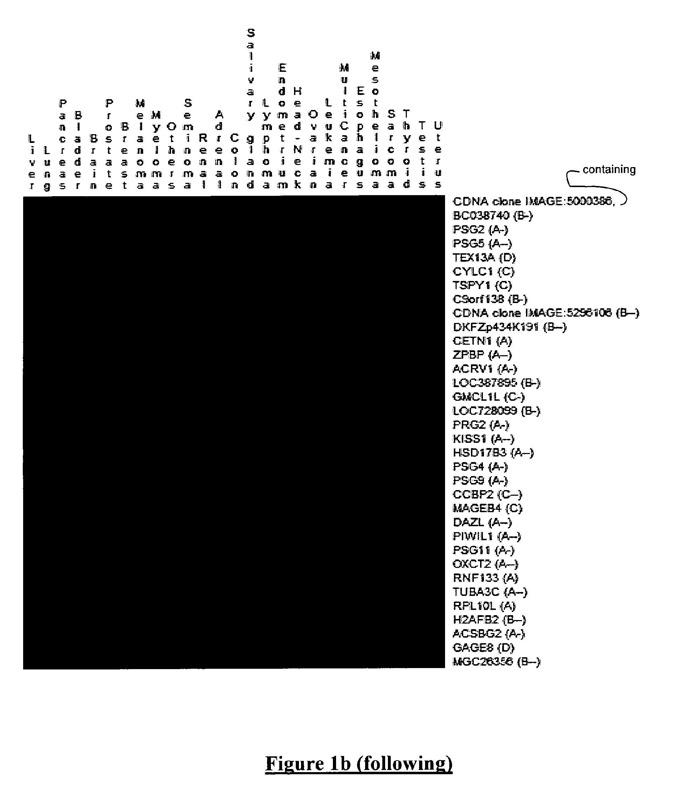

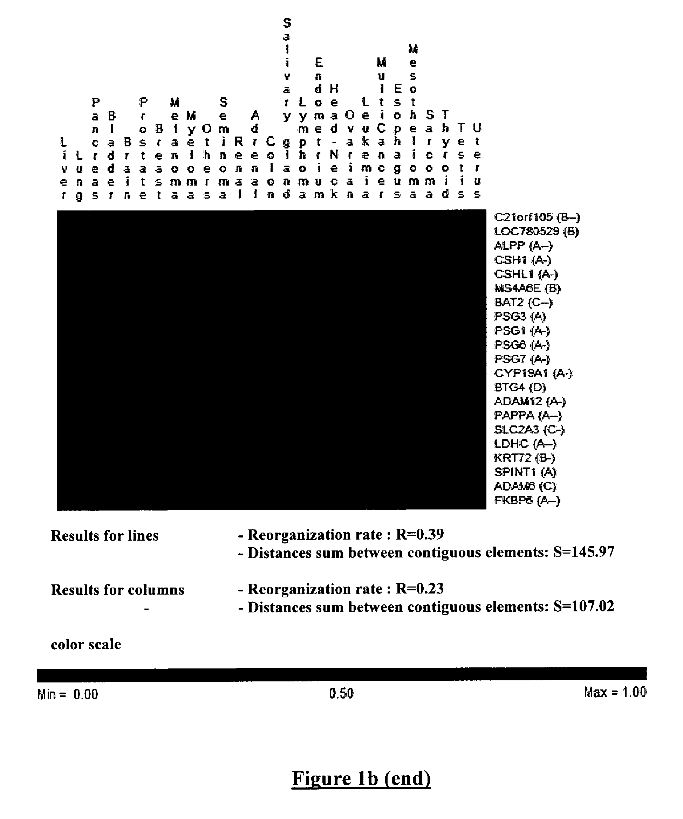

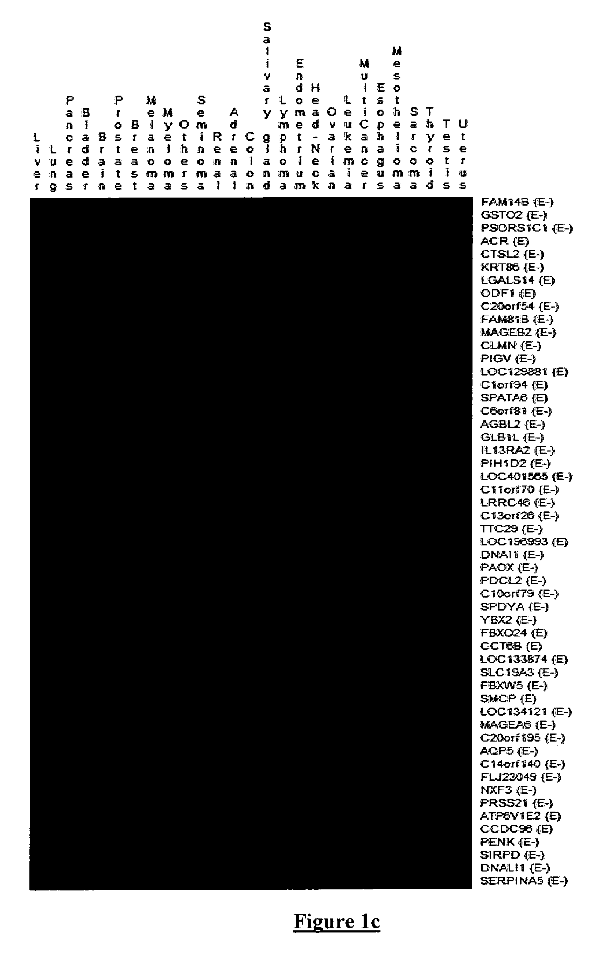

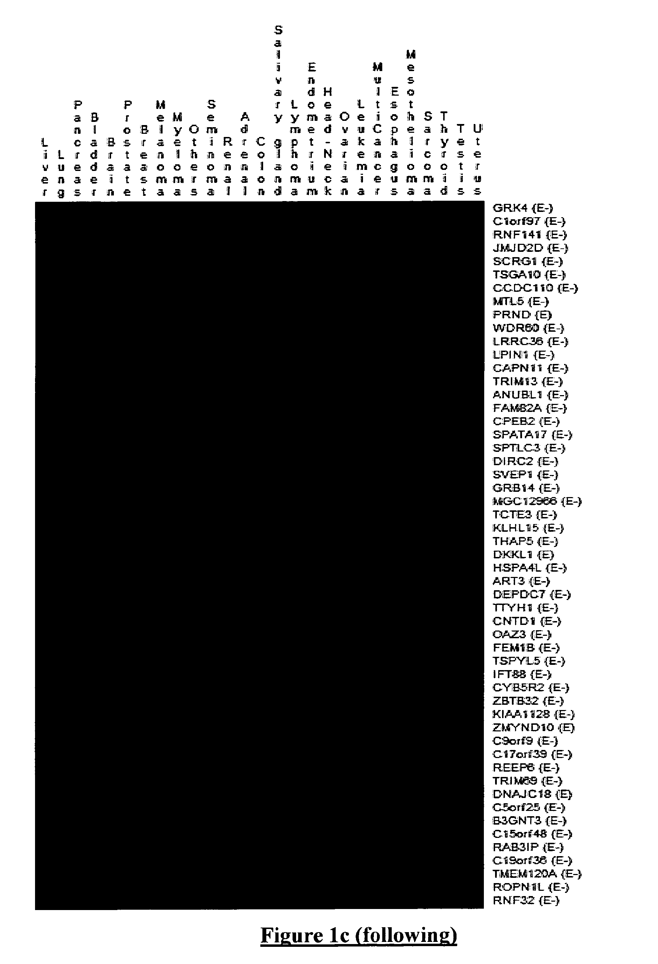

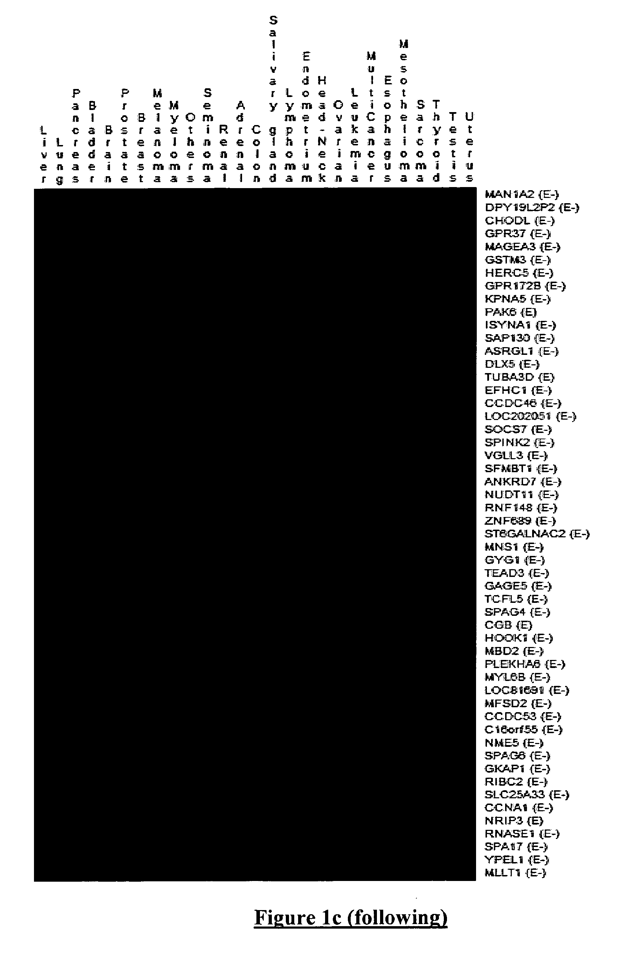

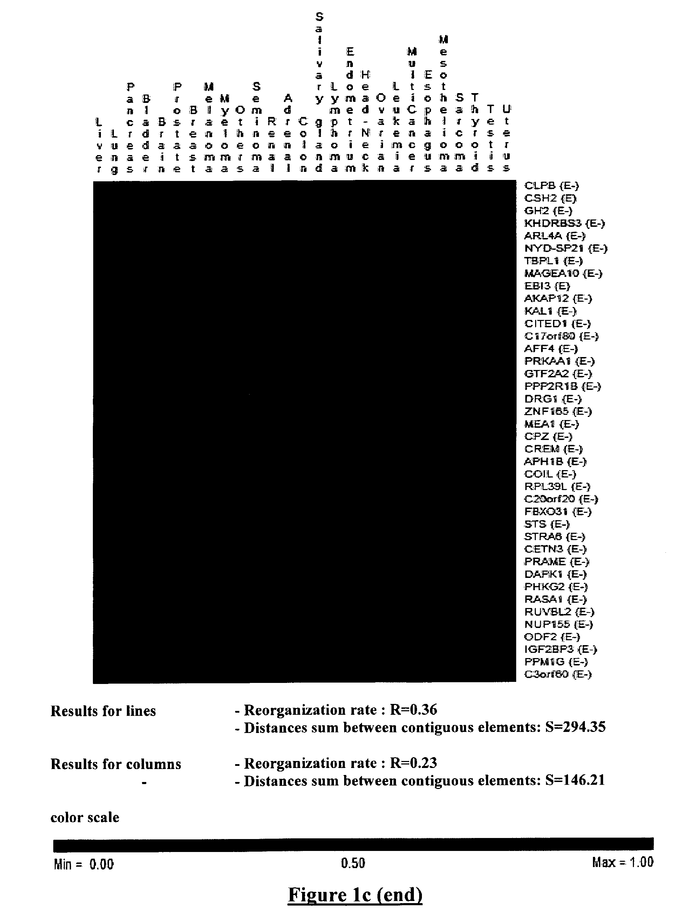

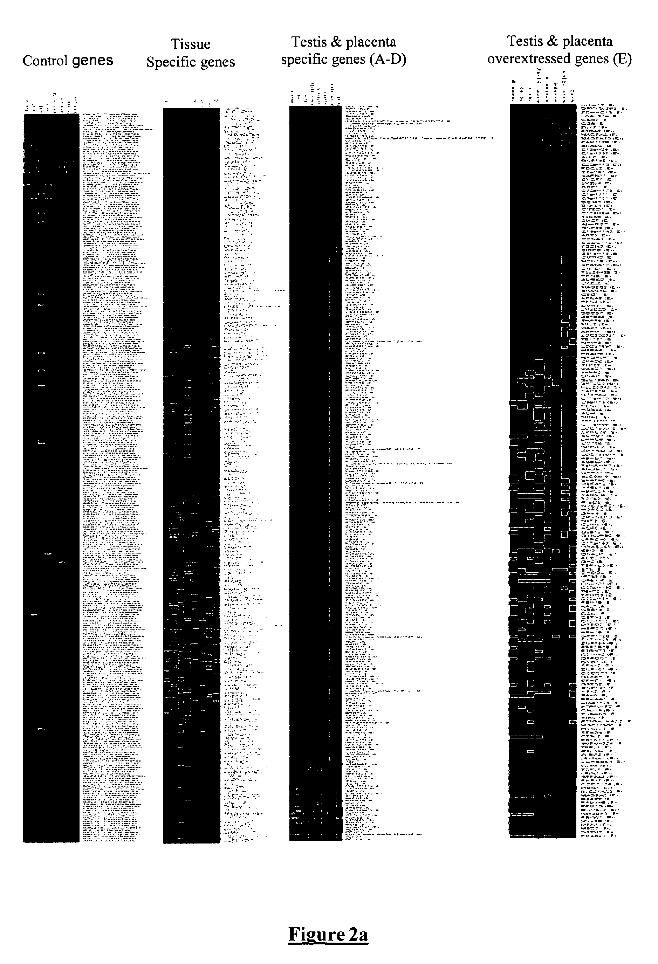

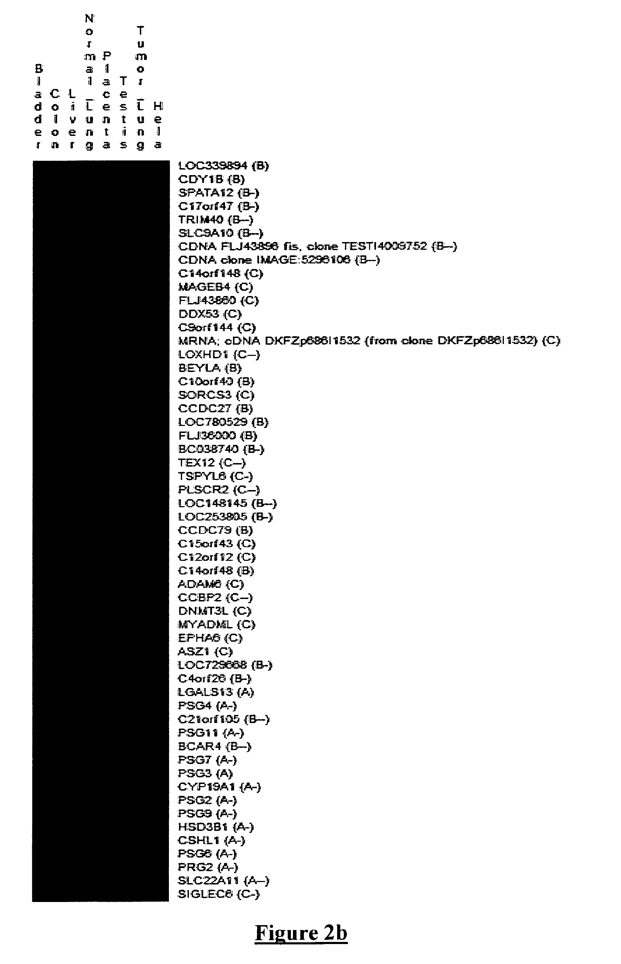

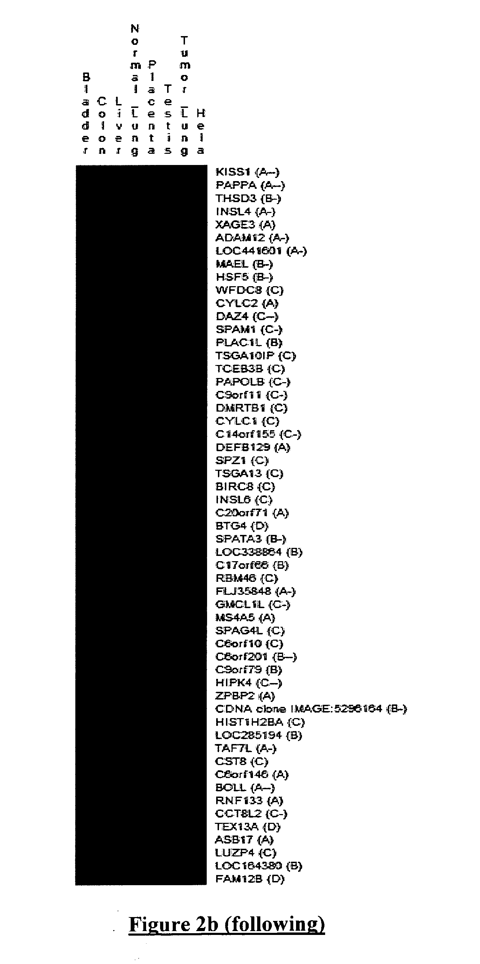

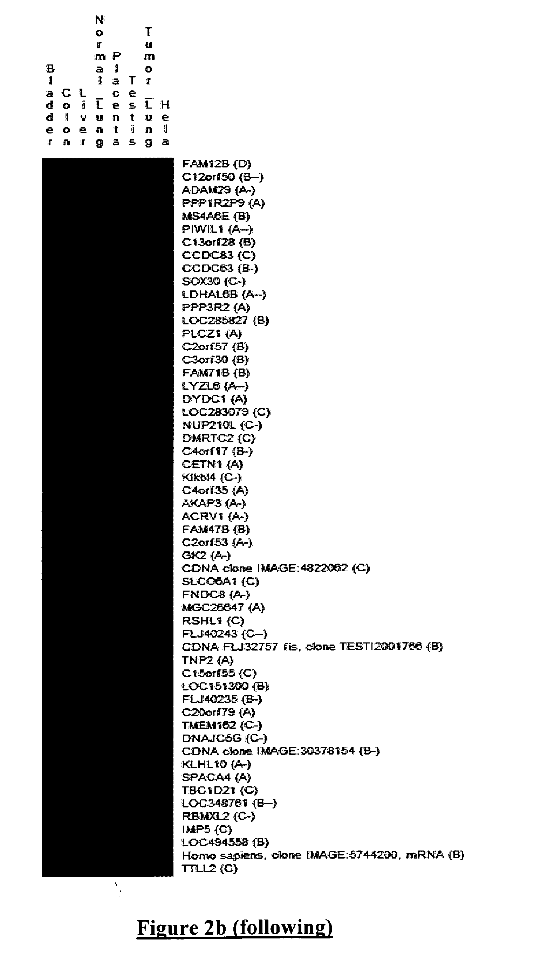

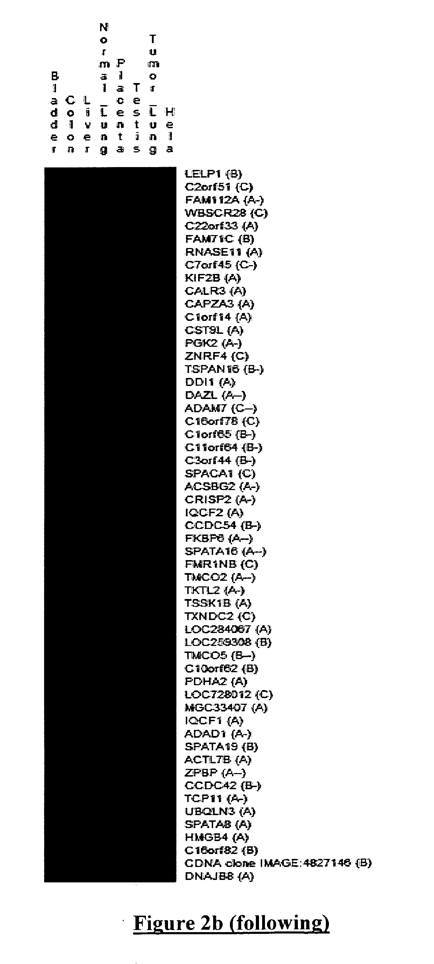

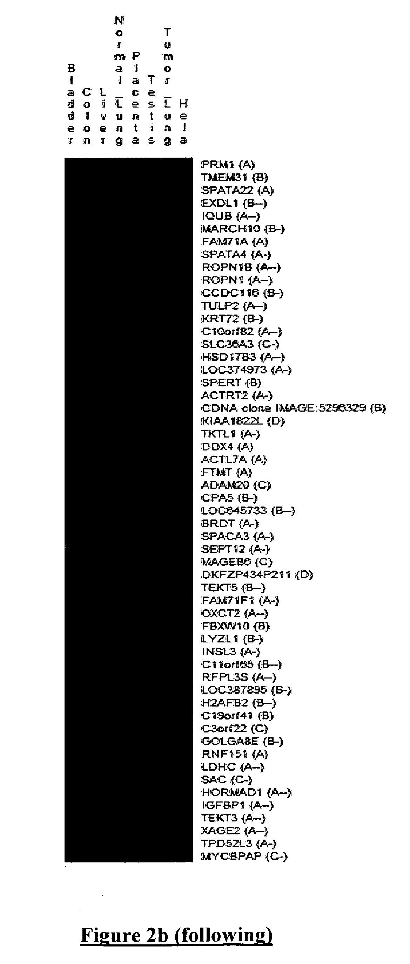

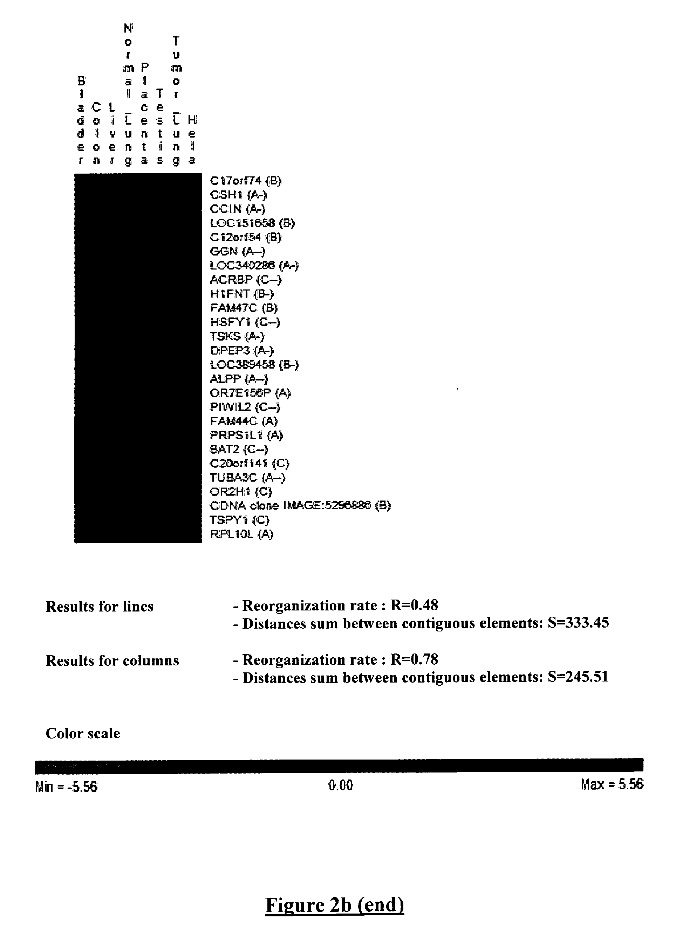

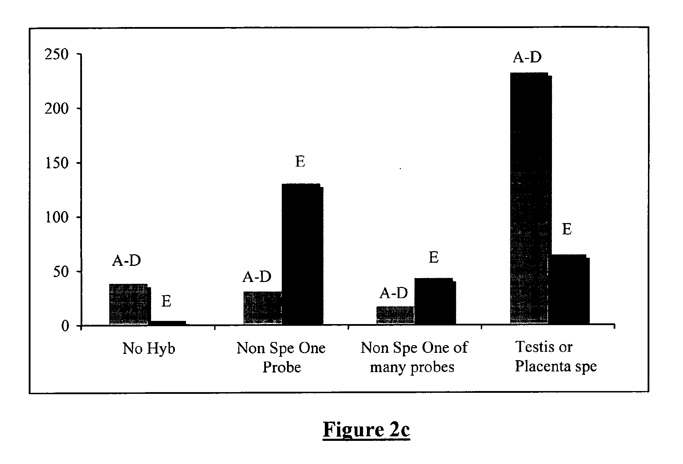

Examples 1 and 2, and figures 1 and 2 that follow, illustrate the invention.
Figure 1a represents a meta-analysis of Oncomine data, showing the aberrant expression, in somatic or ovarian cancers, of testis- or placenta- specific genes of our list (classes A to D - includes list of genes described above) and of testis- or placenta-overexpressed genes (class E&E-). The genes are represented vertically, and the different tissue-specific tumours horizontally. Each red square corresponds to a gene overexpressed in at least one study of each type of somatic cancer compared to the corresponding normal tissue (with p<0.001). Genes found expressed in only one type of somatic cancer are displayed at the top of the map, whereas genes found overexpressed in several somatic cancers are found at the bottom of the map.
Figure 1b and 1c respectively represent a magnification of the map results of the genes belonging to class A to D (1b) and E to E- (1c).
Figure 2a shows a hierarchical clustering of the genes belonging to the indicated categories. This was done using "permutmatrix" software (free online http://www.lirmm.fr/∼caraux/PermutMatrix/).
Figures 2b represents a clustering magnification of testis and placenta specific gene.
Figure 2c recapitulates CT chip global results with testis- and placenta- expressed genes. Number of testis- or placenta- specific (A-D) or testis- or placenta- overexpressed (E) genes showing no hybridization (No Hyb), no specific hybridization with one probe or one of many probes, or displaying a testis- or placenta- specific pattern of expression are represented.

NA on chip: number of genes absent from this first version of the CT chip (not analysed here); No hyb : no expression detected in any of the analysed tissues, Non spe one probe: genes found expressed in at least one somatic tissue (with one probe), Non spe one of many probes: genes with different profiles of expression depending on the probe and found expressed in at least one somatic tissue ; Testis or placenta spe: genes with a restricted expression pattern in the testis and/or placenta.

### EXAMPLES

### Example 1 : In silico identification of testis-specific (TS) and placenta-speciric (PS) genes

We have undertaken a large-scale identification of "Cancer Testis" (CT) genes, which are normally specifically expressed in the male germinal cells (and/or the placenta) but illegitimately expressed in somatic cancer cells.
Combining transcriptomic and EST data has led to the identification of 467 human genes, specifically expressed in male germinal cells (TS genes) or placenta (PS genes).
In order to investigate the aberrant expression of these genes in somatic cancers, we took advantage of cancer transcriptomic data and search engine available on the oncomine website (http://www.oncomine.org/main/index.jsp), and found that 250 of the testis- or placenta-specific genes were illegitimately expressed in somatic cancers recorded in Oncomine and each cancer type expressed a variable number of these genes. This database mining approach has been very efficient in identifying new CT genes.

This list of CT genes will provide the basis to develop new tools for the diagnosis, follow-up and treatment of cancers. In addition, the 196 placenta or testis-specific genes which have not been found over-expressed in any somatic cancer in Oncomine, could however be deregulated in other cancer types, for which the expression data have not been studied yet.

### 1-Identification of testis-specific (TS) and placenta-specific (PS) genes

### Identification of human testis-specific (TS) and placenta-specific (PS) sequences using available EST data

In order to search for testis-specific EST sequences, the following query was made in unigene (ncbi website: http:Hwww.ncbi.nlm.nih.gov/entrez/query.fcgi?CMD=search&DB=unigene): " testis"[restricted] AND HOMO SAPIENS.
A similar query was made for the identification of placenta-specific transcripts: " placenta" [restricted] AND HOMO SAPIENS).

### Identification of human testis-specific (TS) and placenta-specific (PS) genes using available transcriptomic data

The Genomics Institute of the Novartis Research Foundation ("GNF") Gene Expression Database SymAtlas displays transcriptomic data obtained from designed custom arrays, which interrogate the expression of most protein-encoding human and mouse genes in a panel of 79 human and 61 mouse tissues (http://symatlas.gnf.org/SymAtlas/) (ref (Su et al. 2004)).
Two query strategies have been undertaken to interrogate the expression of human genes. The first approach searched for testis-overexpressed genes, which expression in testis germinal cells and/or testis seminiferous tubules was ten fold over the median of expression in all tissues, in at least one probe set, using GNF1H gcRMA as well as MAS5 condensed datasets. The expression profile images were then downloaded and the testis-specific expression checked upon.
In the second approach a similar query was made, but searching for testis-overexpressed genes, which expression in germinal cells and/or seminiferous tubules was three folds over the median of expression in all tissues, and the expression in all the other tissues was less than 2 folds over the median. The expression data in the following tissues or cell lines were excluded from this search: 721 B lymphoblasts, colorectal adenocarcinoma, leukaemia chronic myelogenous k562, leukaemia lymphoblastic molt4, leukaemia promyelocytic h160, lymphoma Burkitt Daudi, lymphoma Burkitt Raji, Ovary, Placenta, and other Testis tissues.
Transcriptomic studies are based on the hybridization of labelled cDNA from the different tissue/cells with oligonucleotides attached to a solid surface (oligoprobes). The sequence specificity of the oligonucleotides sequences defines the hybridization specificity. Depending on its sequence, an oligoprobe can be specific for a single gene product or for a family of genes. Moreover, some oligoprobes are only hybridizing with spliced transcripts. In transcriptomic arrays, each gene is represented by one or more oligonucleotide(s), some of them being more specific than others. In the present approach, a gene was considered overexpressed in male germinal cells when hybridization data obtained with all its oligoprobes showed a « testis specific » profile.
A similar approach was undertaken to identify genes specifically over-expressed in the placenta.

These combined approaches allowed the identification of 733 genes, of which 655 were testis-expressed according to the EST and/or transcriptomic data, and 78 were identified as placenta-expressed. A selection procedure was then undertaken in order to select for genes whose pattern of expression was restricted to the testis and/or the placenta.

### Classification of the testis-expressed and placenta-expressed genes according to their tissue-specificity data

The 733 genes identified above were then classified according to their tissue specificity.

For this purpose, it was assumed that the specificity of the EST sequences prevailed for the following reasons. The ESTs are the result of the systematic sequencing of expressed sequences in specific tissues, whereas transcriptomic data highly depend on the specificity of the oligonucleotide(s) present on the array. Depending on its sequence, the latter could be not entirely specific to a particular gene, and could hybridize with the transcripts of the whole gene family, some of them being non testis- or placenta-specific. This would result in a non-specific hybridization profile of a testis- or placenta-specific gene. Conversely, the chosen oligoprobe(s) could represent a testis- or placenta-specific splicing variant of a particular gene, which expresses other somatic-expressed variants. The resulting hybridization profile with one or several probes would be testis- or placenta- specific, but the gene itself would be expressed in several somatic tissues. For the above reasons, the classification of testis and placenta expressed genes was based on the specificity of the corresponding EST sequences.
For each gene, when more than 20 ESTs were available, a ratio "R0" was defined by the number of ESTs found in tissues other than testis or placenta over the total number of ESTs. The ratio R0 represents the proportion of EST sequences, which were not found in testis or placenta.
Another special mention concerns the testis or placenta expressed genes, which are also expressed in the brain. Indeed, many testis- or placenta-expressed genes are also expressed in the brain. One important characteristic of the testis-specific genes is that they encode products, which are normally kept separated from the immune system by the blood-testis barrier. Placenta-specific genes are in a similar situation. Their illegitimate expression in somatic tissues can induce an immune response, and most of the applications of the CTs are based on their immunogenic properties. Since a similar barrier exists in the brain, the genes, which are expressed in the brain, are also protected from the immune system under normal circumstances, and potentially immunogenic if illegitimately expressed in other tissues. Therefore, those of the testis- and placenta- expressed genes, which are also expressed in the brain, but not in other tissues, were here included in the list of testis- or placenta-specific genes.

For each gene for which more than 20 ESTs were available, another ratio, "**R1**", was calculated defined by the number of ESTs found in tissues other than testis or placenta or brain, over the total number of ESTs. Hence, R1 defines the proportion of EST sequences, which were not found in testis or placenta or brain.

The specificity classes of the genes were based on this ratio R1, and defined as follow.

**Class A** was defined by testis- or placenta-expressed genes, which displayed a specific expression profile according to the Symatlas data, as well as a specificity of their ESTs with R1 = 0. Genes of classes A- and A-- also displayed a specific Symatlas expression profile, but showed a small proportion of non-specific ESTs: up to 20% (A- genes) or 30% (A-- genes) of the corresponding ESTs were found in somatic tissues (other than testis or placenta or brain) (A- genes: R1 ratio between 0 and 0.2; A-- genes: R1 ratio between 0.2 and 0.3). For these genes, the arguments for a testis or placenta specificity of expression are strong.

**Class B** was defined by the genes with testis or placenta specific ESTs (R1=0), but for which no transcriptomic data were available in Symatlas. Genes of classes B- and B-- showed small proportions of non-specific ESTs : up to 20% (B- genes) or 30% (B-- genes) of the corresponding ESTs were found in somatic tissues (other than testis or placenta or brain) (B-genes: R1 ratio between 0 and 0.2 ; B-- genes: R1 ratio between 0.2 and 0.3). Since EST specificity does not depend on probe choices or hybridization conditions, this group of genes could be considered as testis- or placenta- specific with high reliability.

The genes of **class C** also showed testis or placenta specificity of their ESTs (R1=0), but their transcriptomic profiles in Symatlas were not. The most likely explanation for this apparent discrepancy is a lack of specificity of the chosen oligoprobes, which could hybridize with somatic expressed genes of the same family sharing similar sequences. Classes C- and Cincluded genes with a small proportion of non-specific ESTs : up to 20% (C- genes) or 30% (C-- genes) of the corresponding ESTs were found in somatic tissues (other than testis or placenta or brain) (C- genes: R1 ratio between 0 and 0.2 ; C-- genes: R1 ratio between 0.2 and 0.3). Although the transcriptomic data available in symatlas did not convincingly show specific patterns of expression, the large predominance of the corresponding ESTs in testis or placenta suggested that they could be considered as testis- or placenta- specific genes with reasonable reliability.

Genes grouped in **class D** displayed a testis- or placenta- specific expression profile according to symatlas transcriptomic data, but too few EST sequences were available in total. Indeed less than 20 recorded transcripts per Million was considered insufficient for tissue-specificity studies.

Genes of **class E and E-** displayed a testis- or placenta- specific expression profile according to symatlas transcriptomic data, but more than 30% (E) or 50% (E-) of non-specific ESTs were found in somatic tissues (other than testis or placenta or brain). The presence of ESTs in some somatic tissues suggested that, although the genes of E and E- classes were overexpressed in testis or placenta, they are not entirely testis-or placenta-specific. For the genes of the E class, the number of testis and/or placenta transcripts exceeded the number of somatic transcripts, whereas for the E- genes, the somatic transcripts outnumbered the testis and placenta transcripts. These genes were excluded from the final list of TS and PS genes.

From this confrontation between EST specificities and SymAtlas transcriptomic data, a classification of our list of TS and PS genes was established according to their specificity.

### Quality control using other available published data

Data available from other published studies were used to check that our list of testis-specific genes was exhaustive.
In the first study (Fox et al. 2003), the authors have identified 800 sequences differentially expressed between human adult normal testes and Sertoli Cell Only testes (testes with no germinal cells). By using Symatlas and EST data (as above), among these sequences, we found **57** testis-specifically expressed genes. All of these genes were redundant with those identified above.
In the second study (Schultz et al. 2003), 385 mouse testis-specific genes were identified and clustered in five groups, according to the time of their expression in pre-pubertal mice. Using a nucleotide blast search (http://www.ncbi.nlm.nih.gov/BLAST/) we have systematically looked for a human homolog for each of the testis-specific mouse gene, which has led to the identification of 233 human genes. By selecting those that presented a testis-specific expression (according to SymAtlas and/or EST data) we were able to identify 29 human testis-specific genes, all of which were also redundant with those identified with the approaches described above.

Finally, the human homologs of rodents genes, with were described as meiotically or post-meiotically differentially expressed in the male germinal cells (Chalmel et al. 2007), were searched for, and their testis-specificity recorded. Of the **244** human homologs, **64** were found testis-specific according to transcriptomic symatlas data, of which **13** also showed testis-specific ESTs, and one gene was specific of the placenta in the human. All these **65** genes were present on our list of testis- and placenta-expressed genes. The other genes, absent from our list, did not show any clear evidence of testis-overexpression.
Finally, a recent study, recording several tissue-specific genes on the basis of transcriptomic data, have listed 242 genes as being "testis-specific" (Bock-Axelsen et al. 2007). However only 51 of them are redundant with our list of TS genes (classes A to D). Another 74 genes were also found in our testis-overexpressed classes of genes (classes E and E-) but could not be considered as "testis-specific" because of the absence of specificity of their ESTs. The other 117 genes either showed a testis-specific profile with only some of the symatlas probes and a non-specific expression with other probes suggesting that they correspond to differentially spliced genes (43 genes), or displayed a non-specific expression profile with all symatlas probes, as well as non-specific ESTs (74 genes).

Hence, all the above sets of data support the evidence that, our list of germline specific genes includes all genes exclusively expressed in the testis, for which expression data are presently available.

The status of DNA methylation of the promoter of a gene could also provide information on its specificity. Indeed Schubeler and collaborators have recently systematically characterized the DNA methylation status of the promoter regions of the whole human genome in primary fibroblasts (representative of normal somatic cells) and in sperm cells (Weber et al. 2007). They have observed that CpG rich promoters were generally hypomethylated in somatic cells, apart from the germline specific genes promoters, which were generally hypermethylated in fibroblasts and hypomethylated in sperm. Checking the DNA methylation data obtained from their study (http://www.fmi.ch/members/dirk.schubeler/supplemental.htm) on the promoters of the genes, which we have listed as testis-specific (TS, classes A to D), we have indeed found i/ that approximately half of them had CpG-rich or intermediate promoters, and ii/ that 70% of these CpG-rich/intermediate promoters were hypermethylated. In contrast, the testis-expressed genes, which had been found overexpressed but not specifically expressed in the testis (classes E and E-), although showing a higher proportion of CpG-rich/intermediate promoters (77%), displayed a much lower percentage of hypermethylated CpG-rich/intermediate promoters (13%). Hence, the testis specificity of the genes of our TS- list is not only confirmed by ESTs and transcriptomic data, but also by epigenetic marks.

### 2- Classification of TS and PS genes according to their expression in cancer

### Known and new CT genes

A review of the literature and the data available online (http://www.cancerimmunity.org/CTdatabase/) show that 72 genes have been so far recorded as CT genes. Twelve of these genes (10 TS and 2 PS) are redundant with our present list of TS-PS genes deregulated in cancer. Therefore 12 of the testis- and placenta- specific genes identified by us had already been described as "Cancer Testis" genes, deregulated in several somatic cancers.
However, 60 known CTs were not identified as CTs by our approach. The main reason is that these genes did not meet the testis or placenta specificity criteria, which were used to establish our list. Indeed, nine of the known CT genes (7 testis-expressed and 2 placenta-expressed) were found among our testis- or placenta overexpressed genes (classes E and E-). The other 51 did not show specific patterns of expression according to symatlas transcriptomic data and/or EST sequences specificity. They therefore did not qualify as CTs on the basis of our criteria.
In addition, Scanlan and collaborators have described the following genes as CTs, in a published work as well as in WO 2006/029176. Nine of the genes they describe are redundant with our TS genes and have been removed from Table 4.

**Table 3 recapitulates the Unigene accession number, name and class specificity of known CT genes.**

| UNIGENE_ACCS | Name | Class Specif | Chen & Scanlan 2005 | WO 2006/029176 (Table and Sequences) | KnownCT |
|---|---|---|---|---|---|
| Hs.2324 | PRM2 | A- | | x | |
| Hs.121084 | SPINLW1 | A-- | x | | |
| Hs.104636 | TULP2 | A-- | x | | |
| Hs.298312 | HORMAD1 | A-- | | x | CT46 |
| Hs.169797 | BOLL | A-- | | x | |
| Hs.116287 | LOC348120 | B | x | x | |
| Hs.651245 | MAEL | B- | | x | |
| Hs.133095 | MGC27016 | C | x | | |
| Hs.411239 | FLJ25339 | C- | x | | |

Altogether, an exhaustive survey of the literature on the subject demonstrates that, although the discovery and study of CTs have raised a lot of interest because of their potential use in cancer diagnosis and/or treatment, these medical applications have so far been hampered by the fact that all known CTs are sporadically expressed in cancers. No "perfect" CT or group of CTs had been found, which could allow a reliable and highly specific detection and/or targeting of all cancer types.

The present list (table 4) records the first large-scale identification of genes with "Cancer Testis" specific restricted patterns of expression. As a whole, this list provides a basis for the development of reliable tests and therapy approaches available for all cancer types. These approaches are based on the known properties of "Cancer Testis" genes.

### Table 4: List of Testis-Specific and (TS) Placenta-Specific (PS) genes annotated with

- name
- unigene number
- tissue-specificity: Testis-Specific or (TS) Placenta-Specific (PS)
- class defining the reliability of their testis or placenta specificity: A, A- and A-- (TS or PS according to Symatlas transcriptomic data and ESTs), B, B- and B-- (TS or PS according to ESTs, but Symatlas transcriptomic data non available), C, C- and C-- (TS or PS according to ESTs, but Symatlas transcriptomic data non specific), D (TS or PS according to Symatlas transcriptomic data, but ESTs non available), E and E- (TS or PS according to Symatlas transcriptomic data, but ESTs non specific)
- Known genes (KG) and New genes (NG) are also indicated in the table.

**Table 4**

| SEQ ID NO | UNIGENE_ ACCS | Gene Name | LOCUSLINK_ ACCS | Class Specification | NG (II) or KG (III) | TS or PS | no CT or CT |
|---|---|---|---|---|---|---|---|
| 1 | Hs.169222 | ACRV1 | 56 | A- | KG (III) | TS | CT |
| 3 | Hs.465720 | ACSBG2 | 81616 | A- | KG (III) | TS | CT |
| 5 | Hs.123530 | ACTL7A | 10881 | A | KG (III) | TS | CT |
| 7 | Hs.534390 | ACTL7B | 10880 | A | KG (III) | TS | CT |
| 9 | Hs.386283 | ADAM12 | 8038 | A- | KG (III) | PS | CT |
| 11 | Hs.177984 | ADAM20 | 8748 | C | KG (III) | TS | CT |
| 13 | Hs.126838 | ADAM29 | 11086 | A- | KG (III) | TS | no CT |
| 15 | Hs.283011 | ADAM30 | 11085 | C | KG (III) | TS | CT |
| 17 | Hs.662288 | ADAM6 | 8755 | C | NG (II) | TS | CT |
| 19 | Hs.668805 Hs.166003 | ADAM7 | 8756 | C-- | KG (III) | TS | CT |
| 21 | Hs.97633 | AKAP4 | 8852 | A | KG (III) | TS | CT |
| 23 | Hs.284255 | ALPP | 250 | A-- | KG (III) | PS | CT |
| 25 | Hs.236635 | ACTRT2 | 140625 | A- | KG (III) | TS | no CT |
| 27 | Hs.125423 | ASB17 | 127247 | A | KG (III) | TS | CT |
| 29 | Hs.352412 | ASZ1 | 136991 | C | KG (III) | TS | no CT |
| 31 | Hs.436093 Hs.123239 | BAT2 | 7916 | C-- | KG (III) | TS | CT |
| 33 | Hs.348263 | BIRC8 | 112401 | C | KG (III) | TS | no CT |
| 35 | Hs.128180 | BTG4 | 54766 | D | KG (III) | TS | CT |
| 37 | Hs.652240 | CC2D2B | 387707 | C-- | NG (II) | TS | CT |
| 39 | Hs.522955 | C10orf40 | 283025 | B | NG (II) | TS | no CT |
| 41 | Hs.662302 | C10orf62 | 414157 | B | NG (II) | TS | CT |
| 43 | Hs.121347 | C10orf82 | 143379 | A-- | NG (II) | TS | CT |
| 45 | Hs.372650 | C11orf64 | 283197 | B- | NG (II) | TS | no CT |
| 47 | Hs.653180 | C11orf65 | 160140 | B-- | NG (II) | TS | no CT |
| 49 | Hs.128574 | C12orf12 | 196477 | C | NG (II) | TS | no CT |
| 51 | Hs.97592 | C13orf28 | 122258 | B | NG (II) | TS | no CT |
| 53 | Hs.97556 | C14orf148 | 122945 | C | KG (III) | TS | no CT |
| 55 | Hs.307086 | C14orf155 | 84075 | C-- | NG (II) | TS | no CT |
| 57 | Hs.147276 | C14orf166B | 145497 | C | KG (III) | TS | no CT |
| 59 | Hs.143845 | C14orf48 | 256369 | B | NG (II) | TS | CT |
| 61 | Hs.125875 | C16orf78 | 123970 | C | NG (II) | TS | no CT |
| 63 | Hs.646574 | C17orf47 | 284083 | B- | NG (II) | TS | no CT |
| 65 | Hs.414175 | C19orf41 | 126123 | B | NG (II) | TS | no CT |
| 67 | Hs.497034 | C1orf14 | 81626 | A | NG (II) | TS | CT |
| 69 | Hs.666621 | C1orf141 | 400757 | B- | NG (II) | TS | CT |
| 71 | Hs.664180 | C20orf141 | 128653 | C | NG (II) | TS | no CT |
| 73 | Hs.447626 | FAM112A | 149699 | A- | NG (II) | TS | CT |
| 75 | Hs.360989 | C20orf71 | 128861 | A | KG (III) | TS | no CT |
| 77 | Hs.112794 | C20orf79 | 140856 | A | KG (III) | TS | CT |
| 79 | Hs.386685 | C21orf105 | 90625 | B-- | NG (II) | PS | CT |
| 81 | Hs.132104 | C2orf51 | 200523 | C | NG (II) | TS | no CT |
| 83 | Hs.632574 | C3orf30 | 152405 | B | NG (II) | TS | no CT |
| 85 | Hs.97501 | C4orf17 | 84103 | B- | NG (II) | TS | CT |
| 87 | Hs.567414 | C6orf10 | 10665 | C | KG (III) | TS | CT |
| 89 | Hs.657342 | C6orf146 | 222826 | A | NG (II) | TS | CT |
| 91 | Hs.519855 Hs.558944 | C6orf201 | 404220 | B-- | NG (II) | TS | CT |
| 93 | Hs.351816 | C7orf45 | 136263 | C- | NG (II) | TS | no CT |
| 95 | Hs.371776 | C8orf74 | 203076 | C | NG (II) | TS | CT |
| 97 | Hs.163070 | C9orf11 | 54586 | C- | NG (II) | TS | CT |
| 99 | Hs.98943 | C9orf138 | 158297 | B- | NG (II) | TS | CT |
| 101 | Hs.522093 | C9orf144 | 389715 | C | NG (II) | TS | CT |
| 103 | Hs.632073 | C9orf153 | 389766 | B- | NG (II) | TS | no CT |
| 105 | Hs.130672 | C9orf79 | 286234 | B | KG (III) | TS | no CT |
| 107 | Hs.304020 | CALR3 | 125972 | A | KG (III) | TS | no CT |
| 109 | Hs.131288 | CAPZA3 | 93661 | A | KG (III) | TS | no CT |
| 111 | Hs.24286 Hs.656595 | CCBP2 | 1238 | C-- | KG (III) | PS | CT |
| 113 | Hs.131615 | CCDC116 | 164592 | B- | NG (II) | TS | no CT |
| 115 | Hs.513635 | CCDC135 | 84229 | B- | NG (II) | TS | CT |
| 117 | Hs.376505 | CCDC79 | 283847 | B | NG (II) | TS | no CT |
| 119 | Hs.115460 | CCIN | 881 | A- | KG (III) | TS | CT |
| 121 | Hs.689473 | CDNA FLJ46080 fis, clone TESTI2004971 | | B | NG (II) | TS | no CT |
| 123 | Hs.562095 | CDY1B | 253175 | B | KG (III) | TS | CT |
| 125 | Hs.128342 | CCT8L2 | 150160 | C- | KG (III) | TS | CT |
| 127 | Hs.122511 | CETN1 | 1068 | A | KG (III) | TS | CT |
| 129 | Hs.567642 | CPA5 | 93979 | B- | KG (III) | TS | CT |
| 131 | Hs.347963 | CSH1 | 1442 | A- | KG (III) | PS | CT |
| 133 | Hs.447490 | CSHL1 | 1444 | A- | KG (III) | PS | CT |
| 135 | Hs.121602 | CST8 | 10047 | C | KG (III) | TS | CT |
| 137 | Hs.121554 | CST9L | 128821 | A | KG (III) | TS | no CT |
| 139 | Hs.444230 | CYLC1 | 1538 | C | KG (III) | TS | CT |
| 141 | Hs.511367 | CYP19A1 | 1588 | A- | KG (III) | PS | CT |
| 143 | Hs.70936 | DAZ4 | 57135 | C-- | KG (III) | TS | CT |
| 145 | Hs.131179 | DAZL | 1618 | A-- | KG (III) | TS | CT |
| 147 | Hs.591941 | DD11 | 414301 | A | KG (III) | TS | no CT |
| 149 | Hs.223581 | DDX4 | 54514 | A | KG (III) | TS | no CT |
| 151 | Hs.124211 | DEFB126 | 81623 | A | KG (III) | TS | CT |
| 153 | Hs.112087 | DEFB129 | 140881 | A | KG (III) | TS | CT |
| 155 | Hs.406265 | TMCO2 | 127391 | A-- | NG (II) | TS | CT_est |
| 157 | Hs.120369 | FNDC8 | 54752 | A- | NG (II) | TS | CT |
| 159 | Hs.120573 | CCDC70 | 83446 | A-- | KG (III) | TS | CT |
| 161 | Hs.318898 | DKFZp434K191 | 29797 | B-- | NG (II) | TS | CT |
| 163 | Hs.303923 | TKTL2 | 84076 | A- | KG (III) | TS | CT |
| 165 | Hs.558087 | DKFZp43400320 | 55542 | C | NG (II) | TS | CT |
| 167 | | FU39660 | 284992 | D | NG (II) | TS | CT |
| 169 | Hs.328458 Hs.645245 | DKFZP434P211 | 29774 | D | NG (II) | TS | CT |
| 171 | Hs.465414 | DKFZP781G0119 | 644041 | B | NG (II) | TS | no CT |
| 173 | Hs.131654 | DMRTB1 | 63948 | C | KG (III) | TS | no CT |
| 175 | Hs.350507 | DMRTC2 | 63946 | C | KG (III) | TS | no CT |
| 177 | Hs.518241 | DNAJB8 | 165721 | A | KG (III) | TS | no CT |
| 179 | Hs.116303 | DNAJC5G | 285126 | C- | KG (III) | TS | no CT |
| 181 | Hs.517326 | DNMT3L | 29947 | C | KG (III) | PS | CT |
| 183 | Hs.302028 | DPEP3 | 64180 | A- | KG (III) | TS | CT |
| 185 | Hs.653244 | EPHA6 | 285220 | C | KG (III) | TS | CT |
| 187 | Hs.525202 | FAM12B | 64184 | D | KG (III) | TS | no CT |
| 189 | Hs.127882 | FAM44C | 284257 | A | NG (II) | TS | CT |
| 191 | Hs.197801 | FAM47B | 170062 | B | NG (II) | TS | no CT |
| 193 | Hs.535216 | FAM47C | 442444 | B | KG (III) | TS | no CT |
| 195 | Hs.666099 | FAM71 B | 153745 | B | NG (II) | TS | no CT |
| 197 | Hs.591011 | FAM71 C | 196472 | B | NG (II) | TS | no CT |
| 199 | Hs.592128 | FBXW10 | 10517 | B | NG (II) | TS | CT |
| 201 | Hs.567369 | FKBP6 | 8468 | A-- | KG (III) | TS | CT |
| 203 | Hs.123515 | KIAA1822L | 79802 | D | NG (II) | TS | CT |
| 205 | Hs.24510 | C4orf26 | 152816 | B- | NG (II) | PS | no CT |
| 207 | Hs.631621 | FLJ25328 | 148231 | B-- | NG (II) | TS | no CT |
| 209 | Hs.126780 | SEPT12 | 124404 | A- | KG (III) | TS | no CT |
| 211 | Hs.44329 | FLJ32214 | 147664 | B | KG (III) | PS | no CT |
| 213 | Hs.510812 | FLJ32679 | 440321 | C | NG (II) | TS | no CT |
| 215 | Hs.121438 | CCDC42 | 146849 | B- | NG (II) | TS | no CT |
| 217 | Hs.129293 | FAM71A | 149647 | A | NG (II) | TS | CT |
| 219 | Hs.348700 | CCDC27 | 148870 | B | NG (II) | TS | no CT |
| 221 | Hs.514820 | C17orf66 | 256957 | B | KG (III) | TS | no CT |
| 223 | Hs.192090 | C1orf65 | 164127 | B- | NG (II) | TS | CT |
| 225 | Hs.112930 | C12orf50 | 160419 | B-- | NG (II) | TS | no CT |
| 227 | Hs.159650 | IQUB | 154865 | A-- | KG (III) | TS | CT |
| 229 | Hs.437141 | CCDC63 | 160762 | B- | NG (II) | TS | no CT |
| 231 | Hs.554939 Hs.349758 | FLJ35848 | 284071 | A- | NG (II) | TS | CT |
| 233 | Hs.310247 | FLJ36000 | 284124 | B | NG (II) | TS | CT |
| 235 | Hs.210377 | CCDC38 | 120935 | B | NG (II) | TS | no CT |
| 237 | Hs.381087 | FLJ40235 | 284369 | B- | NG (II) | TS | CT |
| 239 | Hs.97714 | FLJ40243 | 133558 | C-- | NG (II) | TS | CT |
| 241 | Hs.689547 | FLJ43860 | 389690 | C | NG (II) | TS | no CT |
| 243 | Hs.167595 | FLJ43944 | 400593 | B | NG (II) | PS | CT |
| 245 | Hs.105324 | FTMT | 94033 | A | KG (III) | TS | no CT |
| 247 | Hs.23632 | Full-length cDNA clone CS0DI054YF04 of Placenta Cot 25-normalized of Homo sapiens (human) | | B-- | NG (II) | PS | no CT |
| 249 | Hs.447948 | GGN | 199720 | A-- | KG (III) | TS | CT |
| 251 | Hs.98008 | GK2 | 2712 | A- | KG (III) | TS | CT |
| 253 | Hs.591738 | GMCL1L | 64396 | C- | KG (III) | TS | CT |
| 255 | Hs.155833 | H1FNT | 341567 | B- | KG (III) | TS | no CT |
| 257 | Hs.534498 | H2AFB2 | 474381 | B-- | KG (III) | TS | CT |
| 259 | Hs.376474 | H2BFM | 286436 | C | KG (III) | TS | CT |
| 261 | Hs.127778 | H2BFWT | 158983 | B | KG (III) | TS | no CT |
| 263 | Hs.631880 | hCG2040054 | 644093 | B- | NG (II) | TS | no CT |
| 265 | Hs.79363 | HIPK4 | 147746 | C-- | KG (III) | TS | CT |
| 267 | Hs.371887 | HIST1H2BA | 255626 | C | KG (III) | TS | CT |
| 269 | Hs.568628 | HMGB4 | 127540 | A | KG (III) | TS | CT |
| 271 | Hs.121605 | RBMXL2 | 27288 | C- | KG (III) | TS | CT |
| 273 | Hs.477 | HSD17B3 | 3293 | A-- | KG (III) | TS | CT |
| 275 | Hs.364941 | HSD3B1 | 3283 | A- | KG (III) | PS | CT |
| 277 | Hs.380061 | HSF5 | 124535 | B- | KG (III) | TS | CT |
| 279 | Hs.662281 | HSFY1 | 86614 | C- | KG (III) | TS | CT |
| 281 | Hs.368451 | Hypothetical FLJ40434, mRNA (cDNA clone MGC:1 49199 IMAGE:40112246) | | B- | NG (II) | TS | no CT |
| 283 | Hs.401316 | IGFBP1 | 3484 | A-- | KG (III) | PS | CT |
| 285 | Hs.144491 | IMP5 | 162540 | C | KG (III) | TS | CT |
| 287 | Hs.37062 | INSL3 | 3640 | A- | KG (III) | TS | CT |
| 289 | Hs.418506 | INSL4 | 3641 | A- | KG (III) | PS | CT |
| 291 | Hs.567095 | INSL6 | 11172 | C | KG (III) | TS | no CT |
| 293 | Hs.381854 | IQCF1 | 132141 | A | NG (II) | TS | CT |
| 295 | Hs.412294 | IQCF2 | 389123 | A | NG (II) | TS | no CT |
| 297 | Hs.254808 | IQCF3 | 401067 | B- | NG (II) | TS | CT |
| 299 | Hs.515444 | IRGC | 56269 | C | KG (III) | TS | CT |
| 301 | Hs.299127 | KCTD19 | 146212 | C- | KG (III) | TS | CT |
| 303 | Hs.115429 | KIAA1666 | 85376 | B-- | NG (II) | TS | CT |
| 305 | Hs.226805 | KIF2B | 84643 | A | KG (III) | TS | CT |
| 307 | Hs.95008 | KISS1 | 3814 | A-- | KG (III) | PS | CT |
| 309 | Hs.127510 | KLHL10 | 317719 | A- | KG (III) | TS | CT |
| 311 | Hs.662013 | KRT72 | 140807 | B- | KG (III) | TS | CT |
| 313 | Hs.307052 | LDHAL6B | 92483 | A- | KG (III) | TS | CT |
| 315 | Hs.23671 | LGALS13 | 29124 | A | KG (III) | PS | CT |
| 317 | Hs.131824 | LOC126536 | 126536 | B | NG (II) | TS | no CT |
| 319 | Hs.131469 | LOC133491 | 133491 | B- | NG (II) | TS | CT |
| 321 | Hs.407751 | DYDC1 | 143241 | A | NG (II) | TS | CT |
| 323 | Hs.125785 | LELP1 | 149018 | B | NG (II) | TS | CT |
| 325 | Hs.420716 | LOC151300 | 151300 | B | NG (II) | TS | CT |
| 327 | Hs.380704 | C17orf74 | 201243 | B | NG (II) | TS | CT |
| 329 | Hs.498620 | LOC254312 | 254312 | B- | NG (II) | TS | no CT |
| 331 | Hs.571603 | LOC259308 | 259308 | B | NG (II) | TS | no CT |
| 333 | Hs.525933 | LOC283688 | 283688 | B-- | NG (II) | TS | no CT |
| 335 | Hs.252707 | LOC284067 | 284067 | A | NG (II) | TS | no CT |
| 337 | Hs.120364 | LOC285194 | 285194 | B | NG (II) | TS | CT |
| 339 | Hs.558748 | LOC285679 | 285679 | B | NG (II) | TS | no CT |
| 341 | Hs.454277 | LOC347376 | 347376 | B | NG (II) | PS | no CT |
| 343 | Hs.314114 | LOC348021 | 348021 | B | NG (II) | TS | no CT |
| 345 | Hs.443299 | LOC374973 | 374973 | A- | NG (II) | TS | no CT |
| 347 | Hs.534660 | LOC387895 | 387895 | B- | NG (II) | TS | CT |
| 349 | Hs.28704 | KRTAP26-1 | 388818 | B | KG (III) | PS | CT |
| 351 | Hs.664098 | LOC388965 | 388965 | B | NG (II) | TS | CT |
| 353 | Hs.396178 | LOC389458 | 389458 | B- | NG (II) | TS | no CT |
| 355 | Hs.454647 | GOLGA8E | 390535 | B- | NG (II) | TS | no CT |
| 357 | Hs.599397 | LOC401351 | 401351 | C | NG (II) | TS | no CT |
| 359 | Hs.439998 | LOC440268 | 440268 | B-- | NG (II) | TS | no CT |
| 361 | Hs.643596 | LOC440896 | 440896 | B | NG (II) | TS | no CT |
| 363 | Hs.535004 | LOC441009 | 441009 | B- | NG (II) | TS | no CT |
| 365 | Hs.652956 | FAM75C1 | 441452 | B | NG (II) | TS | CT |
| 367 | Hs.431161 | LOC643406 | 643406 | B | NG (II) | TS | no CT |
| 369 | Hs.369462 | LOC644753 | 644753 | B-- | NG (II) | TS | CT |
| 371 | Hs.116176 | LOC645733 | 645733 | B-- | NG (II) | TS | CT |
| 373 | Hs.434366 | LOC728012 | 728012 | C | NG (II) | TS | no CT |
| 375 | Hs.127963 | LOC728460 | 728460 | B- | NG (II) | TS | CT |
| 377 | Hs.404175 | LOC728883 | 728883 | B- | NG (II) | TS | no CT |
| 379 | Hs.629498 | LOC729461 | 729461 | B | NG (II) | TS | no CT |
| 381 | Hs.345877 | LOXHD1 | 125336 | C-- | NG (II) | TS | CT_est |
| 383 | Hs.558572 | LYZL1 | 84569 | B- | KG (III) | TS | no CT |
| 385 | Hs.97477 | LYZL6 | 57151 | A-- | KG (III) | TS | CT |
| 387 | Hs.673877 | MGC26356 | 642280 | B-- | NG (II) | TS | CT |
| 389 | Hs.112877 | MGC26647 | 219557 | A | NG (II) | TS | CT |
| 391 | Hs.209206 | MGC33407 | 284382 | A | KG (III) | TS | no CT |
| 393 | Hs.307999 | EXDL1 | 161829 | B-- | KG (III) | TS | CT |
| 395 | Hs.567701 | C15orf43 | 145645 | C | NG (II) | TS | no CT |
| 397 | Hs.178210 | C3orf22 | 152065 | C | NG (II) | TS | no CT |
| 399 | Hs.567774 | CCDC83 | 220047 | C | NG (II) | TS | no CT |
| 401 | Hs.98202 | C12orf54 | 121273 | B | NG (II) | TS | CT |
| 403 | Hs.179646 | TMCO5 | 145942 | B-- | NG (II) | TS | CT |
| 405 | Hs.98104 | C2orf57 | 165100 | B | NG (II) | TS | no CT |
| 407 | Hs.124502 | C22orf33 | 339669 | A | NG (II) | TS | CT |
| 409 | Hs.147128 | C3orf44 | 131831 | B- | NG (II) | TS | no CT |
| 411 | Hs.98660 | LYPD4 | 147719 | B- | KG (III) | TS | CT |
| 413 | Hs.136555 | C2orf53 | 339779 | A- | NG (II) | TS | no CT |
| 415 | Hs.356069 | XKR3 | 150165 | C | KG (III) | TS | no CT |
| 417 | Hs.178066 | MS4A5 | 64232 | A | KG (III) | TS | CT |
| 419 | Hs.662211 | MS4A6E | 245802 | B | KG (III) | TS | CT |
| 421 | Hs.398196 | MYCBPAP | 84073 | C- | KG (III) | TS | CT |
| 423 | Hs.144075 | LOC340286 | 340286 | A- | NG (II) | TS | no CT |
| 425 | Hs.511849 | NBPF4 | 148545 | B | NG (II) | TS | CT |
| 427 | Hs.120319 | NT5C1B | 93034 | A | KG (III) | TS | CT |
| 429 | Hs.67639 | NUP210L | 91181 | C- | KG (III) | TS | CT |
| 431 | Hs.525769 | NUT | 256646 | C | KG (III) | TS | CT |
| 433 | Hs.164799 | CCDC54 | 84692 | B- | KG (III) | TS | no CT |
| 435 | Hs.131098 | FAM71F1 | 84691 | A- | NG (II) | TS | CT |
| 437 | Hs.351068 | SPATA22 | 84690 | A | NG (II) | TS | CT |
| 439 | Hs.351815 | TPD52L3 | 89882 | A- | KG (III) | TS | CT |
| 441 | Hs.120316 | C4orf35 | 85438 | A | NG (II) | TS | no CT |
| 443 | Hs.434715 | OR2H1 | 26716 | C | KG (III) | TS | CT |
| 445 | Hs.568153 | OR7E104P | 81137 | B | NG (II) | TS | CT |
| 447 | Hs.472491 | OXCT2 | 64064 | A-- | KG (III) | TS | CT |
| 449 | Hs.487409 | PAPOLB | 56903 | C- | KG (III) | TS | CT |
| 451 | Hs.494928 | PAPPA | 5069 | A-- | KG (III) | PS | CT |
| 453 | Hs.131361 | PDHA2 | 5161 | A | KG (III) | TS | CT |
| 455 | Hs.367727 | PGK2 | 5232 | A- | KG (III) | TS | CT |
| 457 | Hs.372719 Hs.693655 | PHF7 | 51533 | A-- | KG (III) | TS | CT |
| 459 | Hs.405659 | PIWIL1 | 9271 | A-- | KG (III) | TS | CT |
| 461 | Hs.132310 | PLAC1L | 219990 | B | NG (II) | TS | CT |
| 463 | Hs.472492 | PLAC4 | 191585 | A- | NG (II) | PS | CT |
| 465 | Hs.97542 | PLCZ1 | 89869 | A | KG (III) | TS | no CT |
| 467 | Hs.147305 | PLSCR2 | 57047 | C-- | KG (III) | TS | no CT |
| 469 | Hs.151167 | PPP3R2 | 5535 | A | KG (III) | TS | CT |
| 471 | Hs.512633 | PRG2 | 5553 | A- | KG (III) | PS | CT |
| 473 | Hs.2909 | PRM1 | 5619 | A | KG (III) | TS | CT |
| 475 | Hs.259636 | PRM3 | 58531 - | D | KG (III) | TS | CT |
| 477 | Hs.169284 | PRPS1L1 | 221823 | A | KG (III) | TS | CT |
| 479 | Hs.466843 | PSG1 | 5669 | A- | KG (III) | PS | CT |
| 481 | Hs.502097 | PSG11 | 5680 | A- | KG (III) | PS | CT |
| 483 | Hs.466854 | PSG2 | 5670 | A- | KG (III) | PS | CT |
| 485 | Hs.555887 | PSG3 | 5671 | A | KG (III) | PS | CT |
| 487 | Hs.466860 | PSG4 | 5672 | A- | KG (III) | PS | CT |
| 489 | Hs.558372 | PSG5 | 5673 | A-- | KG (III) | PS | CT |
| 491 | Hs.466849 | PSG6 | 5675 | A- | KG (III) | PS | CT |
| 493 | Hs.512647 | PSG7 | 5676 | A- | KG (III) | PS | CT |
| 495 | Hs.502092 | PSG9 | 5678 | A- | KG (III) | PS | CT |
| 497 | Hs.97124 | HDGFL1 | 154150 | C- | NG (II) | TS | CT |
| 499 | Hs.121200 | RGSL1 | 353299 | B | KG (III) | TS | no CT |
| 501 | Hs.112761 | RNASE11 | 122651 | A | KG (III) | TS | CT |
| 503 | Hs.567756 Hs.126730 | RNF133 | 168433 | A | KG (III) | TS | CT |
| 505 | Hs.99354 | RNF151 | 146310 | A | KG (III) | TS | no CT |
| 507 | Hs.97464 | RNF17 | 56163 | C- | KG (III) | TS | CT |
| 509 | Hs.665663 | MARCH10 | 162333 | B- | KG (III) | TS | no CT |
| 511 | Hs.567516 | ROPN1 | 54763 | A-- | KG (III) | TS | CT |
| 513 | Hs.528203 | ROPN1B | 152015 | A-- | KG (III) | TS | CT_est |
| 515 | Hs.308332 | RPL10L | 140801 | A | KG (III) | TS | CT |
| 517 | Hs.146544 | RSHL1 | 81492 | C | NG (II) | TS | CT |
| 519 | Hs.320892 | SAC | 55811 | C- | KG (III) | TS | CT |
| 521 | Hs.397255 | SIGLEC6 | 946 | C- | KG (III) | PS | CT |
| 523 | Hs.220844 | SLC22A11 | 55867 | A-- | KG (III) | PS | CT |
| 525 | Hs.655169 | SLC2A3 | 6515 | C- | KG (III) | TS | CT |
| 527 | Hs.132482 | SLC36A3 | 285641 | C- | NG (II) | TS | no CT |
| 529 | Hs.680112 | SLC9A10 | 285335 | B-- | KG (III) | TS | no CT |
| 531 | Hs.348526 | SORCS3 | 22986 | C | KG (III) | TS | CT |
| 533 | Hs.529462 | SOX30 | 11063 | C- | KG (III) | TS | CT |
| 535 | Hs.161241 | SPACA1 | 81833 | C | KG (III) | TS | CT |
| 537 | Hs.122599 | SPACA4 | 171169 | A | KG (III) | TS | CT |
| 539 | Hs.375186 | SPAG4L | 140732 | C | KG (III) | TS | CT |
| 541 | Hs.121494 | SPAM1 | 6677 | C- | KG (III) | TS | CT |
| 543 | Hs.97541 | SPATA19 | 219938 | B | KG (III) | TS | no CT |
| 545 | Hs.129794 | SPATA12 | 353324 | B- | KG (III) | TS | no CT |
| 547 | Hs.444236 | SPATA16 | 83893 | A-- | KG (III) | TS | CT |
| 549 | Hs.481235 | SPATA4 | 132851 | A- | KG (III) | TS | CT |
| 551 | Hs.326528 | SPATA8 | 145946 | A | NG (II) | TS | CT |
| 553 | Hs.97726 | SPATC1 | 375686 | C | KG (III) | TS | CT |
| 555 | Hs.186363 | SPERT | 220082 | B | KG (III) | TS | CT |
| 557 | Hs.370724 | SPINT1 | 6692 | A | KG (III) | TS | CT |
| 559 | Hs.519403 | SPZ1 | 84654 | C | KG (III) | TS | no CT |
| 561 | Hs.309767 | STK31 | 56164 | A-- | KG (III) | TS | CT |
| 563 | Hs.223806 | TAF7L | 54457 | A- | KG (III) | TS | CT |
| 565 | Hs.124512 | TBC1D21 | 161514 | C | KG (III) | TS | CT |
| 567 | Hs.448884 | TBC1D3P2 | 440452 | B- | NG (II) | TS | no CT |
| 569 | Hs.375035 | TCEB3B | 51224 | C | KG (III) | TS | CT |
| 571 | Hs.435371 | TCP11 | 6954 | A- | KG (III) | TS | CT |
| 573 | Hs.414648 | TEKT3 | 64518 | A-- | KG (III) | TS | CT |
| 575 | Hs.143519 | TEKT5 | 146279 | B-- | KG (III) | TS | CT_est |
| 577 | Hs.518957 | ADAD1 | 132612 | A- | KG (III) | TS | CT |
| 579 | Hs.524039 | TEX12 | 56158 | C-- | NG (II) | TS | no CT |
| 581 | Hs.567543 | TEX13A | 56157 | D | KG (III) | TS | CT |
| 583 | Hs.29742 | THSD3 | 145501 | B- | KG (III) | PS | CT |
| 585 | Hs.102866 | TKTL1 | 8277 | A- | KG (III) | TS | CT |
| 587 | Hs.128069 | TMEM162 | 148109 | C- | KG (III) | TS | no CT |
| 589 | Hs.98843 | TMEM31 | 203562 | B | KG (III) | TS | no CT |
| 591 | Hs.3017 | TNP1 | 7141 | A- | KG (III) | TS | CT |
| 593 | Hs.513349 | TNP2 | 7142 | A | KG (III) | TS | CT |
| 595 | Hs.120831 | C16orf82 | 162083 | B | NG (II) | TS | no CT |
| 597 | Hs.509439 | TRIM40 | 135644 | B-- | KG (III) | PS | CT |
| 599 | Hs.350671 | TSGA10IP | 254187 | C | NG (II) | TS | no CT |
| 601 | Hs.592266 | TSGA13 | 114960 | C | NG (II) | TS | no CT |
| 603 | Hs.515858 | TSKS | 60385 | A- | KG (III) | TS | CT |
| 605 | Hs.579784 | TSPAN16 | 26526 | B- | KG (III) | TS | no CT |
| 607 | Hs.620508 | TSPYL6 | 388951 | C- | KG (III) | TS | no CT |
| 609 | Hs.519507 | TSSK1B | 83942 | A | NG (II) | TS | no CT |
| 611 | Hs.520554 | TTLL2 | 83887 | C | KG (III) | TS | CT |
| 613 | Hs.349695 | TUBA3C | 7278 | A-- | KG (III) | TS | CT |
| 615 | Hs.433336 | TUBA3E | 112714 | B | KG (III) | TS | no CT |
| 617 | Hs.98712 | TXNDC2 | 84203 | C | KG (III) | TS | CT |
| 619 | Hs.189184 | UBQLN3 | 50613 | A | KG (III) | TS | no CT |
| 621 | Hs.591394 | UNQ9391 | 203074 | C | KG (III) | TS | no CT |
| 623 | Hs.170076 Hs.567386 | VCY | 9084 | C | NG (II) | TS | CT |
| 625 | Hs.647026 | WBSCR28 | 135886 | C | NG (II) | TS | no CT |
| 627 | Hs.213307 | WDR21B | 285429 | B | NG (II) | TS | CT |
| 629 | Hs.211046 | WDR88 | 126248 | B- | KG (III) | TS | CT |
| 631 | Hs.116128 | WFDC8 | 90199 | C | KG (III) | TS | CT |
| 633 | Hs.126496 | ZNRF4 | 148066 | C | KG (III) | TS | CT |
| 635 | Hs.388841 | ZPBP | 11055 | A-- | KG (III) | TS | CT |
| 637 | Hs.367245 | ZPBP2 | 124626 | A | KG (III) | TS | CT |
| 639 | Hs.375054 | ZSWIM2 | 151112 | C | KG (III) | TS | no CT |
| 641 | Hs.98848 | ADAM3A | 1587 | C | NG (II) | TS | no CT |
| 642 | Hs.662209 | BC038740 | 415056 | B- | NG (II) | TS | CT |
| 643 | Hs.406982 | BEYLA | 497634 | B | NG (II) | TS | no CT |
| 644 | Hs.190267 | CDNA clone IMAGE:30378154 | | B- | NG (II) | TS | CT |
| 645 | Hs.449459 | CDNA clone IMAGE:40146443 | | B- | NG (II) | TS | CT_est |
| 646 | Hs.247868 | CDNA clone IMAGE:4822062 | | C | NG (II) | TS | no CT |
| 647 | Hs.456065 | CDNA clone IMAGE:4822139 | | B-- | NG (II) | TS | CT |
| 648 | Hs.568804 | CDNA clone IMAGE:4822215 | | B | NG (II) | TS | no CT |
| 649 | Hs.405628 | CDNA clone IMAGE:4823383 | | B | NG (II) | TS | CT |
| 650 | Hs.125998 | CDNA clone IMAGE:4826738 | | B | NG (II) | TS | no CT |
| 651 | Hs.350698 | CDNA clone IMAGE:4827146 | | B | NG (II) | TS | no CT |
| 652 | Hs.547104 | CDNA clone IMAGE:4828503 | | B- | NG (II) | TS | CT |
| 653 | Hs.122199 | CDNA clone IMAGE:4829423 | | B- | NG (II) | TS | CT |
| 654 | Hs.662260 | CDNA clone IMAGE:4831354 | | B | NG (II) | TS | no CT |
| 655 | Hs.574781 | CDNA clone IMAGE:5000386, containing frame-shift errors | | B- | NG (II) | PS | CT |
| 656 | Hs.160063 | CDNA clone IMAGE:5267179 | | C | NG (II) | TS | CT |
| 657 | Hs.97712 | CDNA clone IMAGE:5269981 | | B | NG (II) | TS | CT |
| 658 | Hs.662373 | CDNA clone IMAGE:5270126 | | B | NG (II) | TS | no CT |
| 659 | Hs.662213 | CDNA clone IMAGE:5271096 | | B | NG (II) | TS | no CT |
| 660 | Hs.409982 | CDNA clone IMAGE:5272174 | | B | NG (II) | TS | no CT |
| 661 | Hs.532159 | CDNA clone IMAGE:5295011 | | B-- | NG (II) | TS | CT |
| 662 | Hs.662207 | CDNA clone IMAGE:5295305 | | B | NG (II) | TS | no CT |
| 663 | Hs.662309 | CDNA clone IMAGE:5295621 | | B | NG (II) | TS | no CT |
| 664 | Hs.562766 | CDNA clone IMAGE:5296106 | | B- | NG (II) | TS | CT |
| 665 | Hs.369473 | CDNA clone IMAGE:5296164 | | B- | NG (II) | TS | CT |
| 666 | Hs.519941 | CDNA clone IMAGE:5296266 | | B-- | NG (II) | TS | no CT |
| 667 | Hs.662423 | CDNA clone IMAGE:5296329 | | B | NG (11) | TS | no CT |
| 668 | Hs.156197 | CDNA clone IMAGE:5296720 | | B-- | NG (II) | TS | no CT |
| 669 | Hs.606987 | CDNA clone IMAGE:5296886 | | B | NG (II) | TS | no CT |
| 670 | Hs.655296 | CDNA clone IMAGE:5299000 | | B- | NG (II) | TS | no CT |
| 671 | Hs.536920 | CDNA FLJ11322 fis, clone PLACE1010329 | | B | NG (II) | PS | CT |
| 672 | Hs.535454 | CDNA FLJ25810 fis, clone TST07303 | | B | NG (II) | TS | CT |
| 673 | Hs.112680 | CDNA FLJ32757 fis, clone TEST12001766 | | B | NG (II) | TS | CT |
| 674 | Hs.149004 | CDNA FLJ32805 fis, clone TESTI2002690 | | B- | NG (II) | TS | CT |
| 675 | Hs.253209 | CDNA FLJ35886 fis, clone TESTI2009091 | | B | NG (II) | TS | CT |
| 676 | Hs.662304 | CDNA FLJ35959 fis, clone TESTI2012444 | | B | NG (II) | TS | no CT |
| 677 | Hs.177930 | CDNA FLJ35971fis, clone TESTI2013257 | | B- | NG (II) | TS | CT |
| 678 | Hs.637057 | CDNA FLJ36135 fis, clone TESTI2024718 | | B- | NG (II) | TS | no CT |
| 679 | Hs.664588 | CDNA FLJ40222 fis, clone TESTI2021785 | | B | NG (II) | TS | no CT |
| 680 | Hs.666581 | CDNA FLJ40460 fis, clone TESTI2041947 | | B- | NG (II) | TS | no CT |
| 681 | Hs.220558 | CDNA FLJ40468 fis, clone TESTI2042400 | | B- | NG (II) | TS | CT_est |
| 682 | Hs.551951 | CDNA FLJ43814 fis, clone TESTI4001473 | | B-- | NG (II) | TS | no CT |
| 683 | Hs.656557 | CDNA FU43896 fis, clone TESTI4009752 | | B-- | NG (II) | TS | no CT |
| 684 | Hs.560509 | Full length insert cDNA clone YA77F06 | | B | NG (II) | PS | CT |
| 685 | Hs.572445 | Full-length cDNA clone CS0DI008YP18 of Placenta Cot 25-normalized of Homo sapiens (human) | | B | NG (II) | PS | no CT |
| 686 | Hs.657576 | Homo sapiens, clone IMAGE:4991280, mRNA (AK056728) | | A | NG (II) | PS | no CT |
| 687 | Hs.98945 | Homo sapiens, clone IMAGE:5744200, mRNA | | B | NG (II) | TS | no CT |
| 688 | Hs.112651 | LOC148145 | 148145 | B-- | NG (II) | TS | CT |
| 689 | Hs.159043 | LOC151658 | 151658 | B | NG (II) | TS | no CT |
| 690 | Hs.400007 | LOC164380 | 164380 | B | NG (II) | TS | no CT |
| 691 | Hs.667492 | LOC253805 | 253805 | B- | NG (II) | TS | CT |
| 692 | Hs.119207 | LOC283079 | 283079 | C | NG (II) | TS | CT |
| 693 | Hs.99161 | LOC285827 | 285827 | B | NG (II) | TS | no CT |
| 694 | Hs.97408 | LOC338864 | 338864 | B | NG (II) | TS | no CT |
| 695 | Hs.478050 | LOC339894 | 339894 | B | NG (II) | TS | CT |
| 696 | Hs.343631 | LOC340178 | 340178 | B | NG (II) | TS | no CT |
| 697 | Hs.591823 | LOC340239 | 340239 | B | NG (II) | TS | CT |
| 698 | Hs.404807 | LOC348761 | 348761 | B-- | NG (II) | TS | no CT |
| 699 | Hs.92520 | LOC388414 | 388414 | B-- | NG (II) | PS | no CT |
| 700 | Hs.666207 | LOC402779 | 402779 | B | NG (II) | TS | CT |
| 701 | Hs.144683 | LOC441601 | 441601 | A- | KG (III) | TS | no CT |
| 702 | Hs.446327 | LOC494141 | 494141 | B | NG (II) | TS | no CT |
| 703 | Hs.130722 | LOC494558 | 494558 | B | NG (II) | TS | no CT |
| 704 | Hs.662663 | LOC613126 | 613126 | B | NG (II) | TS | CT |
| 705 | Hs.680123 | LOC728099 | 728099 | B- | NG (II) | TS | CT |
| 706 | Hs.535746 | LOC728186 | 728186 | B | NG (II) | TS | CT |
| 707 | Hs.648270 | LOC728451 | 728451 | B | NG (II) | TS | no CT |
| 708 | Hs.648235 | LOC729266 | 729266 | B | NG (II) | TS | no CT |
| 709 | Hs.652158 | LOC729668 | 729668 | B- | NG (II) | TS | no CT |
| 710 | Hs.587755 | LOC780529 | 780529 | B | NG (II) | TS | CT |
| 711 | Hs.552781 | LOC90520 | 90520 | B- | NG (II) | TS | no CT |
| 712 | Hs.585529 (from | MRNA; CDNA DKFZp434F1626 clone DKFZp434F1626) | | B- | NG (II) | TS | CT |
| 713 | Hs.116324 | MRNA; CDNA DKFZp434P0626 (from clone DKFZp434P0626) | | B | NG (II) | TS | no CT |
| 714 | Hs.531306 | MRNA; cDNA DKFZp686I1532 (from clone DKFZp686I1532) | | C | NG (II) | TS | CT |
| 715 | Hs.699840 | MRNA; cDNA DKFZp781G0123 (from clone DKFZp781G0123) | | B | NG (II) | TS | no CT |
| 716 | Hs.528847 | MYADML | 151325 | C | KG (III) | TS | no CT |
| 717 | Hs.24611 | BCAR4 | 400500 | B- | NG (II) | PS | CT |
| 718 | Hs.278513 | LOC390705 | 390705 | B | NG (II) | TS | CT |
| 719 | Hs.529729 | This record was discontinued on 06-Mar-2006. | 401464 | B- | NG (II) | PS | CT |
| 720 | Hs.363054 | OR7E156P | 283491 | A | NG (II) | TS | CT |
| 721 | Hs.127689 | PPP1R2P9 | 80316 | A | KG (III) | TS | no CT |
| 722 | Hs.274285 | RFPL3S | 10737 | A- | NG (II) | TS | CT |
| 723 | Hs.121021 | SPATA3 | 130560 | B- | KG (III) | TS | no CT |
| 724 | Hs.112596 | Transcribed locus | | C | NG (II) | TS | CT |
| 725 | Hs.112804 | Transcribed locus | | B | NG (II) | TS | CT |
| 726 | Hs.112924 | Transcribed locus | | B | NG (II) | PS | no CT |
| 727 | Hs.121392 | Transcribed locus | | B | NG (II) | TS | no CT |
| 728 | Hs.125251 | Transcribed locus | | B- | NG (II) | TS | CT |
| 729 | Hs.126866 | Transcribed locus | | B- | NG (II) | TS | CT |
| 730 | Hs.146614 | Transcribed locus | | B | NG (II) | TS | no CT |
| 731 | Hs.178185 | Transcribed locus | | B | NG (II) | TS | no CT |
| 732 | Hs.179715 | Transcribed locus | | B- | NG (II) | TS | no CT |
| 733 | Hs.259426 | Transcribed locus | | B | NG (II) | TS | no CT |
| 734 | Hs.42265 | Transcribed locus | | B | NG (II) | PS | CT |
| 735 | Hs.446169 | Transcribed locus | | B | NG (II) | TS | no CT |
| 736 | Hs.468280 | Transcribed locus | | B | NG (II) | TS | CT |
| 737 | Hs.507924 | Transcribed locus | | B | NG (II) | TS | CT |
| 738 | Hs.517091 | Transcribed locus | | B- | NG (II) | TS | CT |
| 739 | Hs.534101 | Transcribed locus | | B | NG (II) | TS | no CT |
| 740 | Hs.568772 | Transcribed locus | | B | NG (II) | TS | no CT |
| 741 | Hs.570056 | Transcribed locus | | B- | NG (II) | TS | no CT |
| 742 | Hs.571481 | Transcribed locus | | B | NG (II) | TS | no CT |
| 743 | Hs.585699 | Transcribed locus | | B- | NG (II) | TS | no CT |
| 744 | Hs.599607 | Transcribed locus | | B | NG (II) | TS | no CT |
| 745 | Hs.632775 | Transcribed locus | | B- | NG (II) | TS | no CT |
| 746 | Hs.652566 | Transcribed locus | | B | NG (II) | TS | no CT |
| 747 | Hs.662336 | Transcribed locus | | B | NG (II) | TS | no CT |
| 748 | Hs.664960 | Transcribed locus | | B | NG (II) | TS | no CT |
| 749 | Hs.666095 | Transcribed locus | | B- | NG (II) | TS | no CT |
| 750 | Hs.694285 | Transcribed locus | | B- | NG (II) | TS | no CT |
| 751 | Hs.86761 | Transcribed locus | 390009 | B | NG (II) | PS | no CT |
| 752 | Hs.98394 | Transcribed locus | | B | NG (II) | TS | no CT |
| 753 | Hs.650196 | Transcribed locus, strongly similar to XP_001126757.1 | | B- | NG (II) | TS | no CT |
| 754 | Hs.632585 | Transcribed locus, weakly similar to XP_001101794.1 | | B- | NG (II) | TS | no CT |

### Expression of testis- or placenta- specific genes in somatic cancers

In order to extend this observation to other somatic cancers, we then took advantage of the cancer transcriptomic data and search engine available on the oncomine website.
The cancer profiling database Oncomine (http://www.oncomine.org/main/index.jsp) (Rhodes et al. 2007; Rhodes et al. 2004) combines data from more than 20,000 cancer transcriptome profiles with an analysis engine and web application for data mining and visualization.

For each of the 467 testis-specific and placenta-specific genes listed above, we searched the Oncomine database for an overexpression in tumour versus normal tissue, with a p<0.001.

Thirty five of the original 467 testis- or placenta- specific genes were absent from the Oncomine database. Of the remaining 432 TS-PS- genes, this analysis revealed that 250 (58%) were found aberrantly expressed in tumours in at least one of the transcriptomic studies recorded in Oncomine. Some of these genes were aberrantly expressed in studies comparing samples of a somatic cancer versus samples of its normal tissue counterpart (ECN: 157 genes), whereas others were overexpressed in studies comparing cancer samples with other cancer samples (CC: 93 genes). Moreover, every single cancer type recorded in Oncomine expressed a sub-set of these genes. A total of 182 TS-PS- genes were not found expressed in any of the recorded cancer studies recorded in oncomine despite being tested (NEC).

The transcriptomic data in Oncomine therefore shows that more than half of the testis specific and placenta specific genes are illegitimately expressed in at least one somatic cancer type, and that each cancer type is associated with the deregulation of a subset of TS-PS genes.
Expression of TS and PS genes in cancer, in sillico approach
This in silico approach has enabled the Inventors to identify 250 CT genes (TS and PS genes deregulated in cancers), most of which had not been previously described as CTs.

The following table 5 illustrates the result data. Table 5 describes the expression of the testis- and placenta- specific genes of the list (the numbers of the corresponding sequences SEQ ID are displayed in the left column) in a meta analysis of the studies recorded in Oncomine comparing transcriptomic data of somatic or ovarian tumours with the corresponding normal somatic tissue samples (the 27 columns are labelled according to the type of cancerous tissue - the values indicate the number of studies where each gene was found significantly overexpressed in the tumor samples compared to the normal corresponding tissue with p<0.001. Oncomime column represents gene expression in studies recorded in Oncomine : illegitimate expression in studies comparing cancer samples versus their somatic counterpart (ECN) (also illustrated in figures 1a, 1b and 1c), or comparing cancer samples with other cancer samples (CC), or not found expressed in any of the Oncomine studies described above (NEC), or not recorded in Oncomine (NA).

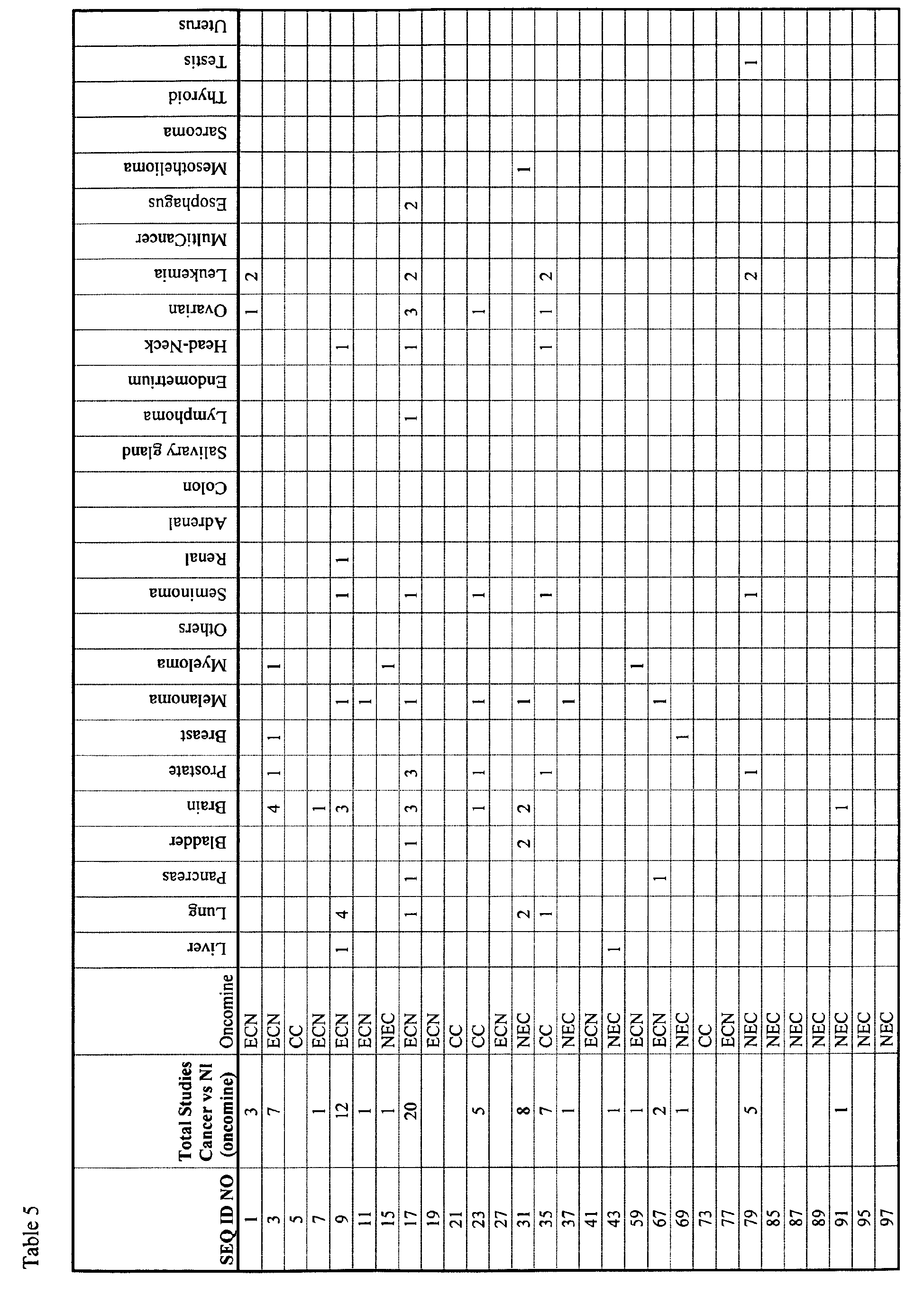

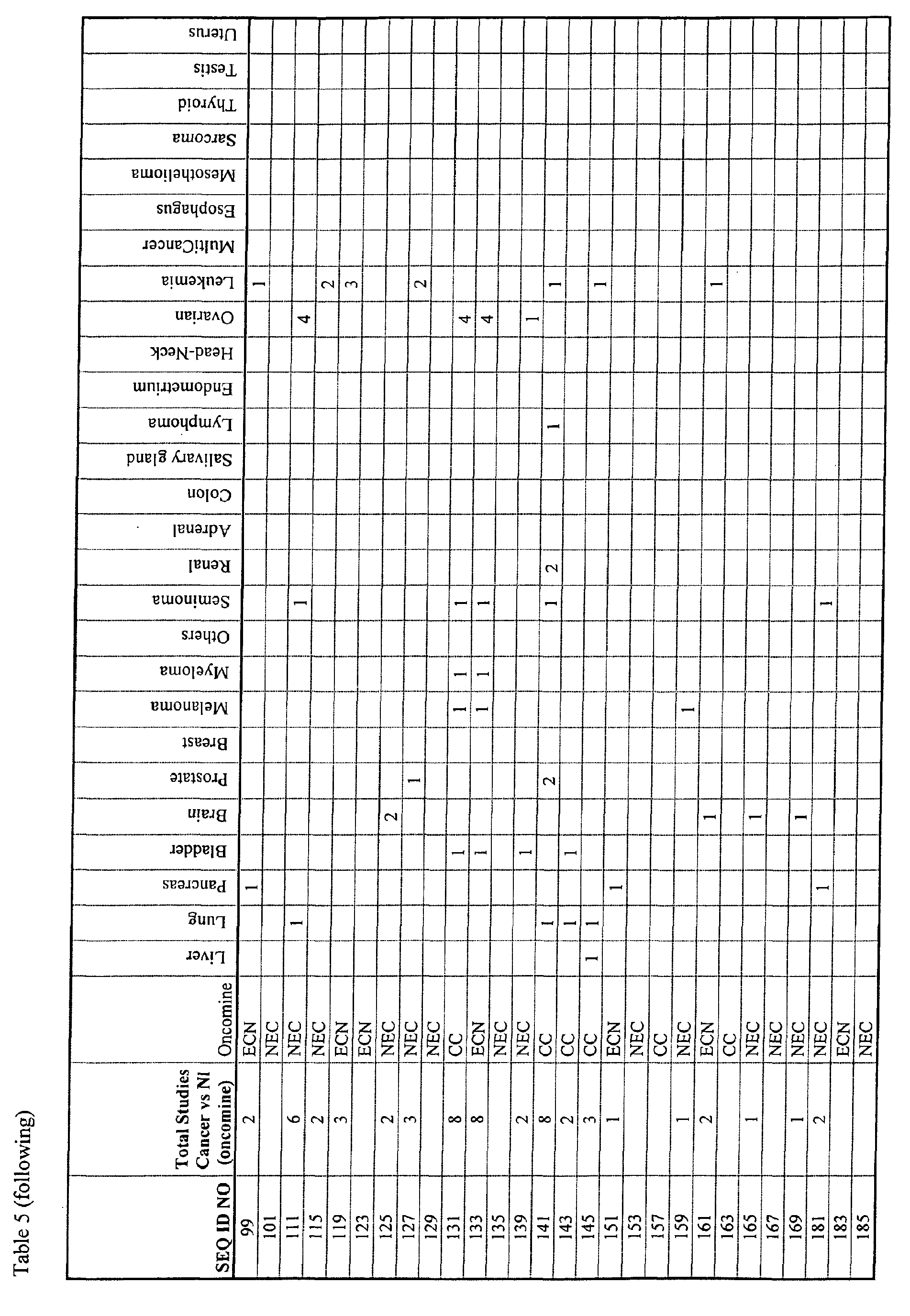

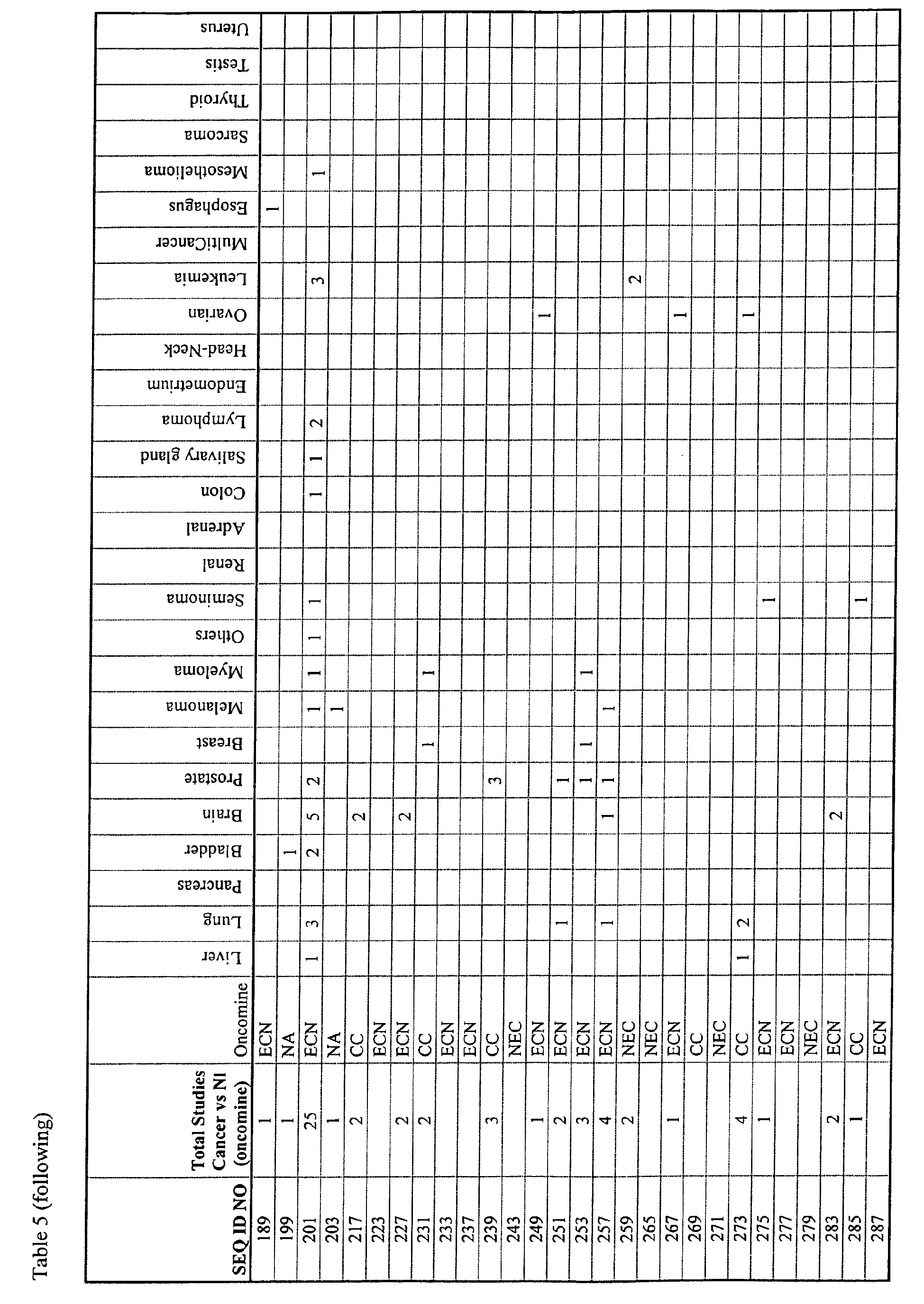

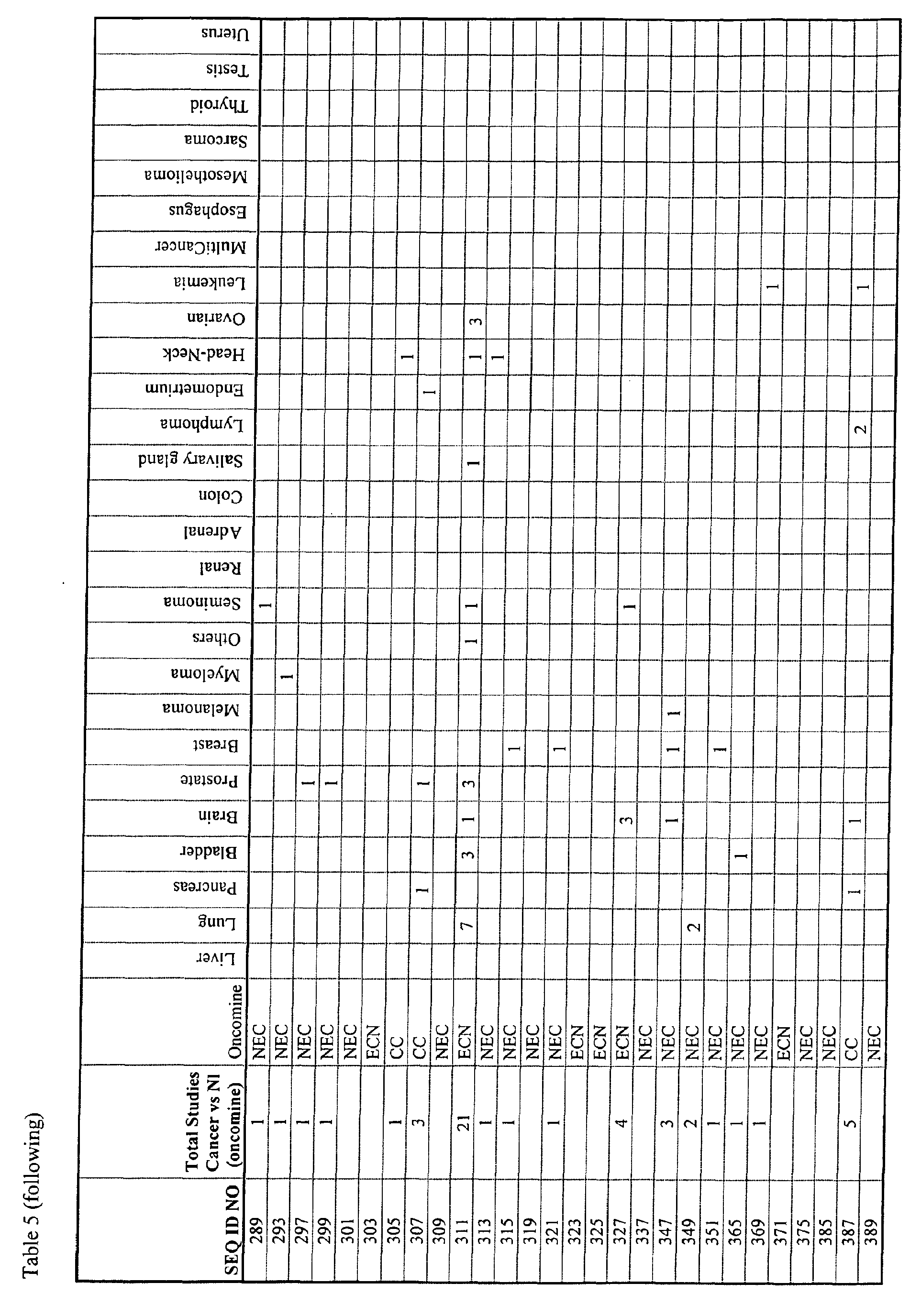

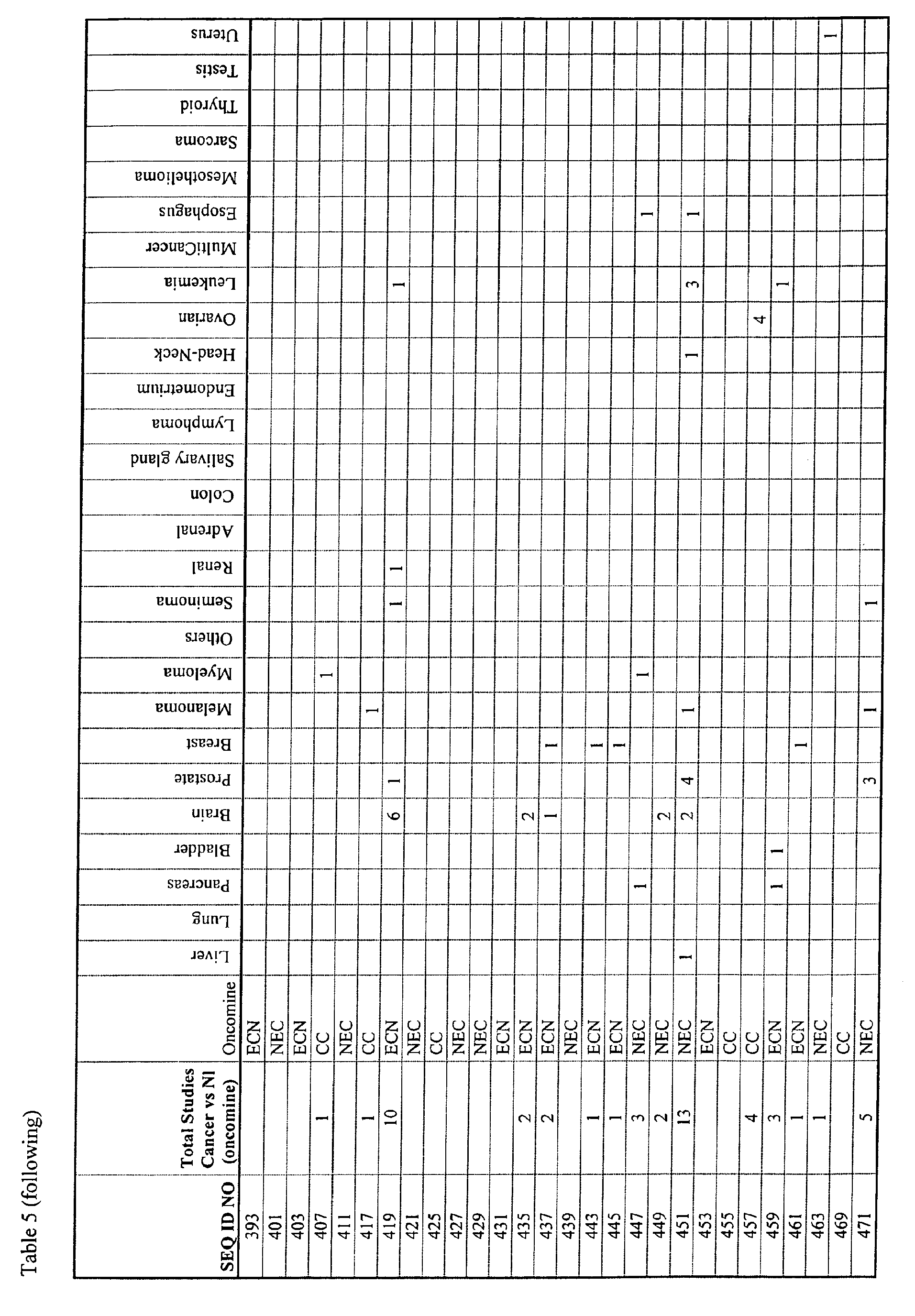

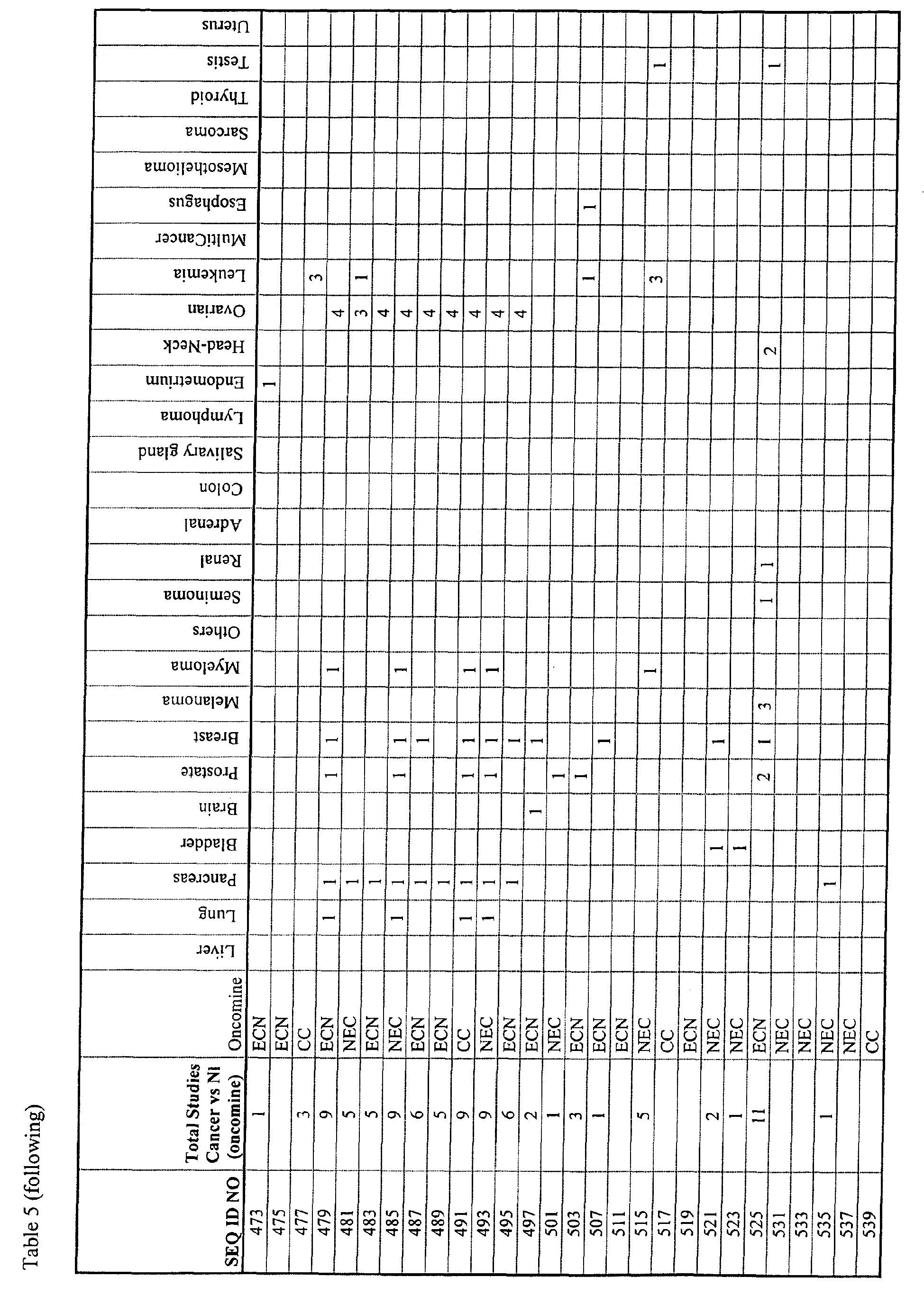

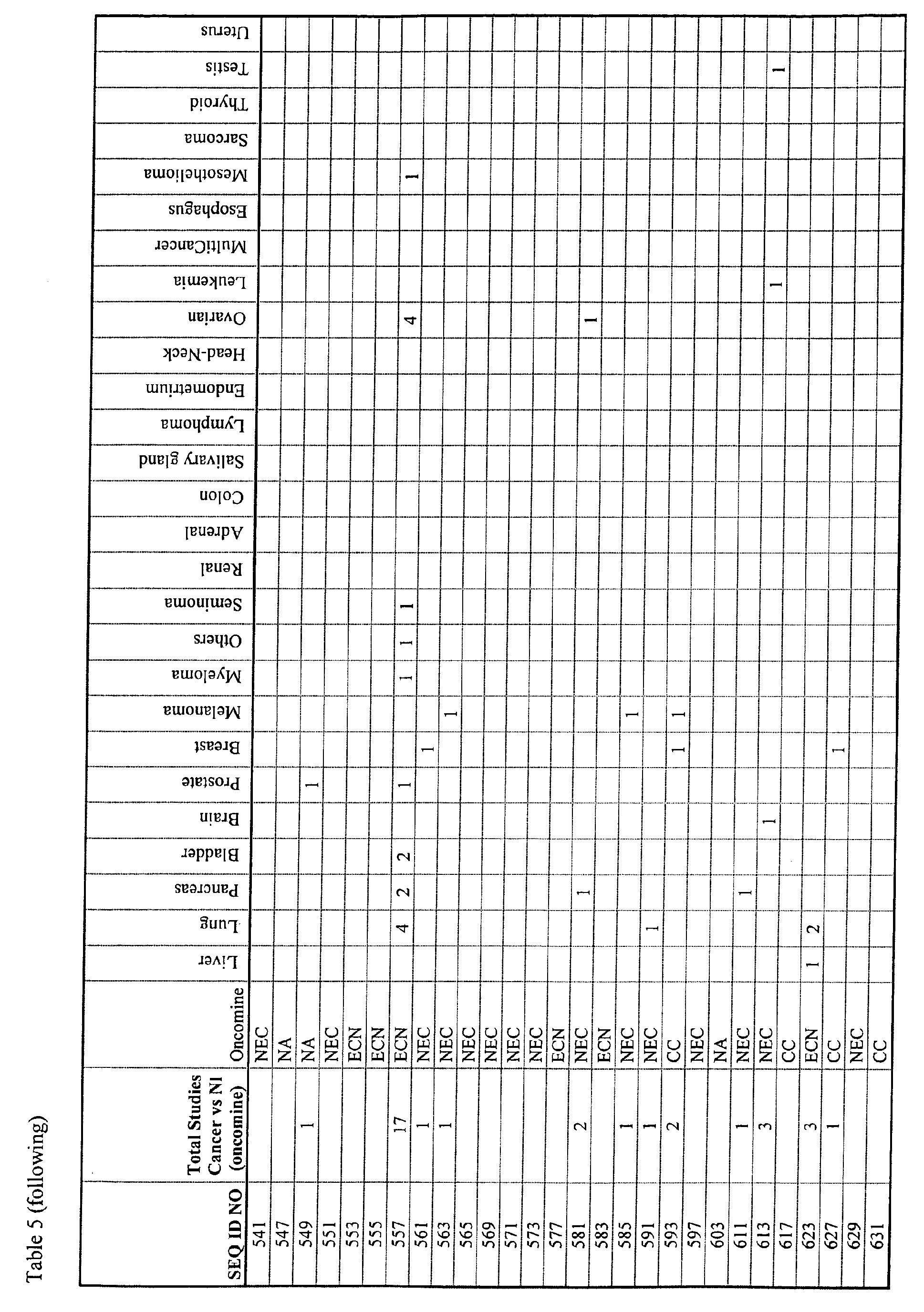

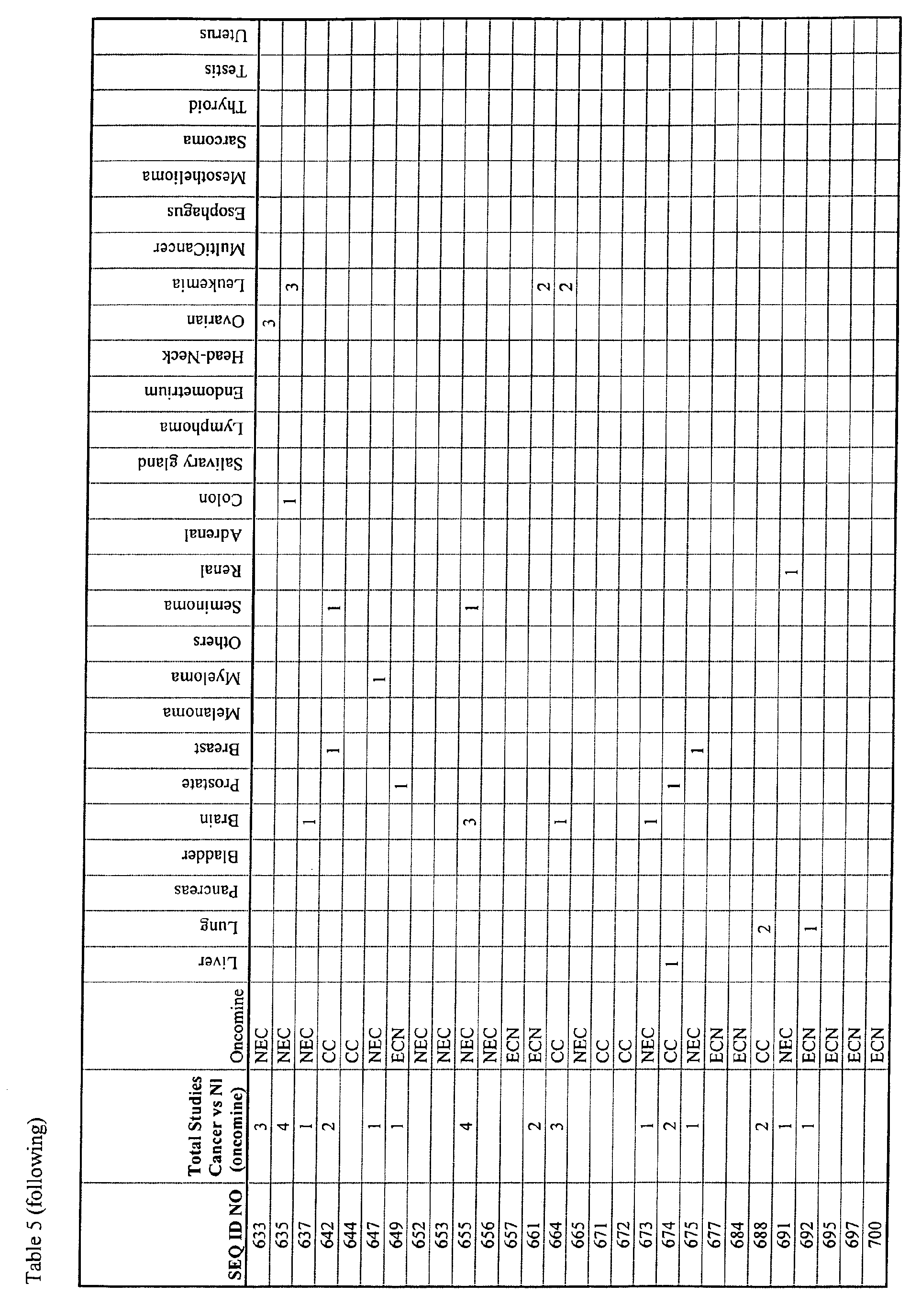

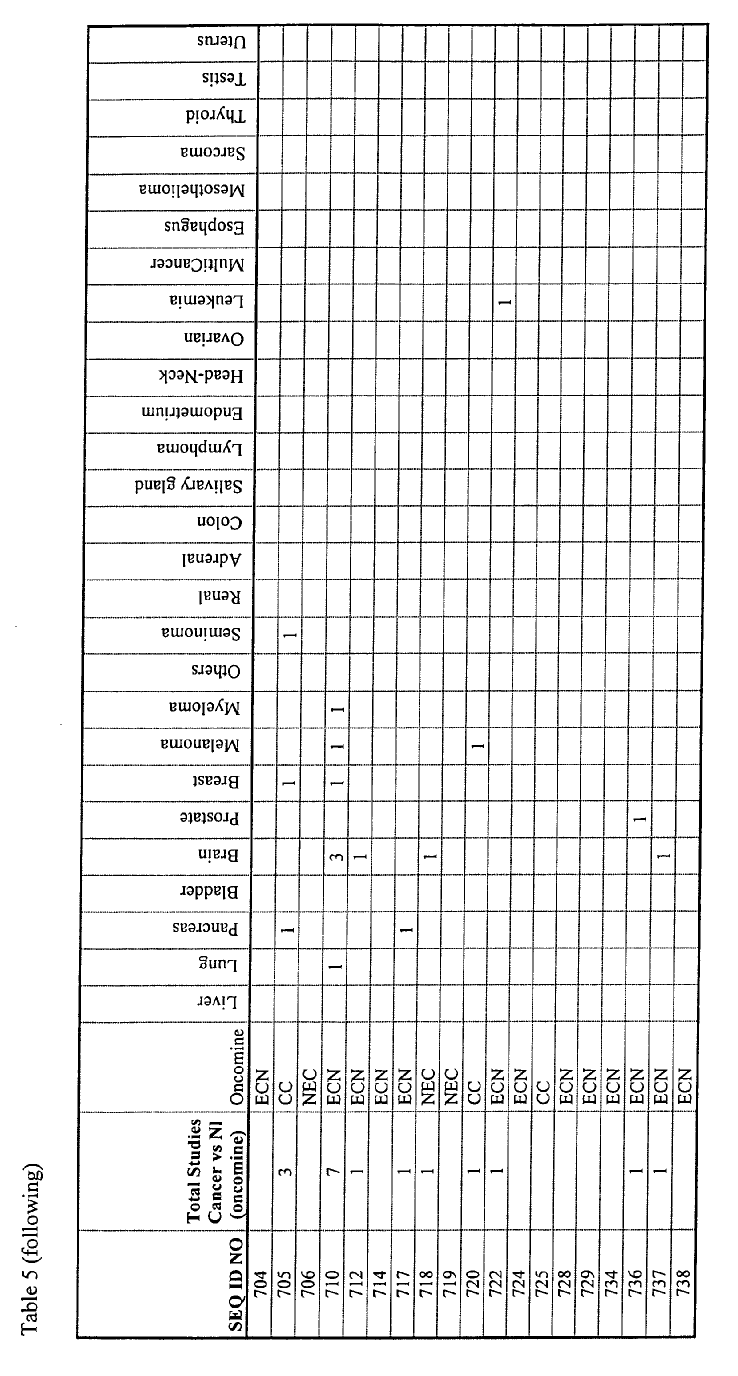

### Example 2: Development and validation of a macro-array, named "CT-chip" dedicated to analyze the expression of TS and PS genes in normal and tumoral tissues

### Objectives

The aim of this work is to design a macroarray (CTchip), based on the in silico data, which enables the detection and quantification of TS and PS genes in normal human tissues and somatic tumours. A first macroarray was evaluated by studying the expression profile of these genes in eight samples of human tissues, including six normal tissues (placenta, testis, bladder, colon, liver, normal lung) a cancer cell line (Hela 53) and a tumour (lung tumour).

### Strategy and method

### 1- Identification of the genes and probes to include in the CTchip

The following categories of genes were included
- TS or PS genes (classes A to D, as defined above and mentioned in Table 2), n=318
- Testis- or placenta overexpressed genes (Classes E and E-, as defined above), n=241
- Genes with several splice variants including one at least overexpressed in testis or placenta and at least another one not specifically expressed in testis or placenta (Class F), or genes with no specificity or clear overexpression in testis or placenta (Classes G and H), n= 247
- Tissue-specific genes (selected according to symatlas transcriptomic data) expressed in one of the following tissues: bladder (Bl), brain (Bra), breast (Bre), colon (Co), kidney (K), liver (Li), lung (Lu), Ovary (O), prostate (Pros), skin (Sk), Thyroid (T), Uterus (U), n= 647
- Control genes expressed in all tissues, n= 334, used as hybridization controls

For each gene of the above lists, at least one specific probe was designed, corresponding to a 60 bp sequence specific to the open reading frame or transcribed sequence of the gene. In particular, probes corresponding to SEQ ID NO 755 to SEQ ID NO 1088 were used.
When available the existing *Agilent Technologies* probes were used. For some genes (within the first three categories above) comprising several transcripts, with or without restricted expression to testis or placenta, several probes were designed in order to detect all known variants.

### 2- CTchip design

The online software eArray (Agilent Technologies) was used to design this first version of the CTchip. The 8x15K was chosen. In order to optimize the data extraction by Feature Extraction, it was necessary to have at least 10 000 spots per array. Therefore, all the above lists of genes, apart from the control genes were replicated six times.

### 3- The following RNA samples were purchased from Applied Biosystems (France)

- Bladder
- Hela 53
- Normal lung
- Tumor lung
- Colon
- Liver
- Testis
- Placenta

### 4- Hybridization

Approach: RNA samples were hybridized on the CT chip in a one-colour approach. This allows a direct comparison of fluorescent intensities between slides, without requirement for a common reference. Each RNA sample was hybridized three times, so that a total of 24 expression profiles were obtained.

RNA samples were first evaluated quantitatively (Nanodrop ND-1000) and qualitatively (analysis of ribosomal RNA electrophoretic profile by the Agilent BioAnalyser 2100), and then 400 ng of each sample was labelled in triplicates by incorporation of a CTP coupled with Cy3 using the Low RNA input linear amplification kit (Agilent Technologies). The concentrations of labelled cDNA were checked, adjusted at 200ng/ml, and their electrophoretic profiles were analysed as above. The labelled cDNAs were then fragmented (by denaturation at 60C for 30 min in a specific buffer) in order to obtain 50 to 200 bp fragments, which were necessary for an optimal hybridization with the 60-mers probes.
600ng of Cy3-cDNA were then hybridized at 65C during 17 hours. The hybridized slides were read in green using an Agilent scanner device.

### 5- Data extraction

The data were extracted by the software "Feature Extraction 9.1", using a one-colour protocol (GE1-v5_95_Feb97). Three files were generated, which include an image file (.jpg), a data file (.text) and a quality report (.pdf). The quality parameters were satisfactory, with a variation coefficient between signals obtained with the same probe of less than 10%, indicating a high reproducibility of the experiments.
In the data file, after background noise signal substraction, the mean signal obtained for each probe was normalized to give the value of the "processed signal". The value in "IsWellAboveBG" indicates if the mean signal is significantly superior to background, and whether this signal is 2.6 times superior to the standard deviation of the background noise for Cy3 (0= not significant; 1= significant).

### 6- Analysis of expression profiles

The aim was
- to identify transcripts expressed in each tissue
- to compare levels of expression in somatic tissues with those in testis or placenta and validate the tissue-specificity of the analysed genes
- to identify testis- or placenta-specific genes expressed in HeLA cells or in tumor lung.

The expression profiles of the genes of the categories described above are summarized in the following table 6 and Figures 2 show a hierarchical clustering of the genes belonging to the different categories. This was done using "permutmatrix" software (free online http://www.lirmm.fr/∼caraux/PermutMatrix/).

The following table 6 recapitulates the expression data of genes studies on the CTchip. The data of all the studied genes belonging to the A-D category, as well as some of the data of the genes belonging to the two other categories are indicated (28 genes E and 14 genes F-H).

CTchip_CT = testis or placenta specific genes, which are also found expressed in Hela cells and/or lung tumour. Genes found as CT genes are indicated in bold text.
All the other columns have been described above.

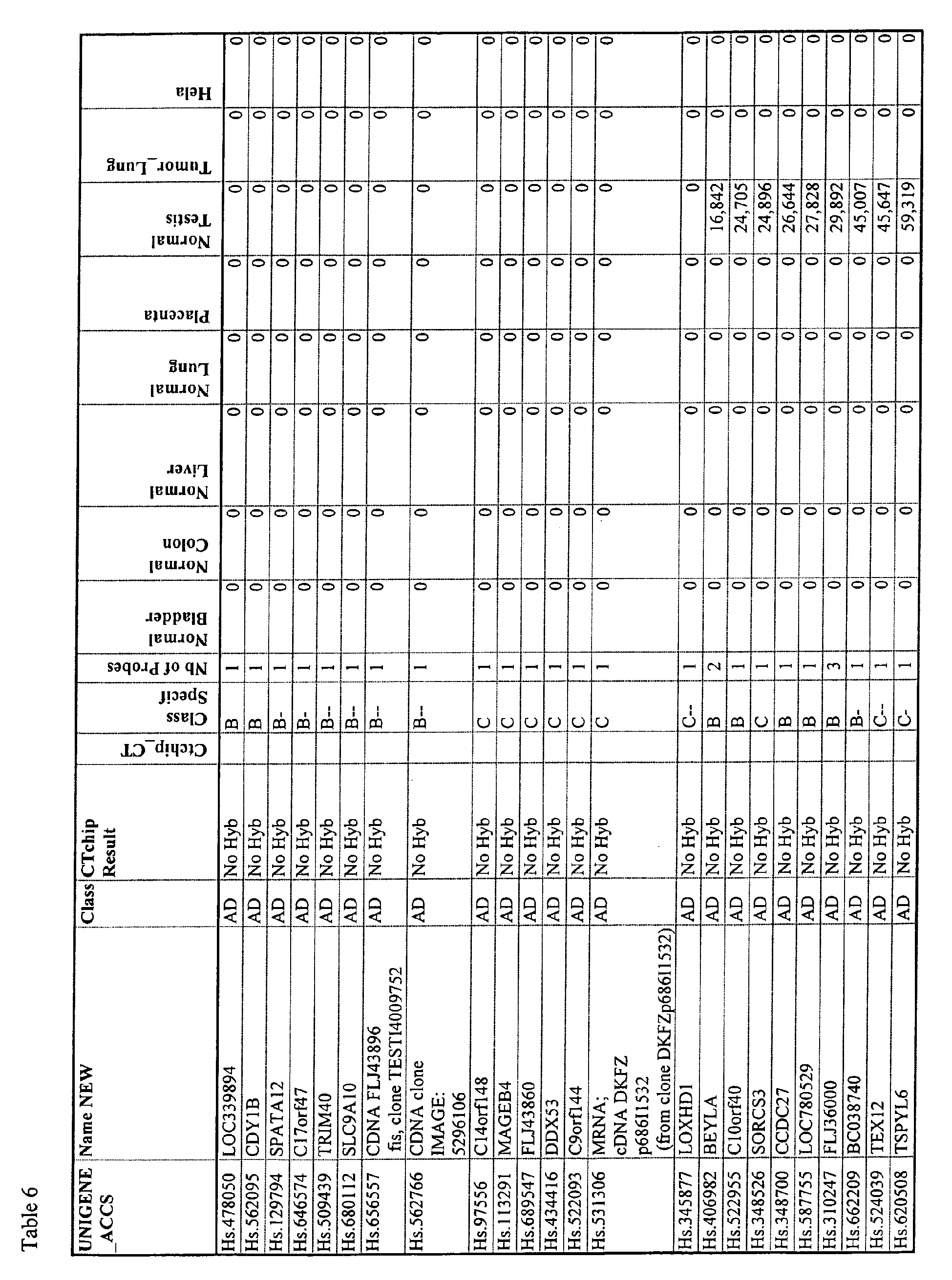

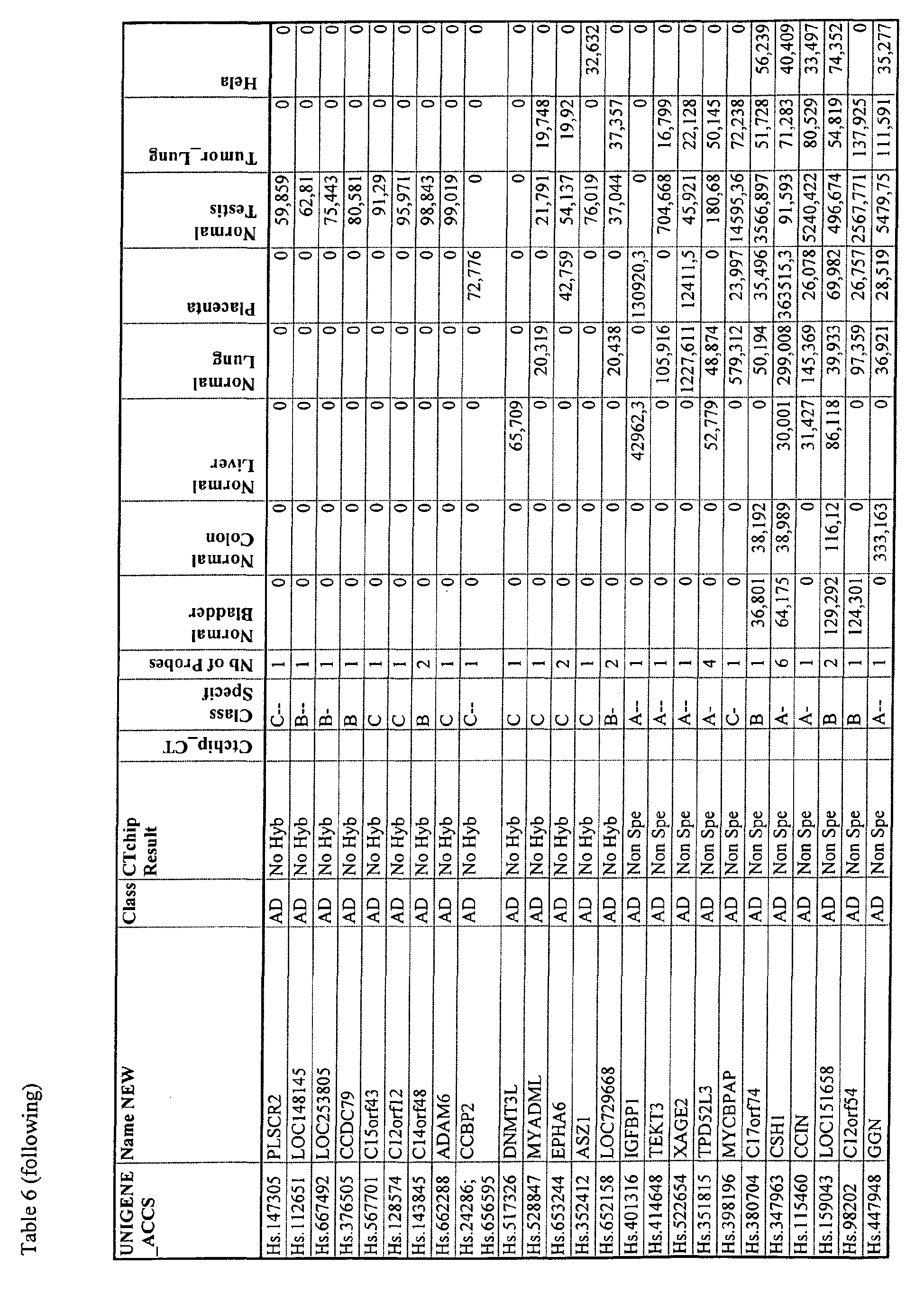

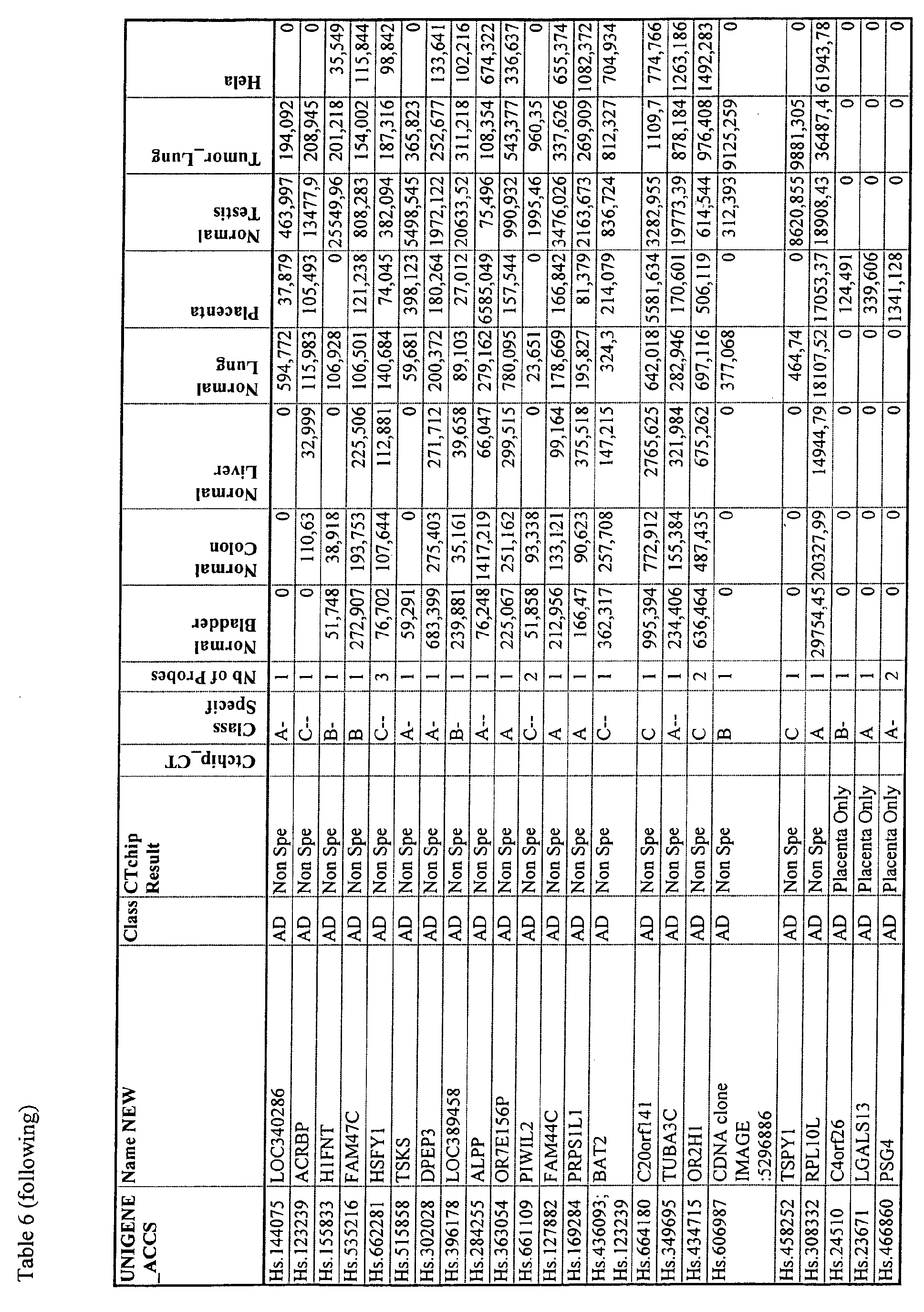

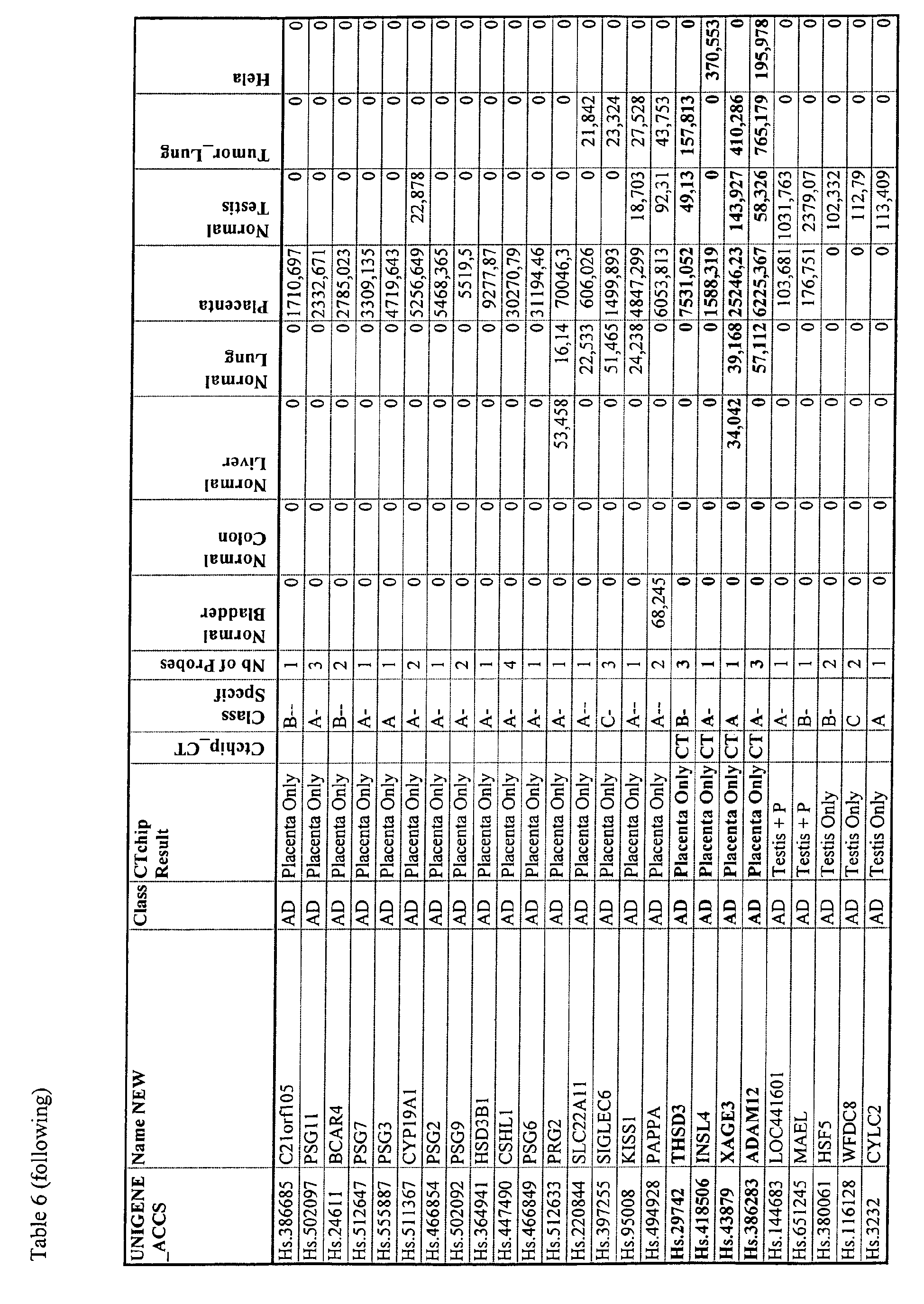

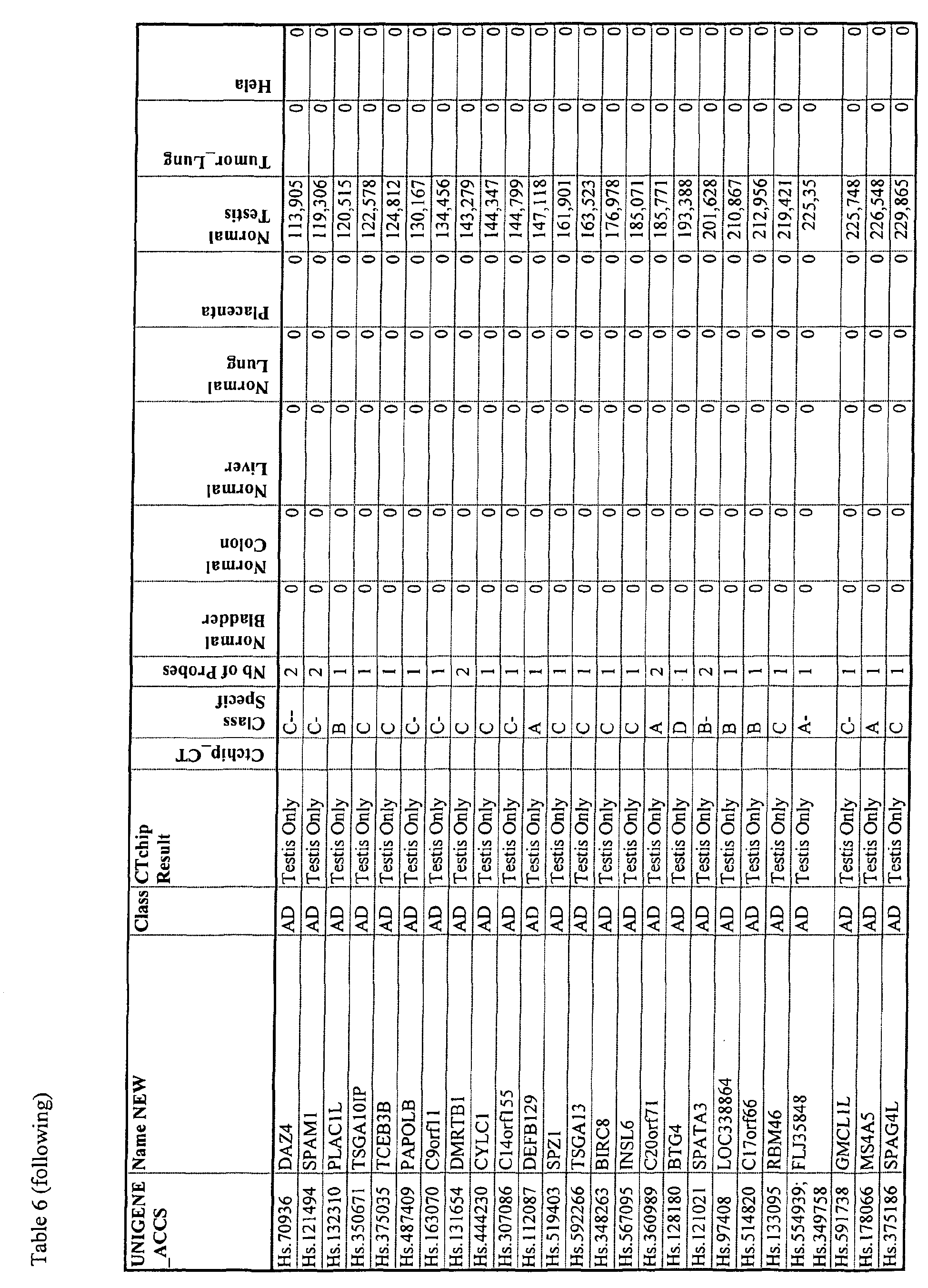

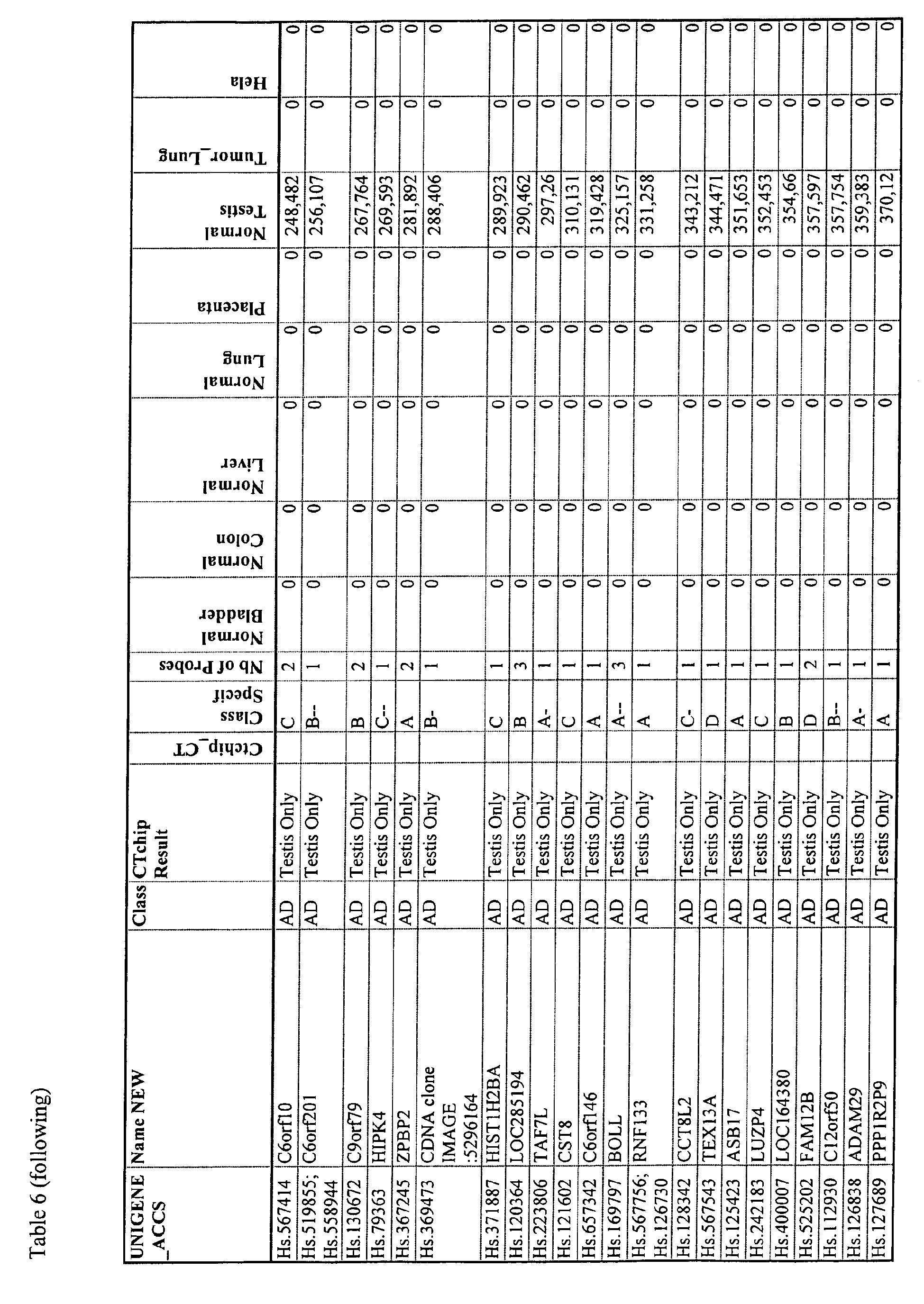

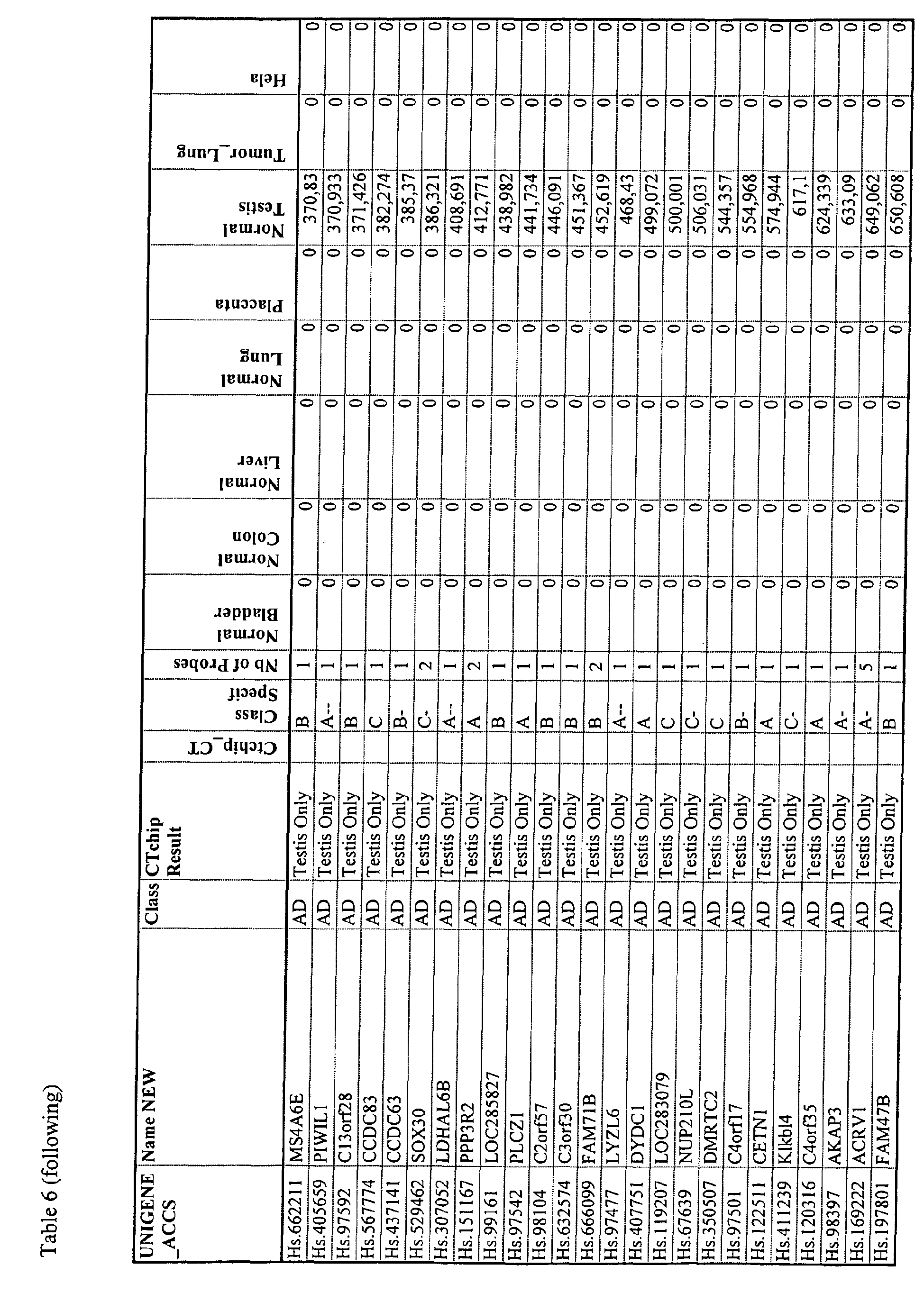

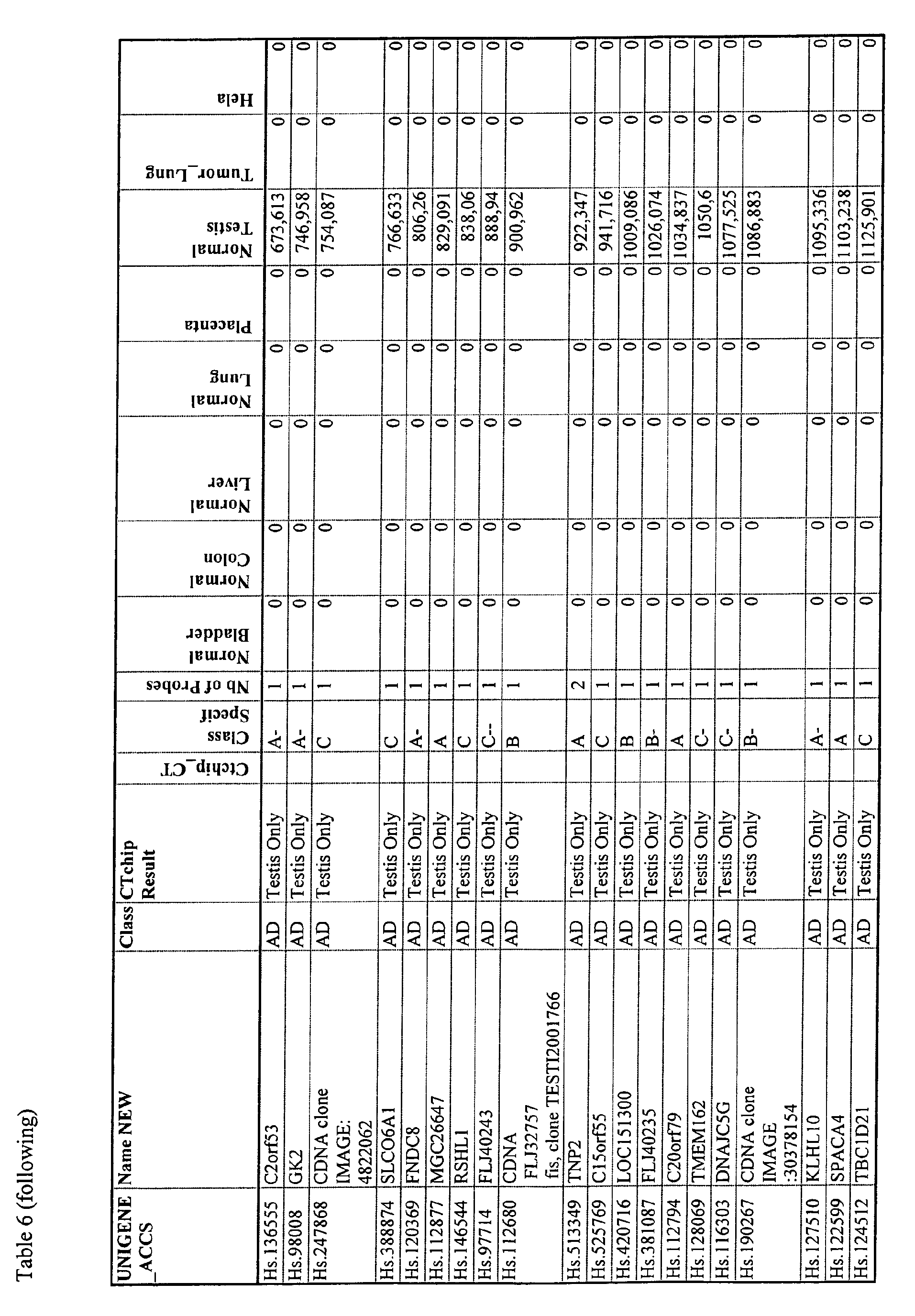

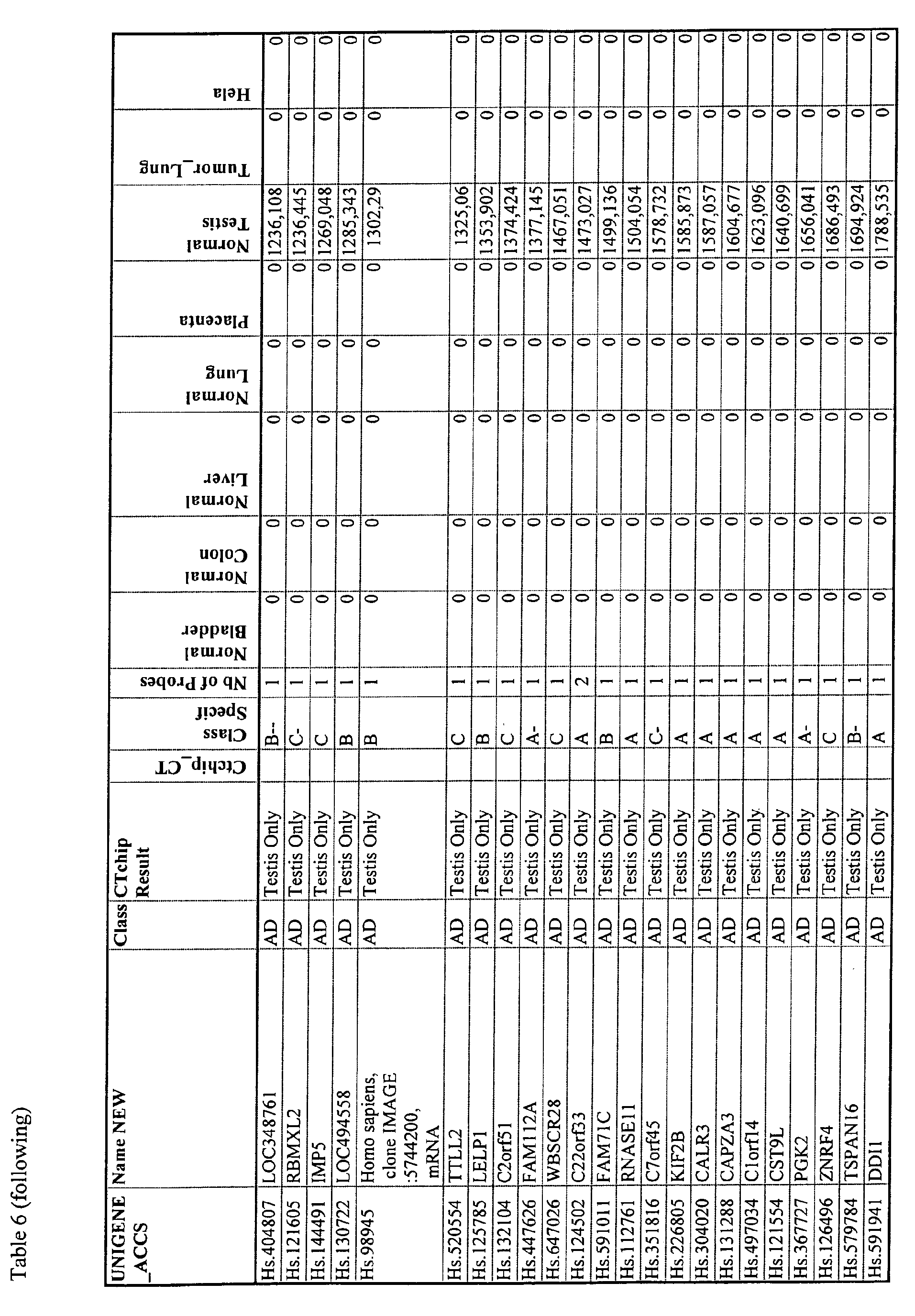

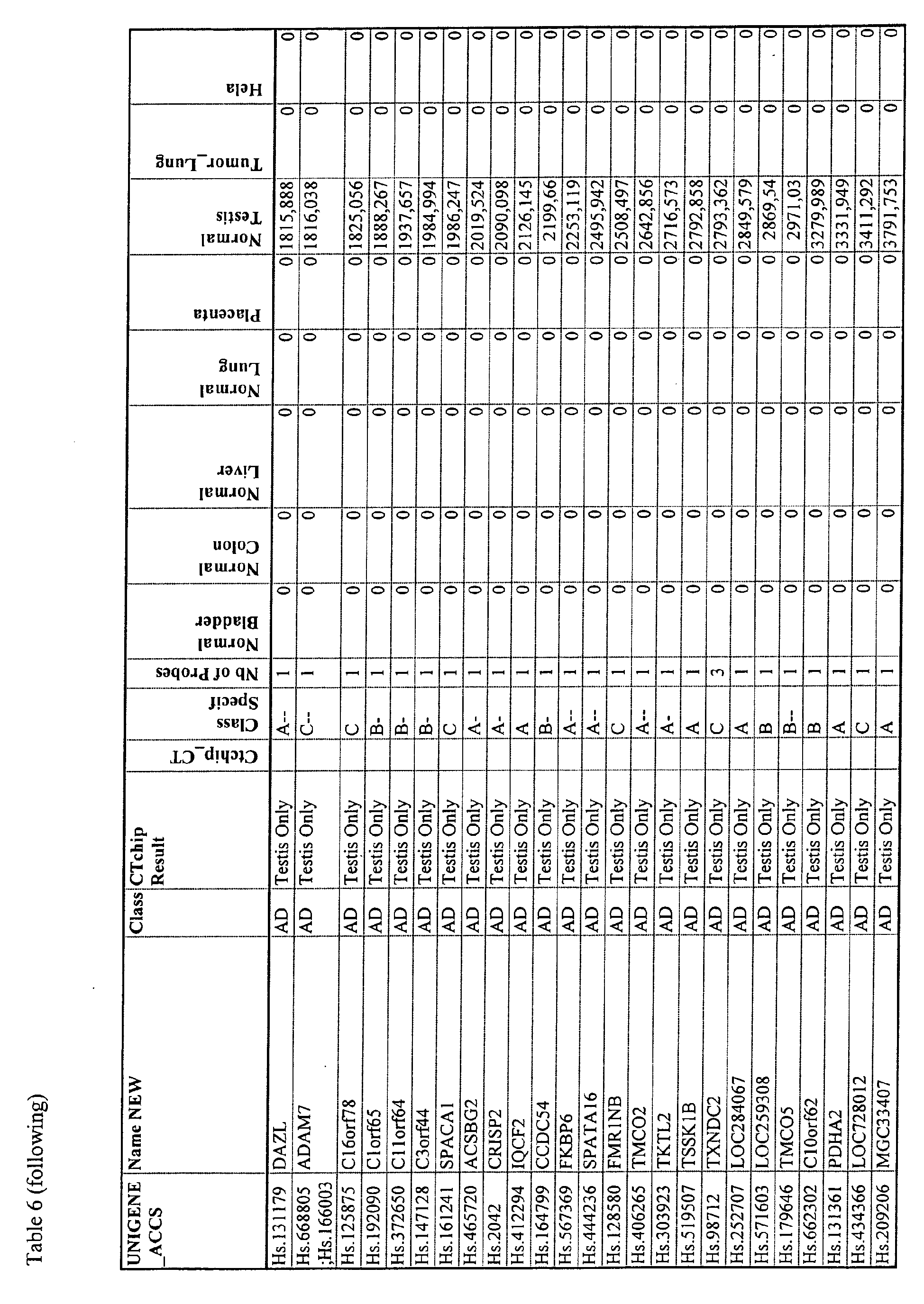

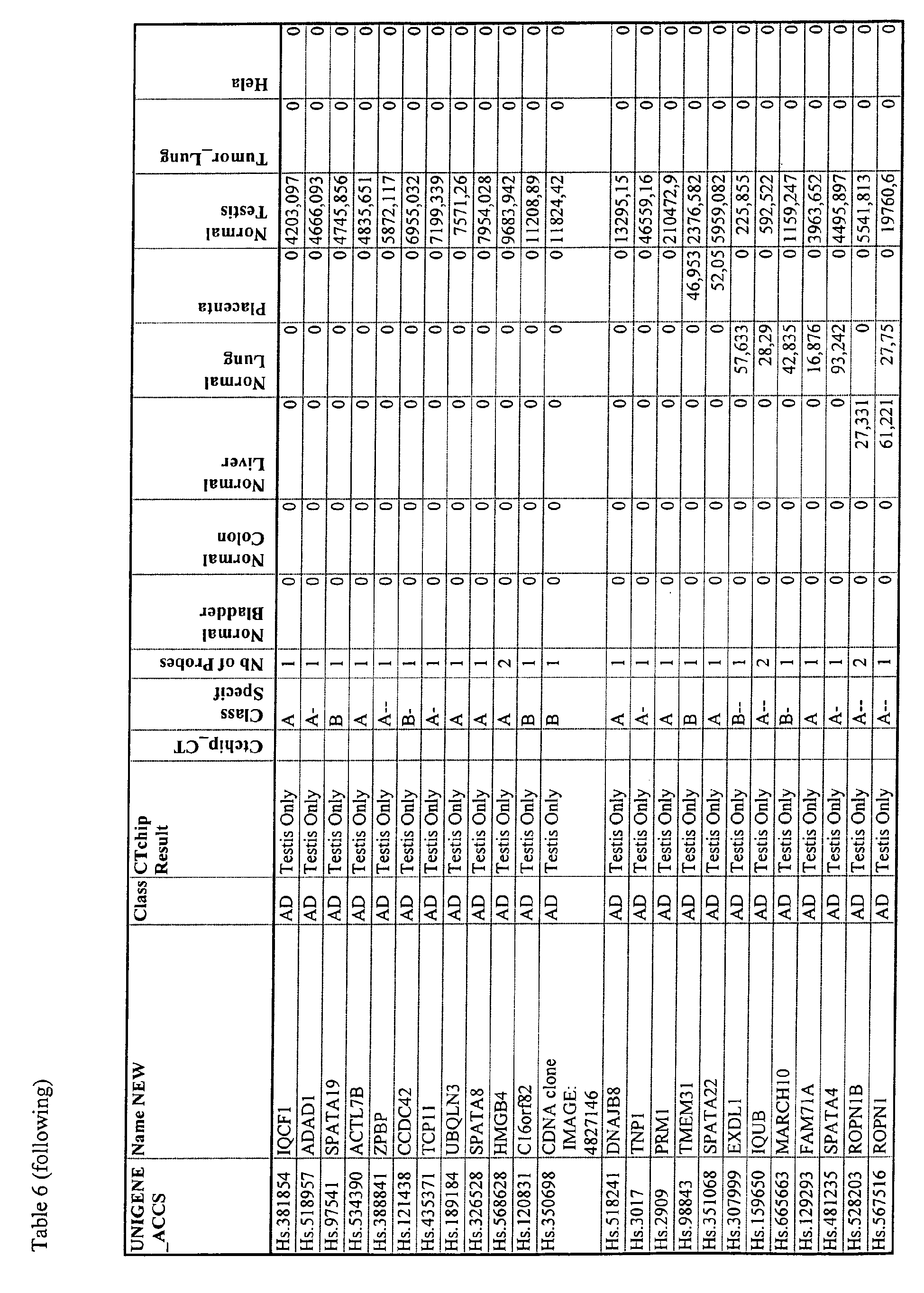

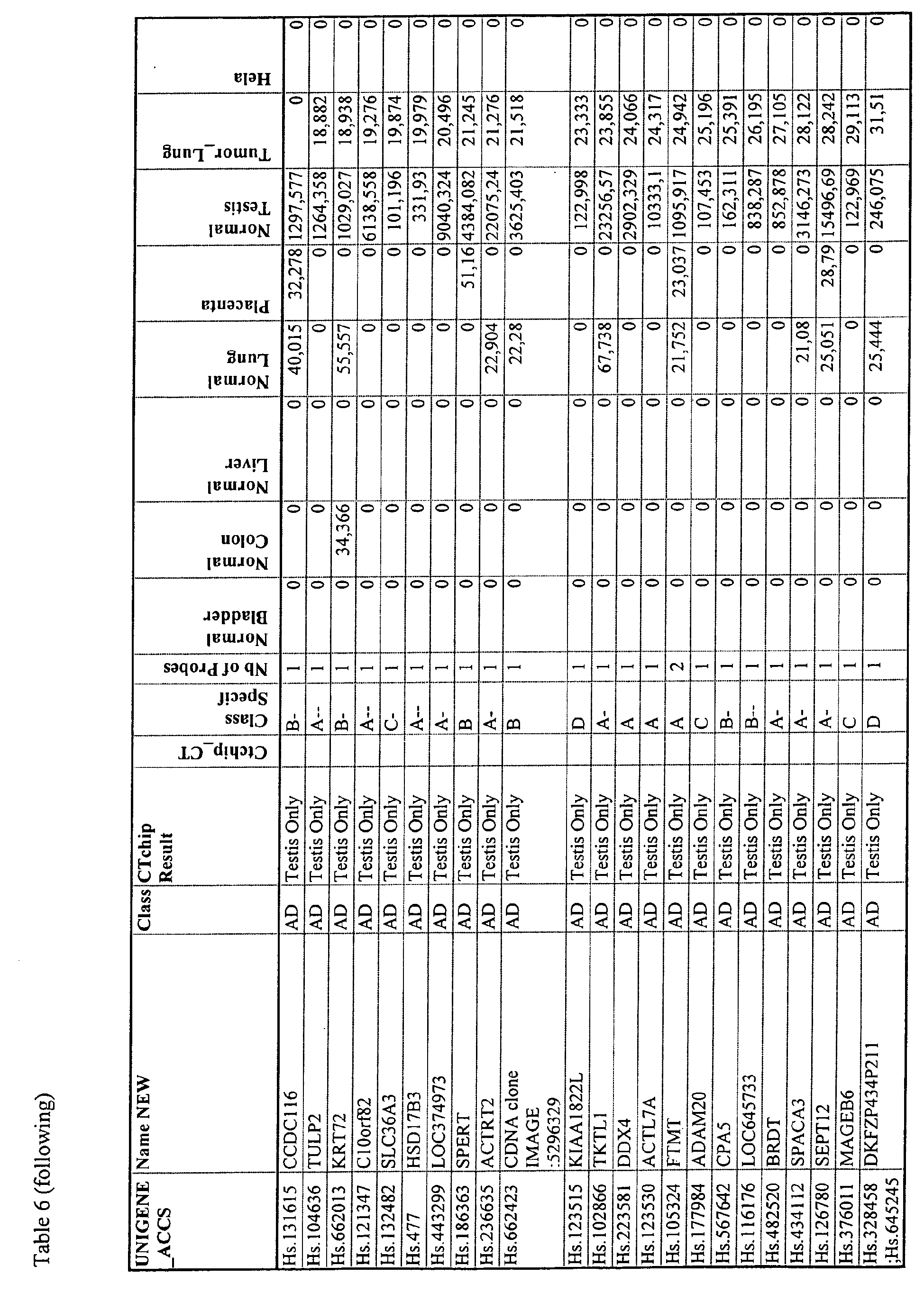

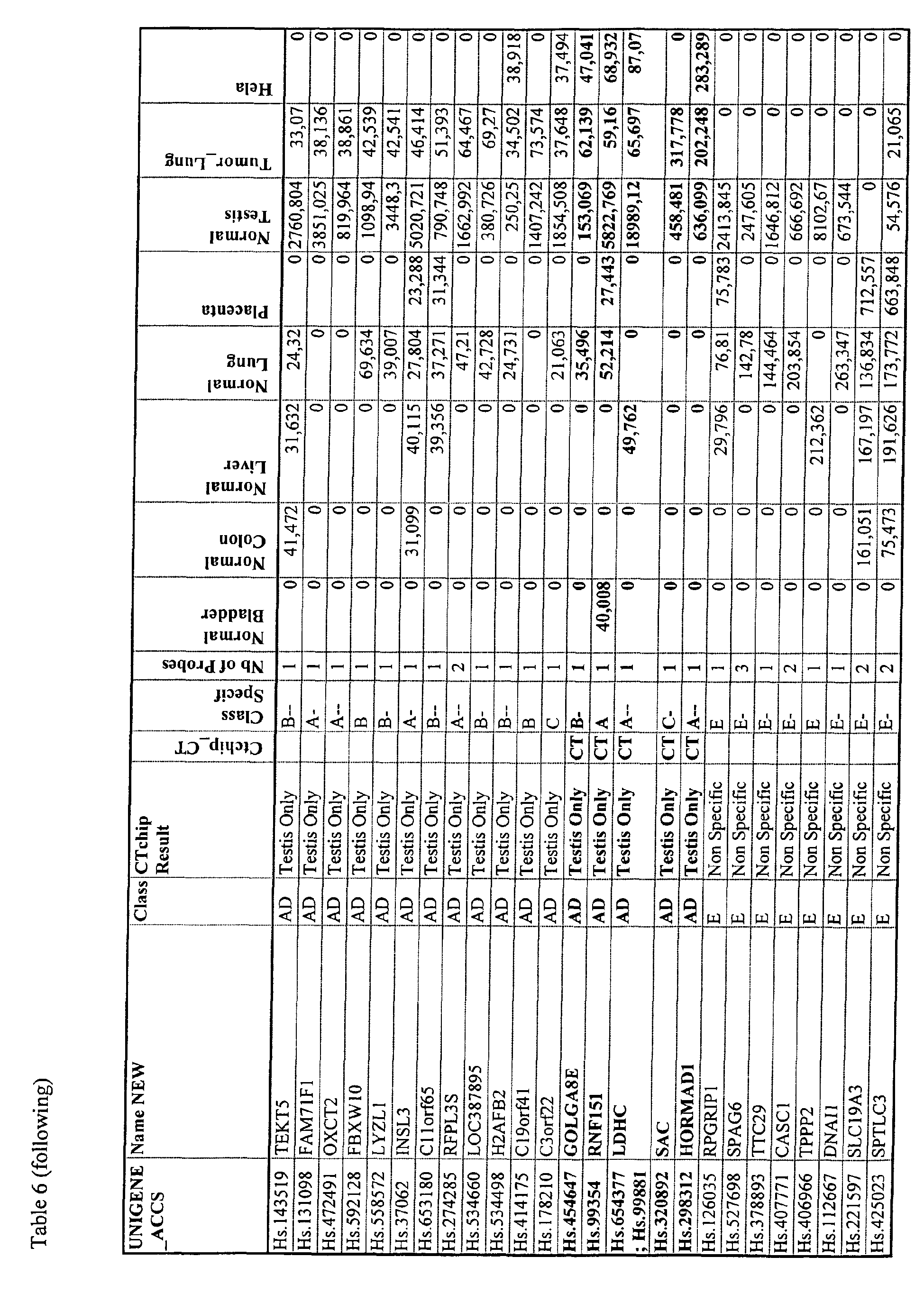

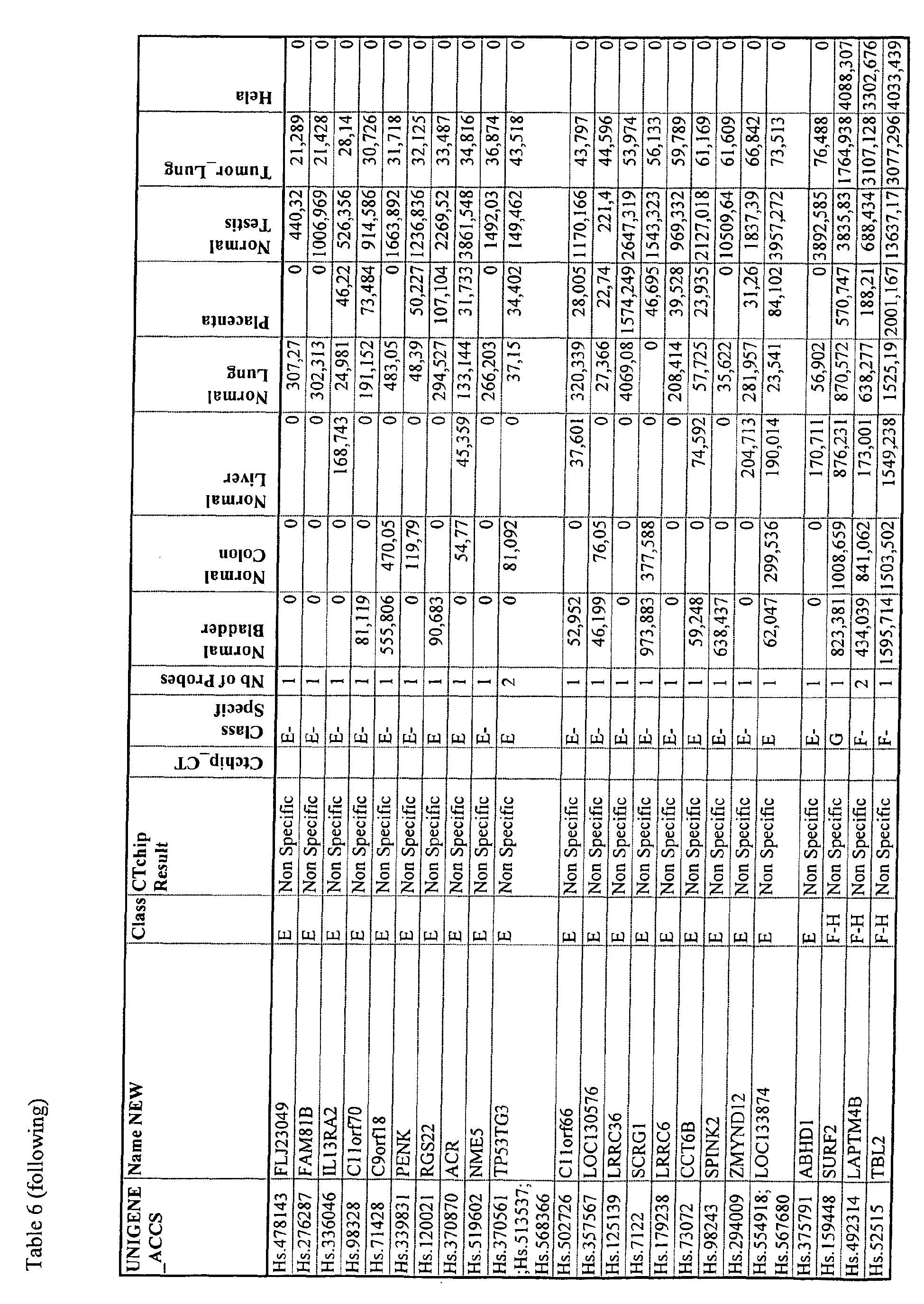

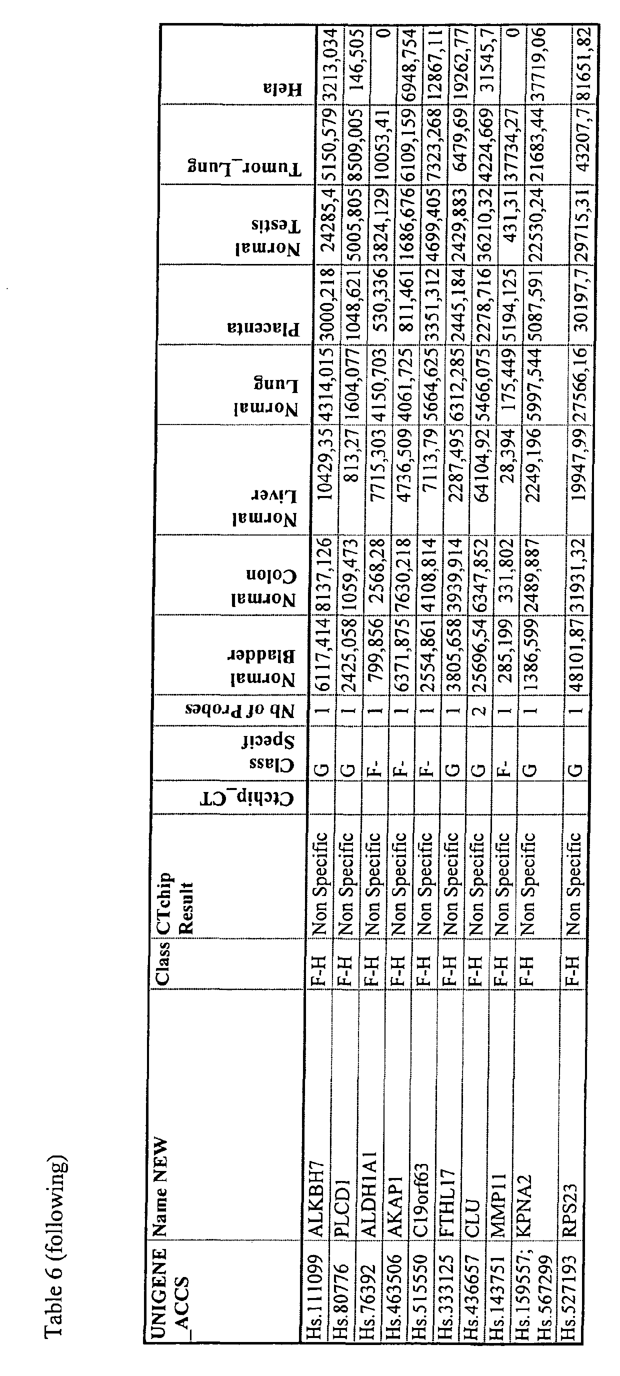

The results obtained from the CTchip indicate that a large majority of the genes analyzed on this first version of the CT chip display the expected profile of expression in normal somatic tissues. These results therefore validate the in silico approach to identify a large number of new testis- or placenta- specific genes (see figure 2b).
These results also validate the specificity of the above described approach, which relies on the concept that the use of this large number of genes enables the detection of a cancer-related gene deregulation in any case of somatic cancer (fig 1a, also refer to fig 2a describing the Oncomine data).
To enforce the data, it is to be noticed that most of the control genes with an expected expression in all tissues were positive in all tissues in the experiment.

A large proportion of the tissue-specific genes showed a rather tissue-restricted pattern of expression, although some of them did not have this restricted pattern of expression. It should be reminded that these genes were selected for a restricted expression pattern according to symatlas transcriptomic data, but were not classified according to the specificities of their ESTs. Therefore these non-specific expression profiles were to be expected.
In particular, a high proportion of the testis-specific and placenta-specific genes (as defined previously, belonging to specificity classes A to D) clearly show a testis- or placenta- restricted pattern of expression in the normal tissues (Figure 2a, third column, and figure 2b).

Among the testis- or placenta-specific genes, nine were found expressed in Hela cells and/or the lung tumor sample analyzed in this series of experiments (see table 6), showing that they can be considered as "CT" genes, which could be deregulated in somatic cancers. The present results demonstrate that this CT-chip approach can indeed detect the deregulation of testis- or placenta-specific genes in somatic cancers and identify a set of deregulated CT genes in somatic cancers.

Altogether the data obtained with this first CT-chip enable to validate the in silico approaches for the identification of testis- or placenta- specific genes and confirm most of the initial data, and in particular the expression specificity of the genes.
These results also demonstrate that this tool can be extremely powerful to systematically identify genes aberrantly expressed in all tumours, and be used to define any cancer type and stage of evolution (see Oncomine data).

These results therefore validate the concept that at least one of testis and placenta specific genes can be found abnormally expressed in cancer cells of at least one type of the somatic or ovarian cancers, and that each type of somatic or ovarian cancer cells can abnormally express at least one of the testis and placenta specific genes.

### References

Bock-Axelsen J, Lotem J, Sachs L, Domany E (2007) Genes overexpressed in different human solid cancers exhibit different tissue-specific expression profiles. Proc Natl Acad Sci U SA
Chalmel F, Rolland AD, Niederhauser-Wiederkehr C, Chung SS, Demougin P, Gattiker A, Moore J, Patard JJ, Wolgemuth DJ, Jegou B, Primig M (2007) The conserved transcriptome in human and rodent male gametogenesis. Proc Natl Acad Sci U S A
Chen YT, Scanlan MJ, Venditti CA, Chua R, Theiler G, Stevenson BJ, Iseli C, Gure AO, Vasicek T, Strausberg RL, Jongeneel CV, Old LJ, Simpson AJ (2005) Identification of cancer/testis-antigen genes by massively parallel signature sequencing. Proc Natl Acad Sci U S A 102: 7940-5
Costa FF, Le Blanc K, Brodin B (2007) Concise review: cancer/testis antigens, stem cells, and cancer. Stem Cells 25: 707-11
Fox MS, Ares VX, Turek PJ, Haqq C, Reijo Pera RA (2003) Feasibility of global gene expression analysis in testicular biopsies from infertile men. Mol Reprod Dev 66: 403-21
Kalejs M, Erenpreisa J (2005) Cancer/testis antigens and gametogenesis: a review and "brain-storming" session. Cancer Cell Int 5: 4
Meklat F, Li Z, Wang Z, Zhang Y, Zhang J, Jewell A, Lim SH (2007) Cancer-testis antigens in haematological malignancies. Br J Haematol 136: 769-76
Rhodes DR, Kalyana-Sundaram S, Mahavisno V, Varambally R, Yu J, Briggs BB, Barrette TR, Anstet MJ, Kincead-Beal C, Kulkarni P, Varambally S, Ghosh D, Chinnaiyan AM (2007) Oncomine 3.0: genes, pathways, and networks in a collection of 18,000 cancer gene expression profiles. Neoplasia 9: 166-80
Rhodes DR, Yu J, Shanker K, Deshpande N, Varambally R, Ghosh D, Barrette T, Pandey A, Chinnaiyan AM (2004) ONCOMINE: a cancer microarray database and integrated data-mining platform. Neoplasia 6: 1-6
Scanlan MJ, Gordon CM, Williamson B, Lee SY, Chen YT, Stockert E, Jungbluth A, Ritter G, Jager D, Jager E, Knuth A, Old LJ (2002) Identification of cancer/testis genes by database mining and mRNA expression analysis. Int J Cancer 98: 485-92
Scanlan MJ, Simpson AJ, Old LJ (2004) The cancer/testis genes: review, standardization, and commentary. Cancer Immun 4: 1
Schultz N, Hamra FK, Garbers DL (2003) A multitude of genes expressed solely in meiotic or postmeiotic spermatogenic cells offers a myriad of contraceptive targets. Proc Natl Acad Sci U S A 100: 12201-6
Simpson AJ, Caballero OL, Jungbluth A, Chen YT, Old LJ (2005) Cancer/testis antigens, gametogenesis and cancer. Nat Rev Cancer 5: 615-25
Su AI, Wiltshire T, Batalov S, Lapp H, Ching KA, Block D, Zhang J, Soden R, Hayakawa M, Kreiman G, Cooke MP, Walker JR, Hogenesch JB (2004) A gene atlas of the mouse and human protein-encoding transcriptomes. Proc Natl Acad Sci U S A
Weber M, Hellmann I, Stadler MB, Ramos L, Paabo S, Rebhan M, Schubeler D (2007) Distribution, silencing potential and evolutionary impact of promoter DNA methylation in the human genome. Nat Genet

## Claims

1. Use of one element chosen among:
- at least a nucleic acid molecule of the group comprising or constituted by :
• a nucleotide sequence of the group consisting in SEQ ID NO 641 to SEQ ID NO 754, or
• a nucleotide sequence of the group consisting in SEQ ID NO 2q-1, q varying from 1 to 320, coding for a protein comprising or constituted by an amino acid sequence belonging to the group consisting in SEQ ID NO 2q, q varying from 1 to 320, or
• the complementary sequence of the nucleic acid molecule thereof,
- at least a fragment of said nucleic acid molecule, said fragment comprising at least 18 contiguous nucleotides of said nucleic acid molecule, and
- at least a variant of the said nucleic acid molecule, wherein the variant presents a sequence homology of at least 70%, particularly 80%, and more particularly 90% compared to said nucleic acid molecule,
for the *in vitro* or *ex vivo* diagnosis of any type of somatic or ovarian cancers, wherein at least one of any of the above described elements is abnormally expressed in cancer cells of at least one type of the somatic or ovarian cancers, and
wherein each type of somatic or ovarian cancer cells abnormally expresses at least one of the above described elements.

2. Use according to claim 1, wherein the nucleic acid molecule belongs to the group consisting in SEQ ID NO 17, SEQ ID NO 37, SEQ ID NO 39, SEQ ID NO 41, SEQ ID NO 43, SEQ ID NO 45, SEQ ID NO 47, SEQ ID NO 49, SEQ ID NO 51, SEQ ID NO 55, SEQ ID NO 59, SEQ ID NO 61, SEQ ID NO 63, SEQ ID NO 65, SEQ ID NO 67, SEQ ID NO 69, SEQ ID NO 71, SEQ ID NO 73, SEQ ID NO 79, SEQ ID NO 81, SEQ ID NO 83, SEQ ID NO 85, SEQ ID NO 89, SEQ ID NO 91, SEQ ID NO 93, SEQ ID NO 95, SEQ ID NO 97, SEQ ID NO 99, SEQ ID NO 101, SEQ ID NO 103, SEQ ID NO 113, SEQ ID NO 115, SEQ ID NO 117, SEQ ID NO 121, SEQ ID NO 155, SEQ ID NO 157, SEQ ID NO 161, SEQ ID NO 165, SEQ ID NO 167, SEQ ID NO 169, SEQ ID NO 171, SEQ ID NO 189, SEQ ID NO 191, SEQ ID NO 195, SEQ ID NO 197, SEQ ID NO 199, SEQ ID NO 203, SEQ ID NO 205, SEQ ID NO 207, SEQ ID NO 213, SEQ ID NO 215, SEQ ID NO 217, SEQ ID NO 219, SEQ ID NO 223, SEQ ID NO 225, SEQ ID NO 229, SEQ ID NO 231, SEQ ID NO 233, SEQ ID NO 235, SEQ ID NO 237, SEQ ID NO 239, SEQ ID NO 241, SEQ ID NO 243, SEQ ID NO 247, SEQ ID NO 263, SEQ ID NO 281, SEQ ID NO 293, SEQ ID NO 295, SEQ ID NO 297, SEQ ID NO 303, SEQ ID NO 317, SEQ ID NO 319, SEQ ID NO 321, SEQ ID NO 323, SEQ ID NO 325, SEQ ID NO 327, SEQ ID NO 329, SEQ ID NO 331, SEQ ID NO 333, SEQ ID NO 335, SEQ ID NO 337, SEQ ID NO 339, SEQ ID NO 341, SEQ ID NO 343, SEQ ID NO 345, SEQ ID NO 347, SEQ ID NO 351, SEQ ID NO 353, SEQ ID NO 355, SEQ ID NO 357, SEQ ID NO 359, SEQ ID NO 361, SEQ ID NO 363, SEQ ID NO 365, SEQ ID NO 367, SEQ ID NO 369, SEQ ID NO 371, SEQ ID NO 373, SEQ ID NO 375, SEQ ID NO 377, SEQ ID NO 379, SEQ ID NO 381, SEQ ID NO 387, SEQ ID NO 389, SEQ ID NO 395, SEQ ID NO 397, SEQ ID NO 399, SEQ ID NO 401, SEQ ID NO 403, SEQ ID NO 405, SEQ ID NO 407, SEQ ID NO 409, SEQ ID NO 413, SEQ ID NO 423, SEQ ID NO 425, SEQ ID NO 435, SEQ ID NO 437, SEQ ID NO 441, SEQ ID NO 445, SEQ ID NO 461, SEQ ID NO 463, SEQ ID NO 497, SEQ ID NO 517, SEQ ID NO 527, SEQ ID NO 551, SEQ ID NO 567, SEQ ID NO 579, SEQ ID NO 595, SEQ ID NO 599, SEQ ID NO 601, SEQ ID NO 609, SEQ ID NO 623, SEQ ID NO 625, SEQ ID NO 627, SEQ ID NO 641, SEQ ID NO 642, SEQ ID NO 643, SEQ ID NO 644, SEQ ID NO 645, SEQ ID NO 646, SEQ ID NO 647, SEQ ID NO 648, SEQ ID NO 649, SEQ ID NO 650, SEQ ID NO 651, SEQ ID NO 652, SEQ ID NO 653, SEQ ID NO 654, SEQ ID NO 655, SEQ ID NO 656, SEQ ID NO 657, SEQ ID NO 658, SEQ ID NO 659, SEQ ID NO 660, SEQ ID NO 661, SEQ ID NO 662, SEQ ID NO 663, SEQ ID NO 664, SEQ ID NO 665, SEQ ID NO 666, SEQ ID NO 667, SEQ ID NO 668, SEQ ID NO 669, SEQ ID NO 670, SEQ ID NO 671, SEQ ID NO 672, SEQ ID NO 673, SEQ ID NO 674, SEQ ID NO 675, SEQ ID NO 676, SEQ ID NO 677, SEQ ID NO 678, SEQ ID NO 679, SEQ ID NO 680, SEQ ID NO 681, SEQ ID NO 682, SEQ ID NO 683, SEQ ID NO 684, SEQ ID NO 685, SEQ ID NO 686, SEQ ID NO 687, SEQ ID NO 688, SEQ ID NO 689, SEQ ID NO 690, SEQ ID NO 691, SEQ ID NO 692, SEQ ID NO 693, SEQ ID NO 694, SEQ ID NO 695, SEQ ID NO 696, SEQ ID NO 697, SEQ ID NO 698, SEQ ID NO 699, SEQ ID NO 700, SEQ ID NO 702, SEQ ID NO 703, SEQ ID NO 704, SEQ ID NO 705, SEQ ID NO 706, SEQ ID NO 707, SEQ ID NO 708, SEQ ID NO 709, SEQ ID NO 710, SEQ ID NO 711, SEQ ID NO 712, SEQ ID NO 713, SEQ ID NO 714, SEQ ID NO 715, SEQ ID NO 717, SEQ ID NO 718, SEQ ID NO 719, SEQ ID NO 720, SEQ ID NO 722, SEQ ID NO 724, SEQ ID NO 725, SEQ ID NO 726, SEQ ID NO 727, SEQ ID NO 728, SEQ ID NO 729, SEQ ID NO 730, SEQ ID NO 731, SEQ ID NO 732, SEQ ID NO 733, SEQ ID NO 734, SEQ ID NO 735, SEQ ID NO 736, SEQ ID NO 737, SEQ ID NO 738, SEQ ID NO 739, SEQ ID NO 740, SEQ ID NO 741, SEQ ID NO 742, SEQ ID NO 743, SEQ ID NO 744, SEQ ID NO 745, SEQ ID NO 746, SEQ ID NO 747, SEQ ID NO 748, SEQ ID NO 749, SEQ ID NO 750, SEQ ID NO 751, SEQ ID NO 752, SEQ ID NO 753 and SEQ ID NO 754.

3. Use according to claim 2, wherein the nucleic acid molecule belongs to the group consisting in SEQ ID NO 17, SEQ ID NO 37, SEQ ID NO 39, SEQ ID NO 41, SEQ ID NO 43, SEQ ID NO 45, SEQ ID NO 47, SEQ ID NO 49, SEQ ID NO 51, SEQ ID NO 55, SEQ ID NO 59, SEQ ID NO 61, SEQ ID NO 63, SEQ ID NO 65, SEQ ID NO 67, SEQ ID NO 69, SEQ ID NO 71, SEQ ID NO 73, SEQ ID NO 81, SEQ ID NO 83, SEQ ID NO 85, SEQ ID NO 89, SEQ ID NO 91, SEQ ID NO 93, SEQ ID NO 95, SEQ ID NO 97, SEQ ID NO 99, SEQ ID NO 101, SEQ ID NO 103, SEQ ID NO 113, SEQ ID NO 115, SEQ ID NO 117, SEQ ID NO 121, SEQ ID NO 155, SEQ ID NO 157, SEQ ID NO 161, SEQ ID NO 165, SEQ ID NO 167, SEQ ID NO 169, SEQ ID NO 171, SEQ ID NO 189, SEQ ID NO 191, SEQ ID NO 195, SEQ ID NO 197, SEQ ID NO 199, SEQ ID NO 203, SEQ ID NO 207, SEQ ID NO 213, SEQ ID NO 215, SEQ ID NO 217, SEQ ID NO 219, SEQ ID NO 223, SEQ ID NO 225, SEQ ID NO 229, SEQ ID NO 231, SEQ ID NO 233, SEQ ID NO 235, SEQ ID NO 237, SEQ ID NO 239, SEQ ID NO 241, SEQ ID NO 263, SEQ ID NO 281, SEQ ID NO 293, SEQ ID NO 295, SEQ ID NO 297, SEQ ID NO 303, SEQ ID NO 317, SEQ ID NO 319, SEQ ID NO 321, SEQ ID NO 323, SEQ ID NO 325, SEQ ID NO 327, SEQ ID NO 329, SEQ ID NO 331, SEQ ID NO 333, SEQ ID NO 335, SEQ ID NO 337, SEQ ID NO 339, SEQ ID NO 343, SEQ ID NO 345, SEQ ID NO 347, SEQ ID NO 351, SEQ ID NO 353, SEQ ID NO 355, SEQ ID NO 357, SEQ ID NO 359, SEQ ID NO 361, SEQ ID NO 363, SEQ ID NO 365, SEQ ID NO 367, SEQ ID NO 369, SEQ ID NO 371, SEQ ID NO 373, SEQ ID NO 375, SEQ ID NO 377, SEQ ID NO 379, SEQ ID NO 381, SEQ ID NO 387, SEQ ID NO 389, SEQ ID NO 395, SEQ ID NO 397, SEQ ID NO 399, SEQ ID NO 401, SEQ ID NO 403, SEQ ID NO 405, SEQ ID NO 407, SEQ ID NO 409, SEQ ID NO 413, SEQ ID NO 423, SEQ ID NO 425, SEQ ID NO 435, SEQ ID NO 437, SEQ ID NO 441, SEQ ID NO 445, SEQ ID NO 461, SEQ ID NO 497, SEQ ID NO 517, SEQ ID NO 527, SEQ ID NO 551, SEQ ID NO 567, SEQ ID NO 579, SEQ ID NO 595, SEQ ID NO 599, SEQ ID NO 601, SEQ ID NO 609, SEQ ID NO 623, SEQ ID NO 625, SEQ ID NO 627, SEQ ID NO 641, SEQ ID NO 642, SEQ ID NO 643, SEQ ID NO 644, SEQ ID NO 645, SEQ ID NO 646, SEQ ID NO 647, SEQ ID NO 648, SEQ ID NO 649, SEQ ID NO 650, SEQ ID NO 651, SEQ ID NO 652, SEQ ID NO 653, SEQ ID NO 654, SEQ ID NO 656, SEQ ID NO 657, SEQ ID NO 658, SEQ ID NO 659, SEQ ID NO 660, SEQ ID NO 661, SEQ ID NO 662, SEQ ID NO 663, SEQ ID NO 664, SEQ ID NO 665, SEQ ID NO 666, SEQ ID NO 667, SEQ ID NO 668, SEQ ID NO 669, SEQ ID NO 670, SEQ ID NO 672, SEQ ID NO 673, SEQ ID NO 674, SEQ ID NO 675, SEQ ID NO 676, SEQ ID NO 677, SEQ ID NO 678, SEQ ID NO 679, SEQ ID NO 680, SEQ ID NO 681, SEQ ID NO 682, SEQ ID NO 683, SEQ ID NO 687, SEQ ID NO 688, SEQ ID NO 689, SEQ ID NO 690, SEQ ID NO 691, SEQ ID NO 692, SEQ ID NO 693, SEQ ID NO 694, SEQ ID NO 695, SEQ ID NO 696, SEQ ID NO 697, SEQ ID NO 698, SEQ ID NO 700, SEQ ID NO 702, SEQ ID NO 703, SEQ ID NO 704, SEQ ID NO 705, SEQ ID NO 706, SEQ ID NO 707, SEQ ID NO 708, SEQ ID NO 709, SEQ ID NO 710, SEQ ID NO 711, SEQ ID NO 712, SEQ ID NO 713, SEQ ID NO 714, SEQ ID NO 715, SEQ ID NO 718, SEQ ID NO 720, SEQ ID NO 722, SEQ ID NO 724, SEQ ID NO 725, SEQ ID NO 727, SEQ ID NO 728, SEQ ID NO 729, SEQ ID NO 730, SEQ ID NO 731, SEQ ID NO 732, SEQ ID NO 733, SEQ ID NO 735, SEQ ID NO 736, SEQ ID NO 737, SEQ ID NO 738, SEQ ID NO 739, SEQ ID NO 740, SEQ ID NO 741, SEQ ID NO 742, SEQ ID NO 743, SEQ ID NO 744, SEQ ID NO 745, SEQ ID NO 746, SEQ ID NO 747, SEQ ID NO 748, SEQ ID NO 749, SEQ ID NO 750, SEQ ID NO 752, SEQ ID NO 753 and SEQ ID NO 754.

4. Use according to claim 2, wherein the nucleic acid molecule belongs to the group consisting in SEQ ID NO 79, SEQ ID NO 205, SEQ ID NO 243, SEQ ID NO 247, SEQ ID NO 341, SEQ ID NO 463, SEQ ID NO 655, SEQ ID NO 671, SEQ ID NO 684, SEQ ID NO 685, SEQ ID NO 686, SEQ ID NO 699, SEQ ID NO 717, SEQ ID NO 719, SEQ ID NO 726, SEQ ID NO 734 and SEQ ID NO 751.

5. Use according to claim 2, wherein the nucleic acid molecule belongs to the group consisting in SEQ ID NO 17, SEQ ID NO 37, SEQ ID NO 41, SEQ ID NO 43, SEQ ID NO 59, SEQ ID NO 67, SEQ ID NO 69, SEQ ID NO 73, SEQ ID NO 79, SEQ ID NO 85, SEQ ID NO 89, SEQ ID NO 91, SEQ ID NO 95, SEQ ID NO 97, SEQ ID NO 99, SEQ ID NO 101, SEQ ID NO 115, SEQ ID NO 155, SEQ ID NO 157, SEQ ID NO 161, SEQ ID NO 165, SEQ ID NO 167, SEQ ID NO 169, SEQ ID NO 189, SEQ ID NO 199, SEQ ID NO 203, SEQ ID NO 217, SEQ ID NO 223, SEQ ID NO 231, SEQ ID NO 233, SEQ ID NO 237, SEQ ID NO 239, SEQ ID NO 243, SEQ ID NO 293, SEQ ID NO 297, SEQ ID NO 303, SEQ ID NO 319, SEQ ID NO 321, SEQ ID NO 323, SEQ ID NO 325, SEQ ID NO 327, SEQ ID NO 337, SEQ ID NO 347, SEQ ID NO 351, SEQ ID NO 365, SEQ ID NO 369, SEQ ID NO 371, SEQ ID NO 375, SEQ ID NO 381, SEQ ID NO 387, SEQ ID NO 389, SEQ ID NO 401, SEQ ID NO 403, SEQ ID NO 407, SEQ ID NO 425, SEQ ID NO 435, SEQ ID NO 437, SEQ ID NO 445, SEQ ID NO 461, SEQ ID NO 463, SEQ ID NO 497, SEQ ID NO 517, SEQ ID NO 551, SEQ ID NO 623, SEQ ID NO 627, SEQ ID NO 642, SEQ ID NO 644, SEQ ID NO 645, SEQ ID NO 647, SEQ ID NO 649, SEQ ID NO 652, SEQ ID NO 653, SEQ ID NO 655, SEQ ID NO 656, SEQ ID NO 657, SEQ ID NO 661, SEQ ID NO 664, SEQ ID NO 665, SEQ ID NO 671, SEQ ID NO 672, SEQ ID NO 673, SEQ ID NO 674, SEQ ID NO 675, SEQ ID NO 677, SEQ ID NO 681, SEQ ID NO 684, SEQ ID NO 688, SEQ ID NO 691, SEQ ID NO 692, SEQ ID NO 695, SEQ ID NO 697, SEQ ID NO 700, SEQ ID NO 704, SEQ ID NO 705, SEQ ID NO 706, SEQ ID NO 710, SEQ ID NO 712, SEQ ID NO 714, SEQ ID NO 717, SEQ ID NO 718, SEQ ID NO 719, SEQ ID NO 720, SEQ ID NO 722, SEQ ID NO 724, SEQ ID NO 725, SEQ ID NO 728, SEQ ID NO 729, SEQ ID NO 734, SEQ ID NO 736, SEQ ID NO 737 and SEQ ID NO 738.

6. Use according to claim 5, wherein the nucleic acid molecule belongs to the group consisting in SEQ ID NO 17, SEQ ID NO 37, SEQ ID NO 41, SEQ ID NO 43, SEQ ID NO 59, SEQ ID NO 67, SEQ ID NO 69, SEQ ID NO 73, SEQ ID NO 85, SEQ ID NO 89, SEQ ID NO 91, SEQ ID NO 95, SEQ ID NO 97, SEQ ID NO 99, SEQ ID NO 101, SEQ ID NO 115, SEQ ID NO 155, SEQ ID NO 157, SEQ ID NO 161, SEQ ID NO 165, SEQ ID NO 167, SEQ ID NO 169, SEQ ID NO 189, SEQ ID NO 199, SEQ ID NO 203, SEQ ID NO 217, SEQ ID NO 223, SEQ ID NO 231, SEQ ID NO 233, SEQ ID NO 237, SEQ ID NO 239, SEQ ID NO 293, SEQ ID NO 297, SEQ ID NO 303, SEQ ID NO 319, SEQ ID NO 321, SEQ ID NO 323, SEQ ID NO 325, SEQ ID NO 327, SEQ ID NO 337, SEQ ID NO 347, SEQ ID NO 351, SEQ ID NO 365, SEQ ID NO 369, SEQ ID NO 371, SEQ ID NO 375, SEQ ID NO 381, SEQ ID NO 387, SEQ ID NO 389, SEQ ID NO 401, SEQ ID NO 403, SEQ ID NO 407, SEQ ID NO 425, SEQ ID NO 435, SEQ ID NO 437, SEQ ID NO 445, SEQ ID NO 461, SEQ ID NO 497, SEQ ID NO 517, SEQ ID NO 551, SEQ ID NO 623, SEQ ID NO 627, SEQ ID NO 642, SEQ ID NO 644, SEQ ID NO 645, SEQ ID NO 647, SEQ ID NO 649, SEQ ID NO 652, SEQ ID NO 653, SEQ ID NO 656, SEQ ID NO 657, SEQ ID NO 661, SEQ ID NO 664, SEQ ID NO 665, SEQ ID NO 672, SEQ ID NO 673, SEQ ID NO 674, SEQ ID NO 675, SEQ ID NO 677, SEQ ID NO 681, SEQ ID NO 688, SEQ ID NO 691, SEQ ID NO 692, SEQ ID NO 695, SEQ ID NO 697, SEQ ID NO 700, SEQ ID NO 704, SEQ ID NO 705, SEQ ID NO 706, SEQ ID NO 710, SEQ ID NO 712, SEQ ID NO 714, SEQ ID NO 718, SEQ ID NO 720, SEQ ID NO 722, SEQ ID NO 724, SEQ ID NO 725, SEQ ID NO 728, SEQ ID NO 729, SEQ ID NO 736, SEQ ID NO 737 and SEQ ID NO 738.

7. Use according to claim 5, wherein the nucleic acid molecule belongs to the group consisting in SEQ ID NO 79, SEQ ID NO 243, SEQ ID NO 463, SEQ ID NO 655, SEQ ID NO 671, SEQ ID NO 684, SEQ ID NO 717, SEQ ID NO 719 and SEQ ID NO 734.

8. Use according to claim 1, wherein the nucleic acid molecule belongs to the group consisting in SEQ ID NO 1, SEQ ID NO 3, SEQ ID NO 5, SEQ ID NO 7, SEQ ID NO 9, SEQ ID NO 11, SEQ ID NO 13, SEQ ID NO 15, SEQ ID NO 19, SEQ ID NO 21, SEQ ID NO 23, SEQ ID NO 25, SEQ ID NO 27, SEQ ID NO 29, SEQ ID NO 31, SEQ ID NO 33, SEQ ID NO 35, SEQ ID NO 53, SEQ ID NO 57, SEQ ID NO 75, SEQ ID NO 77, SEQ ID NO 87, SEQ ID NO 105, SEQ ID NO 107, SEQ ID NO 109, SEQ ID NO 111, SEQ ID NO 119, SEQ ID NO 123, SEQ ID NO 125, SEQ ID NO 127, SEQ ID NO 129, SEQ ID NO 131, SEQ ID NO 133, SEQ ID NO 135, SEQ ID NO 137, SEQ ID NO 139, SEQ ID NO 141, SEQ ID NO 143, SEQ ID NO 145, SEQ ID NO 147, SEQ ID NO 149, SEQ ID NO 151, SEQ ID NO 153, SEQ ID NO 159, SEQ ID NO 163, SEQ ID NO 173, SEQ ID NO 175, SEQ ID NO 177, SEQ ID NO 179, SEQ ID NO 181, SEQ ID NO 183, SEQ ID NO 185, SEQ ID NO 187, SEQ ID NO 193, SEQ ID NO 201, SEQ ID NO 209, SEQ ID NO 211, SEQ ID NO 221, SEQ ID NO 227, SEQ ID NO 245, SEQ ID NO 249, SEQ ID NO 251, SEQ ID NO 253, SEQ ID NO 255, SEQ ID NO 257, SEQ ID NO 259, SEQ ID NO 261, SEQ ID NO 265, SEQ ID NO 267, SEQ ID NO 269, SEQ ID NO 271, SEQ ID NO 273, SEQ ID NO 275, SEQ ID NO 277, SEQ ID NO 279, SEQ ID NO 283, SEQ ID NO 285, SEQ ID NO 287, SEQ ID NO 289, SEQ ID NO 291, SEQ ID NO 299, SEQ ID NO 301, SEQ ID NO 305, SEQ ID NO 307, SEQ ID NO 309, SEQ ID NO 311, SEQ ID NO 313, SEQ ID NO 315, SEQ ID NO 349, SEQ ID NO 383, SEQ ID NO 385, SEQ ID NO 391, SEQ ID NO 393, SEQ ID NO 411, SEQ ID NO 415, SEQ ID NO 417, SEQ ID NO 419, SEQ ID NO 421, SEQ ID NO 427, SEQ ID NO 429, SEQ ID NO 431, SEQ ID NO 433, SEQ ID NO 439, SEQ ID NO 443, SEQ ID NO 447, SEQ ID NO 449, SEQ ID NO 451, SEQ ID NO 453, SEQ ID NO 455, SEQ ID NO 457, SEQ ID NO 459, SEQ ID NO 465, SEQ ID NO 467, SEQ ID NO 469, SEQ ID NO 471, SEQ ID NO 473, SEQ ID NO 475, SEQ ID NO 477, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 499, SEQ ID NO 501, SEQ ID NO 503, SEQ ID NO 505, SEQ ID NO 507, SEQ ID NO 509, SEQ ID NO 511, SEQ ID NO 513, SEQ ID NO 515, SEQ ID NO 519, SEQ ID NO 521, SEQ ID NO 523, SEQ ID NO 525, SEQ ID NO 529, SEQ ID NO 531, SEQ ID NO 533, SEQ ID NO 535, SEQ ID NO 537, SEQ ID NO 539, SEQ ID NO 541, SEQ ID NO 543, SEQ ID NO 545, SEQ ID NO 547, SEQ ID NO 549, SEQ ID NO 553, SEQ ID NO 555, SEQ ID NO 557, SEQ ID NO 559, SEQ ID NO 561, SEQ ID NO 563, SEQ ID NO 565, SEQ ID NO 569, SEQ ID NO 571, SEQ ID NO 573, SEQ ID NO 575, SEQ ID NO 577, SEQ ID NO 581, SEQ ID NO 583, SEQ ID NO 585, SEQ ID NO 587, SEQ ID NO 589, SEQ ID NO 591, SEQ ID NO 593, SEQ ID NO 597, SEQ ID NO 603, SEQ ID NO 605, SEQ ID NO 607, SEQ ID NO 611, SEQ ID NO 613, SEQ ID NO 615, SEQ ID NO 617, SEQ ID NO 619, SEQ ID NO 621, SEQ ID NO 629, SEQ ID NO 631, SEQ ID NO 633, SEQ ID NO 635, SEQ ID NO 637, SEQ ID NO 639, SEQ ID NO 701, SEQ ID NO 716, SEQ ID NO 721 and SEQ ID NO 723.

9. Use according to claim 8, wherein the nucleic acid molecule belongs to the group consisting in SEQ ID NO 1, SEQ ID NO 3, SEQ ID NO 5, SEQ ID NO 7, SEQ ID NO 11, SEQ ID NO 13, SEQ ID NO 15, SEQ ID NO 19, SEQ ID NO 21, SEQ ID NO 25, SEQ ID NO 27, SEQ ID NO 29, SEQ ID NO 31, SEQ ID NO 33, SEQ ID NO 35, SEQ ID NO 53, SEQ ID NO 57, SEQ ID NO 75, SEQ ID NO 77, SEQ ID NO 87, SEQ ID NO 105, SEQ ID NO 107, SEQ ID NO 109, SEQ ID NO 119, SEQ ID NO 123, SEQ ID NO 125, SEQ ID NO 127, SEQ ID NO 129, SEQ ID NO 135, SEQ ID NO 137, SEQ ID NO 139, SEQ ID NO 143, SEQ ID NO 145, SEQ ID NO 147, SEQ ID NO 149, SEQ ID NO 151, SEQ ID NO 153, SEQ ID NO 159, SEQ ID NO 163, SEQ ID NO 173, SEQ ID NO 175, SEQ ID NO 177, SEQ ID NO 179, SEQ ID NO 183, SEQ ID NO 185, SEQ ID NO 187, SEQ ID NO 193, SEQ ID NO 201, SEQ ID NO 209, SEQ ID NO 221, SEQ ID NO 227, SEQ ID NO 245, SEQ ID NO 249, SEQ ID NO 251, SEQ ID NO 253, SEQ ID NO 255, SEQ ID NO 257, SEQ ID NO 259, SEQ ID NO 261, SEQ ID NO 265, SEQ ID NO 267, SEQ ID NO 269, SEQ ID NO 271, SEQ ID NO 273, SEQ ID NO 277, SEQ ID NO 279, SEQ ID NO 285, SEQ ID NO 287, SEQ ID NO 291, SEQ ID NO 299, SEQ ID NO 301, SEQ ID NO 305, SEQ ID NO 309, SEQ ID NO 311, SEQ ID NO 313, SEQ ID NO 383, SEQ ID NO 385, SEQ ID NO 391, SEQ ID NO 393, SEQ ID NO 411, SEQ ID NO 415, SEQ ID NO 417, SEQ ID NO 419, SEQ ID NO 421, SEQ ID NO 427, SEQ ID NO 429, SEQ ID NO 431, SEQ ID NO 433, SEQ ID NO 439, SEQ ID NO 443, SEQ ID NO 447, SEQ ID NO 449, SEQ ID NO 453, SEQ ID NO 455, SEQ ID NO 457, SEQ ID NO 459, SEQ ID NO 465, SEQ ID NO 467, SEQ ID NO 469, SEQ ID NO 473, SEQ ID NO 475, SEQ ID NO 477, SEQ ID NO 499, SEQ ID NO 501, SEQ ID NO 503, SEQ ID NO 505, SEQ ID NO 507, SEQ ID NO 509, SEQ ID NO 511, SEQ ID NO 513, SEQ ID NO 515, SEQ ID NO 519, SEQ ID NO 525, SEQ ID NO 529, SEQ ID NO 531, SEQ ID NO 533, SEQ ID NO 535, SEQ ID NO 537, SEQ ID NO 539, SEQ ID NO 541, SEQ ID NO 543, SEQ ID NO 545, SEQ ID NO 547, SEQ ID NO 549, SEQ ID NO 553, SEQ ID NO 555, SEQ ID NO 557, SEQ ID NO 559, SEQ ID NO 561, SEQ ID NO 563, SEQ ID NO 565, SEQ ID NO 569, SEQ ID NO 571, SEQ ID NO 573, SEQ ID NO 575, SEQ ID NO 577, SEQ ID NO 581, SEQ ID NO 585, SEQ ID NO 587, SEQ ID NO 589, SEQ ID NO 591, SEQ ID NO 593, SEQ ID NO 603, SEQ ID NO 605, SEQ ID NO 607, SEQ ID NO 611, SEQ ID NO 613, SEQ ID NO 615, SEQ ID NO 617, SEQ ID NO 619, SEQ ID NO 621, SEQ ID NO 629, SEQ ID NO 631, SEQ ID NO 633, SEQ ID NO 635, SEQ ID NO 637, SEQ ID NO 639, SEQ ID NO 701, SEQ ID NO 716, SEQ ID NO 721 and SEQ ID NO 723

10. Use according to claim 8, wherein the nucleic acid molecule belongs to the group consisting in SEQ ID NO 9, SEQ ID NO 23, SEQ ID NO 111, SEQ ID NO 131, SEQ ID NO 133, SEQ ID NO 141, SEQ ID NO 181, SEQ ID NO 275, SEQ ID NO 283, SEQ ID NO 289, SEQ ID NO 307, SEQ ID NO 315, SEQ ID NO 349, SEQ ID NO 451, SEQ ID NO 471, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 521, SEQ ID NO 523, SEQ ID NO 583 and SEQ ID NO 597.

11. Use according to claim 8, wherein the nucleic acid molecule belongs to the group consisting in SEQ ID NO 1, SEQ ID NO 3, SEQ ID NO 5, SEQ ID NO 7, SEQ ID NO 9, SEQ ID NO 11, SEQ ID NO 15, SEQ ID NO 19, SEQ ID NO 21, SEQ ID NO 23, SEQ ID NO 27, SEQ ID NO 31, SEQ ID NO 35, SEQ ID NO 77, SEQ ID NO 87, SEQ ID NO 111, SEQ ID NO 119, SEQ ID NO 123, SEQ ID NO 125, SEQ ID NO 127, SEQ ID NO 129, SEQ ID NO 131, SEQ ID NO 133, SEQ ID NO 135, SEQ ID NO 139, SEQ ID NO 141, SEQ ID NO 143, SEQ ID NO 145, SEQ ID NO 151, SEQ ID NO 153, SEQ ID NO 159, SEQ ID NO 163, SEQ ID NO 181, SEQ ID NO 183, SEQ ID NO 185, SEQ ID NO 201, SEQ ID NO 227, SEQ ID NO 249, SEQ ID NO 251, SEQ ID NO 253, SEQ ID NO 257, SEQ ID NO 259, SEQ ID NO 265, SEQ ID NO 267, SEQ ID NO 269, SEQ ID NO 271, SEQ ID NO 273, SEQ ID NO 275, SEQ ID NO 277, SEQ ID NO 279, SEQ ID NO 283, SEQ ID NO 285, SEQ ID NO 287, SEQ ID NO 289, SEQ ID NO 299, SEQ ID NO 301, SEQ ID NO 305, SEQ ID NO 307, SEQ ID NO 309, SEQ ID NO 311, SEQ ID NO 313, SEQ ID NO 315, SEQ ID NO 349, SEQ ID NO 385, SEQ ID NO 393, SEQ ID NO 411, SEQ ID NO 417, SEQ ID NO 419, SEQ ID NO 421, SEQ ID NO 427, SEQ ID NO 429, SEQ ID NO 431, SEQ ID NO 439, SEQ ID NO 443, SEQ ID NO 447, SEQ ID NO 449, SEQ ID NO 451, SEQ ID NO 453, SEQ ID NO 455, SEQ ID NO 457, SEQ ID NO 459, SEQ ID NO 469, SEQ ID NO 471, SEQ ID NO 473, SEQ ID NO 475, SEQ ID NO 477, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 501, SEQ ID NO 503, SEQ ID NO 507, SEQ ID NO 511, SEQ ID NO 513, SEQ ID NO 515, SEQ ID NO 519, SEQ ID NO 521, SEQ ID NO 523, SEQ ID NO 525, SEQ ID NO 531, SEQ ID NO 533, SEQ ID NO 535, SEQ ID NO 537, SEQ ID NO 539, SEQ ID NO 541, SEQ ID NO 547, SEQ ID NO 549, SEQ ID NO 553, SEQ ID NO 555, SEQ ID NO 557, SEQ ID NO 561, SEQ ID NO 563, SEQ ID NO 565, SEQ ID NO 569, SEQ ID NO 571, SEQ ID NO 573, SEQ ID NO 575, SEQ ID NO 577, SEQ ID NO 581, SEQ ID NO 583, SEQ ID NO 585, SEQ ID NO 591, SEQ ID NO 593, SEQ ID NO 597, SEQ ID NO 603, SEQ ID NO 611, SEQ ID NO 613, SEQ ID NO 617, SEQ ID NO 629, SEQ ID NO 631, SEQ ID NO 633, SEQ ID NO 635 and SEQ ID NO 637.

12. Use according to claim 11, wherein the nucleic acid molecule belongs to the group consisting in SEQ ID NO 1, SEQ ID NO 3, SEQ ID NO 5, SEQ ID NO 7, SEQ ID NO 11, SEQ ID NO 15, SEQ ID NO 19, SEQ ID NO 21, SEQ ID NO 27, SEQ ID NO 31, SEQ ID NO 35, SEQ ID NO 77, SEQ ID NO 87, SEQ ID NO 119, SEQ ID NO 123, SEQ ID NO 125, SEQ ID NO 127, SEQ ID NO 129, SEQ ID NO 135, SEQ ID NO 139, SEQ ID NO 143, SEQ ID NO 145, SEQ ID NO 151, SEQ ID NO 153, SEQ ID NO 159, SEQ ID NO 163, SEQ ID NO 183, SEQ ID NO 185, SEQ ID NO 201, SEQ ID NO 227, SEQ ID NO 249, SEQ ID NO 251, SEQ ID NO 253, SEQ ID NO 257, SEQ ID NO 259, SEQ ID NO 265, SEQ ID NO 267, SEQ ID NO 269, SEQ ID NO 271, SEQ ID NO 273, SEQ ID NO 277, SEQ ID NO 279, SEQ ID NO 285, SEQ ID NO 287, SEQ ID NO 299, SEQ ID NO 301, SEQ ID NO 305, SEQ ID NO 309, SEQ ID NO 311, SEQ ID NO 313, SEQ ID NO 385, SEQ ID NO 393, SEQ ID NO 411, SEQ ID NO 417, SEQ ID NO 419, SEQ ID NO 421, SEQ ID NO 427, SEQ ID NO 429, SEQ ID NO 431, SEQ ID NO 439, SEQ ID NO 443, SEQ ID NO 447, SEQ ID NO 449, SEQ ID NO 453, SEQ ID NO 455, SEQ ID NO 457, SEQ ID NO 459, SEQ ID NO 469, SEQ ID NO 473, SEQ ID NO 475, SEQ ID NO 477, SEQ ID NO 501, SEQ ID NO 503, SEQ ID NO 507, SEQ ID NO 511, SEQ ID NO 513, SEQ ID NO 515, SEQ ID NO 519, SEQ ID NO 525, SEQ ID NO 531, SEQ ID NO 533, SEQ ID NO 535, SEQ ID NO 537, SEQ ID NO 539, SEQ ID NO 541, SEQ ID NO 547, SEQ ID NO 549, SEQ ID NO 553, SEQ ID NO 555, SEQ ID NO 557, SEQ ID NO 561, SEQ ID NO 563, SEQ ID NO 565, SEQ ID NO 569, SEQ ID NO 571, SEQ ID NO 573, SEQ ID NO 575, SEQ ID NO 577, SEQ ID NO 581, SEQ ID NO 585, SEQ ID NO 591, SEQ ID NO 593, SEQ ID NO 603, SEQ ID NO 611, SEQ ID NO 613, SEQ ID NO 617, SEQ ID NO 629, SEQ ID NO 631, SEQ ID NO 633, SEQ ID NO 635 and SEQ ID NO 637.

13. Use according to claim 11, wherein the nucleic acid molecule belongs to the group consisting in SEQ ID NO 9, SEQ ID NO 23, SEQ ID NO 111, SEQ ID NO 131, SEQ ID NO 133, SEQ ID NO 141, SEQ ID NO 181, SEQ ID NO 275, SEQ ID NO 283, SEQ ID NO 289, SEQ ID NO 307, SEQ ID NO 315, SEQ ID NO 349, SEQ ID NO 451, SEQ ID NO 471, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 521, SEQ ID NO 523, SEQ ID NO 583 and SEQ ID NO 597.

14. Use according to claim 10, wherein the nucleic acid molecule belongs to the group consisting in SEQ ID NO 9, SEQ ID NO 11, SEQ ID NO 23, SEQ ID NO 25, SEQ ID NO 29, SEQ ID NO 31, SEQ ID NO 33, SEQ ID NO 35, SEQ ID NO 53, SEQ ID NO 57, SEQ ID NO 75, SEQ ID NO 77, SEQ ID NO 87, SEQ ID NO 105, SEQ ID NO 107, SEQ ID NO 109, SEQ ID NO 111, SEQ ID NO 123, SEQ ID NO 125, SEQ ID NO 127, SEQ ID NO 129, SEQ ID NO 131, SEQ ID NO 133, SEQ ID NO 139, SEQ ID NO 143, SEQ ID NO 147, SEQ ID NO 151, SEQ ID NO 153, SEQ ID NO 159, SEQ ID NO 163, SEQ ID NO 173, SEQ ID NO 175, SEQ ID NO 177, SEQ ID NO 179, SEQ ID NO 181, SEQ ID NO 185, SEQ ID NO 187, SEQ ID NO 193, SEQ ID NO 201, SEQ ID NO 209, SEQ ID NO 211, SEQ ID NO 221, SEQ ID NO 227, SEQ ID NO 249, SEQ ID NO 253, SEQ ID NO 257, SEQ ID NO 259, SEQ ID NO 261, SEQ ID NO 265, SEQ ID NO 269, SEQ ID NO 275, SEQ ID NO 277, SEQ ID NO 279, SEQ ID NO 283, SEQ ID NO 285, SEQ ID NO 289, SEQ ID NO 301, SEQ ID NO 305, SEQ ID NO 307, SEQ ID NO 311, SEQ ID NO 315, SEQ ID NO 349, SEQ ID NO 383, SEQ ID NO 391, SEQ ID NO 393, SEQ ID NO 411, SEQ ID NO 415, SEQ ID NO 419, SEQ ID NO 421, SEQ ID NO 427, SEQ ID NO 429, SEQ ID NO 431, SEQ ID NO 433, SEQ ID NO 439, SEQ ID NO 443, SEQ ID NO 447, SEQ ID NO 451, SEQ ID NO 465, SEQ ID NO 467, SEQ ID NO 471, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 499, SEQ ID NO 501, SEQ ID NO 503, SEQ ID NO 505, SEQ ID NO 509, SEQ ID NO 511, SEQ ID NO 519, SEQ ID NO 521, SEQ ID NO 523, SEQ ID NO 525, SEQ ID NO 529, SEQ ID NO 531, SEQ ID NO 539, SEQ ID NO 543, SEQ ID NO 545, SEQ ID NO 547, SEQ ID NO 553, SEQ ID NO 555, SEQ ID NO 557, SEQ ID NO 559, SEQ ID NO 563, SEQ ID NO 565, SEQ ID NO 573, SEQ ID NO 575, SEQ ID NO 581, SEQ ID NO 583, SEQ ID NO 587, SEQ ID NO 589, SEQ ID NO 593, SEQ ID NO 597, SEQ ID NO 605, SEQ ID NO 607, SEQ ID NO 611, SEQ ID NO 615, SEQ ID NO 617, SEQ ID NO 621, SEQ ID NO 629, SEQ ID NO 631, SEQ ID NO 639, SEQ ID NO 701, SEQ ID NO 716 and SEQ ID NO 721.

15. Use according to claim 8, wherein the nucleic acid molecule belongs to the group consisting in SEQ ID NO 9, SEQ ID NO 11, SEQ ID NO 23, SEQ ID NO 27, SEQ ID NO 31, SEQ ID NO 35, SEQ ID NO 77, SEQ ID NO 87, SEQ ID NO 111, SEQ ID NO 123, SEQ ID NO 125, SEQ ID NO 127, SEQ ID NO 129, SEQ ID NO 131, SEQ ID NO 133, SEQ ID NO 135, SEQ ID NO 139, SEQ ID NO 143, SEQ ID NO 151, SEQ ID NO 153, SEQ ID NO 159, SEQ ID NO 163, SEQ ID NO 181, SEQ ID NO 185, SEQ ID NO 201, SEQ ID NO 227, SEQ ID NO 249, SEQ ID NO 253, SEQ ID NO 257, SEQ ID NO 259, SEQ ID NO 265, SEQ ID NO 267, SEQ ID NO 269, SEQ ID NO 275, SEQ ID NO 277, SEQ ID NO 279, SEQ ID NO 283, SEQ ID NO 285, SEQ ID NO 289, SEQ ID NO 301, SEQ ID NO 305, SEQ ID NO 307, SEQ ID NO 309, SEQ ID NO 311, SEQ ID NO 315, SEQ ID NO 349, SEQ ID NO 393, SEQ ID NO 411, SEQ ID NO 419, SEQ ID NO 421, SEQ ID NO 427, SEQ ID NO 429, SEQ ID NO 431, SEQ ID NO 439, SEQ ID NO 443, SEQ ID NO 447, SEQ ID NO 449, SEQ ID NO 451, SEQ ID NO 469, SEQ ID NO 471, SEQ ID NO 475, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 501, SEQ ID NO 503, SEQ ID NO 507, SEQ ID NO 511, SEQ ID NO 515, SEQ ID NO 519, SEQ ID NO 521, SEQ ID NO 523, SEQ ID NO 525, SEQ ID NO 531, SEQ ID NO 533, SEQ ID NO 535, SEQ ID NO 537, SEQ ID NO 539, SEQ ID NO 541, SEQ ID NO 547, SEQ ID NO 549, SEQ ID NO 553, SEQ ID NO 555, SEQ ID NO 557, SEQ ID NO 561, SEQ ID NO 563, SEQ ID NO 565, SEQ ID NO 569, SEQ ID NO 573, SEQ ID NO 575, SEQ ID NO 577, SEQ ID NO 581, SEQ ID NO 583, SEQ ID NO 593, SEQ ID NO 597, SEQ ID NO 611, SEQ ID NO 617, SEQ ID NO 629, SEQ ID NO 631 and SEQ ID NO 637.

16. Use according to claim 15, wherein the nucleic acid molecule belongs to the group consisting in SEQ ID NO 11, SEQ ID NO 27, SEQ ID NO 31, SEQ ID NO 35, SEQ ID NO 77, SEQ ID NO 87, SEQ ID NO 123, SEQ ID NO 125, SEQ ID NO 127, SEQ ID NO 129, SEQ ID NO 135, SEQ ID NO 139, SEQ ID NO 143, SEQ ID NO 151, SEQ ID NO 153, SEQ ID NO 159, SEQ ID NO 163, SEQ ID NO 185, SEQ ID NO 201, SEQ ID NO 227, SEQ ID NO 249, SEQ ID NO 253, SEQ ID NO 257, SEQ ID NO 259, SEQ ID NO 265, SEQ ID NO 267, SEQ ID NO 269, SEQ ID NO 277, SEQ ID NO 279, SEQ ID NO 285, SEQ ID NO 301, SEQ ID NO 305, SEQ ID NO 309, SEQ ID NO 311, SEQ ID NO 393, SEQ ID NO 411, SEQ ID NO 419, SEQ ID NO 421, SEQ ID NO 427, SEQ ID NO 429, SEQ ID NO 431, SEQ ID NO 439, SEQ ID NO 443, SEQ ID NO 447, SEQ ID NO 449, SEQ ID NO 469, SEQ ID NO 475, SEQ ID NO 501, SEQ ID NO 503, SEQ ID NO 507, SEQ ID NO 511, SEQ ID NO 515, SEQ ID NO 519, SEQ ID NO 525, SEQ ID NO 531, SEQ ID NO 533, SEQ ID NO 535, SEQ ID NO 537, SEQ ID NO 539, SEQ ID NO 541, SEQ ID NO 547, SEQ ID NO 549, SEQ ID NO 553, SEQ ID NO 555, SEQ ID NO 557, SEQ ID NO 561, SEQ ID NO 563, SEQ ID NO 565, SEQ ID NO 569, SEQ ID NO. 573, SEQ ID NO 575, SEQ ID NO 577, SEQ ID NO 581, SEQ ID NO 593, SEQ ID NO 611, SEQ ID NO 617, SEQ ID NO 629, SEQ ID NO 631 and SEQ ID NO 637.

17. Use according to claim 8, wherein the nucleic acid molecule belongs to the group consisting in SEQ ID NO 9, SEQ ID NO 23, SEQ ID NO 111, SEQ ID NO 131, SEQ ID NO 133, SEQ ID NO 181, SEQ ID NO 275, SEQ ID NO 283, SEQ ID NO 289, SEQ ID NO 307, SEQ ID NO 315, SEQ ID NO 349, SEQ ID NO 451, SEQ ID NO 471, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 521, SEQ ID NO 523, SEQ ID NO 583 and SEQ ID NO 597.

18. Use according to claims 2 and 8, wherein the nucleic acid molecule belongs to the group consisting in SEQ ID NO 1, SEQ ID NO 3, SEQ ID NO 5, SEQ ID NO 7, SEQ ID NO 9, SEQ ID NO 11, SEQ ID NO 15, SEQ ID NO 17, SEQ ID NO 19, SEQ ID NO 21, SEQ ID NO 23, SEQ ID NO 27, SEQ ID NO 31, SEQ ID NO 35, SEQ ID NO 37, SEQ ID NO 41, SEQ ID NO 43, SEQ ID NO 59, SEQ ID NO 67, SEQ ID NO 69, SEQ ID NO 73, SEQ ID NO 77, SEQ ID NO 79, SEQ ID NO 85, SEQ ID NO 87, SEQ ID NO 89, SEQ ID NO 91, SEQ ID NO 95, SEQ ID NO 97, SEQ ID NO 99, SEQ ID NO 101, SEQ ID NO 111, SEQ ID NO 115, SEQ ID NO 119, SEQ ID NO 123, SEQ ID NO 125, SEQ ID NO 127, SEQ ID NO 129, SEQ ID NO 131, SEQ ID NO 133, SEQ ID NO 135, SEQ ID NO 139, SEQ ID NO 141, SEQ ID NO 143, SEQ ID NO 145, SEQ ID NO 151, SEQ ID NO 153, SEQ ID NO 155, SEQ ID NO 157, SEQ ID NO 159, SEQ ID NO 161, SEQ ID NO 163, SEQ ID NO 165, SEQ ID NO 167, SEQ ID NO 169, SEQ ID NO 181, SEQ ID NO 183, SEQ ID NO 185, SEQ ID NO 189, SEQ ID NO 199, SEQ ID NO 201, SEQ ID NO 203, SEQ ID NO 217, SEQ ID NO 223, SEQ ID NO 227, SEQ ID NO 231, SEQ ID NO 233, SEQ ID NO 237, SEQ ID NO 239, SEQ ID NO 243, SEQ ID NO 249, SEQ ID NO 251, SEQ ID NO 253, SEQ ID NO 257, SEQ ID NO 259, SEQ ID NO 265, SEQ ID NO 267, SEQ ID NO 269, SEQ ID NO 271, SEQ ID NO 273, SEQ ID NO 275, SEQ ID NO 277, SEQ ID NO 279, SEQ ID NO 283, SEQ ID NO 285, SEQ ID NO 287, SEQ ID NO 289, SEQ ID NO 293, SEQ ID NO 297, SEQ ID NO 299, SEQ ID NO 301, SEQ ID NO 303, SEQ ID NO 305, SEQ ID NO 307, SEQ ID NO 309, SEQ ID NO 31 l, SEQ ID NO 313, SEQ ID NO 315, SEQ ID NO 319, SEQ ID NO 321, SEQ ID NO 323, SEQ ID NO 325, SEQ ID NO 327, SEQ ID NO 337, SEQ ID NO 347, SEQ ID NO 349, SEQ ID NO 351, SEQ ID NO 365, SEQ ID NO 369, SEQ ID NO 371, SEQ ID NO 375, SEQ ID NO 381, SEQ ID NO 385, SEQ ID NO 387, SEQ ID NO 389, SEQ ID NO 393, SEQ ID NO 401, SEQ ID NO 403, SEQ ID NO 407, SEQ ID NO 411, SEQ ID NO 417, SEQ ID NO 419, SEQ ID NO 421, SEQ ID NO 425, SEQ ID NO 427, SEQ ID NO 429, SEQ ID NO 431, SEQ ID NO 435, SEQ ID NO 437, SEQ ID NO 439, SEQ ID NO 443, SEQ ID NO 445, SEQ ID NO 447, SEQ ID NO 449, SEQ ID NO 451, SEQ ID NO 453, SEQ ID NO 455, SEQ ID NO 457, SEQ ID NO 459, SEQ ID NO 461, SEQ ID NO 463, SEQ ID NO 469, SEQ ID NO 471, SEQ ID NO 473, SEQ ID NO 475, SEQ ID NO 477, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 497, SEQ ID NO 501, SEQ ID NO 503, SEQ ID NO 507, SEQ ID NO 511, SEQ ID NO 513, SEQ ID NO 515, SEQ ID NO 517, SEQ ID NO 519, SEQ ID NO 521, SEQ ID NO 523, SEQ ID NO 525, SEQ ID NO 531, SEQ ID NO 533, SEQ ID NO 535, SEQ ID NO 537, SEQ ID NO 539, SEQ ID NO 541, SEQ ID NO 547, SEQ ID NO 549, SEQ ID NO 551, SEQ ID NO 553, SEQ ID NO 555, SEQ ID NO 557, SEQ ID NO 561, SEQ ID NO 563, SEQ ID NO 565, SEQ ID NO 569, SEQ ID NO 571, SEQ ID NO 573, SEQ ID NO 575, SEQ ID NO 577, SEQ ID NO 581, SEQ ID NO 583, SEQ ID NO 585, SEQ ID NO 591, SEQ ID NO 593, SEQ ID NO 597, SEQ ID NO 603, SEQ ID NO 611, SEQ ID NO 613, SEQ ID NO 617, SEQ ID NO 623, SEQ ID NO 627, SEQ ID NO 629, SEQ ID NO 631, SEQ ID NO 633, SEQ ID NO 635, SEQ ID NO 637, SEQ ID NO 642, SEQ ID NO 644, SEQ ID NO 645, SEQ ID NO 647, SEQ ID NO 649, SEQ ID NO 652, SEQ ID NO 653, SEQ ID NO 655, SEQ ID NO 656, SEQ ID NO 657, SEQ ID NO 661, SEQ ID NO 664, SEQ ID NO 665, SEQ ID NO 671, SEQ ID NO 672, SEQ ID NO 673, SEQ ID NO 674, SEQ ID NO 675, SEQ ID NO 677, SEQ ID NO 681, SEQ ID NO 684, SEQ ID NO 688, SEQ ID NO 691, SEQ ID NO 692, SEQ ID NO 695, SEQ ID NO 697, SEQ ID NO 700, SEQ ID NO 704, SEQ ID NO 705, SEQ ID NO 706, SEQ ID NO 710, SEQ ID NO 712, SEQ ID NO 714, SEQ ID NO 717, SEQ ID NO 718, SEQ ID NO 719, SEQ ID NO 720, SEQ ID NO 722, SEQ ID NO 724, SEQ ID NO 725, SEQ ID NO 728, SEQ ID NO 729, SEQ ID NO 734, SEQ ID NO 736, SEQ ID NO 737 and SEQ ID NO 738.

19. Use according to claim 18, wherein the nucleic acid molecule belongs to the group consisting in SEQ ID NO 9, SEQ ID NO 11, SEQ ID NO 17, SEQ ID NO 23, SEQ ID NO 27, SEQ ID NO 31, SEQ ID NO 35, SEQ ID NO 37, SEQ ID NO 41, SEQ ID NO 43, SEQ ID NO 59, SEQ ID NO 69, SEQ ID NO 73, SEQ ID NO 77, SEQ ID NO 79, SEQ ID NO 85, SEQ ID NO 87, SEQ ID NO 89, SEQ ID NO 91, SEQ ID NO 95, SEQ ID NO 97, SEQ ID NO 99, SEQ ID NO 101, SEQ ID NO 111, SEQ ID NO 115, SEQ ID NO 123, SEQ ID NO 125, SEQ ID NO 127, SEQ ID NO 129, SEQ ID NO 131, SEQ ID NO 133, SEQ ID NO 135, SEQ ID NO 139, SEQ ID NO 143, SEQ ID NO 151, SEQ ID NO 153, SEQ ID NO 155, SEQ ID NO 157, SEQ ID NO 159, SEQ ID NO 161, SEQ ID NO 163, SEQ ID NO 165, SEQ ID NO 167, SEQ ID NO 181, SEQ ID NO 185, SEQ ID NO 189, SEQ ID NO 199, SEQ ID NO 201, SEQ ID NO 203, SEQ ID NO 217, SEQ ID NO 223, SEQ ID NO 227, SEQ ID NO 231, SEQ ID NO 233, SEQ ID NO 237, SEQ ID NO 239, SEQ ID NO 243, SEQ ID NO 249, SEQ ID NO 253, SEQ ID NO 257, SEQ ID NO 259, SEQ ID NO 265, SEQ ID NO 267, SEQ ID NO 269, SEQ ID NO 275, SEQ ID NO 277, SEQ ID NO 279, SEQ ID NO 283, SEQ ID NO 285, SEQ ID NO 289, SEQ ID NO 293, SEQ ID NO 297, SEQ ID NO 301, SEQ ID NO 303, SEQ ID NO 305, SEQ ID NO 307, SEQ ID NO 309, SEQ ID NO 311, SEQ ID NO 315, SEQ ID NO 319, SEQ ID NO 321, SEQ ID NO 323, SEQ ID NO 325, SEQ ID NO 327, SEQ ID NO 337, SEQ ID NO 347, SEQ ID NO 349, SEQ ID NO 351, SEQ ID NO 365, SEQ ID NO 369, SEQ ID NO 371, SEQ ID NO 375, SEQ ID NO 381, SEQ ID NO 387, SEQ ID NO 389, SEQ ID NO 393, SEQ ID NO 401, SEQ ID NO 403, SEQ ID NO 407, SEQ ID NO 411, SEQ ID NO 419, SEQ ID NO 421, SEQ ID NO 425, SEQ ID NO 427, SEQ ID NO 429, SEQ ID NO 431, SEQ ID NO 435, SEQ ID NO 437, SEQ ID NO 439, SEQ ID NO 443, SEQ ID NO 445, SEQ ID NO 447, SEQ ID NO 449, SEQ ID NO 451, SEQ ID NO 461, SEQ ID NO 463, SEQ ID NO 469, SEQ ID NO 471, SEQ ID NO 475, SEQ ID NO 479, SEQ ID NO 481, SEQ ID NO 483, SEQ ID NO 485, SEQ ID NO 487, SEQ ID NO 489, SEQ ID NO 491, SEQ ID NO 493, SEQ ID NO 495, SEQ ID NO 497, SEQ ID NO 501, SEQ ID NO 503, SEQ ID NO 507, SEQ ID NO 511, SEQ ID NO 515, SEQ ID NO 517, SEQ ID NO 519, SEQ ID NO 521, SEQ ID NO 523, SEQ ID NO 525, SEQ ID NO 531, SEQ ID NO 533, SEQ ID NO 535, SEQ ID NO 537, SEQ ID NO 539, SEQ ID NO 541, SEQ ID NO 547, SEQ ID NO 549, SEQ ID NO 551, SEQ ID NO 553, SEQ ID NO 555, SEQ ID NO 557, SEQ ID NO 561, SEQ ID NO 563, SEQ ID NO 565, SEQ ID NO 569, SEQ ID NO 573, SEQ ID NO 575, SEQ ID NO 577, SEQ ID NO 581, SEQ ID NO 583, SEQ ID NO 593, SEQ ID NO 597, SEQ ID NO 611, SEQ ID NO 617, SEQ ID NO 623, SEQ ID NO 627, SEQ ID NO 629, SEQ ID NO 631, SEQ ID NO 637, SEQ ID NO 642, SEQ ID NO 644, SEQ ID NO 645, SEQ ID NO 647, SEQ ID NO 649, SEQ ID NO 652, SEQ ID NO 653, SEQ ID NO 655, SEQ ID NO 656, SEQ ID NO 657, SEQ ID NO 661, SEQ ID NO 664, SEQ ID NO 665, SEQ ID NO 671, SEQ ID NO 672, SEQ ID NO 673, SEQ ID NO 674, SEQ ID NO 675, SEQ ID NO 677, SEQ ID NO 681, SEQ ID NO 684, SEQ ID NO 688, SEQ ID NO 691, SEQ ID NO 692, SEQ ID NO 695, SEQ ID NO 697, SEQ ID NO 700, SEQ ID NO 704, SEQ ID NO 705, SEQ ID NO 706, SEQ ID NO 710, SEQ ID NO 712, SEQ ID NO 714, SEQ ID NO 717, SEQ ID NO 718, SEQ ID NO 719, SEQ ID NO 722, SEQ ID NO 724, SEQ ID NO 725, SEQ ID NO 728, SEQ ID NO 729, SEQ ID NO 734, SEQ ID NO 736, SEQ ID NO 737 and SEQ ID NO 738.

20. Use of one element chosen among :
- at least a protein coded by a nucleic acid molecule according to anyone of claims 1 to 19, said protein comprising or constituted by an amino acid sequence belonging to the group consisting in SEQ ID NO 2q, q varying from 1 to 320, or
- at least a variant of said protein, said variant presenting a sequence identity of at least 70%, preferably 80%, more preferably 90% with respect to said protein,
- at least a fragment of said protein, said fragment being able to be recognized by an antibody specifically directed against said protein.

21. Use of at least one antibody **characterized in that** it specifically recognize a protein according to claim 20.

22. Use according to any of claims 1-21, wherein the somatic or ovarian cancer cells are a solid tumors or hematological neoplasms.

23. One at least of the nucleic acid molecules belonging to the group consisting in SEQ ID NO 17, SEQ ID NO 37, SEQ ID NO 39, SEQ ID NO 41, SEQ ID NO 43, SEQ ID NO 45, SEQ ID NO 47, SEQ ID NO 49, SEQ ID NO 51, SEQ ID NO 55, SEQ ID NO 59, SEQ ID NO 61, SEQ ID NO 63, SEQ ID NO 65, SEQ ID NO 67, SEQ ID NO 69, SEQ ID NO 71, SEQ ID NO 73, SEQ ID NO 79, SEQ ID NO 81, SEQ ID NO 83, SEQ ID NO 85, SEQ ID NO 89, SEQ ID NO 91, SEQ ID NO 93, SEQ ID NO 95, SEQ ID NO 97, SEQ ID NO 99, SEQ ID NO 101, SEQ ID NO 103, SEQ ID NO 113, SEQ ID NO 115, SEQ ID NO 117, SEQ ID NO 121, SEQ ID NO 155, SEQ ID NO 157, SEQ ID NO 161, SEQ ID NO 165, SEQ ID NO 167, SEQ ID NO 169, SEQ ID NO 171, SEQ ID NO 189, SEQ ID NO 191, SEQ ID NO 195, SEQ ID NO 197, SEQ ID NO 199, SEQ ID NO 203, SEQ ID NO 205, SEQ ID NO 207, SEQ ID NO 213, SEQ ID NO 215, SEQ ID NO 217, SEQ ID NO 219, SEQ ID NO 223, SEQ ID NO 225, SEQ ID NO 229, SEQ ID NO 231, SEQ ID NO 233, SEQ ID NO 235, SEQ ID NO 237, SEQ ID NO 239, SEQ ID NO 241, SEQ ID NO 243, SEQ ID NO 247, SEQ ID NO 263, SEQ ID NO 281, SEQ ID NO 293, SEQ ID NO 295, SEQ ID NO 297, SEQ ID NO 303, SEQ ID NO 317, SEQ ID NO 319, SEQ ID NO 321, SEQ ID NO 323, SEQ ID NO 325, SEQ ID NO 327, SEQ ID NO 329, SEQ ID NO 331, SEQ ID NO 333, SEQ ID NO 335, SEQ ID NO 337, SEQ ID NO 339, SEQ ID NO 341, SEQ ID NO 343, SEQ ID NO 345, SEQ ID NO 347, SEQ ID NO 351, SEQ ID NO 353, SEQ ID NO 355, SEQ ID NO 357, SEQ ID NO 359, SEQ ID NO 361, SEQ ID NO 363, SEQ ID NO 365, SEQ ID NO 367, SEQ ID NO 369, SEQ ID NO 371, SEQ ID NO 373, SEQ ID NO 375, SEQ ID NO 377, SEQ ID NO 379, SEQ ID NO 381, SEQ ID NO 387, SEQ ID NO 389, SEQ ID NO 395, SEQ ID NO 397, SEQ ID NO 399, SEQ ID NO 401, SEQ ID NO 403, SEQ ID NO 405, SEQ ID NO 407, SEQ ID NO 409, SEQ ID NO 413, SEQ ID NO 423, SEQ ID NO 425, SEQ ID NO 435, SEQ ID NO 437, SEQ ID NO 441, SEQ ID NO 445, SEQ ID NO 461, SEQ ID NO 463, SEQ ID NO 497, SEQ ID NO 517, SEQ ID NO 527, SEQ ID NO 551, SEQ ID NO 567, SEQ ID NO 579, SEQ ID NO 595, SEQ ID NO 599, SEQ ID NO 601, SEQ ID NO 609, SEQ ID NO 623, SEQ ID NO 625, SEQ ID NO 627, SEQ ID NO 641, SEQ ID NO 642, SEQ ID NO 643, SEQ ID NO 644, SEQ ID NO 645, SEQ ID NO 646, SEQ ID NO 647, SEQ ID NO 648, SEQ ID NO 649, SEQ ID NO 650, SEQ ID NO 651, SEQ ID NO 652, SEQ ID NO 653, SEQ ID NO 654, SEQ ID NO 655, SEQ ID NO 656, SEQ ID NO 657, SEQ ID NO 658, SEQ ID NO 659, SEQ ID NO 660, SEQ ID NO 661, SEQ ID NO 662, SEQ ID NO 663, SEQ ID NO 664, SEQ ID NO 665, SEQ ID NO 666, SEQ ID NO 667, SEQ ID NO 668, SEQ ID NO 669, SEQ ID NO 670, SEQ ID NO 671, SEQ ID NO 672, SEQ ID NO 673, SEQ ID NO 674, SEQ ID NO 675, SEQ ID NO 676, SEQ ID NO 677, SEQ ID NO 678, SEQ ID NO 679, SEQ ID NO 680, SEQ ID NO 681, SEQ ID NO 682, SEQ ID NO 683, SEQ ID NO 684, SEQ ID NO 685, SEQ ID NO 686, SEQ ID NO 687, SEQ ID NO 688, SEQ ID NO 689, SEQ ID NO 690, SEQ ID NO 691, SEQ ID NO 692, SEQ ID NO 693, SEQ ID NO 694, SEQ ID NO 695, SEQ ID NO 696, SEQ ID NO 697, SEQ ID NO 698, SEQ ID NO 699, SEQ ID NO 700, SEQ ID NO 702, SEQ ID NO 703, SEQ ID NO 704, SEQ ID NO 705, SEQ ID NO 706, SEQ ID NO 707, SEQ ID NO 708, SEQ ID NO 709, SEQ ID NO 710, SEQ ID NO 711, SEQ ID NO 712, SEQ ID NO 713, SEQ ID NO 714, SEQ ID NO 715, SEQ ID NO 717, SEQ ID NO 718, SEQ ID NO 719, SEQ ID NO 720, SEQ ID NO 722, SEQ ID NO 724, SEQ ID NO 725, SEQ ID NO 726, SEQ ID NO 727, SEQ ID NO 728, SEQ ID NO 729, SEQ ID NO 730, SEQ ID NO 731, SEQ ID NO 732, SEQ ID NO 733, SEQ ID NO 734, SEQ ID NO 735, SEQ ID NO 736, SEQ ID NO 737, SEQ ID NO 738, SEQ ID NO 739, SEQ ID NO 740, SEQ ID NO 741, SEQ ID NO 742, SEQ ID NO 743, SEQ ID NO 744, SEQ ID NO 745, SEQ ID NO 746, SEQ ID NO 747, SEQ ID NO 748, SEQ ID NO 749, SEQ ID NO 750, SEQ ID NO 751, SEQ ID NO 752, SEQ ID NO 753 and SEQ ID NO 754, or fragment thereof, or variants of said nucleic acid molecules,
or a set comprising at least two of said nucleic acid molecules, or fragment thereof, or variants.

24. One at least of the nucleic acid molecules belonging to the group consisting in SEQ ID NO 17, SEQ ID NO 37, SEQ ID NO 39, SEQ ID NO 41, SEQ ID NO 43, SEQ ID NO 45, SEQ ID NO 47, SEQ ID NO 49, SEQ ID NO 51, SEQ ID NO 55, SEQ ID NO 59, SEQ ID NO 61, SEQ ID NO 63, SEQ ID NO 65, SEQ ID NO 67, SEQ ID NO 69, SEQ ID NO 71, SEQ ID NO 73, SEQ ID NO 81, SEQ ID NO 83, SEQ ID NO 85, SEQ ID NO 89, SEQ ID NO 91, SEQ ID NO 93, SEQ ID NO 95, SEQ ID NO 97, SEQ ID NO 99, SEQ ID NO 101, SEQ ID NO 103, SEQ ID NO 113, SEQ ID NO 115, SEQ ID NO 117, SEQ ID NO 121, SEQ ID NO 155, SEQ ID NO 157, SEQ ID NO 161, SEQ ID NO 165, SEQ ID NO 167, SEQ ID NO 169, SEQ ID NO 171, SEQ ID NO 189, SEQ ID NO 191, SEQ ID NO 195, SEQ ID NO 197, SEQ ID NO 199, SEQ ID NO 203, SEQ ID NO 207, SEQ ID NO 213, SEQ ID NO 215, SEQ ID NO 217, SEQ ID NO 219, SEQ ID NO 223, SEQ ID NO 225, SEQ ID NO 229, SEQ ID NO 231, SEQ ID NO 233, SEQ ID NO 235, SEQ ID NO 237, SEQ ID NO 239, SEQ ID NO 241, SEQ ID NO 263, SEQ ID NO 281, SEQ ID NO 293, SEQ ID NO 295, SEQ ID NO 297, SEQ ID NO 303, SEQ ID NO 317, SEQ ID NO 319, SEQ ID NO 321, SEQ ID NO 323, SEQ ID NO 325, SEQ ID NO 327, SEQ ID NO 329, SEQ ID NO 331, SEQ ID NO 333, SEQ ID NO 335, SEQ ID NO 337, SEQ ID NO 339, SEQ ID NO 343, SEQ ID NO 345, SEQ ID NO 347, SEQ ID NO 351, SEQ ID NO 353, SEQ ID NO 355, SEQ ID NO 357, SEQ ID NO 359, SEQ ID NO 361, SEQ ID NO 363, SEQ ID NO 365, SEQ ID NO 367, SEQ ID NO 369, SEQ ID NO 371, SEQ ID NO 373, SEQ ID NO 375, SEQ ID NO 377, SEQ ID NO 379, SEQ ID NO 381, SEQ ID NO 387, SEQ ID NO 389, SEQ ID NO 395, SEQ ID NO 397, SEQ ID NO 399, SEQ ID NO 401, SEQ ID NO 403, SEQ ID NO 405, SEQ ID NO 407, SEQ ID NO 409, SEQ ID NO 413, SEQ ID NO 423, SEQ ID NO 425, SEQ ID NO 435, SEQ ID NO 437, SEQ ID NO 441, SEQ ID NO 445, SEQ ID NO 461, SEQ ID NO 497, SEQ ID NO 517, SEQ ID NO 527, SEQ ID NO 551, SEQ ID NO 567, SEQ ID NO 579, SEQ ID NO 595, SEQ ID NO 599, SEQ ID NO 601, SEQ ID NO 609, SEQ ID NO 623, SEQ ID NO 625, SEQ ID NO 627, SEQ ID NO 641, SEQ ID NO 642, SEQ ID NO 643, SEQ ID NO 644, SEQ ID NO 645, SEQ ID NO 646, SEQ ID NO 647, SEQ ID NO 648, SEQ ID NO 649, SEQ ID NO 650, SEQ ID NO 651, SEQ ID NO 652, SEQ ID NO 653, SEQ ID NO 654, SEQ ID NO 656, SEQ ID NO 657, SEX ID NO 658, SEQ ID NO 659, SEQ ID NO 660, SEQ ID NO 661, SEQ ID NO 662, SEQ ID NO 663, SEQ ID NO 664, SEQ ID NO 665, SEQ ID NO 666, SEQ ID NO 667, SEQ ID NO 668, SEQ ID NO 669, SEQ ID NO 670, SEQ ID NO 672, SEQ ID NO 673, SEQ ID NO 674, SEQ ID NO 675, SEQ ID NO 676, SEQ ID NO 677, SEQ ID NO 678, SEQ ID NO 679, SEQ ID NO 680, SEQ ID NO 681, SEQ ID NO 682, SEQ ID NO 683, SEQ ID NO 687, SEQ ID NO 688, SEQ ID NO 689, SEQ ID NO 690, SEQ ID NO 691, SEQ ID NO 692, SEQ ID NO 693, SEQ ID NO 694, SEQ ID NO 695, SEQ ID NO 696, SEQ ID NO 697, SEQ ID NO 698, SEQ ID NO 700, SEQ ID NO 702, SEQ ID NO 703, SEQ ID NO 704, SEQ ID NO 705, SEQ ID NO 706, SEQ ID NO 707, SEQ ID NO 708, SEQ ID NO 709, SEQ ID NO 710, SEQ ID NO 711, SEQ ID NO 712, SEQ ID NO 713, SEQ ID NO 714, SEQ ID NO 715, SEQ ID NO 718, SEQ ID NO 720, SEQ ID NO 722, SEQ ID NO 724, SEQ ID NO 725, SEQ ID NO 727, SEQ ID NO 728, SEQ ID NO 729, SEQ ID NO 730, SEQ ID NO 731, SEQ ID NO 732, SEQ ID NO 733, SEQ ID NO 735, SEQ ID NO 736, SEQ ID NO 737, SEQ ID NO 738, SEQ ID NO 739, SEQ ID NO 740, SEQ ID NO 741, SEQ ID NO 742, SEQ ID NO 743, SEQ ID NO 744, SEQ ID NO 745, SEQ ID NO 746, SEQ ID NO 747, SEQ ID NO 748, SEQ ID NO 749, SEQ ID NO 750, SEQ ID NO 752, SEQ ID NO 753 and SEQ ID NO 754, or fragment thereof, or variants of said nucleic acid molecules,
or a set comprising at least two of said nucleic acid molecules, or fragment thereof, or variants.

25. One at least of the nucleic acid molecules belonging to the group consisting in SEQ ID NO 79, SEQ ID NO 205, SEQ ID NO 243, SEQ ID NO 247, SEQ ID NO 341, SEQ ID NO 463, SEQ ID NO 655, SEQ ID NO 671, SEQ ID NO 684, SEQ ID NO 685, SEQ ID NO 686, SEQ ID NO 699, SEQ ID NO 717, SEQ ID NO 719, SEQ ID NO 726, SEQ ID NO 734 and SEQ ID NO 751, or fragment thereof, or variants of said nucleic acid molecules,
or a set comprising at least two of said nucleic acid molecules, or fragment thereof, or variants.

26. One at least of the nucleic acid molecules belonging to the group consisting in SEQ ID NO 17, SEQ ID NO 37, SEQ ID NO 41, SEQ ID NO 43, SEQ ID NO 59, SEQ ID NO 67, SEQ ID NO 69, SEQ ID NO 73, SEQ ID NO 79, SEQ ID NO 85, SEQ ID NO 89, SEQ ID NO 91, SEQ ID NO 95, SEQ ID NO 97, SEQ ID NO 99, SEQ ID NO 101, SEQ ID NO 115, SEQ ID NO 155, SEQ ID NO 157, SEQ ID NO 161, SEQ ID NO 165, SEQ ID NO 167, SEQ ID NO 169, SEQ ID NO 189, SEQ ID NO 199, SEQ ID NO 203, SEQ ID NO 217, SEQ ID NO 223, SEQ ID NO 231, SEQ ID NO 233, SEQ ID NO 237, SEQ ID NO 239, SEQ ID NO 243, SEQ ID NO 293, SEQ ID NO 297, SEQ ID NO 303, SEQ ID NO 319, SEQ ID NO 321, SEQ ID NO 323, SEQ ID NO 325, SEQ ID NO 327, SEQ ID NO 337, SEQ ID NO 347, SEQ ID NO 351, SEQ ID NO 365, SEQ ID NO 369, SEQ ID NO 371, SEQ ID NO 375, SEQ ID NO 381, SEQ ID NO 387, SEQ ID NO 389, SEQ ID NO 401, SEQ ID NO 403, SEQ ID NO 407, SEQ ID NO 425, SEQ ID NO 435, SEQ ID NO 437, SEQ ID NO 445, SEQ ID NO 461, SEQ ID NO 463, SEQ ID NO 497, SEQ ID NO 517, SEQ ID NO 551, SEQ ID NO 623, SEQ ID NO 627, SEQ ID NO 642, SEQ ID NO 644, SEQ ID NO 645, SEQ ID NO 647, SEQ ID NO 649, SEQ ID NO 652, SEQ ID NO 653, SEQ ID NO 655, SEQ ID NO 656, SEQ ID NO 657, SEQ ID NO 661, SEQ ID NO 664, SEQ ID NO 665, SEQ ID NO 671, SEQ ID NO 672, SEQ ID NO 673, SEQ ID NO 674, SEQ ID NO 675, SEQ ID NO 677, SEQ ID NO 681, SEQ ID NO 684, SEQ ID NO 688, SEQ ID NO 691, SEQ ID NO 692, SEQ ID NO 695, SEQ ID NO 697, SEQ ID NO 700, SEQ ID NO 704, SEQ ID NO 705, SEQ ID NO 706, SEQ ID NO 710, SEQ ID NO 712, SEQ ID NO 714, SEQ ID NO 717, SEQ ID NO 71'8, SEQ ID NO 719, SEQ ID NO 720, SEQ ID NO 722, SEQ ID NO 724, SEQ ID NO 725, SEQ ID NO 728, SEQ ID NO 729, SEQ ID NO 734, SEQ ID NO 736, SEQ ID NO 737 and SEQ ID NO 738, or fragment thereof, or variants of said nucleic acid molecules,
or a set comprising at least two of said nucleic acid molecules, or fragment thereof, or variants.

27. One at least of the nucleic acid molecule belonging to the group consisting in SEQ ID NO 17, SEQ ID NO 37, SEQ ID NO 41, SEQ ID NO 43, SEQ ID NO 59, SEQ ID NO 67, SEQ ID NO 69, SEQ ID NO 73, SEQ ID NO 85, SEQ ID NO 89, SEQ ID NO 91, SEQ ID NO 95, SEQ ID NO 97, SEQ ID NO 99, SEQ ID NO 101, SEQ ID NO 115, SEQ ID NO 155, SEQ ID NO 157, SEQ ID NO 161, SEQ ID NO 165, SEQ ID NO 167, SEQ ID NO 169, SEQ ID NO 189, SEQ ID NO 199, SEQ ID NO 203, SEQ ID NO 217, SEQ ID NO 223, SEQ ID NO 231, SEQ ID NO 233, SEQ ID NO 237, SEQ ID NO 239, SEQ ID NO 293, SEQ ID NO 297, SEQ ID NO 303, SEQ ID NO 319, SEQ ID NO 321, SEQ ID NO 323, SEQ ID NO 325, SEQ ID NO 327, SEQ ID NO 337, SEQ ID NO 347, SEQ ID NO 351, SEQ ID NO 365, SEQ ID NO 369, SEQ ID NO 371, SEQ ID NO 375, SEQ ID NO 381, SEQ ID NO 387, SEQ ID NO 389, SEQ ID NO 401, SEQ ID NO 403, SEQ ID NO 407, SEQ ID NO 425, SEQ ID NO 435, SEQ ID NO 437, SEQ ID NO 445, SEQ ID NO 461, SEQ ID NO 497, SEQ ID NO 517, SEQ ID NO 551, SEQ ID NO 623, SEQ ID NO 627, SEQ ID NO 642, SEQ ID NO 644, SEQ ID NO 645, SEQ ID NO 647, SEQ ID NO 649, SEQ ID NO 652, SEQ ID NO 653, SEQ ID NO 656, SEQ ID NO 657, SEQ ID NO 661, SEQ ID NO 664, SEQ ID NO 665, SEQ ID NO 672, SEQ ID NO 673, SEQ ID NO 674, SEQ ID NO 675, SEQ ID NO 677, SEQ ID NO 681, SEQ ID NO 688, SEQ ID NO 691, SEQ ID NO 692, SEQ ID NO 695, SEQ ID NO 697, SEQ ID NO 700, SEQ ID NO 704, SEQ ID NO 705, SEQ ID NO 706, SEQ ID NO 710, SEQ ID NO 712, SEQ ID NO 714, SEQ ID NO 718, SEQ ID NO 720, SEQ ID NO 722, SEQ ID NO 724, SEQ ID NO 725, SEQ ID NO 728, SEQ ID NO 729, SEQ ID NO 736, SEQ ID NO 737 and SEQ ID NO 738, or fragment thereof, or variants of said nucleic acid molecules,
or a set comprising at least two of said nucleic acid molecules, or fragment thereof, or variants.

28. One at least of the nucleic acid molecule belonging to the group consisting in SEQ ID NO 79, SEQ ID NO 243, SEQ ID NO 463, SEQ ID NO 655, SEQ ID NO 671, SEQ ID NO 684, SEQ ID NO 717, SEQ ID NO 719 and SEQ ID NO 734, or fragment thereof, or variants of said nucleic acid molecules,
or a set comprising at least two of said nucleic acid molecules, or fragment thereof, or variants.

29. Complementary sequence of any nucleic acid molecule according to anyone of the claims 23 to 28.

30. Protein or fragment thereof encoded by a nucleic acid molecule according to anyone of claims 23 to 28.

31. Antibody, **characterized in that** is specifically directed against one of the protein of claim 30.

32. Antibody of claim 31, wherein antibody is monoclonal or polyclonal.

33. Anti-idiotypic antibody directed against one of the antibodies of claim 31.

34. Fab fragment of any anti-idiotypic antibodies of claim 33.

35. Method for the *in vitro* and/or *ex vivo* cancer diagnosis in a subject, by determining the presence or the variation of amount of at least one nucleic acid molecule comprising or constituted by a nucleotide acid sequence consisting in SEQ ID NO 2q-1, q varying from 1 to 320 and SEQ ID NO 641 to SEQ ID NO 754, or a fragment thereof, among nucleic acids from a biological sample from the subject, said presence or variation of amount of said nucleic acid molecule being assessed with respect to the absence or the given amount of said nucleic acid molecule from a sample isolated from an healthy subject, comprising:
- contacting nucleic acids from the biological sample with an agent, said agent being at least one nucleic acid molecule, or a complementary nucleic acid molecule of at least one nucleic acid molecule, comprising or constituted by the group consisting in SEQ ID NO 2q-1, q varying from 1 to 320 and SEQ ID NO 641 to SEQ ID NO 754 or a fragment thereof, and the said agent being able to selectively hybridize with at least one nucleic acid molecule comprising or constituted by the group consisting in SEQ ID NO 2q-1, q varying from 1 to 320 and SEQ ID NO 641 to SEQ ID NO 754 liable to be present among nucleic acids from the biological sample, to form a nucleic acid complex,
- determining the presence or the variation of amount of said nucleic acid complex indicating the fact that the subject is afflicted by cancer.

36. Method according to claim 35, wherein agent is preferably immobilized on a micro-array, said micro-array comprising at least one nucleic acid comprising or consisting by a nucleic acid sequence of the group consisting SEQ ID NO 755 to SEQ ID NO 1088, each nucleic acid being able to specifically recognize one nucleic acid sequence of the group consisting in : SEQ ID NO 1, SEQ ID NO 3, SEQ ID NO 5, SEQ ID NO 7, SEQ ID NO 9, SEQ ID NO 11, SEQ ID NO 13, SEQ ID NO 17, SEQ ID NO 19, SEQ ID NO 21, SEQ ID NO 23, SEQ ID NO 25, SEQ ID NO 27, SEQ ID NO 29, SEQ ID NO 31, SEQ ID NO 33, SEQ ID NO 35, SEQ ID NO 41, SEQ ID NO 43, SEQ ID NO 45, SEQ ID NO 47, SEQ ID NO 49, SEQ ID NO 51, SEQ ID NO 53, SEQ ID NO 55, SEQ ID NO 59, SEQ ID NO 61, SEQ ID NO 63, SEQ ID NO 65, SEQ ID NO 67, SEQ ID NO 71, SEQ ID NO 73, SEQ ID NO 75, SEQ ID NO 77, SEQ ID NO 79, SEQ ID NO 81, SEQ ID NO 83, SEQ ID NO 85, SEQ ID NO 87, SEQ ID NO 89, SEQ ID NO 91, SEQ ID NO 93, SEQ ID NO 97, SEQ ID NO 105, SEQ ID NO 107, SEQ ID NO 109, SEQ ID NO 111, SEQ ID NO 113, SEQ ID NO 115, SEQ ID NO 119, SEQ ID NO 123, SEQ ID NO 125, SEQ ID NO 127, SEQ ID NO 129, SEQ ID NO 131, SEQ ID NO 133, SEQ ID NO 135, SEQ ID NO 137, SEQ ID NO 139, SEQ ID NO 141, SEQ ID NO 145, SEQ ID NO 147, SEQ ID NO 149, SEQ ID NO 151, SEQ ID NO 153, SEQ ID NO 155, SEQ ID NO 157, SEQ ID NO 163, SEQ ID NO 167, SEQ ID NO 169, SEQ ID NO 173, SEQ ID NO 175, SEQ ID NO 177, SEQ ID NO 179, SEQ ID NO 181, SEQ ID NO 183, SEQ ID NO 185, SEQ ID NO 187, SEQ ID NO 189, SEQ ID NO 191, SEQ ID NO 193, SEQ ID NO 195, SEQ ID NO 197, SEQ ID NO 199, SEQ ID NO 201, SEQ ID NO 203, SEQ ID NO 205, SEQ ID NO 209, SEQ ID NO 213, SEQ ID NO 21 S, SEQ ID NO 217, SEQ ID NO 219, SEQ ID NO 221, SEQ ID NO 223, SEQ ID NO 225, SEQ ID NO 227, SEQ ID NO 229, SEQ ID NO 231, SEQ ID NO 233, SEQ ID NO 237, SEQ ID NO 239, SEQ ID NO 241, SEQ ID NO 245, SEQ ID NO 249, SEQ ID NO 251, SEQ ID NO 253, SEQ ID NO 255, SEQ ID NO 257, SEQ ID NO 265, SEQ ID NO 267, SEQ ID NO 269, SEQ ID NO 271, SEQ ID NO 273, SEQ ID NO 275, SEQ ID NO 277, SEQ ID NO 279, SEQ ID NO 283, SEQ ID NO 285, SEQ ID NO 287, SEQ ID NO 289, SEQ ID NO 291, SEQ ID NO 293, SEQ ID NO 295, SEQ ID NO 297, SEQ ID NO 303, SEQ ID NO 305, SEQ ID NO 307, SEQ ID NO 309, SEQ ID NO 311, SEQ ID NO 313, SEQ ID NO 315, SEQ ID NO 319, SEQ ID NO 321, SEQ ID NO 323, SEQ ID NO 325, SEQ ID NO 331, SEQ ID NO 335, SEQ ID NO 341, SEQ ID NO 345, SEQ ID NO 347, SEQ ID NO 353, SEQ ID NO 355, SEQ ID NO 371, SEQ ID NO 373, SEQ ID NO 375, SEQ ID NO 381, SEQ ID NO 383, SEQ ID NO 385, SEQ ID NO 389, SEQ ID NO 391, SEQ ID NO 393, SEQ ID NO 395, SEQ ID NO 397, SEQ ID NO 399, SEQ ID NO 401, SEQ ID NO 403, SEQ ID NO 405, SEQ ID NO 407, SEQ ID NO 409, SEQ ID NO 413, SEQ ID NO 417, SEQ ID NO 419, SEQ ID NO 421, SEQ ID NO 423, SEQ ID NO 427, SEQ ID NO 429, SEQ ID NO 431, SEQ ID NO 433, SEQ ID NO 435, SEQ ID NO 437, SEQ ID NO 439, SEQ ID NO 441, SEQ ID NO 443, SEQ ID NO 447, SEQ ID NO 449, SEQ ID NO 451, SEQ ID NO 453, SEQ ID NO 455, SEQ ID NO 457, SEQ ID NO 459, SEQ ID NO 461, SEQ ID NO 465, SEQ ID NO 467, SEQ ID NO 469, SEQ ID NO 471, SEQ ID NO 473, SEQ ID NO 475, SEQ ID NO 477, SEQ ID NO 481, SEQ ID NO 483, SEQ ID NO 485, SEQ ID NO 487, SEQ ID NO 491, SEQ ID NO 493, SEQ ID NO 495, SEQ ID NO 501, SEQ ID NO 503, SEQ ID NO 505, SEQ ID NO 507, SEQ ID NO 509, SEQ ID NO 511, SEQ ID NO 513, SEQ ID NO 515, SEQ ID NO 517, SEQ ID NO 519, SEQ ID NO 521, SEQ ID NO 523, SEQ ID NO 525, SEQ ID NO 527, SEQ ID NO 529, SEQ ID NO 531, SEQ ID NO 533, SEQ ID NO 535, SEQ ID NO 537, SEQ ID NO 539, SEQ ID NO 541, SEQ ID NO 543, SEQ ID NO 545, SEQ ID NO 547, SEQ ID NO 549, SEQ ID NO 551, SEQ ID NO 555, SEQ ID NO 559, SEQ ID NO 561, SEQ ID NO 563, SEQ ID NO 565, SEQ ID NO 567, SEQ ID NO 569, SEQ ID NO 571, SEQ ID NO 573, SEQ ID NO 575, SEQ ID NO 577, SEQ ID NO 579, SEQ ID NO 581, SEQ ID NO 583, SEQ ID NO 585, SEQ ID NO 587, SEQ ID NO 589, SEQ ID NO 591, SEQ ID NO 593, SEQ ID NO 595, SEQ ID NO 597, SEQ ID NO 599, SEQ ID NO 601, SEQ ID NO 603, SEQ ID NO 605, SEQ ID NO 607, SEQ ID NO 609, SEQ ID NO 611, SEQ ID NO 613, SEQ ID NO 617, SEQ ID NO 619, SEQ ID NO 623, SEQ ID NO 625, SEQ ID NO 629, SEQ ID NO 631, SEQ ID NO 633, SEQ ID NO 635, SEQ ID NO 637, SEQ ID NO 642, SEQ ID NO 644, SEQ ID NO 653, SEQ ID NO 664, SEQ ID NO 669, SEQ ID NO 673, SEQ ID NO 681, SEQ ID NO 683, SEQ ID NO 687, SEQ ID NO 689, SEQ ID NO 690, SEQ ID NO 691, SEQ ID NO 692, SEQ ID NO 693, SEQ ID NO 694, SEQ ID NO 695, SEQ ID NO 698, SEQ ID NO 701, SEQ ID NO 702, SEQ ID NO 703, SEQ ID NO 707, SEQ ID NO 710, SEQ ID NO 712, SEQ ID NO 714, SEQ ID NO 716, SEQ ID NO 717, SEQ ID NO 720, SEQ ID NO 721, SEQ ID NO 722, SEQ ID NO 723, SEQ ID NO 737 and SEQ ID NO 742.

37. Method of claims 35, wherein said agent is able to allow PCR amplification of at least one nucleic acid molecule comprising or constituted by the group consisting SEQ ID NO 2q-1, q varying from 1 to 320 and SEQ ID NO 641 to SEQ ID NO 754 liable to be present among nucleic acids from the biological sample, said PCR amplification being preferably reverse transcription-quantitative PCR, or PCR array.

38. Method for the *in vitro* and/or *ex vivo* cancer diagnosis in a subject, by determining the presence or the variation of amount of at least one protein comprising or constituted by an amino acid sequence consisting in SEQ ID NO 2q, q varying from 1 to 320, or a fragment thereof, among polypeptides from a biological sample from the subject, said presence or variation of amount of said protein being assessed with respect to the absence or the given amount of said protein from a sample isolated from an healthy subject, comprising:
- contacting polypeptides from the biological sample with an agent, said agent being able to recognize at least one protein comprising or constituted by the group consisting in SEQ ID NO 2q, q varying from 1 to 320, or a fragment thereof, liable to be present among polypeptides from the biological sample, to form an immune complex,
- determining the presence or the variation of amount of said immune complex indicating the fact that the subject is afflicted by cancer.

39. Method of claims 39, wherein immune complex results from the specific recognition of a protein comprising or constituted by an amino acid sequence consisting in SEQ ID NO 2q, q varying from 1 to 320, or a fragment thereof, by said agent, said immune complex being liable to be determined for instance by immunohistochemistry, immunocytochemistry, immunofluorescence, western blotting and immunoprecipitation.

40. Method for the *in vitro* and/or *ex vivo* cancer diagnosis in a subject, by determining the presence or the variation of amount of at least one antibody that specifically recognizes protein comprising or constituted by an amino acid sequence consisting in SEQ ID NO 2q, q varying from 1 to 320, or a fragment thereof, among antibodies that specifically recognize polypeptides from a biological sample from the subject, said presence or variation of amount of said antibody that specifically recognizes protein being assessed with respect to the absence or the given amount of said antibody that specifically recognizes protein from a sample isolated from an healthy subject, comprising:
- contacting antibodies that specifically recognize polypeptides from the biological sample with an agent, said agent being able to recognize at least one antibody that specifically recognize protein comprising or constituted by the group consisting in SEQ ID NO 2q, q varying from 1 to 320, or a fragment thereof, liable to be present among antibodies that specifically recognize polypeptides from the biological sample, to form an immune complex,
- determining the presence or the variation of amount of said immune complex indicating the fact that the subject is afflicted by cancer.

41. Method for the *in vitro* and/or *ex vivo* cancer diagnosis in a subject, by determining the presence of an immune response in a biological sample from the subject comprising:
- contacting a biological sample from the subject with an agent, said agent being able to recognize at least one fragment of a protein comprising or constituted by the group consisting in SEQ ID NO 2q, q varying from 1 to 320, liable to be presented by the MCH molecules of T-cells,
- determining the presence of said immune response indicating the fact that the subject is afflicted by cancer.

42. Method of claims 35 to 42, wherein the sample is a body fluid, a body effusion, a cell, a tissue or a tumor.

43. Kit for the *in vitro* and/or *ex vivo* cancer diagnosis comprising:
- a nucleic acid micro-array comprising or constituted by at least one nucleic acid molecule comprising or consisting by a nucleic acid sequence of the group consisting SEQ ID NO 755 to SEQ ID NO 1088,
- possibly material for preparation of nucleic acids of the biological sample from a patient suspected to be afflicted by cancer, in particular the preparation of cDNAs,
- possibly labelled molecules for labelling said nucleic nucleic acids,
- possibly a negative control corresponding to nucleic acids from a biological sample from an healthy subject.

44. Kit for the *in vitro* and/or *ex vivo* cancer diagnosis comprising:
- ELISA support comprising or constituted by at least one protein comprising or constituted by an amino acid sequence of the group consisting in SEQ ID NO 2q, q varying from 1 to 320, or fragment thereof,
- possibly labelled antibodies directed against antibody that recognizes specifically said protein, said protein being liable to be present among polypeptides from a sample from a patient suspected to be afflicted by cancer,
- possibly a negative control corresponding to antibodies polypeptides from a sample from an healthy subject.

45. Kit for the *in vitro* and/or *ex vivo* cancer diagnosis comprising:
- ELISA support comprising or constituted by at least one antibody that recognizes specifically a protein comprising or consisting by an amino acid sequence of the group consisting in SEQ ID NO 2q, q varying from 1 to 320, or fragment thereof,
- possibly labelled antibody directed against a protein specifically recognized by said antibody, said antibody being liable to be present among antibodies from a sample from a patient suspected to be afflicted by cancer,
- possibly a negative control corresponding to polypeptides from a sample from an healthy subject.

46. Pharmaceutical composition comprising at least, as active substance, one of the elements chosen among the group consisting in:
- a nucleic acid molecule according to claims 1 to 19 and
- a protein according to claim 20,
- an antibody according to claim 21 1
in association with a pharmaceutically acceptable vehicle.

47. Vaccine composition comprising as active ingredient an antibody, fragments or derivatives thereof of any of claims 31 to 34, in association with a pharmaceutically acceptable vehicle.

48. Pharmaceutical composition for the treatment of cancers comprising as active ingredient is at least one RNAi molecule, said RNAi molecule being able to hybridize with a nucleic acid molecule according to anyone claim of the claims 1 to 19, in association with a pharmaceutically acceptable vehicle.

49. Pharmaceutical composition according to claim 47, wherein said RNAi specifically hybridize to at least a nucleic acid molecule of the group comprising or constituted by a nucleotide sequence of the group consisting in SEQ ID NO 1 to SEQ ID NO 476, or at least a nucleotide acid molecule coding for protein comprising or constituted by an amino acid sequence belonging to the group consisting in SEQ ID NO 2q, q varying from 1 to 320, said RNAi containing a 17-25 nucleotide sense sequence (siRNA).

50. Pharmaceutical composition according to claim 47, wherein said RNAi specifically binds to at least a nucleic acid molecule of the group comprising or constituted by a nucleotide sequence of the group consisting in SEQ ID NO 1 to SEQ ID NO 476, or at least a nucleotide acid molecule coding for protein comprising or constituted by an amino acid sequence belonging to the group consisting SEQ ID NO 2q, q varying from 1 to 320, said RNAi containing an oligonucleotide composed 17-25 nucleotides sense sequence, a 7-11 nucleotides hairpin loop sequence and an antisense sequence binding complementarily to the sense sequence (shRNA), said shRNA being contained in an expression vector allowing sRNA expression in mammal cells.
